# EUROPEAN PATENT APPLICATION

(11) **EP 2 368 570 A2**
(43) Date of publication of application: **28.09.2011**
(21) Application number: 11167985.8
(22) Date of filing: 18.01.2007
(51) Int. Cl.: A61K 39/00

(54) **Compositions and methods related to staphylococcal bacterium proteins**

(30) Priority: 18.01.2006 US 760008 P; 31.08.2006 US 841521 P
(62) Divisional of application: 07840104.9
(71) Applicant: UNIVERSITY OF CHICAGO, Chicago, IL 60637 (US)
(72) Inventor: Missiakas, Dominique, Chicago, IL Illinois IL 60637 (US); Straner-Jones, Yukiko, Vancouver, British Columbia BC V5Y 3A6 (CA); Burts, Monica, Alexandria, VA Virginia VA 22304 (US); Schneewind, Olaf, Chicago, IL Illinois IL 60637 (US)
(74) Representative: Roberts, Joanne Nicola

(57) **Abstract**

The present invention concerns methods and compositions for treating or preventing a bacterial infection, particularly infection by a Staphylococcus bacteria. The invention provides methods and compositions for stimulating an immune response against the bacteria. In certain embodiments, the methods and compositions involve a secreted virulence factor, which may be EsxA and/or EsxB, and/or peptide processed by sortase, which may be SdrD, SdrE, IsdA, IsdB SdrC, Spa, IsdC, ClfA, ClfB, SasF, or combinations thereof.

## Description

This application claims priority to U.S. Provisional Patent applications serial number 60/760,008 filed January 18, 2006 and serial number 60/841,521 filed August 31, 2006, each of which is incorporated herein by reference in its entirety.

This invention was made with government support under grant number R01 AI38897 awarded by the National Insitutes of Health (NIH). The government has certain rights in the invention.

### BACKGROUND OF THE INVENTION

### I. FIELD OF THE INVENTION

The present invention relates generally to the fields of immunology, microbiology, and pathology. More particularly, it concerns methods and compositions involving extracellular bacterial proteins, which can be used to invoke an immune response against the bacteria. Extracellular proteins include proteins of the Ess pathway and/or peptides or proteins processed by the sortase pathway, including proteins or polypeptides of Staphylococcal and other gram-positive bacteria.

### II. BACKGROUND

*Staphylococcus aureus* and many other Gram-positive bacteria are known to secrete virulence factors or exotoxins into the extracellular milieu. These factors are secreted via the Sec translocon across the membrane by a mechanism that requires N-terminal signal peptides for recognition. Recent advances in *Streptococcus pyogenes* and *Bacillus subtilis* revealed that the translocon may be part of a larger complex (ExPortal), that organizes transport of signal peptide-bearing precursor proteins to dedicated sites within the bacterial cell envelope (Rosch and Caparon, 2004; Campo *et al.,* 2004). Sec-independent translocation of virulence factors has been observed in group A streptococci and other Gram-positive species (Chaatwal, 2002). The mechanism of substrate recognition and transport for these "signal peptide less" polypeptides has thus far not been revealed (Pancholi and Fischetti, 1992). In group A streptococci, secretion across the plasma membrane and injection of polypeptides into the cytosol of host cells uses a cytolysin-mediated transport mechanism (Madden *et al.,* 2001), that functionally resembles type III secretion of Gram-negative bacteria (Galan and Collmer, 1999).

*Mycobacterium tuberculosis* secretes ESAT-6, a virulence factor that triggers cell-mediated immune responses and interferon-γ production during tuberculosis. ESAT-6 and its relative, CFP-10 encoded by genes *esxA* (Rv3875) and *esxB* (Rv3874), respectively, are transported across the membrane of mycobacteria by a specialized secretion system that requires multiple membrane proteins. Some of these proteins carry one or more FtsK-SpoIIIE-like domain(s) (FSD), suggesting that ATP hydrolysis may be required for ESAT-6 and CFP-10 transport. Although ESAT-6 secretion system (Ess) genes have been identified in the sequenced genomes of other microbes, the possibility that this pathway represents a general virulence strategy has hitherto not been addressed.

Mycobacteria are equipped with the typical Sec machinery. However, much attention has been drawn to the secretion of two virulence factors, ESAT-6 (early secreted antigen target 6 kDa) and CFP-10 (culture filtrate antigen 10 kDa), encoded by *Mycobacterium tuberculosis* H37Rv *esxA* (Rv3875) and *esxB* (Rv3874). *M*. *tuberculosis* variants lacking *esxA* display defects in the establishment of tuberculosis and EsxA induces a strong T-cell response during infection (Sorenson *et al.,* 1995). Comparative genomics of mycobacteria related to *M. tuberculosis* H37Rv identified multiple regions of difference that have been deleted from both virulent and avirulent species (Mahairas *et al.,* 1996; Cole *et al.,* 1998; Pym *et al.,* 2002). *esxA* (Rv3875) is located within region of difference 1 (RD1). RD1 is the only locus specifically deleted in the attenuated *Mycobacterium* bovis bacillus Calmette-Gue'rin (bacille Calmette-Gue'rin) vaccine strain and avirulent *Mycobacterium microi* strain (Mahairas *et al.,* 1996; Pym *et al.,* 2002; Calmette, 1927; Harboe *et al.* 1996; Philipp *et al.,* 2003). Although RD1 is certainly not the only virulence trait of mycobacteria, it appears to be a prominent factor in attenuating vaccine strains (Pym *et al.,* 2002; Pym *et al.,* 2003; Hsu *et al.,* 2003).

A survey of *M. tuberculosis* H37Rv genome sequence revealed at least 23 ESAT-6 homologues (Cole *et al.,* 1998), 10 of which are encoded within five gene clusters that specify large soluble and membrane-bound ATPases with two or more FtsK/SpoIIIE-like domains (FSDs) (Gey Van Pittius *et al.,* 2001; Pallen *et al.,* 2002). Pallen *et al.* (2002) discovered ESAT-6 homologues in the sequenced genomes of other Gram-positive bacteria including *Bacillus subtilis, Bacillus anthracis, Clostridium acetobutylicum, Listeria monocytogenes,* and *S. aureus.* The genes for the ESAT-6-like proteins are clustered with at least one gene encoding an FSD-type membrane protein, leading Pallen to propose that FSD ATPases may represent a universal portal for excretion of ESAT-6-like proteins (Pallen *et al.,* 2002).

Support for this hypothesis was garnered when deletions of FSD-like genes (Rv3870, Rv3871) prevented the secretion of *M. tuberculosis* ESAT-6 and CFP-10 (Pym *et al.,* 2003; Hsu *et al.,* 2003; Stanley *et al.,* 2003; Guinn *et al.,* 2004). A third membrane protein encoded by Rv3877/snm4 was found to be required for secretion of ESAT-6 and CFP-10 (Stanley *et al.,* 2003). Therefore, in mycobacteria secretion of ESAT-6 and CFP-10 requires a specialized mechanism encoded by genes flanking *esxA* and *esxB* on the chromosome. This specialized secretion system has been referred to as, Snm for secretion in mycobacteria (Stanley *et al.,* 2003). Most importantly, this dedicated secretion pathway may explain the absence of processing of ESAT-6 or CFP-10 during secretion (Sorenson *et al.,* 1995). Although it has been surmised that FSD proteins may represent a general Gram-positive secretion system (Pallen *et al.,* 2002), this hypothesis has only recently received experimental verification (Burts *et al.,* 2005).

Moreover, pathogenic bacteria continue to pose a global health problem. Few vaccines and other preventative measures have been developed to combat bacterial infections. The *Staphylococcus aureus* pathogen is the most common food-borne infection and it is responsible for causing a number of other infections, including infections of open wounds and toxic shock syndrome. There is a continued need for therapeutic and preventative agents for infection by staphylococci, as well as other bacterial pathogens.

### SUMMARY OF THE INVENTION

Therefore, the present invention provides methods and compositions that can be used to treat or prevent bacterial pathogen infection. The invention is based on data that an immune response can be generated against one or more extracellular proteins in bacteria, which includes secreted virulence factors and cell surface proteins.

In some cases, methods for stimulating an immune response involve administering to the subject an effective amount of a composition including an extracellular protein, which include i) secreted virulence factor, and/or a cell surface protein or peptide, or ii) a recombinant nucleic acid molecule encoding a secreted virulence factor, and/or a cell surface protein or peptide. Compositions of the present invention include immunogenic compositions wherein the antigen(s) or epitope(s) are contained in an amount effective to achieve the intended purpose. More specifically, an effective amount means an amount of active ingredients effective to stimulate or elicit an immune response, or provide resistance to or amelioration of infection. In more specific aspects, an effective amount prevents, alleviates or ameliorates symptoms of disease or infection, or prolongs the survival of the subject being treated. Determination of an effective amount is well within the capability of those skilled in the art, especially in light of the detailed disclosure provided herein. For any preparation used in the methods of the invention, an effective amount or dose can be estimated initially from *in vitro,* cell culture, and/or animal model assays. For example, a dose can be formulated in animal models to achieve a desired immune response or circulating antibody concentration or titer. Such information can be used to more accurately determine useful doses in humans.

In certain embodiments a secreted virulence factor is an Esx protein, for instance, all or part of an EsxA or EsxB protein. In certain aspects, a cell surface protein is a sortase substrate, including, but not limited to an isolated SdrC, SdrD, SdrE, IsdA, IsdB, Spa, ClfA, ClfB, IsdC, SasF or combinations thereof. In certain embodiments, 1, 2, 3, 4, 5, 6, 7, 8, 9, 10 or more of EsxA, EsxB, SdrC, SdrD, SdrE, IsdA, IsdB, Spa, ClfA, ClfB, IsdC, or SasF can be specifically excluded from a formulation of the invention. In certain aspects, the composition is not a Staphylococci bacterium or does not contain Staphylococci bacteria. In a particular embodiment a composition comprises either an isolated EsxA or EsxB protein, or both an isolated EsxA and an isolated EsxB proteins. In still further aspects, the isolated EsxA and EsxB proteins are multimerized and preferably dimerized. In certain aspects of the invention, a composition comprises 1, 2, 3, 4, 5, 6, 7, 8, 9, 10 or more isolated cell surface proteins or segments thereof. In a further aspect the cell surface protein is a sortase substrate, including, but not limited to SdrC, SdrD, SdrE, IsdA, IsdB, Spa, ClfA, ClfB, IsdC, or SasF. In certain embodiments, methods and compositions of the invention will include a second, third, fourth, fifth, sixth or more sortase substrate. Alternatively, the composition may be or include a recombinantly engineered Staphylococcus bacterium that has been altered in a way that comprises specifically altering the bacterium with respect to the relevant secreted virulence factor or cell surface protein. For example, the bacteria may be recombinantly modified to express more of the relevant virulence factor or cell surface protein than it would express if unmodified.

The term "isolated" can refer to a nucleic acid or polypeptide that is substantially free of cellular material, bacterial material, viral material, or culture medium (when produced by recombinant DNA techniques) of their source of origin, or chemical precursors or other chemicals (when chemically synthesized). Moreover, an isolated compound refers to one that can be administered to a subject as an isolated compound; in other words, the compound may not simply be considered "isolated" if it is adhered to a column or embedded in an agarose gel. Moreover, an "isolated nucleic acid fragment" or "isolated peptide" is a nucleic acid or protein fragment that is not naturally occurring as a fragment and/or is not typically in the functional state. Moieties of the invention, such as antigens or immunogens, may be conjugated or linked covalently or noncovalently to other moieties such as adjuvants, proteins, peptides, supports, fluorescence moieties, or labels. The term "conjugate" or "immunoconjugate" is broadly used to define the operative association of one moiety with another agent and is not intended to refer solely to any type of operative association, and is particularly not limited to chemical "conjugation." Recombinant fusion proteins are particularly contemplated. A nucleic acid or polypeptide composition can be at least of a purity of 60, 65, 70, 75, 80, 85, 90, 95, 98, or 100% based on the amount other contaminating substances.

The term EsxA protein refers to a protein that includes isolated wild-type EsxA polypeptides from staphylococcus bacteria, as well as variants that stimulate an immune response against staphylococcus bacteria EsxA proteins in nature. Similarly, the term EsxB protein refers to a protein that includes isolated wild-type EsxB polypeptides from staphylococcus bacteria, as well as variants that stimulate an immune response against staphylococcus bacteria EsxB proteins in nature. Further, the term SdrD protein refers to a protein that includes isolated wild-type SdrD polypeptides from staphylococcus bacteria, as well as variants that stimulate an immune response against staphylococcus bacteria SdrD proteins in nature. Still further, the term SdrE protein refers to a protein that includes isolated wild-type SdrE polypeptides from staphylococcus bacteria, as well as variants that stimulate an immune response against staphylococcus bacteria SdrE proteins in nature. Yet still further, the term IsdA protein refers to a protein that includes isolated wild-type IsdA polypeptides from staphylococcus bacteria, as well as variants that stimulate an immune response against staphylococcus bacteria IsdA proteins in nature. The term IsdB protein refers to a protein that includes isolated wild-type IsdB polypeptides from staphylococcus bacteria, as well as variants that stimulate an immune response against staphylococcus bacteria IsdB proteins in nature. An immune response refers to a humoral response, a cellular response, or both a humoral and cellular response in an organism. An immune response can be measured by assays that include, but are not limited to, assays measuring the presence or amount of antibodies that specifically recognize an Esx protein or cell surface protein, assays measuring T-cell activation or proliferation, and/or assays that measure modulation in terms of activity or expression of one or more cytokines.

Compositions of the invention may further comprise an adjuvant. An adjuvant may be covalently or non-covalently coupled to a polypeptide or peptide of the invention. In certain aspects, the adjuvant is chemically conjugated to a protein, polypeptide, or peptide.

Embodiments of the present invention include methods for eliciting an immune response against a staphylococcus bacterium or staphylococci in a subject comprising providing to the subject an effective amount of 1, 2, 3, 4, 5, 6, 7, 8, 9, 10 or more secreted virulence factors and/or cell surface proteins. A secreted virulence factor includes, but is not limited to an EsxA or EsxB protein. A cell surface protein includes sortase substrates, which include, but are not limited to SdrC, SdrD, SdrE, IsdA, IsdB, Spa, ClfA, ClfB, IsdC and SasF. The term "providing" is used according to its ordinary meaning to indicate "to supply or furnish for use." In some embodiments, the protein is provided directly by administering the protein, while in other embodiments, the protein is effectively provided by administering a nucleic acid that encodes the protein. In certain apsects the invention contemplates compositions comprising various combinations of antigens, peptides, and/or epitope, for example compositions can comprise

EsxA, EsxB, or both EsxA and EsxB in combination with, SdrE, IsdA, IsdB, Spa, ClfA, ClfB, IsdC, or SasF; IsdA, IsdB, Spa, ClfA, ClfB, IsdC, or SasF; IsdB, Spa, ClfA, ClfB, IsdC, or SasF; Spa, ClfA, ClfB, IsdC, or SasF; ClfA, ClfB, IsdC, or SasF; ClfB, IsdC, or SasF; IsdC, or SasF; SasF; SdrC, SdrD, SdrE, IsdA, IsdB, Spa, ClfA, ClfB, or IsdC; SdrC, SdrD, SdrE, IsdA, IsdB, Spa, ClfA, or ClfB; SdrC, SdrD, SdrE, IsdA, IsdB, Spa, or ClfA; SdrC, SdrD, SdrE, IsdA, or IsdB; SdrC, SdrD, SdrE, or IsdA; SdrC, SdrD, or SdrE; SdrC, or SdrD; or SdrC.

In certain aspects, a composition can comprise SdrE and IsdA, IsdB, Spa, ClfA, ClfB, IsdC, and SasF; IsdB, Spa, ClfA, ClfB, IsdC, and SasF; Spa, ClfA, ClfB, IsdC, and SasF; ClfA, ClfB, IsdC and SasF; CIfB, IsdC, and SasF; IsdC and SasF; SasF; IsdA, IsdB, Spa, ClfA, ClfB, and IsdC; IsdA, IsdB, Spa, ClfA, and ClfB; IsdA, IsdB, Spa, and ClfA; IsdA, IsdB, and Spa; IsdA and IsdB; IsdA; IsdB; Spa; ClfA; ClfB; IsdC; and so forth.

In certain aspects, a composition can comprise IsdA and SdrE, IsdB, Spa, ClfA, ClfB, IsdC, and SasF; IsdB, Spa, ClfA, ClfB, IsdC, and SasF; Spa, ClfA, ClfB, IsdC, and SasF; ClfA, ClfB, IsdC, and SasF; ClfB, IsdC, and SasF; IsdC and SasF; SasF; SdrE, IsdB, Spa, ClfA, ClfB, and IsdC; SdrE, IsdB, Spa, ClfA, , and ClfB; SdrE, IsdB, Spa, , and ClfA; SdrE, IsdB, and Spa; SdrE and IsdB; SdrE; IsdB; Spa; ClfA; ClfB; IsdC; and so forth.

In certain aspects, a composition can comprise IsdB and SdrE, IsdA, Spa, ClfA, ClfB, IsdC, and SasF; IsdA, Spa, ClfA, ClfB, IsdC, and SasF; Spa, CIfA, ClfB, IsdC, and SasF; ClfA, ClfB, IsdC, and SasF; ClfB, IsdC, and SasF; IsdC and SasF; SasF; SdrE, IsdA, Spa, ClfA, ClfB, and IsdC; SdrE, IsdA, Spa, ClfA, and ClfB; SdrE, IsdA, Spa, and ClfA; SdrE, IsdA, and Spa; SdrE, and IsdA; SdrE; IsdA; Spa; ClfA; ClfB; IsdC; and so forth.

In certain aspects, a composition can comprise Spa and SdrE, IsdA, IsdB, ClfA, ClfB, IsdC, and SasF; IsdA, IsdB, ClfA, ClfB, IsdC, and SasF; IsdB, ClfA, ClfB, IsdC, and SasF; ClfA, ClfB, IsdC, and SasF; ClfB, IsdC, and SasF; IsdC and SasF; SasF; SdrE, IsdA, IsdB, ClfA, ClfB, and IsdC; SdrE, IsdA, IsdB, ClfA, and ClfB; SdrE, IsdA, IsdB, and ClfA; SdrE, IsdA, and IsdB; SdrE and IsdA; SdrE; IsdA; IsdB; ClfA; ClfB; IsdC; and so forth.

In certain aspects, a composition can comprise ClfA and SdrE, IsdA, IsdB, Spa, ClfB, IsdC, and SasF; IsdA, IsdB, Spa, ClfB, IsdC, and SasF; IsdB, Spa, ClfB, IsdC, and SasF; Spa, ClfB, IsdC, and SasF; ClfB, IsdC, and SasF; IsdC and SasF; SasF; SdrE, IsdA, IsdB, Spa, ClfB, and IsdC; SdrE, IsdA, IsdB, Spa, and CIfB; SdrE, IsdA, IsdB, and Spa; SdrE, IsdA, and IsdB; SdrE and IsdA; SdrE; IsdA; IsdB; Spa; ClfB; IsdC; and so forth.

In certain aspects, a composition can comprise ClfB and SdrE, IsdA, IsdB, Spa, ClfA, IsdC, and SasF; IsdA, IsdB, Spa, ClfA, IsdC, and SasF; IsdB, Spa, ClfA, IsdC, and SasF; Spa, ClfA, IsdC, and SasF; ClfA, IsdC, and SasF; IsdC and SasF; SasF; SdrE, IsdA, IsdB, Spa, ClfA, and IsdC; SdrE, IsdA, IsdB, Spa, and ClfA; SdrE, IsdA, IsdB, and Spa; SdrE, IsdA, and IsdB; SdrE, and IsdA; SdrE; IsdA; IsdB; Spa; ClfA; IsdC; and so forth.

In certain aspects, a composition can comprise IsdC and SdrE, IsdA, IsdB, Spa, ClfA, ClfB, and SasF; IsdA, IsdB, Spa, ClfA, ClfB, and SasF; IsdB, Spa, ClfA, ClfB, and SasF; Spa, ClfA, ClfB, and SasF; ClfA, ClfB, and SasF; ClfB and SasF; SdrE, IsdA, IsdB, Spa, ClfA, and ClfB; SdrE, IsdA, IsdB, Spa, and ClfA; SdrE, IsdA, IsdB, and Spa; SdrE, IsdA, and IsdB; SdrE, and IsdA; SdrE; IsdA; IsdB; Spa; ClfA; ClfB; and so forth.

In certain aspects, a composition can comprise SasF and SdrE, IsdA, IsdB, Spa, ClfA, ClfB, and IsdC; IsdA, IsdB, Spa, ClfA, ClfB, and IsdC; IsdB, Spa, ClfA, ClfB, and IsdC; Spa, ClfA, ClfB, and IsdC; ClfA, ClfB, and IsdC; ClfB and IsdC; SdrE, IsdA, IsdB, Spa, ClfA, and ClfB; SdrE, IsdA, IsdB, Spa, and ClfA; SdrE, IsdA, IsdB, and Spa; SdrE, IsdA, and IsdB; SdrE, and IsdA; SdrE; IsdA; IsdB; Spa; ClfA; ClfB; IsdC and so forth.

Embodiments of the invention include compositions that may include a polypeptide, peptide, or protein that is or is at least 70%, 75%, 80%, 85%, 90%, 95%, 96%, 97%, 98%, or 99% identity or similarity to a secreted virulence protein or a surface protein. In a further embodiment of the invention a composition may include a polypeptide, peptide, or protein that is or is at least 70%, 75%, 80%, 85%, 90%, 95%, 96%, 97%, 98%, or 99% identity or similarity to an EsxA protein, preferably the EsxA protein will have an amino acid sequence of SEQ ID NO:2. Similarity or identity, with identity being preferred, is known in the art, a number of different programs can be used to identify whether a protein (or nucleic acid) has sequence identity or similarity to a known sequence. Sequence identity and/or similarity is determined using standard techniques known in the art, including, but not limited to, the local sequence identity algorithm of Smith & Waterman (1981), by the sequence identity alignment algorithm of Needleman & Wunsch (1970), by the search for similarity method of Pearson & Lipman (1988), by computerized implementations of these algorithms (GAP, BESTFIT, FASTA, and TFASTA in the Wisconsin Genetics Software Package, Genetics Computer Group, 575 Science Drive, Madison, Wis.), the Best Fit sequence program described by Devereux *et al.* (1984), preferably using the default settings, or by inspection. Preferably, percent identity is calculated by using alignment tools known to and readily ascertainable to those of skill in the art.

In still further embodiments of the invention a composition may include a polypeptide, peptide, or protein that is or is at least 70%, 75%, 80%, 85%, 90%, 95%, 96%, 97%, 98%, or 99% identity or similarity to an EsxB protein. In certain aspects the EsxB protein will have the amino acid sequence of SEQ ID NO:4.

In yet still further embodiments of the invention a composition may include a polypeptide, peptide, or protein that is or is at least 70%, 75%, 80%, 85%, 90%, 95%, 96%, 97%, 98%, or 99% identity or similarity to an SdrD protein. In certain aspects the SdrD protein will have the amino acid sequence of SEQ ID NO:6.

In further embodiments of the invention a composition may include a polypeptide, peptide, or protein that is or is at least 70%, 75%, 80%, 85%, 90%, 95%, 96%, 97%, 98%, or 99% identity or similarity to an SdrE protein. In certain aspects the SdrE protein will have the amino acid sequence of SEQ ID NO:8.

In still further embodiments of the invention a composition may include a polypeptide, peptide, or protein that is or is at least 70%, 75%, 80%, 85%, 90%, 95%, 96%, 97%, 98%, or 99% identity or similarity to an IsdA protein. In certain aspects the IsdA protein will have the amino acid sequence of SEQ ID NO:10.

In yet still further embodiments of the invention a composition may include a polypeptide, peptide, or protein that is or is at least 70%, 75%, 80%, 85%, 90%, 95%, 96%, 97%, 98%, or 99% identity or similarity to an IsdB protein. In certain aspects the IsdB protein will have the amino acid sequence of SEQ ID NO:12.

Embodiments of the invention include compositions that include a polypeptide, peptide, or protein that is or is at least 70%, 75%, 80%, 85%, 90%, 95%, 96%, 97%, 98%, or 99% identity or similarity to a Spa protein. In certain aspects the Spa protein will have the amino acid sequence of SEQ ID NO:14.

In a further embodiments of the invention a composition may include a polypeptide, peptide, or protein that is or is at least 70%, 75%, 80%, 85%, 90%, 95%, 96%, 97%, 98%, or 99% identity or similarity to a ClfB protein. In certain aspects the CIfB protein will have the amino acid sequence of SEQ ID NO:16.

In still further embodiments of the invention a composition may include a polypeptide, peptide, or protein that is or is at least 70%, 75%, 80%, 85%, 90%, 95%, 96%, 97%, 98%, or 99% identity or similarity to an IsdC protein. In certain aspects the IsdC protein will have the amino acid sequence of SEQ ID NO:18.

In yet further embodiments of the invention a composition may include a polypeptide, peptide, or protein that is or is at least 70%, 75%, 80%, 85%, 90%, 95%, 96%, 97%, 98%, or 99% identity or similarity to a SasF protein. In certain aspects the SasF protein will have the amino acid sequence of SEQ ID N0:20.

In yet still further embodiments of the invention a composition may include a polypeptide, peptide, or protein that is or is at least 70%, 75%, 80%, 85%, 90%, 95%, 96%, 97%, 98%, or 99% identity or similarity to an SdrC protein. In certain aspects the SdrC protein will have the amino acid sequence of SEQ ID NO:22.

In yet still further embodiments of the invention a composition may include a polypeptide, peptide, or protein that is or is at least 70%, 75%, 80%, 85%, 90%, 95%, 96%, 97%, 98%, or 99% identity or similarity to an ClfA protein. In certain aspects the ClfA protein will have the amino acid sequence of SEQ ID NO: 24.

In the context of a polypeptide or protein, the term "identity" refers to a polypeptide having a sequence that is at least 85%, preferably at least 90%, more preferably at least 95%, and most preferably at least 98% or 99% or more identical to the amino acid sequence of the reference polypeptide. The term "similarity" refers to a polypeptide that has a sequence that has a certain percentage of amino acids that are either identical with the reference polypeptide or constitute conservative substitutions with the reference polypeptides.

The EsxA protein, EsxB protein, or other protein transported by the Ess pathway, and/or SdrC, SdrD, SdrE, IsdA, IsdB, Spa, ClfA, ClfB, IsdC, and SasF or other cell surface proteins may include the following, or at least or at most 3, 4, 5, 6, 7, 8, 9, 10,11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250 contiguous amino acids, or any range derivable therein of SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, and/or SEQ ID NO:24, respectively.

The compositions may be formulated in a pharmaceutically acceptable composition. In certain aspects of the invention the staphylococcus bacterium is an *S. aureus* bacterium.

In further aspects of the invention a composition may be administered more than one time to the subject, and may be administered 1, 2, 3, 4, 5, 6, 7, 8, 9, 10, 15, 20 or more times. The administration of the compositions include, but is not limited to oral, parenteral, subcutaneous, intramuscular, intravenous administration, or various combinations thereof.

Embodiments of the invention include administering to the subject a composition comprising a non-EsxA or non-EsxB Ess protein. The Ess protein may be in the same composition as EsxA protein or EsxB protein, but need not be.

In still further embodiments, a composition comprises a recombinant nucleic acid molecule encoding an EsxA protein or a recombinant nucleic acid molecule encoding an EsxB protein, or both a nucleic acid molecule encoding an EsxA protein and a nucleic acid molecule encoding an EsxB protein. In certain aspects a nucleic acid molecule encodes both an EsxA protein and an EsxB protein. Typically a recombinant nucleic acid molecule encoding an EsxA or EsxB protein contains a heterologous promoter. In certain aspects, a recombinant nucleic acid molecule of the invention is a vector, in still other aspects the vector is a plasmid. In certain embodiments the vector is a viral vector. Aspects of the invention include compositions that further comprise a nucleic acid encoding another Ess protein. In certain aspects a composition includes a recombinant, non-staphylococcus bacterium containing the EsxA or EsxB protein. In particular aspects the recombinant non-staphylococcus bacteria is Salmonella or another gram-positive bacteria. A composition is typically administered to human subjects, but administration to other animals that are capable of eliciting an immune response is contemplated. In further aspects the staphylococcus bacterium is a *Staphylococcus aureus.* In further embodiments the immune response is a protective immune response.

In further embodiments a composition comprises a recombinant nucleic acid molecule encoding a cell surface protein such as an SdrD protein, a recombinant nucleic acid molecule encoding an SdrE protein, a recombinant nucleic acid molecule encoding an IsdA protein, and/or a recombinant nucleic acid molecule encoding an IsdB protein,. Alternatively, a composition may include nucleic acid molecules encoding two or more cell surface proteins. Cell surface proteins include, but are not limited to a SdrD, SdrE, IsdA, or IsdB protein. In certain aspects a nucleic acid molecule encodes two or more cell surface proteins, such as SdrC, SdrD, SdrE, IsdA, IsdB, Spa, ClfA, ClfB, IsdC and/or SasF protein. Typically a recombinant nucleic acid molecule contains a heterologous promoter. In certain aspects, a recombinant nucleic acid molecule of the invention is a vector, in still other aspects the vector is a plasmid. In certain embodiments the vector is a viral vector. Aspects of the invention include compositions that further comprise a nucleic acid encoding another sortase substrate protein. In certain aspects a composition includes a recombinant, non-staphylococcus bacterium containing the SdrC, SdrD, SdrE, IsdA, IsdB, Spa, ClfA, ClfB, IsdC and/or SasF protein. In particular aspects the recombinant non-staphylococcus bacteria is Salmonella or another gram-positive bacteria. A composition is typically administered to human subjects, but administration to other animals that are capable of eliciting an immune response is contemplated, particularly cattle, horses, goats, sheep and other domestic animals. In further aspects the staphylococcus bacterium is a *Staphylococcus aureus.* In further embodiments the immune response is a protective immune response.

In still further aspects, the methods and compositions of the invention can be used to prevent, ameliorate, reduce, or treat infection of glands, *e.g.*, mammary glands, particularly mastitis and other infections. Other methods include, but are not limited to prophylatically reducing bacterial burden in a subject not exhibiting signs of infection, particularly those subjects suspected of or at risk of being colonized by a target bacteria, *e.g.*, patients that are or will be at risk or susceptible to infection during a hospital stay, treatment, and/or recovery.

Still further embodiments include methods for stimulating in a subject a protective or therapeutic immune response against a staphylococcus bacterium comprising administering to the subject an effective amount of a composition including (i) an EsxA and/or EsxB protein or peptide thereof; or, (ii) a nucleic acid molecule encoding an EsxA and/or EsxB protein or peptide thereof, or (iii) any combination or permutation of bacterial proteins described herein. In a preferred embodiment the composition is not a staphylococcus bacteria. In certain aspects the subject is a human or a cow. In a further aspect the composition is formulated in a pharmaceutically acceptable formulation. The staphylococci may be *Staphylococcus aureus.*

Yet still further embodiments include vaccines comprising a pharmaceutically acceptable composition having an isolated EsxA and/or EsxB, or any other combination or permutation of protein(s) or peptide(s) described, wherein the composition is capable of stimulating an immune response against a staphylococcus bacterium. The vaccine may comprise an isolated EsxA and an isolated EsxB, or any other combination or permutation of protein(s) or peptide(s) described. In certain aspects of the invention the isolated EsxA and isolated EsxB, or any other combination or permutation of protein(s) or peptide(s) described are multimerized, *e.g.*, dimerized. In a further aspect, the vaccine composition is contaminated by less than about 10, 9, 8, 7, 6, 5, 4, 3, 2, 1, 0.5, 0.25, 0.05% (or any range derivable therein) of other Staphylococcal proteins. A composition may further comprise an isolated non-EsxA or non-EsxB Ess protein. Typically the vaccine comprises an adjuvant. In certain aspects a protein or peptide of the invention is linked (covalently or non-covalently coupled) to the adjuvant, preferably the adjuvant is chemically conjugated to the protein.

In still yet further embodiments, a vaccine composition is a pharmaceutically acceptable composition having a recombinant nucleic acid encoding all or part of an EsxA or EsxB protein, or any other combination or permutation of protein(s) or peptide(s) described, wherein the composition is capable of stimulating an immune response against a staphylococcus bacteria. The vaccine composition may comprise a recombinant nucleic acid encoding all or part of an EsxA protein, all or part of an EsxB protein, or all or part of both an EsxA protein and EsxB protein, or any other combination or permutation of protein(s) or peptide(s) described. A recombinant nucleic acid of the invention may encode all or part of both EsxA and EsxB proteins in the same or a separate nucleic acid. In certain embodiments the recombinant nucleic acid contains a heterologous promoter. Preferably the recombinant nucleic acid is a vector. More preferably the vector is a plasmid or a viral vector. A vaccine may also comprise a nucleic acid encoding another member of the Ess proteins. In some aspects the vaccine includes a recombinant, non-staphylococcus bacterium containing the nucleic acid. The recombinant non-staphylococci may be Salmonella or another gram-positive bacteria. The vaccine may comprise an adjuvant.

Still further embodiments include methods for stimulating in a subject a protective or therapeutic immune response against a staphylococcus bacterium comprising administering to the subject an effective amount of a composition including i) a SdrC, SdrD, SdrE, IsdA, IsdB, Spa, ClfA, ClfB, IsdC and/or SasF protein or peptide thereof; or, ii) a nucleic acid molecule encoding a SdrC, SdrD, SdrE, IsdA, IsdB, Spa, ClfA, ClfB, IsdC and/or SasF protein or peptide thereof. In a preferred embodiment the composition is not a staphylococcus bacteria. In certain aspects the subject is a human or4 cow. In a further aspect the composition is formulated in a pharmaceutically acceptable formulation. The staphylococci may be *Staphylococcus aureus.* Methods of the invention also include compositions that contain 1, 2, 3, 4, 5, 6, 7, 8, 9, 10 or more secreted virulence factors and/or cell surface proteins, such as EsxA, EsxB, SdrC, SdrD, SdrE, IsdA, IsdB, Spa, ClfA, ClfB, IsdC and/or SasF in various combinations. In certain aspects a vaccine formulation includes SdrD, SdrE, IsdA and IsdB; or SdrC, SdrD, SdrE, IsdA, IsdB, Spa, ClfA, ClfB, IsdC, and SasF.

Yet still further embodiments include vaccines comprising a pharmaceutically acceptable composition having an isolated SdrC, SdrD, SdrE, IsdA, IsdB, Spa, ClfA, ClfB, IsdC and/or SasF protein or peptide thereof, wherein the composition is capable of stimulating an immune response against a staphylococcus bacterium. The vaccine may comprise two or more isolated SdrC, SdrD, SdrE, IsdA, IsdB, Spa, ClfA, ClfB, IsdC and/or SasF proteins or peptides thereof. In certain aspects of the invention the isolated SdrC, SdrD, SdrE, IsdA, IsdB, Spa, ClfA, ClfB, IsdC and/or SasF proteins are multimerized, *e.g.*, dimerized. In a further aspect, the vaccine composition is contaminated by less than about 10, 9, 8, 7, 6, 5, 4, 3, 2, 1, 0.5, 0.25, 0.05% (or any range derivable therein) of other Staphylococcal proteins. Typically the vaccine comprises an adjuvant. In certain aspects a protein or peptide of the invention is linked (covalently or non-covalently coupled) to the adjuvant, preferably the adjuvant is chemically conjugated to the protein. Vaccines of the invention may include 1, 2, 3, 4, 5, 6, or more secreted virulence factors and/or cell surface proteins, such as EsxA, EsxB, SdrC, SdrD, SdrE, IsdA, IsdB, Spa, ClfA, ClfB, IsdC and/or SasF in various combinations.

In still yet further embodiments, a vaccine composition is a pharmaceutically acceptable composition having a recombinant nucleic acid encoding all or part of an SdrC, SdrD, SdrE, IsdA, IsdB, Spa, ClfA, ClfB, IsdC or SasF protein, wherein the composition is capable of stimulating an immune response against a staphylococcus bacteria. The vaccine composition may comprise a recombinant nucleic acid encoding all or part of a SdrD protein, all or part of an SdrE protein, all or part of an IsdA protein, all or part of an IsdB or all or part of two or more of SdrD, SdrE, IsdA, or IsdB protein. A recombinant nucleic acid of the invention may encode all or part of SdrD, SdrE, IsdA, IsdB, Spa, ClfB, IsdC and/or SasF proteins in the same or a separate nucleic acid. In certain embodiments the recombinant nucleic acid contains a heterologous promoter. Preferably the recombinant nucleic acid is a vector. More preferably the vector is a plasmid or a viral vector. A vaccine may also comprise a nucleic acid encoding another member of the sortase substrate proteins. In some aspects the vaccine includes a recombinant, non-staphylococcus bacterium containing the nucleic acid. The recombinant non-staphylococci may be Salmonella or another gram-positive bacteria. The vaccine may comprise an adjuvant. Aspects of the invention also include nucleic acids encoding 1, 2, 3, 4, 5, 6, 7, 8, 9, 10 or more surface proteins or secreted virulence factors. In certain aspects the proteins or peptides may be encoded in 1, 2, 3, 4, 5, 6, 7, 8, 9, 10 or more expression vectors (*e.g*., one or more nucleic acid or plasmid) and/or delivery vectors (*e.g.*, one or more bacteria or virus).

Any embodiment discussed with respect to one aspect of the invention applies to other aspects of the invention as well. In particular, any embodiment discussed in the context of an Esx protein or nucleic acid may be implemented with respect to other secreted virulence factors, and/or cell surface proteins, such as SdrC, SdrD, SdrE, IsdA, IsdB, Spa, ClfA, ClfB, IsdC and/or SasF proteins (or nucleic acids), and vice versa.

The embodiments in the Example section are understood to be embodiments of the invention that are applicable to all aspects of the invention.

The use of the term "or" in the claims is used to mean "and/or" unless explicitly indicated to refer to alternatives only or the alternatives are mutually exclusive, although the disclosure supports a definition that refers to only alternatives and "and/or."

Throughout this application, the term "about" is used to indicate that a value includes the standard deviation of error for the device or method being employed to determine the value.

Following long-standing patent law, the words "a" and "an," when used in conjunction with the word "comprising" in the claims or specification, denotes one or more, unless specifically noted.

Other objects, features and advantages of the present invention will become apparent from the following detailed description. It should be understood, however, that the detailed description and the specific examples, while indicating specific embodiments of the invention, are given by way of illustration only, since various changes and modifications within the spirit and scope of the invention will become apparent to those skilled in the art from this detailed description.

### DESCRIPTION OF THE DRAWINGS

So that the matter in which the above-recited features, advantages and objects of the invention as well as others which will become clear are attained and can be understood in detail, more particular descriptions and certain embodiments of the invention briefly summarized above are illustrated in the appended drawings. These drawings form a part of the specification. It is to be noted, however, that the appended drawings illustrate certain embodiments of the invention and therefore are not to be considered limiting in their scope.

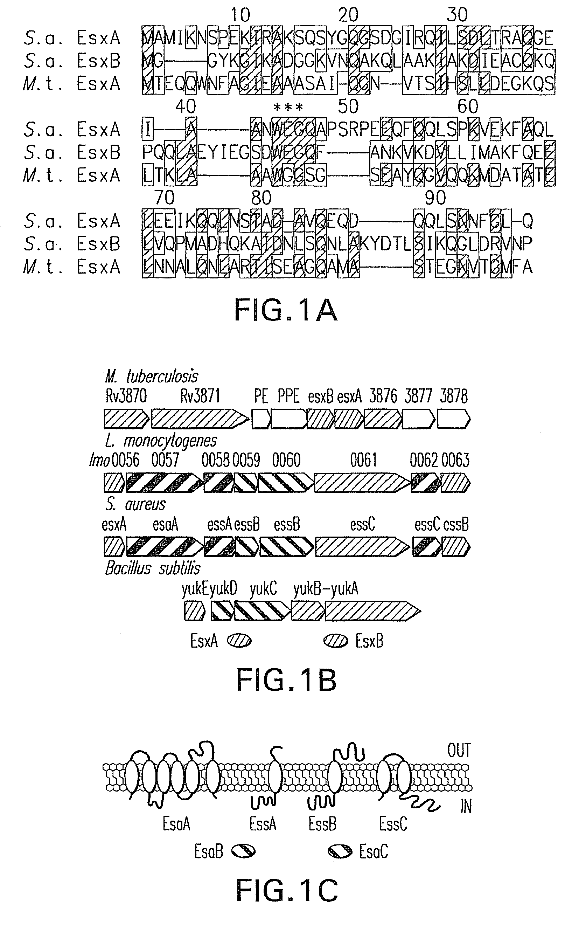

**FIGs. 1A-1C****.** *S. aureus* ess locus encoding ESAT-6-like proteins. (FIG. 1A) Protein sequence alignment of *S*. *aureus* (S.a.) EsxA and EsxB with M. tuberculosis (M.t.) EsxA. *M. tuberculosis* and *S*. *aureus* EsxA display 20.8% identity and 25% similarity, whereas *S*. *aureus* EsxB and M. tuberculosis EsxA are 17.8% identical and 35% similar. All three proteins contain the WXG motif. (FIG. 1B) Comparison of the *M. tuberculosis* H37Rv ESAT-6 locus with the *S*. *aureus, L. monocytogenes* ess loci and the *B. subtilis* yuk locus. Genes and proteins indicate FSD factors, ESAT-6-like, mycobacterial genes, and staphylococcal (also in Listeria or bacilli). (FIG. 1C) Membrane topology or soluble character of proteins encoded by the *S*. *aureus* ess locus.

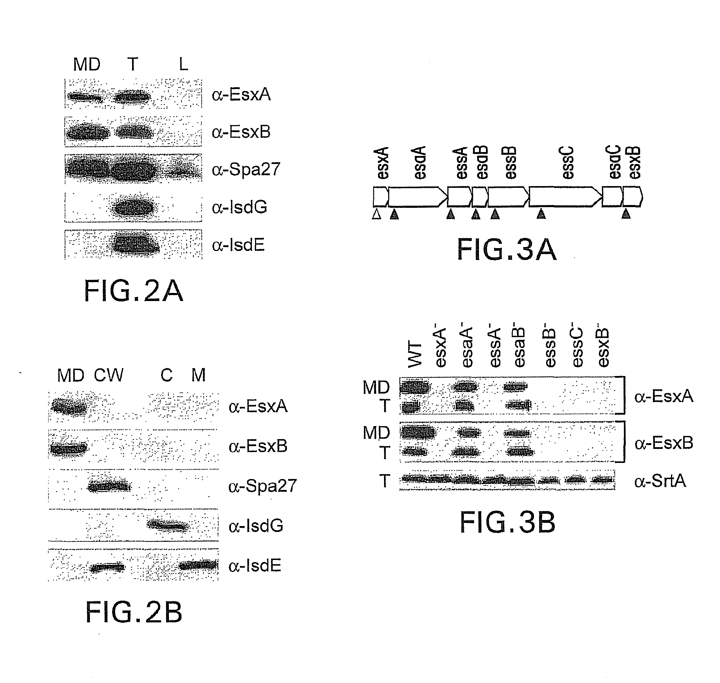

**FIGs. 2A-2B**. Subcellular location of EsxA and EsxB. (FIG. 2A) *S. aureus* strain Newman cultures were separated into medium (MD), total (T), and loosely wall associated (L) fractions. Proteins were precipitated with TCA, separated on SDS-PAGE, and detected by immunoblotting with specific antibodies [α-EsxA, α-EsxB, α-Spa (protein A), α-IsdG, and α-IsdE]. (FIG. 2B) *S*. *aureus* strain Newman cultures were fractionated into medium (MD), cell wall (CW), cytoplasm (C), and membrane (M) compartments. TCA-precipitated proteins in each compartment were revealed by SDS-PAGE and immunoblotting.
**FIGs. 3A-3B****.** Factors affecting the production and secretion of EsxA and EsxB. (FIG. 3A) Schematic drawing of the Ess cluster with the position of *bursa aurealis* insertions (filled triangles) and *esxA24* mutation (open triangles). (FIG. 3B) Medium (MD) and total protein fractions (T) were separated on SDS-15% PAGE and immunoblotted with α-EsxA and α-EsxB. α-SrtA is used as a control for total protein loaded.

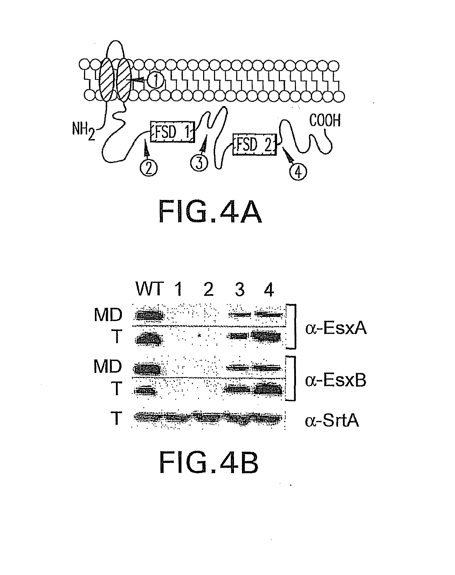

**FIGs. 4A-4B****.** Role of EssC in EsxA and EsxB secretion. (FIG. 4A) Predicted topology of EssC with insertion sites of *bursa aurealis* in each strain (filled triangles). EssC contains two FstK-SpoIIIE-like domains (FSD). (FIG. 4B) Immunoblotting of total cellular protein (T) or culture medium(MD) with α-EsxA and α-EsxB antibodies. Insertional mutations at codon 264 (mutant 1) and codon 618 (mutant 2) prevented production/secretion of EsxA and EsxB. Mutants 1-4 are strains ΦN 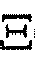 04464 (insertion at codon 264), ΦN 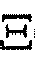 02191 (insertion at codon 618), ΦN 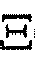 02832 (insertion at codon 721), and ΦN 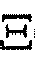 13038 (insertion at codon 1279), respectively. α-SrtA was used as a control for the total protein loaded.

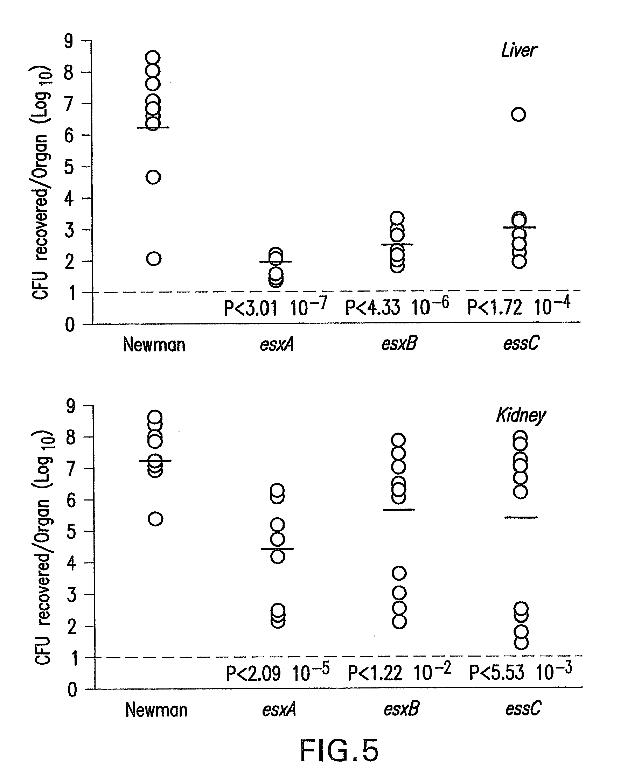

**FIG. 5****.** *S. aureus esxA, esxB,* and *essC* mutants are defective in the pathogenesis of murine abscesses. Ten 6-week-old BALB-c mice were injected retro-orbitally with 10⁶ cfu for each strain. Mice were killed 4 days after infection. Kidneys and liver were removed and homogenized. Viable bacteria were counted after dilution and colony formation on tryptic soy agar. Statistical significance was examined with the Student t test, and P values were recorded. The limit of detection (solid line) was determined to be 10 cfu (10¹).

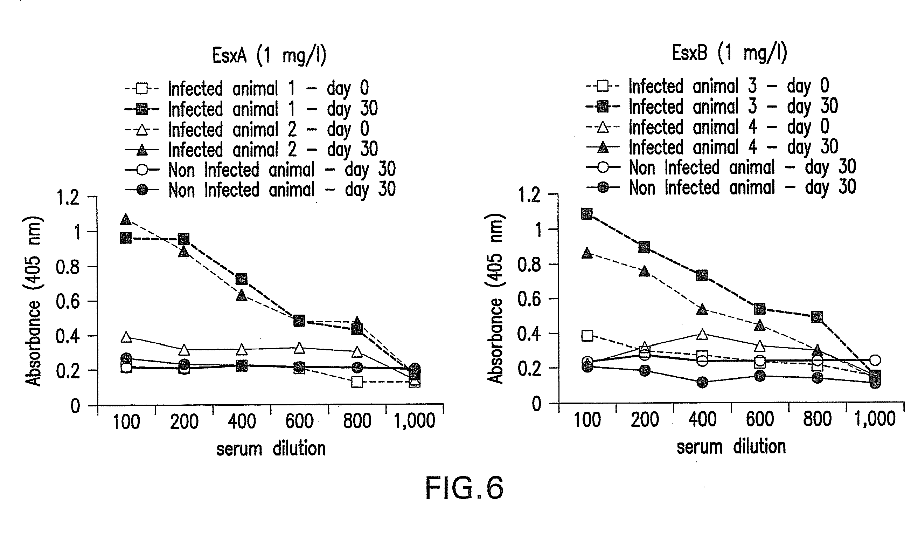

**FIG. 6****.** Serum IgG titers of animals acutely infected with *S*. *aureus.* Three-week old BALB/c mice were infected by intravenous inoculation with 1 x 10⁷ cfu of *S. aureus* Newman or PBS buffer (mock control). Mice were bled on day 0 and 30 and various dilutions of serum samples were analyzed for EsxA and EsxB specific IgG by custom ELISA.

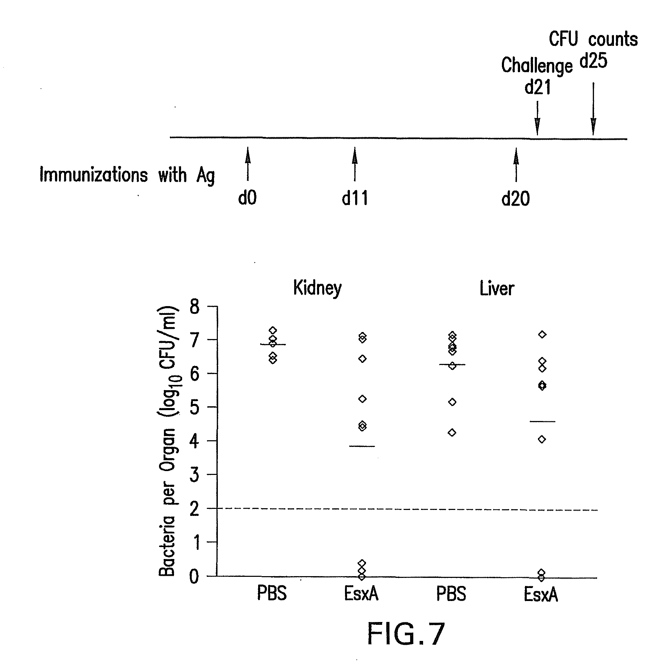

**FIG. 7****.** Purified EsxA confers partial immunity to *S*. *aureus* infection in the mouse model of disease.

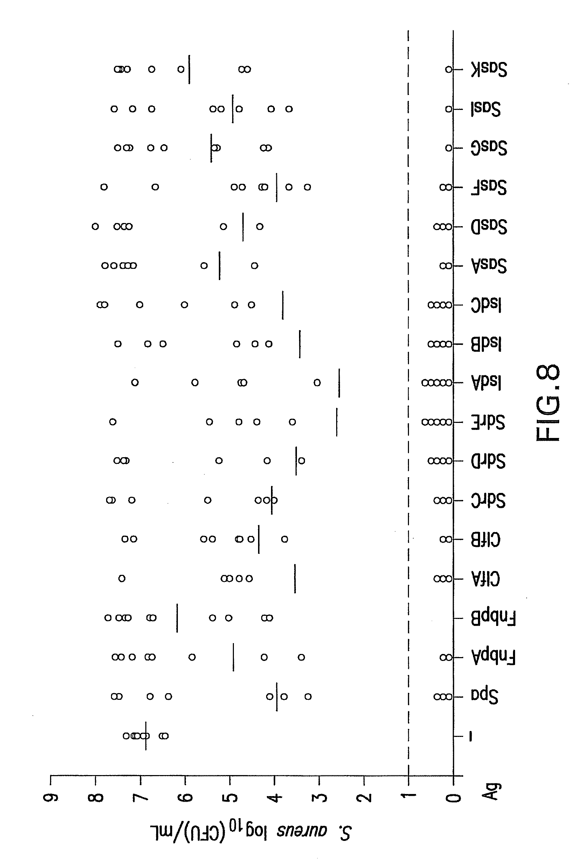

**FIG. 8****.** Protection conferred by surface proteins of *S*. *aureus,* as assessed by the renal abscess model. BALB/c mice received two doses of 100 µg protein or phosphate-buffered saline (PBS) adsorbed to CFA (day 0) or IFA (day 11). Mice were challenged with *S*. *aureus* strain Newman (∼10⁶ CFU) and 96 hours after infection the animals were killed and the kidneys excised, homogenized and plated. The dashed line indicates the limit of detection of staphylococci in renal tissues.

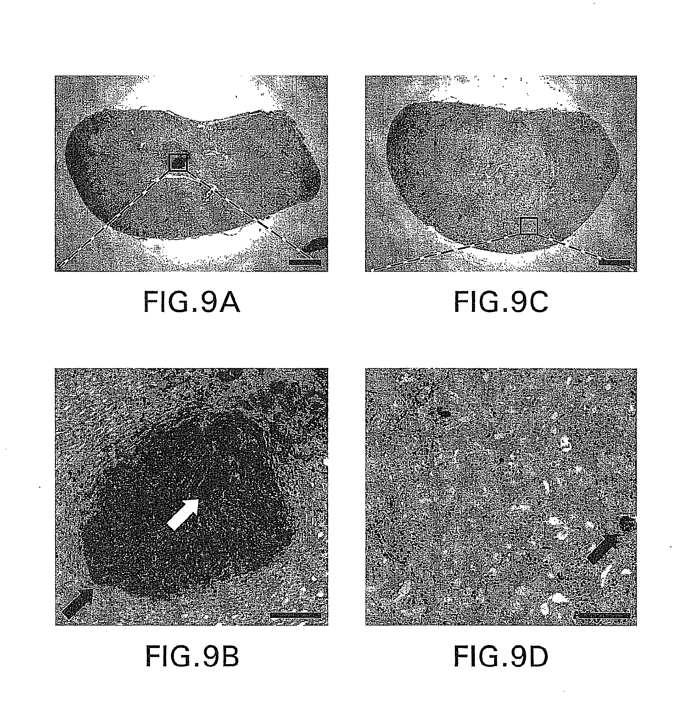

**FIGs. 9A-9D****.** Immunization with the combined vaccine (IsdA, IsdB, SdrD and SdrE) generates protective immunity against *S*. *aureus* abscess formation in mice. BALB/c mice were mock immunized (FIG. 9A and FIG. 9B) or immunized with the combined vaccine (FIG. 9C and FIG. 9D) and challenged by retro-orbital infection with *S*. *aureus* Newman. Four days after infection, animals were killed and kidneys removed. Organ tissue was fixed in formalin, thin sectioned and stained with hematoxylin/eosin. Microscopic images of whole kidneys (FIG. 9A and FIG. 9C) or organ tissue at higher magnification (FIG. 9B and FIG. 9D) revealed abscess formation only in mock immunized animals. Similar results were obtained for six organ tissues in each group. Bars indicate 1 mm (FIG. 9A and FIG. 9C) or 0.1 mm (FIG. 9B and FIG. 9D). Arrows identify a staphylococcal abscess with a central concentration of staphylococci (FIG. 9B) as well as polymorphonuclear cell infiltrate (FIG. 9D).

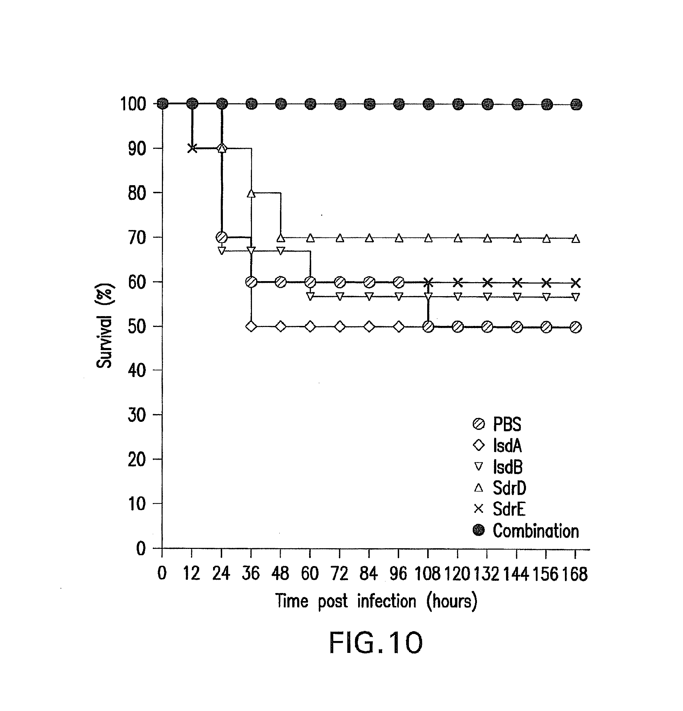

**FIG. 10****.** Immunization with the combined vaccine (IsdA, IsdB, SdrD and SdrE) generates protective immunity against lethal *S*. *aureus* challenge. Mice (n = 8-10) were immunized with individual surface protein antigens (IsdA, IsdB, SdrD or SdrE), with the combined vaccine (IsdA, IsdB, SdrD and SdrE) or with PBS. Animals were challenged by intra-peritoneal injection of *S. aureus* Newman (2 x 10¹⁰ CFU) and monitored over seven days. When compared to animals receiving mock immunization (PBS), the significance of protective immunity generated by various antigens was recorded with the Fisher's exact test as follows: IsdA (P= 0.34372), IsdB (P= 0.22049), SdrD (P= 0.24006), SdrE (P=0.31508) and Combined Vaccine (P= 0.02941). When compared to animals receiving the combined vaccine, significance of protective immunity was recorded with the Fisher's exact test as follows: PBS (P= 0.02941), IsdA (P= 0.02941), IsdB (P= 0.05294), SdrD (P= 0.00377) and SdrE (P= 0.01131).

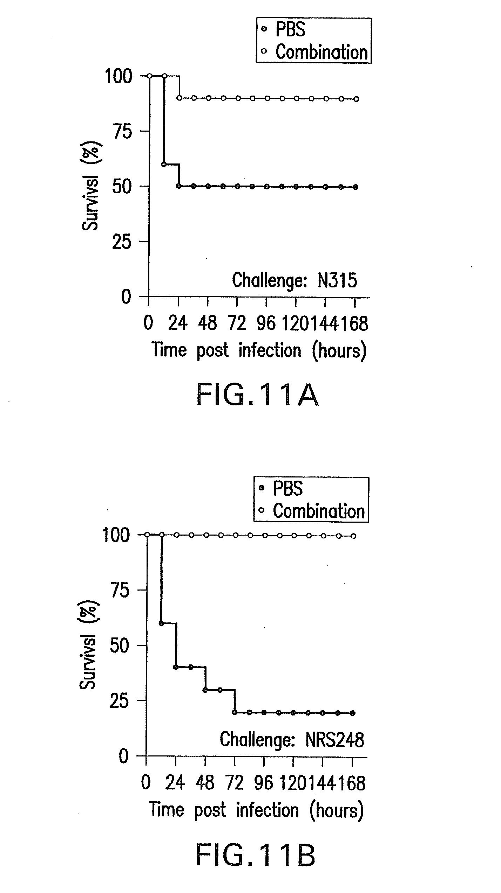

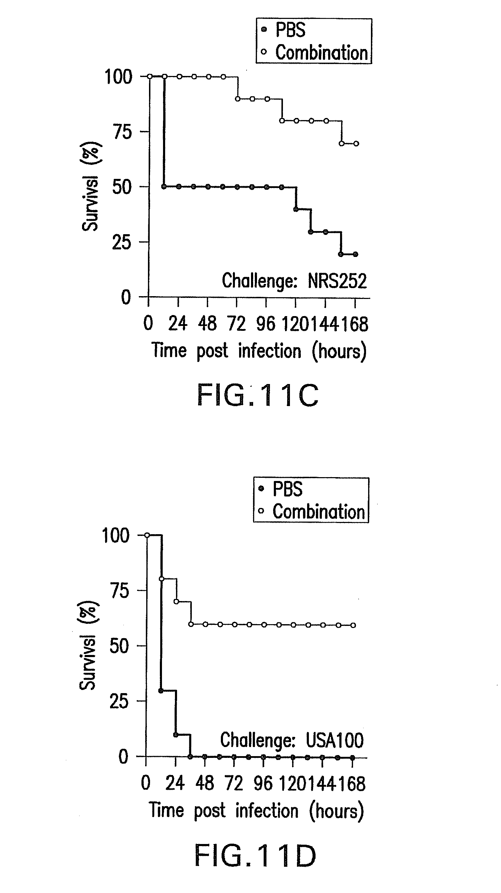

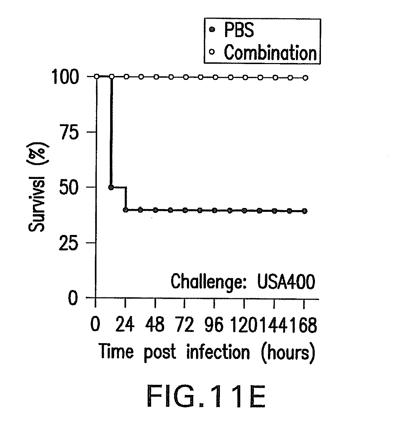

**FIGs. 11A-11E****.** Immunization with the combined vaccine generates protective immunity against lethal challenge with five different clinical *S*. *aureus* isolates. Mice (n = 10) were immunized with the combined vaccine (IsdA, IsdB, SdrD and SdrE) or with PBS, challenged by intra-peritoneal injection with clinical *S*. *aureus* isolates (3-10 x 10⁹ CFU) and monitored over seven days for survival. When compared to animals receiving mock immunization (PBS), the significance of protective immunity generated by the combined vaccine was recorded with the Fisher's exact test as follows: FIG. 11A, N315 (P= 0.06502); FIG. 11B, NRS248 (P= 0.00036); FIG. 11C, NRS252 (P= 0.03215); FIG. 11D, USA100 (P= 0.00542); and FIG. 11E, USA400 (P= 0.00542).

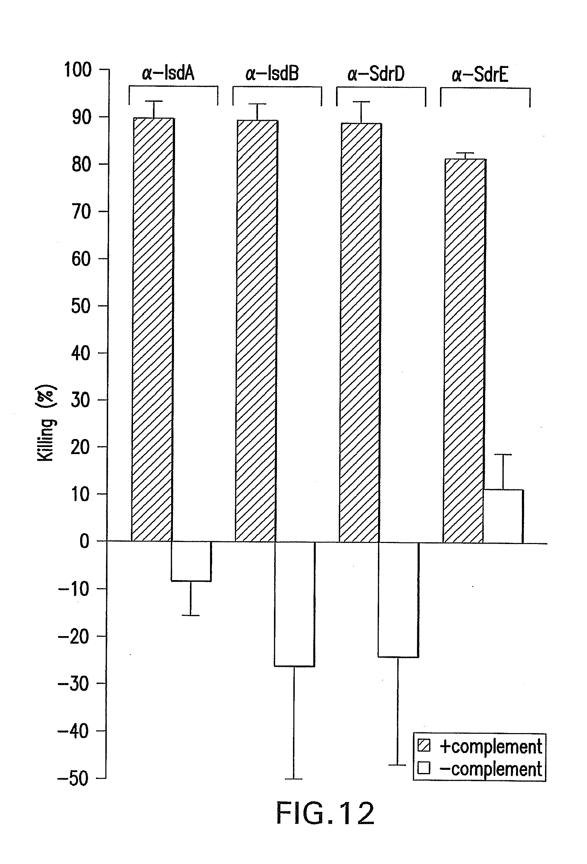

**FIG. 12****.** Antibodies against IsdA, IsdB, SdrD, and SdrE mediate complement-dependent opsonophagocytosis. Phagocytic assays were performed to determine the mechanism of the protection afforded by these surface proteins. Rabbit antibodies, rabbit complement, freshly isolated human PMNs, and ***S*.** *aureus* were incubated and then plated on agar medium to measure bacterial survival as colony forming units (CFU). Percentage killing was calculated. Error bars represent the SEM.

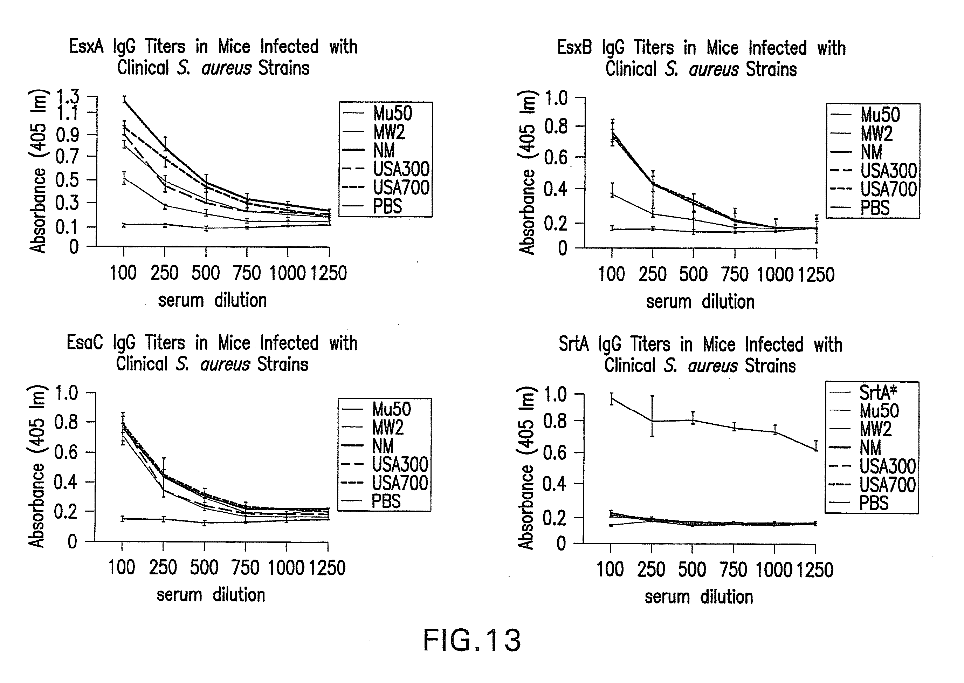

**FIG. 13****.** ELISA showing the titers of EsxA, EsxB, EsaC antigens in the sera of mice infected with either one of the following staphylococcal strains Mu50, MW2, Newman (NM), USA300, USA700. PBS is used as a control (uninfected animals). The last panel shows the same ELISA for SrtA (Sortase A). Sortase A is a protein of the plasma membrane of staphylococci and animals do not develop anti-SrtA IgG during infection. As a positive control, the serum of a rabbit immunized with recombinant SrtA is shown (SrtA+, last panel on the right).

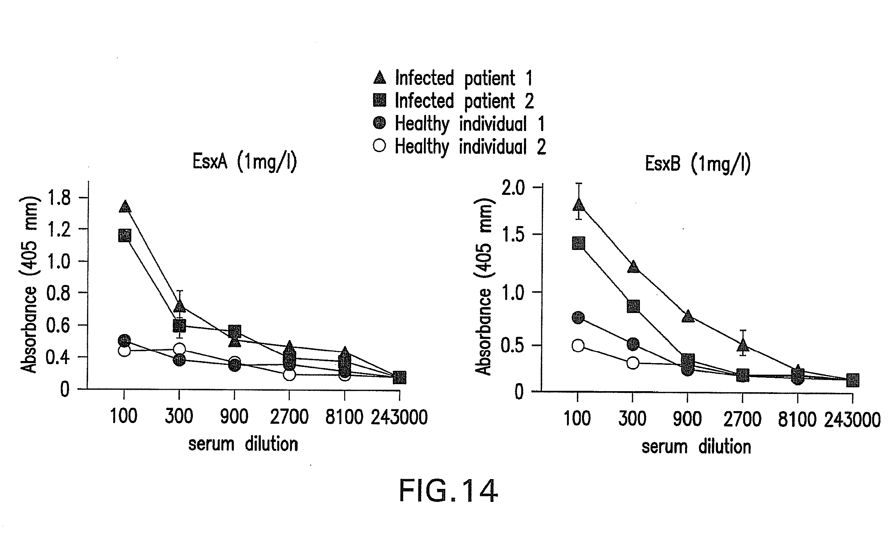

**FIG. 14****.** Serum IgG titers of patients infected with *S. aureus.* Sera of two non infected individuals were used as a control. Various dilutions of sera were analyzed for EsxA and EsxB specific IgG by custom ELISA.

### DETAILED DESCRIPTION OF THE INVENTION

The pathogenesis of staphylococcal infections relies on many different virulence factors such as secreted exotoxins, exopolysaccharides, and surface adhesins. However, deletion of single genes encoding such factors causes either no defect or results in only modest reduction of virulence. The development of staphylococcal vaccines is hindered by the multifaceted nature of staphylococcal invasion strategies. It is well established that live attenuated micro-organisms are highly effective vaccines; immune responses elicited by such vaccines are often of greater magnitude and of longer duration than those produced by non-replicating immunogens. One explanation for this may be that live attenuated strains establish limited infections in the host and mimic the early stages of natural infection. Embodiments of the invention are directed to immunogenic extracellular proteins, polypeptides, and peptides (including both secreted and cell surface proteins or peptides) of gram positive bacteria for the use in mitigating or immunizing against infection. In particular embodiments the bacteria is a staphylococcus. Extracellular proteins, polypeptides, or peptides include, but are not limited to secreted and cell surface proteins of the targeted bacteria.

The human pathogen *S. aureus* secretes EsxA and EsxB, two ESAT-6 like proteins, across the bacterial envelope (Burts *et al.,* 2005, which is incorporated herein by reference). Staphylococcal *esxA* and *esxB* are clustered with six other genes in the order of transcription: *esxA esaA essA esaB essB essC esaC esxB* (FIG. 1). The acronyms esa, ess, and esx stand for ESAT-6 secretion accessory, system, and extracellular, respectively, depending whether the encoded proteins play an accessory (esa) or direct (ess) role for secretion, or are secreted (esx) into the extracellular milieu. The entire cluster of eight genes is herein referred to as the Ess cluster. The inventors have shown that *esxA, esxB, essA, essB,* and *essC* are all required for synthesis or secretion of EsxA and EsxB. Mutants that fail to produce EsxA, EsxB, and EssC display defects in the pathogenesis of *S*. *aureus* murine abscesses, suggesting that this specialized secretion system may be a general strategy of human bacterial pathogenesis.

The *Staphylococcus aureus* Ess pathway can be viewed as a secretion module equipped with specialized transport components (Ess), accessory factors (Esa) and cognate secretion substrates (Esx). EssA, EssB and EssC are required for EsxA and EsxB secretion. Because EssA, EssB and EssC are predicted to be transmembrane proteins (FIG. 1), it is contemplated that these proteins form a secretion apparatus. Some of the proteins in the *ess* gene cluster may actively transport secreted substrates (acting as motor) while others may regulate transport (regulator). Regulation may be achieved, but need not be limited to, transcriptional or post-translational mechanisms for secreted polypeptides, sorting of specific substrates to defined locations (*e.g.*, extracellular medium or host cells), or timing of secretion events during infection. At this point, it is unclear whether all secreted Esx proteins function as toxins or contribute indirectly to pathogenesis. Two hypotheses may be considered. Hypothesis one: secreted EsxA and EsxB directly affect host function and hence encode bona fide toxins. Members of the ESAT-6 family only share between 16-20% identity at the protein level. Conservation of sequence identity may be due to cis-acting elements within peptide sequence required for substrate recognition or secretion while the remainder of the polypeptide may encode toxin activity(ies). Hypothesis two: EsxA and EsxB could serve as chaperones for the secretion of other proteins (effectors) into the extra-cellular medium or into the cytosol of eukaryotic cells. In this model, while one of the machinery components (EssC) and EsxAB are shared by mycobacteria and gram-positive bacteria, and may be identified by sequence similarity, the secreted effectors may be highly specific for a given bacterial species and implement infectious disease strategies unique to this bacterial species. For example, the accessory proteins EsaB and EsaC could be substrates for the EssABC-EsxAB secretion machinery.

Staphylococci rely on surface protein mediated-adhesion to host cells or invasion of tissues as a strategy for escape from immune defenses. Furthermore, *S. aureus* utilize surface proteins to sequester iron from the host during infection. The majority of surface proteins involved in staphylococcal pathogenesis carry C-terminal sorting signals, *i.e*., they are covalently linked to the cell wall envelope by sortase. Further, staphylococcal strains lacking the genes required for surface protein anchoring, *i.e*., sortase A and B, display a dramatic defect in the virulence in several different mouse models of disease. Thus, surface protein antigens represent a validated vaccine target as the corresponding genes are essential for the development of staphylococcal disease and can be exploited in various embodiments of the invention. The sortase enzyme superfamily are Gram-positive transpeptidases responsible for anchoring surface protein virulence factors to the peptidoglycan cell wall layer. Two sortase isoforms have been identified in *Staphylococcus aureus,* SrtA and SrtB. These enzymes have been shown to recognize a LPXTG or NPQTN motif in substrate proteins, respectively. The SrtB isoform appears to be important in heme iron acquisition and iron homeostasis, whereas the SrtA isoform plays a critical role in the pathogenesis of Gram-positive bacteria by modulating the ability of the bacterium to adhere to host tissue via the covalent anchoring of adhesions and other proteins to the cell wall peptidoglycan. Embodiments of the invention include, but are not limited to compositions and methods related to SdrC, SdrD, SdrE, IsdA, IsdB, Spa, ClfA, ClfB, IsdC and/or SasF proteins.

### I. STAPHYLOCOCCAL ANTIGENS

Certain aspects of the invention include methods and compositions concerning proteinaceous compositions including polypeptides, peptides, or nucleic acid encoding such, of an Ess pathway, preferably of the Esx class and more preferably all or part of EsxA, EsxB, or other proteins transported by the Ess pathway, or sortase substrates including, but not limited to SdrC, SdrD, SdrE, IsdA, IsdB, Spa, ClfA, ClfB, IsdC, SasF or combinations thereof. These proteins may be modified by deletion, insertion, and/or substitution. In particular embodiments, modifications of these proteins are capable of eliciting an immune response in a subject.

The Esx polypeptides include the amino acid sequence of Esx proteins from bacteria in the Staphylococcus genus. The Esx sequence may be from a particular staphylococcus species, such as *Staphylococcus aureus,* and may be from a particular strain, such as Newman. In certain embodiments, the EsxA sequence is SAV0282 from strain Mu50 (which is the same amino acid sequence for Newman) and can be accessed using Genbank Accession Number Q99WU4 (gi|68565539) (SEQ ID NO:2), which is hereby incorporated by reference. In other embodiments, the EsxB sequence is SAV0290 from strain Mu50 (which is the same amino acid sequence for Newman) and can be accessed using Genbank Accession Number Q99WT7 (gi|68565532) (SEQ ID NO:4), which is hereby incorporated by reference. In further embodiments, other polypeptides transported by the Ess pathway may be used, the sequences of which may be identified by one of skill in the art using databases and internet accessible resources.

The sortase substrate polypeptides include, but are not limited to the amino acid sequence of SdrC, SdrD, SdrE, IsdA, IsdB, Spa, ClfA, ClfB, IsdC or SasF proteins from bacteria in the Staphylococcus genus. The sortase substrate polypeptide sequence may be from a particular staphylococcus species, such as *Staphylococcus aureus,* and may be from a particular strain, such as Newman. In certain embodiments, the SdrD sequence is from strain N315 and can be accessed using Genbank Accession Number NP_373773.1 (gi|15926240) (SEQ ID NO:6), which is hereby incorporated by reference. In other embodiments, the SdrE sequence is from strain N315 and can be accessed using Genbank Accession Number NP_373774.1 (g|15926241) (SEQ ID NO:8), which is hereby incorporated by reference. In other embodiments, the IsdA sequence is SAV1130 from strain Mu50 (which is the same amino acid sequence for Newman) and can be accessed using Genbank Accession Number NP_371654.1 (gi|15924120) (SEQ ID NO:10), which is hereby incorporated by reference. In other embodiments, the IsdB sequence is SAV1129 from strain Mu50 (which is the same amino acid sequence for Newman) and can be accessed using Genbank Accession Number NP_371653.1 (gi|15924119) (SEQ ID NO:12), which is hereby incorporated by reference. In further embodiments, other polypeptides transported by the Ess pathway or processed by sortase may be used, the sequences of which may be identified by one of skill in the art using databases and internet accessible resources.

Examples of various proteins that can be used in the context of the present invention can be identified by analysis of database submissions of bacterial genomes, including but not limited to Refseq accession numbers NC_002951 (GI:57650036 and GenBank CP000046), NC_002758 (GI:57634611 and GenBank BA000017), NC_002745 (GI:29165615 and GenBank BA000018), NC_003923 (GI:21281729 and GenBank BA000033), NC_002952 (GI:49482253 and GenBank BX571856), NC_002953 (GI:49484912 and GenBank BX571857), NC_007793 (GI:87125858 and GenBank CP000255), NC_007795 (GI:87201381 and GenBank CP000253) each of which are incorporated herein by reference in their entirety.

As used herein, a "protein" or "polypeptide" refers to a molecule comprising at least ten amino acid residues. In some embodiments, a wild-type version of a protein or polypeptide are employed, however, in many embodiments of the invention, a modified protein or polypeptide is employed to generate an immune response. The terms described above may be used interchangeably herein. A "modified protein" or "modified polypeptide" refers to a protein or polypeptide whose chemical structure, particularly its amino acid sequence, is altered with respect to the wild-type protein or polypeptide. In some embodiments, a modified protein or polypeptide has at least one modified activity or function (recognizing that proteins or polypeptides may have multiple activities or functions). It is specifically contemplated that a modified protein or polypeptide may be altered with respect to one activity or function yet retain a wild-type activity or function in other respects, such as immunogenicity.

In certain embodiments the size of a protein or polypeptide (wild-type or modified) may comprise, but is not limited to, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 275, 300, 325, 350, 375, 400, 425, 450, 475, 500, 525, 550, 575, 600, 625, 650, 675, 700, 725, 750, 775, 800, 825, 850, 875, 900, 925, 950, 975, 1000, 1100, 1200, 1300, 1400, 1500, 1750, 2000, 2250, 2500 amino molecules or greater, and any range derivable therein, or derivative thereof. It is contemplated that polypeptides may be mutated by truncation, rendering them shorter than their corresponding wild-type form, but also they might be altered by fusing or conjugating a heterologous protein sequence with a particular function (*e.g.*, for targeting or localization, for enhanced immunogenicity, for purification purposes, *etc*.).

As used herein, an "amino molecule" refers to any amino acid, amino acid derivative, or amino acid mimic known in the art. In certain embodiments, the residues of the proteinaceous molecule are sequential, without any non-amino molecule interrupting the sequence of amino molecule residues. In other embodiments, the sequence may comprise one or more non-amino molecule moieties. In particular embodiments, the sequence of residues of the proteinaceous molecule may be interrupted by one or more non-amino molecule moieties.

Accordingly, the term "proteinaceous composition" encompasses amino molecule sequences comprising at least one of the 20 common amino acids in naturally synthesized proteins, or at least one modified or unusual amino acid.

Proteinaceous compositions may be made by any technique known to those of skill in the art, including (i) the expression of proteins, polypeptides, or peptides through standard molecular biological techniques, (ii) the isolation of proteinaceous compounds from natural sources, or (iii) the chemical synthesis of proteinaceous materials. The nucleotide as well as the protein, polypeptide, and peptide sequences for various genes have been previously disclosed, and may be found in the recognized computerized databases. One such database is the National Center for Biotechnology Information's Genbank and GenPept databases (on the World Wide Web at ncbi.nlm.nih.gov/). The coding regions for these genes may be amplified and/or expressed using the techniques disclosed herein or as would be know to those of ordinary skill in the art.

Amino acid sequence variants of EsxA, EsxB, and other polypeptides transported by the Ess pathway ("other Ess pathway polypeptides"), and/or SdrC, SdrD, SdrE, IsdA, IsdB, Spa, ClfA, ClfB, IsdC, SasF or other sortase substrates can be substitutional, insertional, or deletion variants. A modification in a polypeptide of the invention may affect 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500 or more non-contiguous or contiguous amino acids of the polypeptide, as compared to wild-type. A polypeptide processed or secreted by the Ess pathway, and/or SdrC, SdrD, SdrE, IsdA, IsdB, Spa, ClfA, ClfB, IsdC, SasF or other surface proteins (see Table 1) or sortase substrates from any staphylococcus species and strain are contemplated for use in methods of the invention.

Deletion variants typically lack one or more residues of the native or wild-type protein. Individual residues can be deleted or a number of contiguous amino acids can be deleted. A stop codon may be introduced (by substitution or insertion) into an encoding nucleic acid sequence to generate a truncated protein. Insertional mutants typically involve the addition of material at a non-terminal point in the polypeptide. This may include the insertion of one or more residues. Terminal additions, called fusion proteins, may also be generated.

Substitutional variants typically contain the exchange of one amino acid for another at one or more sites within the protein, and may be designed to modulate one or more properties of the polypeptide, with or without the loss of other functions or properties. Substitutions may be conservative, that is, one amino acid is replaced with one of similar shape and charge. Conservative substitutions are well known in the art and include, for example, the changes of: alanine to serine; arginine to lysine; asparagine to glutamine or histidine; aspartate to glutamate; cysteine to serine; glutamine to asparagine; glutamate to aspartate; glycine to proline; histidine to asparagine or glutamine; isoleucine to leucine or valine; leucine to valine or isoleucine; lysine to arginine; methionine to leucine or isoleucine; phenylalanine to tyrosine, leucine or methionine; serine to threonine; threonine to serine; tryptophan to tyrosine; tyrosine to tryptophan or phenylalanine; and valine to isoleucine or leucine. Alternatively, substitutions may be non-conservative such that a function or activity of the polypeptide is affected. Non-conservative changes typically involve substituting a residue with one that is chemically dissimilar, such as a polar or charged amino acid for a nonpolar or uncharged amino acid, and vice versa.

**Table 1. Exemplary surface proteins of S. aureus strains.**

| **SAV #** | **SA#** | Surface | MW2 | Mu50 | N315 | Newman | MRSA252* | MSSA476* |
|---|---|---|---|---|---|---|---|---|
| SAV0111 | SA0107 | Spa | 492 | 450 | 450 | | 516 | 492 |
| SAV2503 | SA2291 | FnBPA | 1015 | 1038 | 1038 | | - | 1015 |
| SAV2502 | SA2290 | FnBPB | 943 | 961 | 961 | | 965 | 957 |
| SAV0811 | SA0742 | ClfA | 946 | 935 | 989 | 933 | 1029 | 928 |
| SAV2630 | SA2423 | ClfB | 907 | 877 | 877 | 913 | 873 | 905 |
| Np | np | Can | 1183 | - | - | | 1183 | 1183 |
| SAV0561 | SA0519 | SdrC | 955 | 953 | 953 | 947 | 906 | 957 |
| SAV0562 | SA0520 | SdrD | 1347 | 1385 | 1385 | 1315 | - | 1365 |
| SAV0563 | SA0521 | SdrE | 1141 | 1141 | 1141 | 1166 | 1137 | 1141 |
| Np | np | Pls | - | - | - | | - | - |
| SAV2654 | SA2447 | SasA | 2275 | 2271 | 2271 | | 1351 | 2275 |
| SAV2160 | SA1964 | SasB | 686 | 2481 | 2481 | | 2222 | 685 |
| | SA1577 | SasC | 2186 | 213 | 2186 | | 2189 | 2186 |
| SAV0134 | SA0129 | SasD | 241 | 241 | 241 | | 221 | 241 |
| SAV1130 | SA0977 | SasE/IsdA | 350 | 350 | 350 | | 354 | 350 |
| SAV2646 | SA2439 | SasF | 635 | 635 | 635 | | 627 | 635 |
| SAV2496 | | SasG | 1371 | 525 | 927 | | - | 1371 |
| SAV0023 | SA0022 | SasH | 772 | - | 772 | | 786 | 786 |
| SAV1731 | SA1552 | SasI | 895 | 891 | 891 | | 534 | 895 |
| SAV1129 | SA0976 | SasJ/IsdB | 645 | 645 | 645 | | 652 | 645 |
| | SA2381 | SasK | 198 | 211 | 211 | | - | 197 |
| | np | SasL | - | 232 | - | | - | - |
| SAV1131 | SA0978 | IsdC | 227 | 227 | 227 | | 227 | 227 |

Proteins of the invention may be recombinant, or synthesized *in vitro.* Alternatively, a non-recombinant or recombinant protein may be isolated from bacteria. It is also contemplated that a bacteria containing such a variant may be implemented in compositions and methods of the invention. Consequently, a protein need not be isolated.

The term "functionally equivalent codon" is used herein to refer to codons that encode the same amino acid, such as the six codons for arginine or serine, and also refers to codons that encode biologically equivalent amino acids (see Table 2, below).

**Table 2 Codon Table**

| Amino Acids | | | Codons |
|---|---|---|---|
| **Alanine** | **Ala** | **A** | **GCA GCC GCG GCU** |
| **Cysteine** | **Cys** | **C** | **UGC UGU** |
| **Aspartic acid** | **Asp** | **D** | **GAC GAU** |
| **Glutamic acid** | **Glu** | **E** | **GAA GAG** |
| **Phenylalanine** | **Phe** | **F** | **UUC UUU** |
| **Glycine** | **Gly** | **G** | **GGA GGC GGG GGU** |
| **Histidine** | **His** | **H** | **CAC CAU** |
| **Isoleucine** | **Ile** | **I** | **AUA AUC AUU** |
| **Lysine** | **Lys** | **K** | **AAA AAG** |
| **Leucine** | **Leu** | **L** | **UUA UUG CUA CUC CUG CUU** |
| **Methionine** | **Met** | **M** | **AUG** |
| **Asparagine** | **Asn** | **N** | **AAC AAU** |
| **Proline** | **Pro** | **P** | **CCA CCC CCG CCU** |
| **Glutamine** | **Gln** | **Q** | **CAA CAG** |
| **Arginine** | **Arg** | **R** | **AGA AGG CGA CGC CGG CGU** |
| **Serine** | **Ser** | **S** | **AGC AGU UCA UCC UCG UCU** |
| **Threonine** | **Thr** | **T** | **ACA ACC ACG ACU** |
| **Valine** | **Val** | **V** | **GUA GUC GUG GUU** |
| **Tryptophan** | **Trp** | **W** | **UGG** |
| **Tyrosine** | **Tyr** | **Y** | **UAC UAU** |

It also will be understood that amino acid and nucleic acid sequences may include additional residues, such as additional N- or C-terminal amino acids, or 5' or 3' sequences, respectively, and yet still be essentially as set forth in one of the sequences disclosed herein, so long as the sequence meets the criteria set forth above, including the maintenance of biological protein activity where protein expression is concerned. The addition of terminal sequences particularly applies to nucleic acid sequences that may, for example, include various non-coding sequences flanking either of the 5' or 3' portions of the coding region.

The following is a discussion based upon changing of the amino acids of a protein to create an equivalent, or even an improved, second-generation molecule. For example, certain amino acids may be substituted for other amino acids in a protein structure without appreciable loss of interactive binding capacity with structures such as, for example, antigen-binding regions of antibodies or binding sites on substrate molecules. Since it is the interactive capacity and nature of a protein that defines that protein's biological functional activity, certain amino acid substitutions can be made in a protein sequence, and in its underlying DNA coding sequence, and nevertheless produce a protein with like properties. It is thus contemplated by the inventors that various changes may be made in the DNA sequences of genes without appreciable loss of their biological utility or activity.

In making such changes, the hydropathic index of amino acids may be considered. The importance of the hydropathic amino acid index in conferring interactive biologic function on a protein is generally understood in the art (Kyte and Doolittle, 1982). It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like.

It also is understood in the art that the substitution of like amino acids can be made effectively on the basis of hydrophilicity. U.S. Patent 4,554,101, incorporated herein by reference, states that the greatest local average hydrophilicity of a protein, as governed by the hydrophilicity of its adjacent amino acids, correlates with a biological property of the protein. It is understood that an amino acid can be substituted for another having a similar hydrophilicity value and still produce a biologically equivalent and immunologically equivalent protein.

As outlined above, amino acid substitutions generally are based on the relative similarity of the amino acid side-chain substituents, for example, their hydrophobicity, hydrophilicity, charge, size, and the like. Exemplary substitutions that take into consideration the various foregoing characteristics are well known and include: arginine and lysine; glutamate and aspartate; serine and threonine; glutamine and asparagine; and valine, leucine and isoleucine.

It is contemplated that in compositions of the invention, there is between about 0.001 mg and about 10 mg of total protein per ml. Thus, the concentration of protein in a composition can be about, at least about or at most about 0.001, 0.010, 0.050, 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0 mg/ml or more (or any range derivable therein). Of this, about, at least about, or at most about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 3, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100% may be EsxA protein, EsxB protein, or another protein transported by the Ess pathway, and/or SdrD, SdrE, IsdA, IsdB, or other sortase substrates.

The present invention contemplates the administration of EsxA or EsxB polypeptides or peptides, as well as any other protein transported by the Ess pathway, and/or SdrD, SdrE, IsdA, IsdB, or other sortase substrates, to effect a preventative therapy against the development of a disease or condition associated with infection by a staphylococcus pathogen.

In addition, U.S. Patent 4,554,101 (Hopp), which is incorporated herein by reference, teaches the identification and preparation of epitopes from primary amino acid sequences on the basis of hydrophilicity. Through the methods disclosed in Hopp, one of skill in the art would be able to identify potential epitopes from within an amino acid sequence and confirm their immunogenicity. Numerous scientific publications have also been devoted to the prediction of secondary structure and to the identification of epitopes, from analyses of amino acid sequences (Chou & Fasman, 1974a,b; 1978a,b, 1979). Any of these may be used, if desired, to supplement the teachings of Hopp in U.S. Patent 4,554,101.

### A. Other antigens

Compositions of the invention can include one or more additional active agents. Such agents include, but are not limited to one or more (a) staphylococcal antigens, (b) non-staphylococcal antigens, (c) nucleic acid molecules encoding (a) or (b), and antibodies which specifically bind to (a) or (b).

### 1. Staphylococcal antigens

Staphylococcal antigens which can be included in compositions of the invention include *S*. *aureus* type 5 and 8 capsular polysaccharides optionally conjugated to nontoxic recombinant Pseudomonas aeruginosa exotoxin A, such as StaphVAX®, antigens derived from extracellular polysaccharides (lipoteichoic acid, cell wall teichoic acid, poly-N-acetylglucosamine (PNAG) exopolysacchride) or antigens derived from surface proteins, invasins (leukocidin, kinases, hyaluronidase), surface factors that inhibit phagocytic engulfment (capsule, Protein A), carotenoids, catalase production, Protein A, coagulase, clotting factor, and/or membrane-damaging toxins (optionally detoxified) that lyse eukaryotic cell membranes (hemolysins, leukotoxin, leukocidin).

### 2. Non-staphylococcal antigens

Compositions of the invention may be administered in conjunction with one or more antigens for use in therapeutic, prophylactic, or diagnostic methods of the present invention. Compositions of the invention optionally can comprise one or more additional polypeptide antigens which are not derived from staphylococcal proteins. Preferred antigens include those listed below. Additionally, the compositions of the present invention may be used to treat or prevent infections caused by any of the below-listed pathogens. In addition to combination with the antigens described below, the compositions of the invention may also be combined with an adjuvant as described herein.

Antigens for use with the invention include, but are not limited to, one or more of the following antigens set forth below, or antigens derived from one or more of the pathogens set forth below:

### a. Bacterial Antigens

Bacterial antigens suitable for use in the invention include proteins, polysaccharides, lipopolysaccharides, and outer membrane vesicles which may be isolated, purified or derived from a bacteria. In addition, bacterial antigens may include bacterial lysates and inactivated bacteria formulations. Bacteria antigens may be produced by recombinant expression. Bacterial antigens preferably include epitopes which are exposed on the surface of the bacteria during at least one stage of its life cycle. Bacterial antigens are preferably conserved across multiple serotypes. Bacterial antigens include antigens derived from one or more of the bacteria set forth below as well as the specific antigens examples identified below.

*Neisseria meningitides*: Meningitides antigens may include proteins, saccharides (including a polysaccharide, oligosaccharide or lipopolysaccharide), or outer-membrane vesicles purified or derived from any *N. meningitides* serogroups. Meningitides protein antigens may be selected from adhesions, autotransporters, toxins, Fe acquisition proteins, and membrane associated proteins (preferably integral outer membrane protein).

*Streptococcus pneumoniae*: *Streptococcus pneumoniae* antigens may include a saccharide (including a polysaccharide or an oligosaccharide) and/or protein from *Streptococcus pneumoniae.* Saccharide antigens may be selected from serotypes 1, 2, 3, 4, 5, 6B, 7F, 8, 9N, 9V, bA, hA, 12F, 14, 15B, 17F, 18C, 19A, 19F, 20, 22F, 23F, and 33F. Protein antigens may be selected from a protein identified in WO 98/18931, WO 98/18930, US Patent No. 6,699,703, US Patent No. 6,800,744, WO 97/43303, and WO 97/37026. *Streptococcus pneumoniae* proteins may be selected from the Poly Histidine Triad family (PhtX), the Choline Binding Protein family (CbpX), CbpX truncates, LytX family, LytX truncates, CbpX truncate-LytX truncate chimeric proteins, pneumolysin (Ply), PspA, PsaA, Sp128, Sp1O1, Sp130, Sp125 or Sp133.

*Streptococcus pyogenes* (Group A Streptococcus): GAS antigens may include a protein identified in WO 02/34771 or WO 2005/032582 (including GBS 40), fusions of fragments of GBS M proteins (including those described in WO 02/094851, and Dale, Vaccine (1999) 17:193-200, and Dale, Vaccine 14(10): 944-948), fibronectin binding protein (STh 1), Streptococcal heme-associated protein (Shp), and Streptolysin S (SagA).

*Streptococcus agalactiae* (Group B Streptococcus): Group B Streptococcus antigens include a protein or saccharide antigen identified in WO 02/34771, WO 03/093306, WO 04/041157, or WO 2005/0026 19 (including proteins GBS 80, GBS 104, GBS 276 and GBS 322, and including saccharide antigens derived from serotypes Ia, Tb, Ia/c, II, III, IV, V, VI, VII and VIII).

*Moraxella catarrhalis*: Moraxella antigens include antigens identified in WO 02/18595 and WO 99/58562, outer membrane protein antigens (HMW-OMP), C-antigen, and/or LPS.

*Bordetella pertussis*: Pertussis antigens include pertussis holotoxin (PT) and filamentous haemagglutinin (FHA) from *B. pertussis,* optionally also combination with pertactin and/or agglutinogens 2 and 3 antigen.

*Clostridium tetani* (Tetanus): Tetanus antigens include tetanus toxoid (TT), preferably used as a carrier protein in conjunction/conjugated with the compositions of the present invention.

*Cornynebacterium diphtheriae* (Diphtheria): Diphtheria antigens include diphtheria toxin, preferably detoxified, such as CRM197. Additionally antigens capable of modulating, inhibiting or associated with ADP ribosylation are contemplated for combination/co-administration/conjugation with the compositions of the present invention. The diphtheria toxoids may be used as carrier proteins.

*Haemophilus influenzae* B (Hib): Hib antigens include a Hib saccharide antigen.

*Pseudomonas aeruginosa*: Pseudomonas antigens include endotoxin A, Wzz protein, *P. aeruginosa* LPS, more particularly LPS isolated from PAO1 (05 serotype), and/or Outer Membrane Proteins, including Outer Membrane Proteins F (OprF) (Infect Immun. 2001 May; 69(5) : 3510-3515).

*Legionella pneumophila:* Bacterial antigens may be derived from *Legionella pneumophila.*

*Neiserria gonorrhoeae*: *Gonorrhoeae* antigens include Por (or porin) protein, such as PorB (see Zhu et al., Vaccine (2004) 22:660 - 669), a transferring binding protein, such as TbpA and TbpB (See Price et al., Infection and Immunity (2004) 71(1):277 - 283), a opacity protein (such as Opa), a reduction-modifiable protein (Rmp), and outer membrane vesicle (OMV) preparations (see Plante et al., J Infectious Disease (2000) 182:848 - 855), also see e.g. WO99/24578, WO99/36544, WO99/57280.

*Chlamydia trachomatis*: *Chlamydia trachomatis* antigens include antigens derived from serotypes A, B, Ba and C (agents of trachoma, a cause of blindness), serotypes Li, L2 & L3 (associated with Lymphogranuloma venereum), and serotypes, D-K. *Chlamydia trachomas* antigens may also include an antigen identified in WO 00/37494, WO 03/049762, WO 03/068811, or WO 05/0026 19, including PepA (CT045), LcrE (CT089), ArtJ (CT381), DnaK (CT396), CT398, OmpH-like (CT242), L7/L12 (CT316), OmcA (CT444), AtosS (CT467), CT547, Eno (CT587), HrtA (CT823), and MurG (CT761).

*Treponeina pallidum* (Syphilis): Syphilis antigens include TmpA antigen.

*Haemophilus ducreyi* (causing chancroid): Ducreyi antigens include outer membrane protein (DsrA).

*Enterococcus faecalis* or *Enterococcus faecium:* Antigens include a trisaccharide repeat or other Enterococcus derived antigens provided in US Patent No. 6,756,361.

*Helicobacter pylori*: *H. pylori* antigens include Cag, Vac, Nap, HopX, HopY and/or urease antigen.

*Yersinia enterocolitica* : *Yersinia enterocolitica* antigens include LPS (Infect Immun. 2002 August; 70(8): 4414).

*E. coli*: *E. coli* antigens may be derived from enterotoxigenic *E. coli* (ETEC), enteroaggregative *E. coli* (EAggEC), diffusely adhering *E. coli* (DAEC), enteropathogenic *E. coli* (EPEC), and/or enterohemorrhagic *E. coli* (EHEC).

*Bacillus anthracis* (anthrax): *B. anthracis* antigens are optionally detoxified and may be selected from A-components (lethal factor (LF) and edema factor (EF)), both of which can share a common B-component known as protective antigen (PA).

*Yersinia pestis* (plague): Plague antigens include F 1 capsular antigen (Infect Immun. 2003 Jan; 71(1): 374-383), LPS (Infect Immun. 1999 Oct; 67(10): 5395), *Yersinia pestis* V antigen (Infect Immun. 1997 Nov; 65(11): 4476-4482).

*Mycobacterium tuberculosis*: Tuberculosis antigens include lipoproteins, LPS, BCG antigens, a fusion protein of antigen 85B (Ag85B) and/or ESAT-6 optionally formulated in cationic lipid vesicles (Infect Immune. 2004 October; 72(10): 6148), Mycobacterium tuberculosis (Mtb) isocitrate dehydrogenase associated antigens (Proc Natl Acad Sci U S A. 2004 Aug 24; 101(34): 12652), and/or MPT51 antigens (Infect Immun. 2004 July; 72(7): 3829).

*Rickettsia*: Antigens include outer membrane proteins, including the outer membrane protein A and/or B (OmpB) (Biochim Biophys Acta. 2004 Nov 1;1702(2):145), LPS, and surface protein antigen (SPA) (J Autoimmun. 1989 Jun;2 Suppl:81).

*Listeria monocytogenes*: Bacterial antigens may be derived from Listeria monocytogenes.

*Chlamydia pneumoniae*: Antigens include those identified in WO 02/02606.

*Vibrio cholera*: Antigens include proteinase antigens, LPS, particularly lipopolysaccharides of *Vibrio cholera,* 0-specific polysaccharides, *V. cholera* 0139, antigens of IEM1O8 vaccine (Infect Immun. 2003 Oct;71(10):5498-504), and/or Zonula occludens toxin (Zot).

*Salmonella typhi* (typhoid fever): Antigens include capsular polysaccharides preferably conjugates (Vi, i.e. vax-TyVi).

*Borrelia burgdorferi* (Lyme disease): Antigens include lipoproteins (such as OspA, OspB, Osp C and Osp D), other surface proteins such as OspE-related proteins (Erps), decorin-binding proteins (such as DbpA), and antigenically variable VI proteins, such as antigens associated with P39 and P13 (an integral membrane protein, Infect Immun. 2001 May; 69(5): 3323-3334), VlsE Antigenic Variation Protein (J Clin Microbiol. 1999 Dec; 37(12): 3997).

*Porphyroinonas gingivalis*: Antigens include *P. gingivalis* outer membrane protein (OMP).

*Kiebsiella*: Antigens include outermembrane proteins, including OmpA, or a polysaccharide optionally conjugated to tetanus toxoid.

Further bacterial antigens of the invention may be capsular antigens, polysaccharide antigens or protein antigens of any of the above. Further bacterial antigens may also include an outer membrane vesicle (OMV) preparation. Additionally, antigens include live, attenuated, and/or purified versions of any of the aforementioned bacteria. The antigens of the present invention may be derived from gram-negative or gram-positive bacteria. The antigens of the present invention may be derived from aerobic or anaerobic bacteria.

Additionally, any of the above bacterial-derived saccharides (polysaccharides, LPS, LOS or oligosaccharides) can be conjugated to another agent or antigen, such as a carrier protein (for example CRM197). Such conjugation may be direct conjugation effected by reductive amination of carbonyl moieties on the saccharide to amino groups on the protein, as provided in US Patent No. 5,360,897 and Can J Biochem Cell Biol. 1984 May;62(5):270-5. Alternatively, the saccharides can be conjugated through a linker, such as, with succinamide or other linkages.

### b. Viral Antigens

Viral antigens suitable for use in the invention include inactivated (or killed) virus, attenuated virus, split virus formulations, purified subunit formulations, viral proteins which may be isolated, purified or derived from a virus, and Virus Like Particles (VLPs). Viral antigens may be derived from viruses propagated on cell culture or other substrate. Alternatively, viral antigens may be expressed recombinantly. Viral antigens preferably include epitopes which are exposed on the surface of the virus during at least one stage of its life cycle. Viral antigens are preferably conserved across multiple serotypes or isolates. Viral antigens include antigens derived from one or more of the viruses set forth below as well as the specific antigens examples identified below.

Orthomyxovirus: Viral antigens may be derived from an Orthomyxovirus, such as Influenza A, B and C. Orthomyxovirus antigens may be selected from one or more of the viral proteins, including haemagglutinin (HA), neuraminidase (NA), nucleoprotein (NP), matrix protein (M1), membrane protein (M2), one or more of the transcriptase components (PB 1, PB2 and PA). Preferred antigens include HA and NA. Influenza antigens may be derived from interpandemic (annual) flu strains. Alternatively influenza antigens may be derived from strains with the potential to cause a pandemic outbreak (i.e., influenza strains with new haemagglutinin compared to the haemagglutinin in currently circulating strains, or influenza strains which are pathogenic in avian subjects and have the potential to be transmitted horizontally in the human population, or influenza strains which are pathogenic to humans).

Paramyxoviridae viruses: Viral antigens may be derived from Paramyxoviridae viruses, such as Pneumoviruses (RSV), Paramyxoviruses (PIV) and Morbilliviruses (Measles).

Pneumovirus: Viral antigens may be derived from a Pneumovirus, such as Respiratory syncytial virus (RSV), Bovine respiratory syncytial virus, Pneumonia virus of mice, and Turkey rhinotracheitis virus. Preferably, the Pneumovirus is RSV. Pneumovirus antigens may be selected from one or more of the following proteins, including surface proteins Fusion (F), Glycoprotein (G) and Small Hydrophobic protein (SH), matrix proteins M and M2, nucleocapsid proteins N, P and L and nonstructural proteins NS 1 and NS2. Preferred Pneumovirus antigens include F, G and M. Pneumovirus antigens may also be formulated in or derived from chimeric viruses. For example, chimeric RSV/PIV viruses may comprise components of both RSV and PIV.

Paramyxovirus: Viral antigens may be derived from a Paramyxovirus, such as Parainfluenza virus types 1 - 4 (PIV), Mumps, Sendai viruses, Simian virus 5, Bovine parainfluenza virus and Newcastle disease virus. Preferably, the Paramyxovirus is PIV or Mumps. Paramyxovirus antigens may be selected from one or more of the following proteins: Hemagglutinin - Neuraminidase (HN), Fusion proteins F1 and F2, Nucleoprotein (NP), Phosphoprotein (P), Large protein (L), and Matrix protein (M). Preferred Paramyxovirus proteins include HIN, F1 and F2. Paramyxovirus antigens may also be formulated in or derived from chimeric viruses. For example, chimeric RSV/PIV viruses may comprise components of both RSV and PIV. Commercially available mumps vaccines include live attenuated mumps virus, in either a monovalent form or in combination with measles and rubella vaccines (MMR).

Morbillivirus: Viral antigens may be derived from a Morbillivirus, such as Measles. Morbillivirus antigens may be selected from one or more of the following proteins: hemagglutinin (H), Glycoprotein (G), Fusion factor (F), Large protein (L), Nucleoprotein (NP), Polymerase phosphoprotein (P), and Matrix (M). Commercially available measles vaccines include live attenuated measles virus, typically in combination with mumps and rubella (MMR).

Picornavirus: Viral antigens may be derived from Picornaviruses, such as Enteroviruses, Rhinoviruses, Heparnavirus, Cardioviruses and Aphthoviruses. Antigens derived from Enteroviruses, such as Poliovirus are preferred.

Enterovirus: Viral antigens may be derived from an Enterovirus, such as Poliovirus types 1, 2 or 3, Coxsackie A virus types 1 to 22 and 24, Coxsackie B virus types 1 to 6, Echovirus (ECHO) virus) types 1 to 9, 11 to 27 and 29 to 34 and Enterovirus 68 to 71. Preferably, the Enterovirus is poliovirus. Enterovirus antigens are preferably selected from one or more of the following Capsid proteins VP1, VP2, VP3 and VP4. Commercially available polio vaccines include Inactivated Polio Vaccine (IPV) and Oral poliovirus vaccine (OPV).

Heparnavirus: Viral antigens may be derived from an Heparnavirus, such as Hepatitis A virus (HAV). Commercially available HAV vaccines include inactivated HAV vaccine.

Togavirus: Viral antigens may be derived from a Togavirus, such as a Rubivirus, an Alphavirus, or an Arterivirus. Antigens derived from Rubivirus, such as Rubella virus, are preferred. Togavirus antigens may be selected from E1, E2, E3, C, NSP-1, NSPO-2, NSP- 3 or NSP-4. Togavirus antigens are preferably selected from E1, E2 or E3. Commercially available Rubella vaccines include a live cold-adapted virus, typically in combination with mumps and measles vaccines (MMR).

Flavivirus: Viral antigens may be derived from a Flavivirus, such as Tick-borne encephalitis (TBE), Dengue (types 1, 2, 3 or 4), Yellow Fever, Japanese encephalitis, West Nile encephalitis, St. Louis encephalitis, Russian spring-summer encephalitis, Powassan encephalitis. Flavivirus antigens may be selected from PrM, M, C, E, NS-1, NS-2a, NS2b, N53, N54a, NS4b, and NS5. Flavivirus antigens are preferably selected from PrM, M and E. Commercially available TBE vaccine include inactivated virus vaccines.

Pestivirus: Viral antigens may be derived from a Pestivirus, such as Bovine viral diarrhea (BVDV), Classical swine fever (CSFV) or Border disease (BDV).

Hepadnavirus: Viral antigens may be derived from a Hepadnavirus, such as Hepatitis B virus. Hepadnavirus antigens may be selected from surface antigens (L, M and S), core antigens (HBc, HBe). Commercially available HBV vaccines include subunit vaccines comprising the surface antigen S protein.

Hepatitis C virus: Viral antigens may be derived from a Hepatitis C virus (HCV). HCV antigens may be selected from one or more of E1, E2, E1/E2, NS345 polyprotein, NS 345-core polyprotein, core, and/or peptides from the nonstructural regions (Houghton et al., Hepatology (1991) 14:381).

Rhabdovirus: Viral antigens may be derived from a Rhabdovirus, such as a Lyssavirus (Rabies virus) and Vesiculovirus (VSV). Rhabdovirus antigens may be selected from glycoprotein (G), nucleoprotein (N), large protein (L), nonstructural proteins (NS). Commercially available Rabies virus vaccine comprise killed virus grown on human diploid cells or fetal rhesus lung cells.

Calciviridae: Viral antigens may be derived from Calciviridae, such as Norwalk virus, and Norwalk-like Viruses, such as Hawaii Virus and Snow Mountain Virus.

Coronavirus: Viral antigens may be derived from a Coronavirus, SARS, Human respiratory coronavirus, Avian infectious bronchitis (IBV), Mouse hepatitis virus (MHV), and Porcine transmissible Gastroenteritis virus (TGEV). Coronavirus antigens may be selected from spike (5), envelope (E), matrix (M), nucleocapsid (N), and Hemagglutinin-esterase glycoprotein (HE). Preferably, the Coronavirus antigen is derived from a SARS virus.

Retrovirus: Viral antigens may be derived from a Retrovirus, such as an Oncovirus, a Lentivirus or a Spumavirus. Oncovirus antigens may be derived from HTLV-1, HTLV-2 or HTLV-5. Lentivirus antigens may be derived from HIV-1 or HIV-2. Retrovirus antigens may be selected from gag, poi, env, tax, tat, rex, rev, nef, vif, vpu, and vpr. HIV antigens may be selected from gag (p24gag and p55gag), env (gp160 and gp41), pol, tat, nef, rev vpu, miniproteins. HIV antigens may be derived from one or more of the following strains: H1V1I1b, HIVSF2, HIVLAV, HIVLAI, HIVMN, HIV-1CM235, HIV-1US4.

Reovirus: Viral antigens may be derived from a Reovirus, such as an Orthoreovirus, a Rotavirus, an Orbivirus, or a Coltivirus. Reovirus antigens may be selected from structural proteins or nonstructural proteins. Preferred Reovirus antigens may be derived from a Rotavirus. Rotavirus antigens may be selected from VP1, VP2, VP3, VP4 (or the cleaved product VP5 and -40 - VP8), NSP 1, VP6, NSP3, NSP2, VP7, NSP4, or NSP5. Preferred Rotavirus antigens include VP4 (or the cleaved product VP5 and VP8), and VP7.

Parvovirus: Viral antigens may be derived from a Parvovirus, such as Parvovirus B 19. Parvovirus antigens may be selected from VP-1, VP-2, VP-3, NS-1 and NS-2. Preferably, the Parvovirus antigen is capsid protein VP-2.

Delta hepatitis virus (HDV): Viral antigens may be derived HDV, particularly s-antigen from HDV (see, e.g., U.S. Patent 5,378,814).

Hepatitis E virus (HEV): Viral antigens may be derived from HEV.

Hepatitis G virus (HGV): Viral antigens may be derived from HGV.

Human Herpesvirus: Viral antigens may be derived from a Human Herpesvirus, such as Herpes Simplex Viruses (HSV), Varicella-zoster virus (VZV), Epstein-Barr virus (EBV), Cytomegalovirus (CMV), Human Herpesvirus 6 (HHV6), Human Herpesvirus 7 (HHV7), and Human Herpesvirus 8 (HHV8). Human Herpesvirus antigens may be selected from immediate early proteins (a), early proteins (J3), and late proteins (y). HSV antigens may be derived from HSV-1 or HSV-2 strains. HSV antigens may be selected from glycoproteins gB, gC, gD and gH, fusion protein (gB), or immune escape proteins (gC, gE, or gI). VZV antigens may be selected from core, nucleocapsid, tegument, or envelope proteins. A live attenuated VZV vaccine is commercially available. EBV antigens may be selected from early antigen (BA) proteins, viral capsid antigen (VCA), and glycoproteins of the membrane antigen (MA). CMV antigens may be selected from capsid proteins, envelope glycoproteins (such as gB and gH), and tegument proteins.

Papovaviruses: Antigens may be derived from Papovaviruses, such as Papillomaviruses and Polyomaviruses. Papillomaviruses include HPV serotypes 1, 2, 4, 5, 6, 8, 11, 13, 16, 18, 31, 33, 35, 39, 41, 42, 47, 51, 57, 58, 63 and 65. Preferably, HPV antigens are derived from serotypes 6, 11, 16 or 18. HPV antigens may be selected from capsid proteins (Li) and (L2), or E1 - E7, or fusions thereof. HPV antigens are preferably formulated into virus-like particles (VLPs). Polyomyavirus viruses include BK virus and JK virus. Polyomavirus antigens may be selected from VP1, VP2 or VP3.

### c. Fungal Antigens

Fungal antigens for use in the invention may be derived from one or more of the fungi set forth below.

Fungal antigens may be derived from Dermatophytes, including: *Epidermophyton floccusum, Microsporum audouini, Microsporum canis, Microsporum distortum, Microsporum equinum, Microsporum gypsum, Microsporum nanum, Trichophyton concentricum, Trichophyton equinum, Trichophyton gallinae, Trichophyton gypseum, Trichophyton megnini, Trichophyton mentagrophytes, Trichophyton quinckeanum, Trichophyton rubrum, Trichophyton schoenleini, Trichophyton tonsurans, Trichophyton verrucosum, T. verrucosum* var. *album,* var. *discoides,* var. *ochraceum, Trichophyton violaceum,* and/or *Trichophyton faviforme.*

Fungal pathogens may be derived from *Aspergillus fumigatus, Aspergillus flavus, Aspergillus niger, Aspergillus nidulans, Aspergillus terreus, Aspergillus sydowi, Aspergillus flavatus, Aspergillus glaucus, Blastoschizomyces capitatus, Candida albicans, Candida enolase, Candida tropicalis, Candida glabrata, Candida krusei, Candida parapsilosis, Candida stellatoidea, Candida kusei, Candida parakwsei, Candida Iusitaniae, Candida pseudotropicalis, Candida guilliermondi, Cladosporium carrionii, Coccidioides immitis, Blastomyces dermatidis, Cryptococcus neoformans, Geotrichum clavatum, Histoplasma capsulatum, Klebsiella pneumoniae, Paracoccidioides brasiliensis, Pneumocystis carinii, Pythiumn insidiosum, Pityrosporum ovale, Sacharomyces cerevisae, Saccharomyces boulardii, Saccharomyces pombe, Scedosporium apiosperum, Sporothrix schenckii, Trichosporon beigelii, Toxoplasma gondii, Penicillium marneffei, Malassezia* spp., *Fonsecaea* spp., *Wangiella* spp., *Sporothrix* spp., *Basidiobolus* spp., *Conidiobolus* spp., *Rhizopus* spp., *Mucor* spp., *Absidia* spp., *Mortierella* spp., *Cunninghamella* spp., *Saksenaea* spp., *Alternaria* spp., *Curvularia* spp., *Helminthosporium* spp., *Fusarium* spp., *Aspergillus* spp., *Penicillium* spp., *Monolinia* spp., *Rhizoctonia* spp., *Paecilomyces* spp., *Pithomyces* spp., and *Cladosporium* spp.

Processes for producing fungal antigens are well known in the art (see US Patent No. 6,333,164). In a preferred method a solubilized fraction extracted and separated from an insoluble fraction obtainable from fungal cells of which cell wall has been substantially removed or at least partially removed, characterized in that the process comprises the steps of: obtaining living fungal cells; obtaining fungal cells of which cell wall has been substantially removed or at least partially removed; bursting the fungal cells of which cell wall has been substantially removed or at least partially removed; obtaining an insoluble fraction; and extracting and separating a solubilized fraction from the insoluble fraction.

### d. STD Antigens

The compositions of the invention may include one or more antigens derived from a sexually transmitted disease (STD). Such antigens may provide for prophylaxis or therapy for STD's such as chlamydia, genital herpes, hepatitis (such as HCV), genital warts, gonorrhea, syphilis and/or chancroid (See, W000/15255). Antigens may be derived from one or more viral or bacterial STD's. Viral STD antigens for use in the invention may be derived from, for example, HIV, herpes simplex virus (HSV-1 and HSV-2), human papillomavirus (HPV), and hepatitis (HCV). Bacterial STD antigens for use in the invention may be derived from, for example, *Neiserria gonorrhoeae, Chlamydia trachomatis, Treponema pallidum, Haemophilus ducreyi, E. coli,* and *Streptococcus agalactiae.* Examples of specific antigens derived from these pathogens are described above.

### e. Respiratory Antigens

The compositions of the invention may include one or more antigens derived from a pathogen which causes respiratory disease. For example, respiratory antigens may be derived from a respiratory virus such as Orthomyxoviruses (influenza), Pneumovirus (RSV), Paramyxovirus (Ply), Morbillivirus (measles), Togavirus (Rubella), VZV, and Coronavirus (SARS). Respiratory antigens may be derived from a bacterium which causes respiratory disease, such as *Streptococcus pneumoniae, Pseudomonas aeruginosa, Bordetella pertussis, Mycobacterium tuberculosis, Mycoplasma pneumoniae, Chlamydia pneumoniae, Bacillus anthracis,* and *Moraxella catarrhalis.* Examples of specific antigens derived from these pathogens are described above.

### f. Pediatric Vaccine Antigens.

The compositions of the invention may include one or more antigens suitable for use in pediatric subjects. Pediatric subjects are typically less than about 3 years old or less than about 2 years old or less than about 1 year old. Pediatric antigens may be administered multiple times over the course of 6 months, 1, 2 or 3 years. Pediatric antigens may be derived from a virus which may target pediatric populations and/or a virus from which pediatric populations are susceptible to infection. Pediatric viral antigens include antigens derived from one or more of Orthomyxovirus (influenza), Pneumovirus (RSV), Paramyxovirus (P1Y and Mumps), Morbillivirus (measles), Togavirus (Rubella), Enterovirus (polio), HBV, Coronavirus (SARS), and Varicella-zoster virus (VZV), Epstein Barr virus (EBV). Pediatric bacterial antigens include antigens derived from one or more of Streptococcus pneumoniae, Neisseria meningitides, Streptococcus pyogenes (Group A Streptococcus), Moraxella catarrhalis, Bordetella pertussis, Clostridium tetani (Tetanus), Cornynebacterium diphtheriae (Diphtheria), Haemophilus influenzae B (Hib), Pseudomonas aeruginosa, Streptococcus agalactiae (Group B Streptococcus), and E. coli. Examples of specific antigens derived from these pathogens are described above.

### g. Antigens suitable for use in Elderly or Immunocompromised Individuals.

The compositions of the invention may include one or more antigens suitable for use in elderly or immunocompromised individuals. Such individuals may need to be vaccinated more frequently, with higher doses or with adjuvanted formulations to improve their immune response to the targeted antigens. Antigens which may be targeted for use in Elderly or Immunocompromised individuals include antigens derived from one or more of the following pathogens: Neisseria meningitides, Streptococcus pneumoniae, Streptococcus pyogenes (Group A Streptococcus), Moraxella catarrhalis, Bordetella pertussis, Staphylococcus epidermis, Clostridium tetani (Tetanus), Cornynebacterium diphtheriae (Diphtheria), Haemophilus influenzae B (Hib), Pseudomonas aeruginosa, Legionella pneumophila, Streptococcus agalactiae (Group B Streptococcus), Enterococcus faecalis, Helicobacter pylon, Clarnydia pneumoniae, Orthomyxovirus (influenza), Pneumovirus (RSV), Paramyxovirus (PIV and Mumps), Morbillivirus (measles), Togavirus (Rubella), Enterovirus (polio), HBV, Coronavirus (SARS), Varicella-zoster virus (VZV) , Epstein Barr virus (EBV), Cytomegalovirus (CMV). Examples of specific antigens derived from these pathogens are described above.

### h. Antigens suitable for use in Adolescent Vaccines

The compositions of the invention may include one or more antigens suitable for use in adolescent subjects. Adolescents may be in need of a boost of a previously administered pediatric antigen. Pediatric antigens which may be suitable for use in adolescents are described above. In addition, adolescents may be targeted to receive antigens derived from an STD pathogen in order to ensure protective or therapeutic immunity before the beginning of sexual activity. STD antigens which may be suitable for use in adolescents are described above.

### II. PROTEIN PRODUCTION

### A. Synthetic Proteins

The present invention describes polypeptides, peptides, and proteins for use in various embodiments of the present invention. For example, specific polypeptides are assayed for their abilities to elicit an immune response. In specific embodiments, all or part of the proteins of the invention can also be synthesized in solution or on a solid support in accordance with conventional techniques. Various automatic synthesizers are commercially available and can be used in accordance with known protocols. See, for example, Stewart and Young, (1984); Tam *et al.,* (1983); Merrifield, (1986); and Barany and Merrifield (1979), each incorporated herein by reference. Alternatively, recombinant DNA technology may be employed wherein a nucleotide sequence which encodes a peptide of the invention is inserted into an expression vector, transformed or transfected into an appropriate host cell and cultivated under conditions suitable for expression.

### 1. In Vitro Protein Production

One embodiment of the invention includes the use of gene transfer to cells, including microorganisms, for the production and/or presentation of proteins. The gene for the protein of interest may be transferred into appropriate host cells followed by culture of cells under the appropriate conditions. A nucleic acid encoding virtually any polypeptide may be employed. The generation of recombinant expression vectors, and the elements included therein, are discussed herein. Alternatively, the protein to be produced may be an endogenous protein normally synthesized by the cell used for protein production.

Another embodiment of the present invention uses autologous B lymphocyte cell lines, which are transfected with a viral vector that expresses an immunogen product, and more specifically, a protein having immunogenic activity. Other examples of mammalian host cell lines include, but are not limited to Vero and HeLa cells, other B- and T- cell lines, such as CEM, 721.221, H9, Jurkat, Raji, as well as cell lines of Chinese hamster ovary, W138, BHK, COS-7, 293, HepG2, 3T3, RIN and MDCK cells. In addition, a host cell strain may be chosen that modulates the expression of the inserted sequences, or that modifies and processes the gene product in the manner desired. Such modifications (*e.g*., glycosylation) and processing (*e.g*., cleavage) of protein products may be important for the function of the protein. Different host cells have characteristic and specific mechanisms for the post-translational processing and modification of proteins. Appropriate cell lines or host systems can be chosen to ensure the correct modification and processing of the foreign protein expressed.

A number of selection systems may be used including, but not limited to HSV thymidine kinase, hypoxanthine-guanine phosphoribosyltransferase, and adenine phosphoribosyltransferase genes, in tk-, hgprt- or aprt- cells, respectively. Also, antimetabolite resistance can be used as the basis of selection: for dhfr, which confers resistance to trimethoprim and methotrexate; gpt, which confers resistance to mycophenolic acid; neo, which confers resistance to the aminoglycoside G418; and hygro, which confers resistance to hygromycin.

Animal cells can be propagated *in vitro* in two modes: as non-anchorage-dependent cells growing in suspension throughout the bulk of the culture or as anchorage-dependent cells requiring attachment to a solid substrate for their propagation (*i.e*., a monolayer type of cell growth).

Non-anchorage dependent or suspension cultures from continuous established cell lines are the most widely used means of large scale production of cells and cell products. However, suspension cultured cells have limitations, such as tumorigenic potential and lower protein production than adherent cells.

### III. NUCLEIC ACIDS

The present invention concerns recombinant polynucleotides encoding the proteins, polypeptides, peptides of the invention. The nucleic acid sequences for wild-type EsxA, EsxB, or any other polypeptide transported by the Ess pathway, and/or SdrD, SdrE, IsdA, IsdB, or other surface proteins or sortase substrates, are included, all of which are incorporated by reference, and can be used to prepare an EsxA, EsxB, or any other polypeptide transported by the Ess pathway, and/or SdrC, SdrD, SdrE, IsdA, IsdB, Spa, ClfA, ClfB, IsdC, SasF or other sortase substrates. Similarly, nucleic acid sequences encoding any other wild-type protein that is transported by the Ess pathway or are substrates for sortase mechanism are included.

As used in this application, the term "polynucleotide" refers to a nucleic acid molecule that either is recombinant or has been isolated free of total genomic nucleic acid. Included within the term "polynucleotide" are oligonucleotides (nucleic acids 100 residues or less in length), recombinant vectors, including, for example, plasmids, cosmids, phage, viruses, and the like. Polynucleotides include, in certain aspects, regulatory sequences, isolated substantially away from their naturally occurring genes or protein encoding sequences. Polynucleotides may be RNA, DNA, analogs thereof, or a combination thereof.

In this respect, the term "gene," "polynucleotide" or "nucleic acid" is used for to refer to a nucleic acid that encodes a protein, polypeptide, or peptide (including any sequences required for proper transcription, post-translational modification, or localization). As will be understood by those in the art, this term encompasses genomic sequences, expression cassettes, cDNA sequences, and smaller engineered nucleic acid segments that express, or may be adapted to express, proteins, polypeptides, domains, peptides, fusion proteins, and mutants. A nucleic acid encoding all or part of a polypeptide may contain a contiguous nucleic acid sequence encoding all or a portion of such a polypeptide of the following lengths: 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 441, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, 1010, 1020, 1030, 1040, 1050, 1060, 1070, 1080, 1090, 1095, 1100, 1500, 2000, 2500, 3000, 3500, 4000, 4500, 5000, 5500, 6000, 6500, 7000, 7500, 8000, 9000, 10000, or more nucleotides, nucleosides, or base pairs. It also is contemplated that a particular polypeptide from a given species may be encoded by nucleic acids containing natural variations that having slightly different nucleic acid sequences but, nonetheless, encode the same or substantially similar protein (see Table 2 above).

In particular embodiments, the invention concerns isolated nucleic acid segments and recombinant vectors incorporating nucleic acid sequences that encode an EsxA, EsxB, or any other protein transported by the Ess pathway, and/or SdrD, SdrE, IsdA, IsdB, or other sortase substrates. Thus, an isolated nucleic acid segment or vector containing a nucleic acid segment may encode, for example, an EsxA, EsxB, or other Ess pathway protein, and/or SdrD, SdrE, IsdA, IsdB, or other sortase substrates that is immunogenic. The term "recombinant" may be used in conjunction with a polypeptide or the name of a specific polypeptide, and this generally refers to a polypeptide produced from a nucleic acid molecule that has been manipulated *in vitro* or that is a replication product of such a molecule.

In other embodiments, the invention concerns isolated nucleic acid segments and recombinant vectors incorporating nucleic acid sequences that encode a EsxA, EsxB, or other Ess transported polypeptide or peptide, and/or SdrD, SdrE, IsdA, IsdB, or other sortase substrate polypeptide or peptide that can be used to generate an immune response in a subject. In various embodiments the nucleic acids of the invention may be used in genetic vaccines.

The nucleic acid segments used in the present invention, regardless of the length of the coding sequence itself, may be combined with other nucleic acid sequences, such as promoters, polyadenylation signals, additional restriction enzyme sites, multiple cloning sites, other coding segments, and the like, such that their overall length may vary considerably. It is therefore contemplated that a nucleic acid fragment of almost any length may be employed, with the total length preferably being limited by the ease of preparation and use in the intended recombinant nucleic acid protocol. In some cases, a nucleic acid sequence may encode a polypeptide sequence with additional heterologous coding sequences, for example to allow for purification of the polypeptide, transport, secretion, post-translational modification, or for therapeutic benefits such as targeting or efficacy. As discussed above, a tag or other heterologous polypeptide may be added to the modified polypeptide-encoding sequence, wherein "heterologous" refers to a polypeptide that is not the same as the modified polypeptide.

The nucleic acid used in the present invention encode EsxA, EsxB, or any other peptide or protein from a polypeptide transported by the Ess pathway, and/or SdrD, SdrE, IsdA, IsdB, or any other peptides or protein processed by the sortase mechanism. Such sequences may arise as a consequence of codon redundancy and functional equivalency that are known to occur naturally within nucleic acid sequences and the proteins thus encoded. Alternatively, functionally equivalent proteins or peptides may be created via the application of recombinant DNA technology, in which changes in the protein structure may be engineered, based on considerations of the properties of the amino acids being exchanged. Changes designed by human may be introduced through the application of site-directed mutagenesis techniques, *e.g.*, to introduce improvements to the antigenicity of the protein.

In certain other embodiments, the invention concerns isolated nucleic acid segments and recombinant vectors that include within their sequence a contiguous nucleic acid sequence from SEQ ID NO:1 (EsxA), SEQ ID NO:3 (EsxB) , SEQ ID NO:5 (SdrD) , SEQ ID NO:7 (SdrE), SEQ ID NO:9 (IsdA), SEQ ID NO:11 (IsdB), SEQ ID NO:13 (Spa), SEQ ID NO:15 (ClfB), SEQ ID NO:17 (IsdC), SEQ ID NO:19 (SasF), SEQ ID NO:21 (SdrC), SEQ ID NO:23 (ClfA) or any other nucleic acid sequences encoding secreted virulence factors and/or surface proteins including proteins transported by the Ess pathway, processed by sortase, or proteins incorporated herein by reference.

### A. Vectors

Polypeptides of the invention may be encoded by a nucleic acid molecule comprised in a vector. The term "vector" is used to refer to a carrier nucleic acid molecule into which a heterologous nucleic acid sequence can be inserted for introduction into a cell where it can be replicated and expressed. A nucleic acid sequence can be "heterologous," which means that it is in a context foreign to the cell in which the vector is being introduced, which includes a sequence homologous to a sequence in the cell but in a position within the host cell where it is ordinarily not found. Vectors include DNAs, RNAs, plasmids, cosmids, viruses (bacteriophage, animal viruses, and plant viruses), and artificial chromosomes (*e.g.*, YACs). One of skill in the art would be well equipped to construct a vector through standard recombinant techniques (for example Sambrook *et al.,* 2001; Ausubel *et al.,* 1996, both incorporated herein by reference). In addition to encoding an EsxA, EsxB, or other Ess transported polypeptide, and/or SdrD, SdrE, IsdA, IsdB, or any other peptides or protein processed by sortase, a vector may encode polypeptide sequences such as a tag or immunogenicity enhancing peptide. Useful vectors encoding such fusion proteins include pIN vectors (Inouye *et al.,* 1985), vectors encoding a stretch of histidines, and pGEX vectors, for use in generating glutathione S-transferase (GST) soluble fusion proteins for later purification and separation or cleavage.

Vectors of the invention may be used in a host cell to produce an EsxA, EsxB, or other Ess transported polypeptide, and/or a SdrD, SdrE, IsdA, IsdB, or any other peptides or protein processed by the sortase mechanism that may subsequently be purified for administration to a subject or the vector may be purified for direct administration to a subject for expression of the protein in the subject.

The term "expression vector" refers to a vector containing a nucleic acid sequence coding for at least part of a gene product capable of being transcribed. In some cases, RNA molecules are then translated into a protein, polypeptide, or peptide. Expression vectors can contain a variety of "control sequences," which refer to nucleic acid sequences necessary for the transcription and possibly translation of an operably linked coding sequence in a particular host organism. In addition to control sequences that govern transcription and translation, vectors and expression vectors may contain nucleic acid sequences that serve other functions as well and are described infra.

### 1. Promoters and Enhancers

A "promoter" is a control sequence. The promoter is typically a region of a nucleic acid sequence at which initiation and rate of transcription are controlled. It may contain genetic elements at which regulatory proteins and molecules may bind such as RNA polymerase and other transcription factors. The phrases "operatively positioned," "operatively linked," "under control," and "under transcriptional control" mean that a promoter is in a correct functional location and/or orientation in relation to a nucleic acid sequence to control transcriptional initiation and expression of that sequence. A promoter may or may not be used in conjunction with an "enhancer," which refers to a cis-acting regulatory sequence involved in the transcriptional activation of a nucleic acid sequence.

A promoter may be one naturally associated with a gene or sequence, as may be obtained by isolating the 5' non-coding sequences located upstream of the coding segment or exon. Such a promoter can be referred to as "endogenous." Similarly, an enhancer may be one naturally associated with a nucleic acid sequence, located either downstream or upstream of that sequence. Alternatively, certain advantages will be gained by positioning the coding nucleic acid segment under the control of a recombinant or heterologous promoter, which refers to a promoter that is not normally associated with a nucleic acid sequence in its natural environment. A recombinant or heterologous enhancer refers also to an enhancer not normally associated with a nucleic acid sequence in its natural state. Such promoters or enhancers may include promoters or enhancers of other genes, and promoters or enhancers isolated from any other prokaryotic, viral, or eukaryotic cell, and promoters or enhancers not "naturally occurring," *i.e.*, containing different elements of different transcriptional regulatory regions, and/or mutations that alter expression. In addition to producing nucleic acid sequences of promoters and enhancers synthetically, sequences may be produced using recombinant cloning and/or nucleic acid amplification technology, including PCR™, in connection with the compositions disclosed herein (see U.S. Patent 4,683,202, U.S. Patent 5,928,906, each incorporated herein by reference).

Naturally, it may be important to employ a promoter and/or enhancer that effectively directs the expression of the DNA segment in the cell type or organism chosen for expression. Those of skill in the art of molecular biology generally know the use of promoters, enhancers, and cell type combinations for protein expression (see Sambrook *et al.,* 2001, incorporated herein by reference). The promoters employed may be constitutive, tissue-specific, or inducible and in certain embodiments may direct high level expression of the introduced DNA segment under specified conditions, such as large-scale production of recombinant proteins or peptides.

Various elements/promoters that may be employed in the context of the present invention to regulate the expression of a gene. Examples of such inducible elements, which are regions of a nucleic acid sequence that can be activated in response to a specific stimulus, include but are not limited to Immunoglobulin Heavy Chain (Banerji *et al.,* 1983; Gilles *et al.,* 1983; Grosschedl *et al.,* 1985; Atchinson *et al.,* 1986, 1987; Imler *et al.,* 1987; Weinberger *et al.,* 1984; Kiledjian *et al.,* 1988; Porton *et al.*; 1990), Immunoglobulin Light Chain (Queen *et al.,* 1983; Picard *et al.,* 1984), T Cell Receptor (Luria *et al.,* 1987; Winoto *et al.,* 1989; Redondo *et al.*; 1990), HLA DQ a and/or DQ β (Sullivan *et al.,* 1987), β Interferon (Goodbourn *et al.,* 1986; Fujita *et al.,* 1987; Goodbourn *et al.,* 1988), Interleukin-2 (Greene *et al*., 1989), Interleukin-2 Receptor (Greene *et al*., 1989; Lin *et al.,* 1990), MHC Class II 5 (Koch *et al.,* 1989), MHC Class II HLA-DRα (Sherman *et al.,* 1989), β-action (Kawamoto *et al*., 1988; Ng *et al.*; 1989), Muscle Creatine Kinase (MCK) (Jaynes *et al.,* 1988; Horlick *et al.,* 1989; Johnson *et al.,* 1989), Prealbumin (Transthyretin) (Costa *et al.,* 1988), Elastase I (Omitz *et al.,* 1987), Metallothionein (MTII) (Karin *et al.,* 1987; Culotta *et al.,* 1989), Collagenase (Pinkert *et al.,* 1987; Angel *et al.,* 1987), Albumin (Pinkert *et al.,* 1987; Tronche *et al.,* 1989, 1990), α-Fetoprotein (Godbout *et al.,* 1988; Campere *et al*., 1989), γ-Globin (*Bodine et al*., 1987; Perez-Stable *et al.,* 1990), β-Globin (Trudel *et al.,* 1987), c-fos (Cohen *et al.,* 1987), c-HA-ras (Triesman, 1986; Deschamps *et al.,* 1985), Insulin (Edlund *et al.,* 1985), Neural Cell Adhesion Molecule (NCAM) (Hirsh *et al.,* 1990), α1-Antitrypain (Latimer *et al.,* 1990), H2B (TH2B) Histone (Hwang *et al.,* 1990), Mouse and/or Type I Collagen (Ripe *et al.,* 1989), Glucose-Regulated Proteins (GRP94 and GRP78) (Chang *et al.,* 1989), Rat Growth Hormone (Larsen *et al.,* 1986), Human Serum Amyloid A (SAA) (Edbrooke *et al.,* 1989), Troponin I (TN I) (Yutzey *et al.,* 1989), Platelet-Derived Growth Factor (PDGF) (Pech *et al.,* 1989), Duchenne Muscular Dystrophy (Klamut *et al.,* 1990), SV40 (Banerji *et al.,* 1981; Moreau *et al.,* 1981; Sleigh *et al.,* 1985; Firak *et al.,* 1986; Herr *et al.,* 1986; Imbra *et al.,* 1986; Kadesch *et al.,* 1986; Wang *et al.,* 1986; Ondek *et al.,* 1987; Kuhl *et al.,* 1987; Schaffner *et al.,* 1988), Polyoma (Swartzendruber *et al.,* 1975; Vasseur *et al.,* 1980; Katinka *et al.,* 1980, 1981; Tyndell *et al.,* 1981; Dandolo *et al.,* 1983; de Villiers *et al.,* 1984; Hen *et al*., 1986; Satake *et al.,* 1988; Campbell *et al.,* 1988), Retroviruses (Kriegler *et al.,* 1982, 1983; Levinson *et al.,* 1982; Kriegler *et al.,* 1983, 1984a, b, 1988; Bosze *et al.,* 1986; Miksicek *et al*., 1986; Celander *et al.,* 1987; Thiesen *et al.,* 1988; Celander *et al.,* 1988; Chol *et al.,* 1988; Reisman *et al.,* 1989), Papilloma Virus (Campo *et al.,* 1983; Lusky *et al*., 1983; Spandidos and Wilkie, 1983; Spalholz *et al*., 1985; Lusky *et al*., 1986; Cripe *et al.,* 1987; Gloss *et al*., 1987; Hirochika *et al*., 1987; Stephens *et al*., 1987), Hepatitis B Virus (Bulla *et al*., 1986; Jameel *et al*., 1986; Shaul *et al.,* 1987; Spandau *et al.,* 1988; Vannice *et al.,* 1988), Human Immunodeficiency Virus (Muesing *et al.,* 1987; Hauber *et al*., 1988; Jakobovits *et al.,* 1988; Feng *et al*., 1988; Takebe *et al*., 1988; Rosen *et al.,* 1988; Berkhout *et al.,* 1989; Laspia *et al*., 1989; Sharp *et al*., 1989; Braddock *et al.,* 1989), Cytomegalovirus (CMV) IE (Weber *et al.,* 1984; Boshart *et al.,* 1985; Foecking *et al.,* 1986), Gibbon Ape Leukemia Virus (Holbrook *et al.,* 1987; Quinn *et al.,* 1989).

Inducible Elements include, but are not limited to MT II - Phorbol Ester (TFA)/Heavy metals (Palmiter *et al*., 1982; Haslinger *et al.,* 1985; Searle *et al.,* 1985; Stuart *et al.,* 1985; Imagawa *et al.,* 1987, Karin *et al*., 1987; Angel *et al*., 1987b; McNeall *et al.,* 1989); MMTV (mouse mammary tumor virus) - Glucocorticoids (Huang *et al.,* 1981; Lee *et al*., 1981; Majors *et al.,* 1983; Chandler *et al*., 1983; Lee *et al.,* 1984; Ponta *et al.,* 1985; Sakai *et al*., 1988); β-Interferon - poly(rI)x/poly(rc) (Tavernier *et al.,* 1983); Adenovirus 5 E2 - E1A (Imperiale *et al.,* 1984); Collagenase - Phorbol Ester (TPA) (Angel *et al.,* 1987a); Stromelysin - Phorbol Ester (TPA) (Angel *et al*., 1987b); SV40 - Phorbol Ester (TPA) (Angel *et al.,* 1987b); Murine MX Gene - Interferon, Newcastle Disease Virus (Hug *et al.,* 1988); GRP78 Gene - A23187 (Resendez *et al.,* 1988); α-2-Macroglobulin - IL-6 (Kunz *et al*., 1989); Vimentin - Serum (Rittling *et al.,* 1989); MHC Class I Gene H-2κb - Interferon (Blanar *et al*., 1989); HSP70 - E1A/SV40 Large T Antigen (Taylor *et al*., 1989, 1990a, 1990b); Proliferin - Phorbol Ester/TPA (Mordacq *et al.,* 1989); Tumor Necrosis Factor - PMA (Hensel *et al.,* 1989); and Thyroid Stimulating Hormone α Gene - Thyroid Hormone (Chatterjee *et al.,* 1989).

Also contemplated as useful in the present invention are the dectin-1 and dectin-2 promoters. Additionally any promoter/enhancer combination (as per the Eukaryotic Promoter Data Base EPDB) could also be used to drive expression of structural genes encoding oligosaccharide processing enzymes, protein folding accessory proteins, selectable marker proteins or a heterologous protein of interest.

The particular promoter that is employed to control the expression of peptide or protein encoding polynucleotide of the invention is not believed to be critical, so long as it is capable of expressing the polynucleotide in a targeted cell, preferably a bacterial cell. Where a human cell is targeted, it is preferable to position the polynucleotide coding region adjacent to and under the control of a promoter that is capable of being expressed in a human cell. Generally speaking, such a promoter might include either a bacterial, human or viral promoter.

In various embodiments, the human cytomegalovirus (CMV) immediate early gene promoter, the SV40 early promoter, and the Rous sarcoma virus long terminal repeat can be used to obtain high level expression of an EsxA-, EsxB-, or other Ess-related polynucleotide, and/or SdrD, SdrE, IsdA, IsdB, or any other sortase substrate related polynucleotide. The use of other viral or mammalian cellular or bacterial phage promoters, which are well known in the art, to achieve expression of polynucleotides is contemplated as well.

In embodiments in which a vector is administered to a subject for expression of the protein, it is contemplated that a desirable promoter for use with the vector is one that is not down-regulated by cytokines or one that is strong enough that even if down-regulated, it produces an effective amount of an EsxA, EsxB, or other Ess transported protein, and/or SdrD, SdrE, IsdA, IsdB, or any other peptides or protein processed by sortase in a subject to elicit an immune response. Non-limiting examples of these are CMV IE and RSV LTR. In other embodiments, a promoter that is up-regulated in the presence of cytokines is employed. The MHC I promoter increases expression in the presence of IFN-γ.

Tissue specific promoters can be used, particularly if expression is in cells in which expression of an antigen is desirable, such as dendritic cells or macrophages. The mammalian MHC I and MHC II promoters are examples of such tissue-specific promoters.

### 2. Initiation Signals and Internal Ribosome Binding Sites (IRES)

A specific initiation signal also may be required for efficient translation of coding sequences. These signals include the ATG initiation codon or adjacent sequences. Exogenous translational control signals, including the ATG initiation codon, may need to be provided. One of ordinary skill in the art would readily be capable of determining this and providing the necessary signals. It is well known that the initiation codon must be "in-frame" with the reading frame of the desired coding sequence to ensure translation of the entire insert. The exogenous translational control signals and initiation codons can be either natural or synthetic and may be operable in bacteria or mammalian cells. The efficiency of expression may be enhanced by the inclusion of appropriate transcription enhancer elements.

In certain embodiments of the invention, the use of internal ribosome entry sites (IRES) elements are used to create multigene, or polycistronic, messages. IRES elements are able to bypass the ribosome scanning model of 5' methylated Cap dependent translation and begin translation at internal sites (Pelletier and Sonenberg, 1988). IRES elements from two members of the picornavirus family (polio and encephalomyocarditis) have been described (Pelletier and Sonenberg, 1988), as well an IRES from a mammalian message (Macejak and Samow, 1991). IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message (see U.S. Patents 5,925,565 and 5,935,819, herein incorporated by reference).

### 3. Multiple Cloning Sites

Vectors can include a multiple cloning site (MCS), which is a nucleic acid region that contains multiple restriction enzyme sites, any of which can be used in conjunction with standard recombinant technology to digest the vector. (See Carbonelli *et al.,* 1999, Levenson *et al.,* 1998, and Cocea, 1997, incorporated herein by reference.) Frequently, a vector is linearized or fragmented using a restriction enzyme that cuts within the MCS to enable exogenous sequences to be ligated to the vector. Techniques involving restriction enzymes and ligation reactions are well known to those of skill in the art of recombinant technology.

### 4. Splicing Sites

Most transcribed eukaryotic RNA molecules will undergo RNA splicing to remove introns from the primary transcripts. Vectors containing genomic eukaryotic sequences may require donor and/or acceptor splicing sites to ensure proper processing of the transcript for protein expression. (See Chandler *et al.,* 1997, incorporated herein by reference.)

### 5. Termination Signals

The vectors or constructs of the present invention will generally comprise at least one termination signal. A "termination signal" or "terminator" is comprised of the DNA sequences involved in specific termination of an RNA transcript by an RNA polymerase. Thus, in certain embodiments a termination signal that ends the production of an RNA transcript is contemplated. A terminator may be necessary *in vivo* to achieve desirable message levels.

In eukaryotic systems, the terminator region may also comprise specific DNA sequences that permit site-specific cleavage of the new transcript so as to expose a polyadenylation site. This signals a specialized endogenous polymerase to add a stretch of about 200 A residues (polyA) to the 3' end of the transcript. RNA molecules modified with this polyA tail appear to more stable and are translated more efficiently. Thus, in other embodiments involving eukaryotes, it is preferred that that terminator comprises a signal for the cleavage of the RNA, and it is more preferred that the terminator signal promotes polyadenylation of the message.

Terminators contemplated for use in the invention include any known terminator of transcription described herein or known to one of ordinary skill in the art, including but not limited to, for example, the bovine growth hormone terminator or viral termination sequences, such as the SV40 terminator. In certain embodiments, the termination signal may be a lack of transcribable or translatable sequence, such as due to a sequence truncation.

### 6. Polyadenylation Signals

In expression, particularly eukaryotic expression, one will typically include a polyadenylation signal to effect proper polyadenylation of the transcript. The nature of the polyadenylation signal is not believed to be crucial to the successful practice of the invention, and/or any such sequence may be employed. Preferred embodiments include the SV40 polyadenylation signal and/or the bovine growth hormone polyadenylation signal, convenient and/or known to function well in various target cells. Polyadenylation may increase the stability of the transcript or may facilitate cytoplasmic transport.

### 7. Origins of Replication

In order to propagate a vector in a host cell, it may contain one or more origins of replication sites (often termed "ori"), which is a specific nucleic acid sequence at which replication is initiated. Alternatively an autonomously replicating sequence (ARS) can be employed if the host cell is yeast.

### 8. Selectable and Screenable Markers

In certain embodiments of the invention, cells containing a nucleic acid construct of the present invention may be identified *in vitro* or *in vivo* by encoding a screenable or selectable marker in the expression vector. When transcribed and translated, a marker confers an identifiable change to the cell permitting easy identification of cells containing the expression vector. Generally, a selectable marker is one that confers a property that allows for selection. A positive selectable marker is one in which the presence of the marker allows for its selection, while a negative selectable marker is one in which its presence prevents its selection. An example of a positive selectable marker is a drug resistance marker.

Usually the inclusion of a drug selection marker aids in the cloning and identification of transformants, for example, markers that confer resistance to neomycin, puromycin, hygromycin, DHFR, GPT, zeocin or histidinol are useful selectable markers. In addition to markers conferring a phenotype that allows for the discrimination of transformants based on the implementation of conditions, other types of markers including screenable markers such as GFP for colorimetric analysis. Alternatively, screenable enzymes such as herpes simplex virus thymidine kinase (tk) or chloramphenicol acetyltransferase (CAT) may be utilized. One of skill in the art would also know how to employ immunologic markers that can be used in conjunction with FACS analysis. The marker used is not believed to be important, so long as it is capable of being expressed simultaneously with the nucleic acid encoding a protein of the invention. Further examples of selectable and screenable markers are well known to one of skill in the art.

### B. Host Cells

As used herein, the terms "cell," "cell line," and "cell culture" may be used interchangeably. All of these terms also include their progeny, which is any and all subsequent generations. It is understood that all progeny may not be identical due to deliberate or inadvertent mutations. In the context of expressing a heterologous nucleic acid sequence, "host cell" refers to a prokaryotic or eukaryotic cell, and it includes any transformable organism that is capable of replicating a vector or expressing a heterologous gene encoded by a vector. A host cell can, and has been, used as a recipient for vectors or viruses. A host cell may be "transfected" or "transformed," which refers to a process by which exogenous nucleic acid, such as a recombinant protein-encoding sequence, is transferred or introduced into the host cell. A transformed cell includes the primary subject cell and its progeny.

Host cells may be derived from prokaryotes or eukaryotes, including bacteria, yeast cells, insect cells, and mammalian cells for replication of the vector or expression of part or all of the nucleic acid sequence(s). Numerous cell lines and cultures are available for use as a host cell, and they can be obtained through the American Type Culture Collection (ATCC), which is an organization that serves as an archive for living cultures and genetic materials (www.atcc.org). An appropriate host can be determined by one of skill in the art based on the vector backbone and the desired result. A plasmid or cosmid, for example, can be introduced into a prokaryote host cell for replication of many vectors or expression of encoded proteins. Bacterial cells used as host cells for vector replication and/or expression include Staphylococcus strains, DH5α, JM109, and KC8, as well as a number of commercially available bacterial hosts such as SURE@ Competent Cells and SOLOPACK™ Gold Cells (STRATAGENE®, La Jolla, CA). Alternatively, bacterial cells such as E. coli LE392 could be used as host cells for phage viruses. Appropriate yeast cells include *Saccharomyces cerevisiae, Saccharomyces pombe,* and *Pichia pastoris*.

Examples of eukaryotic host cells for replication and/or expression of a vector include HeLa, NIH3T3, Jurkat, 293, Cos, CHO, Saos, and PC12. Many host cells from various cell types and organisms are available and would be known to one of skill in the art. Similarly, a viral vector may be used in conjunction with either a eukaryotic or prokaryotic host cell, particularly one that is permissive for replication or expression of the vector.

Some vectors may employ control sequences that allow it to be replicated and/or expressed in both prokaryotic and eukaryotic cells. One of skill in the art would further understand the conditions under which to incubate all of the above described host cells to maintain them and to permit replication of a vector. Also understood and known are techniques and conditions that would allow large-scale production of vectors, as well as production of the nucleic acids encoded by vectors and their cognate polypeptides, proteins, or peptides.

### C. Expression Systems

Numerous expression systems exist that comprise at least a part or all of the compositions discussed above. Prokaryote- and/or eukaryote-based systems can be employed for use with the present invention to produce nucleic acid sequences, or their cognate polypeptides, proteins and peptides. Many such systems are commercially and widely available.

The insect cell/baculovirus system can produce a high level of protein expression of a heterologous nucleic acid segment, such as described in U.S. Patents 5,871,986, 4,879,236, both herein incorporated by reference, and which can be bought, for example, under the name MAXBAC® 2.0 from INVITROGEN® and BACPACK™ BACULOVIRUS EXPRESSION SYSTEM FROM CLONTECH®.

In addition to the disclosed expression systems of the invention, other examples of expression systems include STRATAGENE®'s COMPLETE CONTROL™ Inducible Mammalian Expression System, which involves a synthetic ecdysone-inducible receptor, or its pET Expression System, an *E. coli* expression system. Another example of an inducible expression system is available from INVITROGEN®, which carries the T-REX™ (tetracycline-regulated expression) System, an inducible mammalian expression system that uses the full-length CMV promoter. INVITROGEN® also provides a yeast expression system called the *Pichia methanolica* Expression System, which is designed for high-level production of recombinant proteins in the methylotrophic yeast Pichia methanolica. One of skill in the art would know how to express a vector, such as an expression construct, to produce a nucleic acid sequence or its cognate polypeptide, protein, or peptide.

### D. Amplification of Nucleic Acids

Nucleic acids used as a template for amplification may be isolated from cells, tissues or other samples according to standard methodologies (Sambrook *et al.,* 2001). In certain embodiments, analysis is performed on whole cell or tissue homogenates or biological fluid samples without substantial purification of the template nucleic acid. The nucleic acid may be genomic DNA or fractionated or whole cell RNA. Where RNA is used, it may be desired to first convert the RNA to a complementary DNA.

The term "primer," as used herein, is meant to encompass any nucleic acid that is capable of priming the synthesis of a nascent nucleic acid in a template-dependent process. Typically, primers are oligonucleotides from ten to twenty and/or thirty base pairs in length, but longer sequences can be employed. Primers may be provided in double-stranded and/or single-stranded form, although the single-stranded form is preferred.

Pairs of primers designed to selectively hybridize to nucleic acids corresponding to sequences of genes identified herein are contacted with the template nucleic acid under conditions that permit selective hybridization. Depending upon the desired application, high stringency hybridization conditions may be selected that will only allow hybridization to sequences that are completely complementary to the primers. In other embodiments, hybridization may occur under reduced stringency to allow for amplification of nucleic acids containing one or more mismatches with the primer sequences. Once hybridized, the template-primer complex is contacted with one or more enzymes that facilitate template-dependent nucleic acid synthesis. Multiple rounds of amplification, also referred to as "cycles," are conducted until a sufficient amount of amplification product is produced.

The amplification product may be detected or quantified. In certain applications, the detection may be performed by visual means. Alternatively, the detection may involve indirect identification of the product via chemiluminescence, radioactive scintigraphy of incorporated radiolabel or fluorescent label or even via a system using electrical and/or thermal impulse signals (Bellus, 1994).

A number of template dependent processes are available to amplify the oligonucleotide sequences present in a given template sample. One of the best known amplification methods is the polymerase chain reaction (referred to as PCR™) which is described in detail in U.S. Patents 4,683,195, 4,683,202 and 4,800,159, and in Innis *et al.,* 1988, each of which is incorporated herein by reference in their entirety.

Alternative methods for amplification of target nucleic acid sequences that may be used in the practice of the present invention are disclosed in U.S. Patents 5,843,650, 5,846,709, 5,846,783, 5,849,546, 5,849,497, 5,849,547, 5,858,652, 5,866,366, 5,916,776, 5,922,574, 5,928,905, 5,928,906, 5,932,451, 5,935,825, 5,939,291 and 5,942,391, GB Application No. 2 202 328, and in PCT Application No. PCT/US89/01025, each of which is incorporated herein by reference in its entirety.

### E. Methods of Gene Transfer

Suitable methods for nucleic acid delivery to effect expression of compositions of the present invention are believed to include virtually any method by which a nucleic acid (*e.g*., DNA, including viral and nonviral vectors) can be introduced into a cell, a tissue or an organism, as described herein or as would be known to one of ordinary skill in the art. Such methods include, but are not limited to, direct delivery of DNA such as by injection (U.S. Patents 5,994,624, 5,981,274, 5,945,100, 5,780,448, 5,736,524, 5,702,932, 5,656,610, 5,589,466 and 5,580,859, each incorporated herein by reference), including microinjection (Harland and Weintraub, 1985; U.S. Patent 5,789,215, incorporated herein by reference); by electroporation (U.S. Patent No. 5,384,253, incorporated herein by reference); by calcium phosphate precipitation (Graham and Van Der Eb, 1973; Chen and Okayama, 1987; Rippe *et al.,* 1990); by using DEAE dextran followed by polyethylene glycol (Gopal, 1985); by direct sonic loading (Fechheimer *et al*., 1987); by liposome mediated transfection (Nicolau and Sene, 1982; Fraley *et al.,* 1979; Nicolau *et al.,* 1987; Wong *et al.,* 1980; Kaneda *et al*., 1989; Kato *et al.,* 1991); by microprojectile bombardment (PCT Application Nos. WO 94/09699 and 95/06128; U.S. Patents 5,610,042; 5,322,783 5,563,055, 5,550,318, 5,538,877 and 5,538,880, and each incorporated herein by reference); by agitation with silicon carbide fibers (Kaeppler et al., 1990; U.S. Patents 5,302,523 and 5,464,765, each incorporated herein by reference); by Agrobacterium mediated transformation (U.S. Patents 5,591,616 and 5,563,055, each incorporated herein by reference); or by PEG mediated transformation of protoplasts (Omirulleh et al., 1993; U.S. Patents 4,684,611 and 4,952,500, each incorporated herein by reference); by desiccation/inhibition mediated DNA uptake (Potrykus *et al.,* 1985). Through the application of techniques such as these, organelle(s), cell(s), tissue(s) or organism(s) may be stably or transiently transformed.

### IV. IMMUNE RESPONSE AND ASSAYS

As discussed above, the invention concerns evoking an immune response in a subject against a secreted virulence factor or surface protein, including EsxA, EsxB, or other polypeptide transported by the Ess pathway, and/or SdrC, SdrD, SdrE, IsdA, IsdB, Spa, ClfA, ClfB, SasF, IsdC or any other peptide or protein processed by sortase. In one embodiment, the immune response can protect against or treat a subject having, suspected of having, or at risk of developing an infection or related disease, particularly those related to staphylococci.

### A. Immunoassays

The present invention includes the implementation of serological assays to evaluate whether and to what extent an immune response is induced or evoked by EsxA, EsxB, or any other polypeptide transported by the Ess pathway, and/or SdrD, SdrE, IsdA, IsdB, or any other sortase process peptide or protein. There are many types of immunoassays that can be implemented. Immunoassays encompassed by the present invention include, but are not limited to, those described in U.S. Patent 4,367,110 (double monoclonal antibody sandwich assay) and U.S. Patent 4,452,901 (western blot). Other assays include immunoprecipitation of labeled ligands and immunocytochemistry, both *in vitro* and *in vivo.*

Immunoassays generally are binding assays. Certain preferred immunoassays are the various types of enzyme linked immunosorbent assays (ELISAs) and radioimmunoassays (RIA) known in the art. Immunohistochemical detection using tissue sections is also particularly useful.

In one exemplary ELISA, the antibodies or antigens are immobilized on a selected surface, such as a well in a polystyrene microtiter plate, dipstick, or column support. Then, a test composition suspected of containing the desired antigen or antibody, such as a clinical sample, is added to the wells. After binding and washing to remove non specifically bound immune complexes, the bound antigen or antibody may be detected. Detection is generally achieved by the addition of another antibody, specific for the desired antigen or antibody, that is linked to a detectable label. This type of ELISA is known as a "sandwich ELISA". Detection also may be achieved by the addition of a second antibody specific for the desired antigen, followed by the addition of a third antibody that has binding affinity for the second antibody, with the third antibody being linked to a detectable label.

Variations on ELISA techniques are known to those of skill in the art. In one such variation, the samples suspected of containing a target antigen or antibody are immobilized onto the well surface and then contacted with the antibodies or antigens of the invention. After binding and appropriate washing, the bound immune complexes are detected. Where the initial antigen specific antibodies are linked to a detectable label, the immune complexes may be detected directly. Again, the immune complexes may be detected using a second antibody that has binding affinity for the first antigen specific antibody, with the second antibody being linked to a detectable label.

Competition ELISAs are also possible in which test samples compete for binding with known amounts of labeled antigens or antibodies. The amount of reactive species in the unknown sample is determined by mixing the sample with the known labeled species before or during incubation with coated wells. The presence of reactive species in the sample acts to reduce the amount of labeled species available for binding to the well and thus reduces the ultimate signal.

Irrespective of the format employed, ELISAs have certain features in common, such as coating, incubating or binding, washing to remove non specifically bound species, and detecting the bound immune complexes.

Antigen or antibodies may also be linked to a solid support, such as in the form of plate, beads, dipstick, membrane, or column matrix, and the sample to be analyzed is applied to the immobilized antigen or antibody. In coating a plate with either antigen or antibody, one will generally incubate the wells of the plate with a solution of the antigen or antibody, either overnight or for a specified period. The wells of the plate will then be washed to remove incompletely-adsorbed material. Any remaining available surfaces of the wells are then "coated" with a nonspecific protein that is antigenically neutral with regard to the test antisera. These include bovine serum albumin (BSA), casein, and solutions of milk powder. The coating allows for blocking of nonspecific adsorption sites on the immobilizing surface and thus reduces the background caused by nonspecific binding of antisera onto the surface.

In ELISAs, it is more customary to use a secondary or tertiary detection means rather than a direct procedure. Thus, after binding of the antigen or antibody to the well, coating with a non reactive material to reduce background, and washing to remove unbound material, the immobilizing surface is contacted with the clinical or biological sample to be tested under conditions effective to allow immune complex (antigen/antibody) formation. Detection of the immune complex then requires a labeled secondary binding ligand or antibody, or a secondary binding ligand or antibody in conjunction with a labeled tertiary antibody or third binding ligand.

"Under conditions effective to allow immune complex (antigen/antibody) formation" means that the conditions preferably include diluting the antigens and antibodies with solutions such as BSA, bovine gamma globulin (BGG) and phosphate buffered saline (PBS)/Tween. These added agents also tend to assist in the reduction of nonspecific background.

The suitable conditions also mean that the incubation is at a temperature and for a period of time sufficient to allow effective binding. Incubation steps are typically from about 1 to 2 to 4 hours, at temperatures preferably on the order of 25° to 27°C, or may be overnight at about 4°C or so.

After all incubation steps in an ELISA are followed, the contacted surface is washed so as to remove non complexed material. Washing often includes washing with a solution of PBS/Tween, or borate buffer. Following the formation of specific immune complexes between the test sample and the originally bound material, and subsequent washing, the occurrence of even minute amounts of immune complexes may be determined.

To provide a detecting means, the second or third antibody will have an associated label to allow detection. Preferably, this will be an enzyme that will generate color development upon incubating with an appropriate chromogenic substrate. Thus, for example, one will desire to contact and incubate the first or second immune complex with a urease, glucose oxidase, alkaline phosphatase, or hydrogen peroxidase conjugated antibody for a period of time and under conditions that favor the development of further immune complex formation, *e.g.*, incubation for 2 hours at room temperature in a PBS containing solution such as PBS Tween.

After incubation with the labeled antibody, and subsequent to washing to remove unbound material, the amount of label is quantified, *e.g.*, by incubation with a chromogenic substrate such as urea and bromocresol purple or 2,2' azino-di(3-ethyl benzthiazoline-6-sulfonic acid [ABTS] and H₂O₂, in the case of peroxidase as the enzyme label. Quantification is then achieved by measuring the degree of color generation, *e.g.*, using a visible spectra spectrophotometer. Alternatively, the label may be a chemiluminescent label (see, U.S. Patents 5,310,687, 5,238,808 and 5,221,605).

### B. Diagnosis of Bacterial Infection

In addition to the use of proteins, polypeptides, and/or peptides, as well as antibodies binding these polypeptides, proteins, and/or peptides to treat or prevent infection as described above, the present invention contemplates the use of these polypeptides, proteins, peptides, and/or antibodies in a variety of ways, including the detection of the presence of Staphylococci to diagnose an infection, whether in a patient or on medical equipment which may also become infected. In accordance with the invention, a preferred method of detecting the presence of infections involves the steps of obtaining a sample suspected of being infected by one or more staphylococcal bacteria species or strains, such as a sample taken from an individual, for example, from one's blood, saliva, tissues, bone, muscle, cartilage, or skin. Following isolation of the sample, diagnostic assays utilizing the polypeptides, proteins, peptides, and/or antibodies of the present invention may be carried out to detect the presence of staphylococci, and such assay techniques for determining such presence in a sample are well known to those skilled in the art and include methods such as radioimmunoassay, western blot analysis and ELISA assays. In general, in accordance with the invention, a method of diagnosing an infection is contemplated wherein a sample suspected of being infected with staphylococci has added to it the polypeptide, protein, peptide, antibody, or monoclonal antibody in accordance with the present invention, and staphylococci are indicated by antibody binding to the polypeptides, proteins, and/or peptides, or polypeptides, proteins, and/or peptides binding to the antibodies in the sample.

Accordingly, antibodies in accordance with the invention may be used for the prevention of infection from staphylococcal bacteria, for the treatment of an ongoing infection, or for use as research tools. The term "antibodies" as used herein includes monoclonal, polyclonal, chimeric, single chain, bispecific, simianized, and humanized or primatized antibodies as well as Fab fragments, such as those fragments which maintain the binding specificity of the antibodies, including the products of an Fab immunoglobulin expression library. Accordingly, the invention contemplates the use of single chains such as the variable heavy and light chains of the antibodies. Generation of any of these types of antibodies or antibody fragments is well known to those skilled in the art. Specific examples of the generation of an antibody to a bacterial protein can be found in U.S. Patent Application Pub. No. 20030153022, which is incorporated herein by reference in its entirety.

Any of the above described polypeptides, proteins, peptides, and/or antibodies may be labeled directly with a detectable label for identification and quantification of staphylococcal bacteria. Labels for use in immunoassays are generally known to those skilled in the art and include enzymes, radioisotopes, and fluorescent, luminescent and chromogenic substances, including colored particles such as colloidal gold or latex beads. Suitable immunoassays include enzyme-linked immunosorbent assays (ELISA).

### C. Protective Immunity

In some embodiments of the invention, proteinaceous compositions confer protective immunity on a subject. Protective immunity refers to a body's ability to mount a specific immune response that protects the subject from developing a particular disease or condition that involves the agent against which there is an immune response. An immunogenically effective amount is capable of conferring protective immunity to the subject.

As used herein in the specification and in the claims section that follows, the term polypeptide refers to a stretch of amino acids covalently linked there amongst via peptide bonds. Different polypeptides have different functionalities according to the present invention. While according to one aspect a polypeptide is derived from an immunogen designed to induce an active immune response in a recipient, according to another aspect of the invention, a polypeptide is derived from an antibody which results following the elicitation of an active immune response, in, for example, an animal, and which can serve to induce a passive immune response in the recipient. In both cases, however, the polypeptide is encoded by a polynucleotide according to any possible codon usage.

As used herein the phrase "immune response" or its equivalent "immunological response" refers to the development of a humoral (antibody mediated), cellular (mediated by antigen-specific T cells or their secretion products) or both humoral and cellular response directed against a protein, peptide, or polypeptide of the invention in a recipient patient. Such a response can be an active response induced by administration of immunogen or a passive response induced by administration of antibody, antibody containing material, or primed T-cells. A cellular immune response is elicited by the presentation of polypeptide epitopes in association with Class I or Class II MHC molecules, to activate antigen-specific CD4 (+) T helper cells and/or CD8 (+) cytotoxic T cells. The response may also involve activation of monocytes, macrophages, NK cells, basophils, dendritic cells, astrocytes, microglia cells, eosinophils or other components of innate immunity.

As used herein "active immunity" refers to any immunity conferred upon a subject by administration of an antigen.

As used herein "passive immunity" refers to any immunity conferred upon a subject without administration of an antigen to the subject. "Passive immunity" therefore includes, but is not limited to, administration of activated immune effectors including cellular mediators or protein mediators (*e.g.*, monoclonal and/or polyclonal antibodies) of an immune response. A monoclonal or polyclonal antibody composition may be used in passive immunization for the prevention or treatment of infection by organisms that carry the antigen recognized by the antibody. An antibody composition may include antibodies that bind to a variety of antigens that may in turn be associated with various organisms. The antibody component can be a polyclonal antiserum. In certain aspects the antibody or antibodies are affinity purified from an animal or second subject that has been challenged with an antigen(s). Alternatively, an antibody mixture may be used, which is a mixture of monoclonal and/or polyclonal antibodies to antigens present in the same, related, or different microbes or organisms, such as gram-positive bacteria, gram-negative bacteria, including but not limited to staphylococcus bacteria.

Passive immunity may be imparted to a patient or subject by administering to the patient immunoglobulins (Ig) and/or other immune factors obtained from a donor or other non-patient source having a known immunoreactivity. In other aspects, an antigenic composition of the present invention can be administered to a subject who then acts as a source or donor for globulin, produced in response to challenge from the composition ("hyperimmune globulin"), that contains antibodies directed against Staphylococcus or other organism. A subject thus treated would donate plasma from which hyperimmune globulin would then be obtained, *via* conventional plasma-fractionation methodology, and administered to another subject in order to impart resistance against or to treat staphylococcus infection. Hyperimmune globulins according to the invention are particularly useful for immune-compromised individuals, for individuals undergoing invasive procedures or where time does not permit the individual to produce his own antibodies in response to vaccination. See U.S. Patents 6,936,258, 6,770,278, 6,756,361, 5,548,066, 5,512,282, 4,338,298, and 4,748,018, each of which is incorporated herein by reference in its entirety, for exemplary methods and compositions related to passive immunity.

For purposes of this specification and the accompanying claims the terms "epitope" and "antigenic determinant" are used interchangeably to refer to a site on an antigen to which B and/or T cells respond or recognize. B-cell epitopes can be formed both from contiguous amino acids or noncontiguous amino acids juxtaposed by tertiary folding of a protein. Epitopes formed from contiguous amino acids are typically retained on exposure to denaturing solvents whereas epitopes formed by tertiary folding are typically lost on treatment with denaturing solvents. An epitope typically includes at least 3, and more usually, at least 5 or 8-10 amino acids in a unique spatial conformation. Methods of determining spatial conformation of epitopes include, for example, x-ray crystallography and 2-dimensional nuclear magnetic resonance. See, *e.g.*, Epitope Mapping Protocols (1996). Antibodies that recognize the same epitope can be identified in a simple immunoassay showing the ability of one antibody to block the binding of another antibody to a target antigen. T-cells recognize continuous epitopes of about nine amino acids for CD8 cells or about 13-15 amino acids for CD4 cells. T cells that recognize the epitope can be identified by in vitro assays that measure antigen-dependent proliferation, as determined by ³H-thymidine incorporation by primed T cells in response to an epitope (Burke *et al.,* 1994), by antigen-dependent killing (cytotoxic T lymphocyte assay, Tigges *et al*., 1996) or by cytokine secretion.

The presence of a cell-mediated immunological response can be determined by proliferation assays (CD4 (+) T cells) or CTL (cytotoxic T lymphocyte) assays. The relative contributions of humoral and cellular responses to the protective or therapeutic effect of an immunogen can be distinguished by separately isolating IgG and T-cells from an immunized syngeneic animal and measuring protective or therapeutic effect in a second subject.

As used herein and in the claims, the terms "antibody" or "immunoglobulin" are used interchangeably and refer to any of several classes of structurally related proteins that function as part of the immune response of an animal or recipient, which proteins include IgG, IgD, IgE, IgA, IgM and related proteins.

Under normal physiological conditions antibodies are found in plasma and other body fluids and in the membrane of certain cells and are produced by lymphocytes of the type denoted B cells or their functional equivalent. Antibodies of the IgG class are made up of four polypeptide chains linked together by disulfide bonds. The four chains of intact IgG molecules are two identical heavy chains referred to as H-chains and two identical light chains referred to as L-chains.

In order to produce polyclonal antibodies, a host, such as a rabbit or goat, is immunized with the antigen or antigen fragment, generally with an adjuvant and, if necessary, coupled to a carrier. Antibodies to the antigen are subsequently collected from the sera of the host. The polyclonal antibody can be affinity purified against the antigen rendering it monospecific.

In order to produce monoclonal antibodies, hyperimmunization of an appropriate donor, generally a mouse, with the antigen is undertaken. Isolation of splenic antibody producing cells is then carried out. These cells are fused to a cell characterized by immortality, such as a myeloma cell, to provide a fused cell hybrid (hybridoma) which can be maintained in culture and which secretes the required monoclonal antibody. The cells are then be cultured, in bulk, and the monoclonal antibodies harvested from the culture media for use. By definition, monoclonal antibodies are specific to a single epitope. Monoclonal antibodies often have lower affinity constants than polyclonal antibodies raised against similar antigens for this reason.

Monoclonal antibodies may also be produced *ex-vivo* by use of primary cultures of splenic cells or cell lines derived from spleen (Anavi, 1998). In order to produce recombinant antibody (see generally Huston *et al.,* 1991; Johnson *et al.,* 1991; Mernaugh *et al.,* 1995), messenger RNAs from antibody producing B-lymphocytes of animals, or hybridoma are reverse-transcribed to obtain complementary DNAs (cDNAs). Antibody cDNA, which can be full length or partial length, is amplified and cloned into a phage or a plasmid. The cDNA can be a partial length of heavy and light chain cDNA, separated or connected by a linker. The antibody, or antibody fragment, is expressed using a suitable expression system to obtain recombinant antibody. Antibody cDNA can also be obtained by screening pertinent expression libraries.

The antibody can be bound to a solid support substrate or conjugated with a detectable moiety or be both bound and conjugated as is well known in the art. For a general discussion of conjugation of fluorescent or enzymatic moieties see Johnstone *et al.* (1982). The binding of antibodies to a solid support substrate is also well known in the art (Harlow *et al.,* 1988; Borrebaeck, 1992).

As used herein and in the claims, the phrase "an immunological portion of an antibody" include a Fab fragment of an antibody, a Fv fragment of an antibody, a heavy chain of an antibody, a light chain of an antibody, an unassociated mixture of a heavy chain and a light chain of an antibody, a heterodimer consisting of a heavy chain and a light chain of an antibody, a catalytic domain of a heavy chain of an antibody, a catalytic domain of a light chain of an antibody, a variable fragment of a light chain of an antibody, a variable fragment of a heavy chain of an antibody, and a single chain variant of an antibody, which is also known as scFv. In addition, the term includes chimeric immunoglobulins which are the expression products of fused genes derived from different species, one of the species can be a human, in which case a chimeric immunoglobulin is said to be humanized. Typically, an immunological portion of an antibody competes with the intact antibody from which it was derived for specific binding to an antigen.

Optionally, an antibody or preferably an immunological portion of an antibody, can be chemically conjugated to, or expressed as, a fusion protein with other proteins. For purposes of this specification and the accompanying claims, all such fused proteins are included in the definition of antibodies or an immunological portion of an antibody.

As used herein the terms "immunogenic agent" or "immunogen" or "antigen" are used interchangeably to describe a molecule capable of inducing an immunological response against itself on administration to a recipient, either alone, in conjunction with an adjuvant, or presented on a display vehicle.

### D. Treatment Methods

A method of the present invention includes treatment for a disease or condition caused by a staphylococcus pathogen. An immunogenic polypeptide of the invention can be given to induce an immune response in a person infected with staphylococcus or suspected of having been exposed to staphylococcus. Methods may be employed with respect to individuals who have tested positive for exposure to staphylococcus or who are deemed to be at risk for infection based on possible exposure.

In some embodiments, the treatment is administered in the presence of adjuvants or carriers or other staphylococcal antigens. Furthermore, in some examples, treatment comprises administration of other agents commonly used against bacterial infection, such as one or more antibiotics.

The use of peptides for vaccination typically requires conjugation of the peptide to an immunogenic carrier protein, such as hepatitis B surface antigen, keyhole limpet hemocyanin, or bovine serum albumin. Methods for performing this conjugation are well known in the art.

### V. VACCINE AND OTHER PHARMACEUTICAL COMPOSITIONS AND ADMINISTRATION

### A. Vaccines

The present invention includes methods for preventing or ameliorating staphylococcus infections. As such, the invention contemplates vaccines for use in both active and passive immunization embodiments. Immunogenic compositions, proposed to be suitable for use as a vaccine, may be prepared most readily directly from immunogenic secreted virulence proteins or surface proteins, including EsxA, EsxB, or any other polypeptide transported by the Ess pathway, and/or SdrC, SdrD, SdrE, IsdA, IsdB, IsdC, Spa, ClfA, ClfB, SasF or any other sortase processed peptide or protein prepared in a manner disclosed herein. Preferably the antigenic material is extensively dialyzed to remove undesired small molecular weight molecules and/or lyophilized for more ready formulation into a desired vehicle. The invention includes compositions that can be used to induce an immune response against a polypeptide or peptide derived from a member of the Ess pathway, in certain aspects an Esx protein and more specifically an EsxA, EsxB, or any other polypeptide transported by the Ess pathway, and/or a polypeptide or peptide derived from a peptide or protein processed by the sortase pathway, in certain aspects, SdrD, SdrE, IsdA, IsdB, or any other sortase processed peptide or protein so as to protect against infection by a staphylococcus and against developing a condition or disease caused by such a pathogen.

Alternatively, other viable and important options for a protein/peptide-based vaccine involve introducing nucleic acids encoding the antigen(s) as DNA vaccines. In this regard, recent reports described construction of recombinant vaccinia viruses expressing either 10 contiguous minimal CTL epitopes (Thomson, 1996) or a combination of B cell, CTL, and TH epitopes from several microbes (An, 1997), and successful use of such constructs to immunize mice for priming protective immune responses. Thus, there is ample evidence in the literature for successful utilization of peptides, peptide-pulsed APCs, and peptide-encoding constructs for efficient *in vivo* priming of protective immune responses. The use of nucleic acid sequences as vaccines is exemplified in U.S. Patents 5,958,895 and 5,620,896.

The preparation of vaccines that contain polypeptide or peptide sequence(s) as active ingredients is generally well understood in the art, as exemplified by U.S. Patents 4,608,251; 4,601,903; 4,599,231; 4,599,230; 4,596,792; and 4,578,770, all of which are incorporated herein by reference. Typically, such vaccines are prepared as injectables either as liquid solutions or suspensions: solid forms suitable for solution in or suspension in liquid prior to injection may also be prepared. The preparation may also be emulsified. The active immunogenic ingredient is often mixed with excipients that are pharmaceutically acceptable and compatible with the active ingredient. Suitable excipients are, for example, water, saline, dextrose, glycerol, ethanol, or the like and combinations thereof. In addition, if desired, the vaccine may contain amounts of auxiliary substances such as wetting or emulsifying agents, pH buffering agents, or adjuvants that enhance the effectiveness of the vaccines. In specific embodiments, vaccines are formulated with a combination of substances, as described in U.S. Patents 6,793,923 and 6,733,754, which are incorporated herein by reference.

Vaccines may be conventionally administered parenterally, by injection, for example, either subcutaneously or intramuscularly. Additional formulations which are suitable for other modes of administration include suppositories and, in some cases, oral formulations. For suppositories, traditional binders and carriers may include, for example, polyalkalene glycols or triglycerides: such suppositories may be formed from mixtures containing the active ingredient in the range of about 0.5% to about 10%, preferably about 1% to about 2%. Oral formulations include such normally employed excipients as, for example, pharmaceutical grades of mannitol, lactose, starch, magnesium stearate, sodium saccharine, cellulose, magnesium carbonate and the like. These compositions take the form of solutions, suspensions, tablets, pills, capsules, sustained release formulations or powders and contain about 10% to about 95% of active ingredient, preferably about 25% to about 70%.

The polypeptides and polypeptide-encoding DNA constructs may be formulated into a vaccine as neutral or salt forms. Pharmaceutically-acceptable salts include the acid addition salts (formed with the free amino groups of the peptide) and those that are formed with inorganic acids such as, for example, hydrochloric or phosphoric acids, or such organic acids as acetic, oxalic, tartaric, mandelic, and the like. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

Typically, vaccines are administered in a manner compatible with the dosage formulation, and in such amount as will be therapeutically effective and immunogenic. The quantity to be administered depends on the subject to be treated, including the capacity of the individual's immune system to synthesize antibodies and the degree of protection desired. Precise amounts of active ingredient required to be administered depend on the judgment of the practitioner. However, suitable dosage ranges are of the order of several hundred micrograms active ingredient per vaccination. Suitable regimes for initial administration and booster shots are also variable, but are typified by an initial administration followed by subsequent inoculations or other administrations.

The manner of application may be varied widely. Any of the conventional methods for administration of a vaccine are applicable. These are believed to include oral application on a solid physiologically acceptable base or in a physiologically acceptable dispersion, parenterally, by injection and the like. The dosage of the vaccine will depend on the route of administration and will vary according to the size and health of the subject.

In many instances, it will be desirable to have multiple administrations of the vaccine, usually not exceeding six vaccinations, more usually not exceeding four vaccinations, and preferably one or more, usually at least about three vaccinations. The vaccinations will normally be at two to twelve week intervals, more usually from three to five week intervals. Periodic boosters at intervals of 1-5 years, usually three years, will be desirable to maintain protective levels of the antibodies. The course of the immunization may be followed by assays for antibodies against the antigens, as described supra, U.S. Patents 3,791,932; 4,174,384 and 3,949,064, are illustrative of these types of assays.

### 1. Carriers

A given composition may vary in its immunogenicity. It is often necessary therefore to boost the host immune system, as may be achieved by coupling a peptide or polypeptide to a carrier. Exemplary and preferred carriers are keyhole limpet hemocyanin (KLH) and bovine serum albumin (BSA). Other albumins such as ovalbumin, mouse serum albumin, or rabbit serum albumin can also be used as carriers. Means for conjugating a polypeptide to a carrier protein are well known in the art and include glutaraldehyde, m-maleimidobencoyl-N-hydroxysuccinimide ester, carbodiimyde, and bis-biazotized benzidine.

### 2. Adjuvants

The immunogenicity of polypeptide or peptide compositions can be enhanced by the use of non-specific stimulators of the immune response, known as adjuvants. Suitable adjuvants include all acceptable immunostimulatory compounds, such as cytokines, toxins, or synthetic compositions.

A number of adjuvants can be used to enhance an antibody response against an EsxA, EsxB, or any other polypeptide transported by the Ess pathway and/or against a SdrD, SdrE, IsdA, IsdB, or any other sortase processed peptide or protein. Adjuvants can 1) trap the antigen in the body to cause a slow release; 2) attract cells involved in the immune response to the site of administration; 3) induce proliferation or activation of immune system cells; or 4) improve the spread of the antigen throughout the subject's body.

Adjuvants include, but are not limited to, oil-in-water emulsions, water-in-oil emulsions, mineral salts, polynucleotides, and natural substances. Specific adjuvants that may be used include IL-1, IL-2, IL-4, IL-7, IL-12, γ-interferon, GMCSP, BCG, aluminum hydroxide or other aluminum compound, MDP compounds, such as thur-MDP and nor-MDP, CGP (MTP-PE), lipid A, and monophosphoryl lipid A (MPL). RIBI, which contains three components extracted from bacteria, MPL, trehalose dimycolate (TDM), and cell wall skeleton (CWS) in a 2% squalene/Tween 80 emulsion. MHC antigens may even be used. Others adjuvants or methods are exemplified in U.S. Patents 6,814,971, 5,084,269, 6,656,462, each of which is incorporated herein by reference).

Various methods of achieving adjuvant affect for the vaccine includes use of agents such as aluminum hydroxide or phosphate (alum), commonly used as about 0.05 to about 0.1 % solution in phosphate buffered saline, admixture with synthetic polymers of sugars (Carbopol®) used as an about 0.25% solution, aggregation of the protein in the vaccine by heat treatment with temperatures ranging between about 70° to about 101°C for a 30-second to 2-minute period, respectively. Aggregation by reactivating with pepsin-treated (Fab) antibodies to albumin; mixture with bacterial cells (e.g., *C. parvum*), endotoxins or lipopolysaccharide components of Gram-negative bacteria; emulsion in physiologically acceptable oil vehicles (e.g., mannide mono-oleate (Aracel A)); or emulsion with a 20% solution of a perfluorocarbon (Fluosol-DA®) used as a block substitute may also be employed to produce an adjuvant effect.

Exemplary, often preferred adjuvants include complete Freund's adjuvant (a non-specific stimulator of the immune response containing killed *Mycobacterium tuberculosis*), incomplete Freund's adjuvants, and aluminum hydroxide.

In addition to adjuvants, it may be desirable to co-administer biologic response modifiers (BRM) to enhance immune responses. BRMs have been shown to upregulate T cell immunity or downregulate suppresser cell activity. Such BRMs include, but are not limited to, Cimetidine (CIM; 1200 mg/d) (Smith/Kline, PA); or low-dose Cyclophosphamide (CYP; 300 mg/m²) (Johnson/ Mead, NJ) and cytokines such as γ-interferon, IL-2, or IL-12 or genes encoding proteins involved in immune helper functions, such as B-7.

### B. Lipid Components and Moieties

In certain embodiments, the present invention concerns compositions comprising one or more lipids associated with a nucleic acid or a polypeptide/peptide. A lipid is a substance that is insoluble in water and extractable with an organic solvent. Compounds other than those specifically described herein are understood by one of skill in the art as lipids, and are encompassed by the compositions and methods of the present invention. A lipid component and a non-lipid may be attached to one another, either covalently or non-covalently.

A lipid may be a naturally occurring lipid or a synthetic lipid. However, a lipid is usually a biological substance. Biological lipids are well known in the art, and include for example, neutral fats, phospholipids, phosphoglycerides, steroids, terpenes, lysolipids, glycosphingolipids, glucolipids, sulphatides, lipids with ether and ester-linked fatty acids and polymerizable lipids, and combinations thereof.

A nucleic acid molecule or a polypeptide/peptide, associated with a lipid may be dispersed in a solution containing a lipid, dissolved with a lipid, emulsified with a lipid, mixed with a lipid, combined with a lipid, covalently bonded to a lipid, contained as a suspension in a lipid or otherwise associated with a lipid. A lipid or lipid-poxvirus-associated composition of the present invention is not limited to any particular structure. For example, they may also simply be interspersed in a solution, possibly forming aggregates which are not uniform in either size or shape. In another example, they may be present in a bilayer structure, as micelles, or with a "collapsed" structure. In another non-limiting example, a lipofectamine(Gibco BRL)-poxvirus or Superfect (Qiagen)-poxvirus complex is also contemplated.

In certain embodiments, a composition may comprise about 1%, about 2%, about 3%, about 4% about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, or any range therebetween, of a particular lipid, lipid type, or non-lipid component such as an adjuvant, antigen, peptide, polypeptide, sugar, nucleic acid or other material disclosed herein or as would be known to one of skill in the art. In a non-limiting example, a composition may comprise about 10% to about 20% neutral lipids, and about 33% to about 34% of a cerebroside, and about 1% cholesterol. In another non-limiting example, a liposome may comprise about 4% to about 12% terpenes, wherein about 1% of the micelle is specifically lycopene, leaving about 3% to about 11% of the liposome as comprising other terpenes; and about 10% to about 35% phosphatidyl choline, and about 1% of a non-lipid component. Thus, it is contemplated that compositions of the present invention may comprise any of the lipids, lipid types or other components in any combination or percentage range.

### C. Combination Therapy

The compositions and related methods of the present invention, particularly administration of a secreted virulence factor or surface protein, including a polypeptide or peptide of a EsxA, EsxB, or other polypeptide transported by the Ess pathway, and/or a polypeptide or peptide of a SdrC, SdrD, SdrE, IsdA, IsdB, IsdC, Spa, ClfA, ClfB, SasF or any other sortase processed peptide or protein to a patient/subject, may also be used in combination with the administration of traditional therapies. These include, but are not limited to, the administration of antibiotics such as streptomycin, ciprofloxacin, doxycycline, gentamycin, chloramphenicol, trimethoprim, sulfamethoxazole, ampicillin, tetracycline or various combinations of antibiotics.

In one aspect, it is contemplated that a polypeptide vaccine and/or therapy is used in conjunction with antibacterial treatment. Alternatively, the therapy may precede or follow the other agent treatment by intervals ranging from minutes to weeks. In embodiments where the other agents and/or a proteins or polynucleotides are administered separately, one would generally ensure that a significant period of time did not expire between the time of each delivery, such that the agent and antigenic composition would still be able to exert an advantageously combined effect on the subject. In such instances, it is contemplated that one may administer both modalities within about 12-24 h of each other and, more preferably, within about 6-12 h of each other. In some situations, it may be desirable to extend the time period for administration significantly, however, where several days (2, 3, 4, 5, 6 or 7) to several weeks (1, 2, 3, 4, 5, 6, 7 or 8) lapse between the respective administrations.

Various combinations may be employed, for example antibiotic therapy is "A" and the immunogenic molecule given as part of an immune therapy regime, such as an antigen, is "B":
A/B/A B/A/B B/B/A A/A/B A/BB B/A/A A/B/B/B B/A/B/B
B/B/B/A B/B/A/B A/A/B/B A/B/A/B A/B/B/A B/B/A/A
B/A/B/A B/A/A/B A/A/A/B B/A/A/A A/B/A/A A/A/B/A

Administration of the immunogenic compositions of the present invention to a patient/subject will follow general protocols for the administration of such compounds, taking into account the toxicity, if any, of the EsxA-composition, EsxB-composition, or composition of any other polypeptide transported by the Ess pathway and/or a SdrD-composition, SdrE-composition, IsdA-composition, IsdB-composition, or any other sortase processed peptide or protein. It is expected that the treatment cycles would be repeated as necessary. It also is contemplated that various standard therapies, such as hydration, may be applied in combination with the described therapy.

### D. General Pharmaceutical Compositions

In some embodiments, pharmaceutical compositions are administered to a subject. Different aspects of the present invention involve administering an effective amount of a composition to a subject. In some embodiments of the present invention, members of the Ess pathway and including polypeptides or peptides of the Esx class, and/or members of sortase substrates may be administered to the patient to protect against infection by one or more staphylococcus pathogens. Alternatively, an expression vector encoding one or more such polypeptides or peptides may be given to a patient as a preventative treatment. Additionally, such compounds can be administered in combination with an antibiotic. Such compositions will generally be dissolved or dispersed in a pharmaceutically acceptable carrier or aqueous medium.

The phrases "pharmaceutically acceptable" or "pharmacologically acceptable" refer to molecular entities and compositions that do not produce an adverse, allergic, or other untoward reaction when administered to an animal, or human. As used herein, "pharmaceutically acceptable carrier" includes any and all solvents, dispersion media, coatings, antibacterial and antifungal agents, isotonic and absorption delaying agents, and the like. The use of such media and agents for pharmaceutical active substances is well known in the art. Except insofar as any conventional media or agent is incompatible with the active ingredients, its use in immunogenic and therapeutic compositions is contemplated. Supplementary active ingredients, such as other anti-cancer agents, can also be incorporated into the compositions.

In addition to the compounds formulated for parenteral administration, such as those for intravenous or intramuscular injection, other pharmaceutically acceptable forms include, *e.g.*, tablets or other solids for oral administration; time release capsules; and any other form currently used, including creams, lotions, mouthwashes, inhalants and the like.

The active compounds of the present invention can be formulated for parenteral administration, *e.g.*, formulated for injection via the intravenous, intramuscular, sub-cutaneous, or even intraperitoneal routes. The preparation of an aqueous composition that contains a compound or compounds that increase the expression of an MHC class I molecule will be known to those of skill in the art in light of the present disclosure. Typically, such compositions can be prepared as injectables, either as liquid solutions or suspensions; solid forms suitable for use to prepare solutions or suspensions upon the addition of a liquid prior to injection can also be prepared; and, the preparations can also be emulsified.

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions; formulations including sesame oil, peanut oil, or aqueous propylene glycol; and sterile powders for the extemporaneous preparation of sterile injectable solutions or dispersions. In all cases the form must be sterile and must be fluid to the extent that it may be easily injected. It also should be stable under the conditions of manufacture and storage and must be preserved against the contaminating action of microorganisms, such as bacteria and fungi.

The proteinaceous compositions may be formulated into a neutral or salt form. Pharmaceutically acceptable salts, include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier also can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion, and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques, which yield a powder of the active ingredient, plus any additional desired ingredient from a previously sterile-filtered solution thereof.

Administration of the compositions according to the present invention will typically be via any common route. This includes, but is not limited to oral, nasal, or buccal administration. Alternatively, administration may be by orthotopic, intradermal, subcutaneous, intramuscular, intraperitoneal, intranasal, or intravenous injection. In certain embodiments, a vaccine composition may be inhaled (*e.g*., U.S. Patent 6,651,655, which is specifically incorporated by reference). Such compositions would normally be administered as pharmaceutically acceptable compositions that include physiologically acceptable carriers, buffers or other excipients. As used herein, the term "pharmaceutically acceptable" refers to those compounds, materials, compositions, and/or dosage forms which are, within the scope of sound medical judgment, suitable for contact with the tissues of human beings and animals without excessive toxicity, irritation, allergic response, or other problem complications commensurate with a reasonable benefit/risk ratio. The term "pharmaceutically acceptable carrier," means a pharmaceutically acceptable material, composition or vehicle, such as a liquid or solid filler, diluent, excipient, solvent or encapsulating material, involved in carrying or transporting a chemical agent.

For parenteral administration in an aqueous solution, for example, the solution should be suitably buffered, if necessary, and the liquid diluent first rendered isotonic with sufficient saline or glucose. These particular aqueous solutions are especially suitable for intravenous, intramuscular, subcutaneous, and intraperitoneal administration. In this connection, sterile aqueous media which can be employed will be known to those of skill in the art in light of the present disclosure. For example, one dosage could be dissolved in isotonic NaCl solution and either added to hypodermoclysis fluid or injected at the proposed site of infusion, (see for example, Remington's Pharmaceutical Sciences, 1990). Some variation in dosage will necessarily occur depending on the condition of the subject. The person responsible for administration will, in any event, determine the appropriate dose for the individual subject.

An effective amount of therapeutic or prophylactic composition is determined based on the intended goal. The term "unit dose" or "dosage" refers to physically discrete units suitable for use in a subject, each unit containing a predetermined quantity of the composition calculated to produce the desired responses discussed above in association with its administration, *i.e*., the appropriate route and regimen. The quantity to be administered, both according to number of treatments and unit dose, depends on the protection desired.

Precise amounts of the composition also depend on the judgment of the practitioner and are peculiar to each individual. Factors affecting dose include physical and clinical state of the subject, route of administration, intended goal of treatment (alleviation of symptoms versus cure), and potency, stability, and toxicity of the particular composition.

Upon formulation, solutions will be administered in a manner compatible with the dosage formulation and in such amount as is therapeutically or prophylactically effective. The formulations are easily administered in a variety of dosage forms, such as the type of injectable solutions described above.

### E. In Vitro, Ex Vivo, or In Vivo Administration

As used herein, the term *in vitro* administration refers to manipulations performed on cells removed from or outside of an animal, including, but not limited to cells in culture. The term *ex vivo* administration refers to cells which have been manipulated *in vitro,* and are subsequently administered to a living animal. The term *in vivo* administration includes all manipulations performed within an animal.

In certain aspects of the present invention, the compositions may be administered either *in vitro, ex vivo,* or *in vivo.* In certain *in vitro* embodiments, autologous B-lymphocyte cell lines are incubated with a virus vector of the instant invention for 24 to 48 hours or with EsxA, EsxB, or any other polypeptide transported by the Ess pathway, and/or a polypeptide or peptide of a SdrC, SdrD, SdrE, IsdA, IsdB, IsdC, Spa, ClfA., ClfB, SasF or any other sortase processed peptide or protein (or any combination thereof) for two hours. The transduced cells can then be used for *in vitro* analysis, or alternatively for *ex vivo* administration.

U.S. Patents 4,690,915 and 5,199,942, both incorporated herein by reference, disclose methods for *ex vivo* manipulation of blood mononuclear cells and bone marrow cells for use in therapeutic applications.

### EXAMPLES

The following examples are given for the purpose of illustrating various embodiments of the invention and are not meant to limit the present invention in any fashion. One skilled in the art will appreciate readily that the present invention is well adapted to carry out the objects and obtain the ends and advantages mentioned, as well as those objects, ends and advantages inherent herein. The present examples, along with the methods described herein are presently representative of preferred embodiments, are exemplary, and are not intended as limitations on the scope of the invention. Changes therein and other uses which are encompassed within the spirit of the invention as defined by the scope of the claims will occur to those skilled in the art.

### EXAMPLE 1

### Staphylococcus Mutant in esxA, esxB or essC are Defective in the Pathogenesis of Staphylococcal Abscesses

*Staphylococcus aureus* host infection and dissemination within organ tissues is dependent on staphylococcal synthesis and secretion of a wide variety of virulence factors (Novick, 2003). Because of the astonishing armament of staphylococci with virulence factors, the pathogenesis of *S*. *aureus* infections is considered multi-factorial (Novick, 2003). Thus, with the exception of α-hemolysin (*hla*) mutants (Bhakdi and Tranum-Jensen, 1991), strains carrying mutations that abrogate the expression of individual exoproteins typically do not display significant defects in the pathogenesis of *S*. *aureus* infections. It was contemplated that the Ess secretion pathway is involved in the establishment of staphylococcal disease. *S*. *aureus* Newman variant strains *esxA24, essC::erm* and *esxB::erm* do not produce EsxA and EsxB. These strains were chosen for measurement of bacterial virulence. Viable staphylococci of mutant or wild-type Newman strains (approximately 10⁶ staphylococci) were administered intravenously via retroorbital injection into mice. Four days after infection, the animals were killed. Internal organs were removed, inspected for abscess formation, and then homogenized and spread on agar medium to quantify staphylococcal replication in host tissues via colony formation (Albus *et al.,* 1991). Mutations in *esxA, esxB* and *essC* genes caused a significant reduction in the ability of *S. aureus* Newman to establish kidney or spleen abscesses in infected mice (FIG. 2, data not shown). Kidneys of mice infected with Newman carried a mean value of 3.89 x 10⁷ colony forming units (CFU)/organ while those of mice infected with strains lacking *esxA, esxB* and *essC* harbored a mean value of 3.00 x 10⁴ (p < 2.09 x 10⁻⁵), 6.17 x 10⁵ (p < 1.2 x 10⁻²) and 2.51 x 10⁵ (p < 5.53 x 10⁻³) CFU/organ, respectively. It should be noted that half of the animals infected with mutant bacteria had cleared most staphylococci from the kidneys after four days, suggesting that complete clearing may occur by day five, post infection with the mutant strains. The formation of liver abscesses in infected animals was even more affected when staphylococci carried transposon insertions in *essC* and *esxB.* Homogenized livers of mice infected with Newman were found to contain a mean value of 2.09 x 10⁶ CFU/organ of staphylococci, while the livers of mutants lacking *esxA, esxB* and *essC* contained a mean value of 9 x 10¹ (p < 3.01 x 10⁻⁷), 4.17 x 10² (p < 4.33 x 10⁻⁶) and 1.35 x 10³ (p < 1.72 x 10⁴) CFU/organ, respectively.

### EXAMPLE 2

### Immune Response of Mice to EsxA and EsxB During Staphylococcal Infection

A hallmark of virulence factors secreted by pathogenic microbes is the development of specific humoral or cellular immune responses. Often such acquired immune responses neutralize the pathogenesis functions of such essential virulence factors and thereby contribute to opsono-phagocytic clearances of microbial infections (Mills *et al.,* 1997). As EsxA or EsxB represent secreted virulence factors, the inventors studied the response of the immune system of animals infected with *S*. *aureus* relative to these polypeptides as antigens, in particular the generation of specific humoral immune responses. In addressing this issue, three week old BALB/c mice were infected by intravenous inoculation with 1 x 10⁷ cfu of *S*. *aureus* Newman (a sublethal dose of infection) and compared with mock-infected animals as a control. As reported above, staphylococci invade multiple tissues and seed abscesses in liver and other internal organs, which are resolved when acquired immune responses of BALB/c mice are mounted that eventually clear these infections after 10-14 days. During this time interval, the animals mount a plethora of humoral immune responses, first via the production IgM type antibodies and then via the secretion of mature IgG type antibodies (Janeway *et al.,* 1999). To examine whether animals acutely infected with *S*. *aureus* generate humoral immune responses to EsxA and EsxB, infected animals were euthanized via terminal bleeding on day 0 and 30 after intravenous inoculation. Serum samples were subjected to quantitative analysis for specific IgG responses using purified EsxA and EsxB in an ELISA assay. Data in FIG. 6 show that animals infected with *S*. *aureus* developed IgG type antibodies against EsxA and EsxB, whereas mock-infected animals did not. It is contemplated that the observed humoral immune responses confer protection against staphylococcal infection.

Efficacy of purified EsxA antigen in raising protective immunity was studied in a sub-lethal bacterial mouse challenge model (FIG. 7.). On day 0, ten four-week old BALB/c mice were immunized by intra-muscular injection with 50 µg of purified antigen (EsxA) in phosphate buffered saline and mixed (50% v/v) with complete freund adjuvant (CFA). Immunized animals were boosted with the same antigen formulation at days 11 and 20. Two groups of ten mice from immunized and control animals (PBS/CFA 50% v/v) were challenged on day 21 after the first immunization. Animals were killed 96 h post-infection, and bacterial counts were performed on their kidneys and livers. The experiment suggests that immunization with purified EsxA antigen confers partial protection against *S*. *aureus* disease.

### EXAMPLE 3

### Immunization with Purified EsxA and EsxB

Preliminary studies have shown that *bursa aurealis* insertion in *esxA, essC* or *esxB* caused a 3-4 log reduction in the ability of *S*. *aureus* strain Newman to form liver abscesses following intravenous infection of BALB/c mice (FIG. 5). The ability of mutant staphylococci to seed abscesses in internal organs was also diminished for spleen and kidneys, indicating that Ess-mediated secretion plays generally an important role during the pathogenesis of *S*. *aureus* infections. Similarly severe virulence defects have only been observed for accessory gene regulation two component genes (*agr*) (Cheung *et al.* 1994), sortase (Ton-That *et al.,* 2000) or heme iron transport mutants (Skaar *et al.,* 2004), whereas mutations in single surface protein or exoprotein genes cause only small reductions in the ability of staphylococci to establish murine disease (Ni Eidhin *et al.,* 1998). In the system used by the inventors, staphylococci (1 x 10⁶ or 1 x 10⁷ cfu of staphylococci grown in tryptic soy broth) are injected intravenously (Albus *et al.,* 1991). In the first hour after infection, staphylococci are slowly cleared from the blood stream, in part via phagocytic killing or by bacterial binding to host organ tissues (Thakker *et al.,* 1998). Staphylococci that escape killing by early innate immune responses (Thomas *et al.,* 2000) begin to replicate in infected tissues and generate pro-inflammatory responses with the release of cytokines from macrophages, neutrophils and other immune cells (Jonsson *et al.,* 2003). The resulting massive invasion of immune cells into the site of infection is accompanied by central liquefaction necrosis and formation of peripheral fibrin walls in an effort to prevent infection spread (Jonsson *et al.,* 1985). Eventually, the central liquefaction necrosis is drained to organ or skin surfaces, while infectious material is being phagocytosed and epithelia repaired by wound healing (Jonsson *et al.,* 1985; Patel *et al.,* 1987). All of these events represent physiological host responses, producing abscesses in variable degrees of development as the macroscopic and microscopic anatomic-pathological substrate of *S*. *aureus* infections. The experimental data reported in FIG. 5 however account only for the presence of abscesses as well as for the viability of staphylococci within infected organ tissue.

Previous work in the field sought to identify immuno-dominant antigen epitopes reactive with human serum and individual antibodies were selected because of their high titer of opsonic activity in phagocytic killing assays (Etz *et al.,* 2002). *S*. *aureus* whole genome expression libraries, either used as fusions to *E*. *coli* surface proteins (Etz *et al.,* 2002) or synthesized *via* the ribosome display method (Weichhart *et al.,* 2003) isolated either 60 (Etz *et al.,* 2002) or 84 immuno-dominant antigens (Weichhart *et al.,* 2003), respectively. EsxA and EsxB were not among the identified immuno-dominant antigens, however considering the limitations of gene expression and bacterial surface display or the limitations of using human sera without disease - there are many reasons to suspect that these genome scale studies were far from exhaustive (Weichhart *et al.,* 2003). Since animals acutely infected with *S*. *aureus* mount an immune response to EsxA and EsxB as judged by our ELISA experiment (FIG. 6), the efficacy of purified antigens of raising protective immunity will be tested in sub-lethal bacterial mouse challenge models. On day 0, mice will be immunized by intra-muscular injection with 100 µg of purified antigen (EsxA or EsxB or both) in phosphate buffered saline and absorbed to adjuvant by mixing (50% v/v). Immunized animals will then be boosted with the same antigen formulation after one week (day 7). Blood samples will be withdrawn on day 0, 7 and 21, either before or during immunization for each animal, blood cells, and clotting factors will be removed and the serum examined for immune-reactivity to purified recombinant staphylococcal antigen using an ELISA based assay. The data output of these experiments are the relative levels of IgG or IgM antibodies that bind to each antigen, thereby documenting a successful immunization. Groups of 5 mice from immunized and control animals will be challenged on day 21 after the first immunization. Animals will be euthanized 96 h post-infection, and bacterial counts will be performed on their kidneys, livers, and peritoneal lavage fluids. These experiments will allow us to measure whether specific EsxA and/or EsxB antibodies induced by active immunization confer protection against *S*. *aureus* disease.

### EXAMPLE 4

### Esxa And Esxb Are Secreted By An Esat-6-Like System

### A. Materials and Methods

***Strains, Media, and Growth Conditions.** Escherichia coli* and *S. aureus* were grown in Luria-Bertani broth and tryptic soy broth at 37°C, respectively. Ampicillin and erythromycin were used at 100 mg/liter and 10 mg/liter, respectively. essABC, esaAB, and esxB mutants were obtained from the Phoenix (ΦN 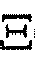 ) library (Bae *et al.,* 2004) (Table 2). Each Phoenix isolate is a derivative of the clinical isolate Newman (Kuroda *et al.,* 2001) and carries the transposon *bursa aurealis* at a site on the chromosome that has been determined by DNA sequencing (Bae *et al.,* 2004) and compared to *S*. *aureus* Mu50 genomic sequence (Kuroda *et al.,* 2001). All *bursa aurealis* insertions were transduced into wild-type *S*. *aureus* Newman by using bacteriophage φ85 (Mazmanian *et al.,* 2000).

***Culture Fractionation.*** Cultures were grown to an optical density of 0.8 at 660 nm (OD660), and 1.5 ml of culture was centrifuged at 10,000 x g for 4 min. Proteins in 1 ml of supernatant were precipitated with 7.5% trichloroacetic acid (TCA), using deoxycholic acid (0.2%) as a carrier, and sedimented by centrifugation 10,000 x g for 10 min (medium fraction). Culture (1.5 ml) was incubated in the presence of lysostaphin (100 µg/ml) for 30 min at 37°C. A 1-ml aliquot was precipitated with TCA (total culture). For extraction of proteins that are associated with the cell wall (loosely associated fraction), the cells of 1.5 ml of culture were washed three times with an equal volume of TSM buffer (50 mM Tris-HCl, pH 7.5 / 0.5 M sucrose / 10 mM MgCl₂).

A 1-ml cell suspension was transferred to a fresh tube and precipitated with TCA. All TCA precipitates were washed with ice-cold acetone, solubilized in 50 µl of 0.5 M Tris-HCl (pH 8.0) / 4% SDS and heated at 90°C for 10 min. Proteins were separated on SDS-PAGE and transferred to poly(vinylidene difluoride) membrane for immunoblot analysis with appropriate polyclonal antibodies. Immunoreactive signals were revealed by using a secondary antibody coupled to horseradish peroxidase and chemiluminescence.

***Staphylococcal Fractionation.*** Cultures were centrifuged as described above and supernatants TCA precipitated in the presence of deoxycholic acid (medium). Cell pellets were suspended in 1 ml TSM buffer containing 100 µg/ml lystostaphin and incubated at 37°C for 30 min. Protoplasts were collected by centrifugation at 10,000 x g for 10 min, and the supernatant (cell wall fraction) was precipitated with TCA.

The protoplasts were suspended in membrane buffer (0.1 M Tris-HCl, pH 7.5 / 0.1 M NaCl / 10 mM MgCl₂) and subjected to five rounds of freeze-thaw in a dry ice ethanol bath. Soluble proteins (cytoplasmic fraction) were separated from insoluble materials and membranes (membrane fraction) by centrifugation at 100,000 x g for 30 min. All samples were TCA-precipitated before immunoblotting.

***Murine Abscess Model.** S. aureus* strains were grown at 37°C overnight in tryptic soy broth, diluted 100-fold in fresh broth and incubated at 37°C until an OD660 of 0.4 was reached. Cells were washed and suspended in PBS, and 100 µl of bacterial suspension was injected intravenously into 10 6- to 8-week-old female BALB-c mice anesthetized by intraperitoneal injection of 100 mg/ml ketamine and 2 mg/ml xylazine. Viable staphylococci were enumerated by colony formation on tryptic soy agar to quantify the infection dose [∼10⁶ colony forming units (cfu)/ml]. Four days after challenge, mice were killed by CO₂ asphyxiation. Spleen, kidneys, and liver were removed, and organs were homogenized in 1 ml of 1% Triton X-100 in PBS. Dilutions of the homogenates were plated on agar for enumeration of viable staphylococci. The statistical analysis of the data were performed with Student's t test using ANALYZE-IT (Analyze-It Software, Leeds, England).

### B. Results

***A Cluster of S. aureus Genes Encoding ESAT-6-Like Proteins.** S. aureus* EsxA and EsxB correspond to the Mu50 ORFs SAV0282 and SAV0290 (Kuroda *et al.,* 2001) and display 20.8% and 17.8% identity as well as 25% and 35% similarity to *M. tuberculosis* EsxA, respectively (FIG. 1A) The peptide sequences of *S*. *aureus* EsxA and EsxB encompass the WXG motif, a signature sequence of ESAT-6-like proteins (Pallen *et al.,* 2002) (FIG. 1A). EsxA and EsxB are encoded within a cluster comprised of eight predicted ORFs (*esxA, esaA, essA, esaB, essB, essC, esaC,* and *esxB*) as shown in FIG. 1B (esa, ESAT-6 secretion accessory gene). Beside *esxA* and *esxB,* the other genes in this cluster appear to be unique in the chromosome. The cluster is referred to as the Ess cluster. Genes located immediately downstream of *esxB* are not depicted in FIG. 1B. However, their conservation in other related clusters from low G+C organisms suggest that they may also be functionally associated with the Ess cluster. The deduced peptide sequence of one of these genes, *essC,* displays similarity with FSD proteins of the *M. tuberculosis* RD1 cluster (FIG. 1B). Because of a sequencing error, *essC* entry in the Mu50 genome appears as two ORFs (SAV0287-SAV0288) when it is, in fact, a single combined ORF (data not shown). FIG. 1C shows the membrane topology of four proteins, EsaA, EssA, EssB, and EssC, as predicted by the TMHMM algorithm (www.cbs.dtu.dk/services/TMHMM-2.0) and PSORT-B (Gardy *et al.,* 2003). Four other factors (EsxA, EsxB, EsaB, and EsaC) are predicted by the same computational methods to be soluble, and not membrane associated. A summary of the relevant features of proteins encoded by the ess locus is presented in Table 3.

**Table 3. S. aureus strains used in this study**

| Strain | Genotype | *bursa aurealis* insertion/mutation | | | Phenotvpe | |
|---|---|---|---|---|---|---|
| | | nt position | AA position | Orientation | EsxA secretion | EsxB secretion |
| Newman | Clinical isolate | | | | Yes | Yes |
| ΦNΞ**187-01** | ***esxA24*** | **24** | **8** | **NA** | **No** | **No** |
| ΦNΞ**09349** | ***esaA::erm*** | **1110** | **370** | **(+)** | **Yes** | **Yes** |
| ΦNΞ**03742** | ***essA::erm*** | **121** | **41** | **(-)** | **No** | **No** |
| ΦNΞ**13152** | ***esaB::erm*** | **43** | **15** | **(+)** | **Yes** | **Yes** |
| ΦNΞ**02411** | ***essB::erm*** | **199** | **67** | **(+)** | **No** | **No** |
| ΦNΞ**04464** | ***essC::erm*** | **792** | **264** | **(-)** | **No** | **No** |
| ΦNΞ**02191** | ***essC::erm*** | **1854** | **618** | **(-)** | **No** | **No** |
| ΦNΞ**02832** | ***essC::erm*** | **2161** | **721** | **(+)** | **Low** | **Low** |
| ΦNΞ**13038** | ***essC::erm*** | **3835** | **1,279** | **(+)** | **Yes** | **Yes** |
| ΦNΞ**07912** | ***esxB::erm*** | **2** | **1** | **(+)** | **No** | **No** |
| **All Phoenix strains (ΦNΞ) are isogenic to *S*. *aureus* Newman (Duthie and Lorenz, 1952) and are part of inventor's laboratory collection (Bae *et al.,* 2004). In strain *esxA24,* a stop codon replaces the coding codon at position 8. AA, amino acid; *erm,* erythromycin resistance gene; nt, nucleotide.** | | | | | | |

***EsxA and EsxB Are Secreted into the Culture Medium of S. aureus.*** To study the location of EsxA and EsxB, cultures of *S*. *aureus* strain Newman were centrifuged and bacterial cells in the sediment were separated from the supernatant containing conditioned culture medium. Staphylococci were suspended in buffer and the cell wall was digested with lysostaphin, an endopeptidase that cleaves staphylococcal peptidoglycan (Schindler and Schuhardt, 1964). Proteins in the total lysate of staphylococci (T) or the culture medium (MD) were precipitated with TCA before separation on SDS-PAGE and immunoblotting. EsxA and EsxB were detected with specific rabbit antisera and were found in the total lysate and in the culture medium, indicating that *S*. *aureus* strain Newman secretes both polypeptides across the bacterial envelope (FIG. 2A).

As a control, *S*. *aureus* IsdG, a cytoplasmic protein, and IsdE, a membrane lipoprotein, were found in total lysates but not in the bacterial culture medium (FIG. 2A) (Jonsson *et al.,* 2003). As a control for polypeptides that are covalently anchored to the cell wall, staphylococcal protein A was examined (Schneewind *et al.,* 1995). Cell wall degradation products, *i.e*., peptidoglycan fragments with linked polypeptide, are being released from the staphylococcal surface, and some protein A can therefore be found in both the total lysate and culture medium (Navarre and Schneewind, 1999) (FIG. 2A). Staphylococci were harvested from cultures by centrifugation and were boiled in hot SDS to release polypeptides that are only loosely associated with the cell wall (L). Boiling in hot SDS released small amounts of protein A, but not IsdE and IsdG. This is an expected result as the cell wall of staphylococci cannot be disintegrated by boiling in ionic detergents (Schneewind *et al.,* 1992). EsxA and EsxB were not found to be associated with the staphylococcal surface after boiling bacteria in hot SDS, suggesting that staphylococci ESAT-6-like proteins are soluble in the extracellular milieu.

***Subcellular Localization of EsxA and EsxB.*** To examine the subcellular localization of EsxA and EsxB in staphylococci, cultures of *S*. *aureus* strain Newman were separated into cytoplasm, membrane, cell wall, and medium fractions (FIG. 2B). Proteins in all fractions were revealed by immunoblotting with specific antibodies. EsxA and EsxB were found exclusively in the culture medium. As a control, protein A was detected in the cell wall fraction, whereas IsdG and IsdE resided in the cytoplasm and the plasma membrane or cell wall of staphylococci, respectively (FIG. 2B). Together, these results demonstrate that *S*. *aureus* strain Newman secretes EsxA and EsxB across the bacterial envelope into the culture medium.

***Factors Affecting the Production and Secretion of EsxA and EsxB.*** Previous work from the inventors' laboratory generated the Phoenix (ΦN 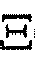 ) library, a collection of *bursa aurealis* insertion mutants in *S*. *aureus* strain Newman that have been mapped by inverted PCR and DNA sequencing (Bae *et al.,* 2004). *S. aureus* strain Newman variants ΦN 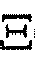 09349, ΦN 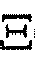 03742, ΦN 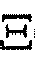 13152, ΦN 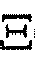 02411, ΦN 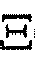 04464, and ΦN 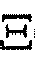 07912 carry *bursa aurealis* insertions in *esaA, essA, esaB, essB, essC,* or *esxB,* respectively (Table 3 and FIG. 3A). *S. aureus* Newman variant ΦN 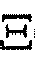 187-01 carries a stop codon at position 8 of *esxA* coding sequence. The ability of these mutant strains to synthesize and secrete EsxA and EsxB was examined by immunoblotting of culture medium and total lysostaphin lysate samples as described in the legend of FIG. 2A.

*Bursa aurealis* insertions at nucleotide positions 1,110 (codon 370) of *esaA,* or 43 (codon 15) of *esaB* did not affect the production or secretion of EsxA and EsxB when examined by immunoblot analysis (FIG. 3B). However, *bursa aurealis* insertions at nucleotide positions 121 (codon 41) of *essA,* 328 (codon 110) of *essB,* or 792 (codon 264) of *essC* abolished production and thus secretion of both EsxA and EsxB polypeptides as immunoreactive species could not be detected in either the medium or total culture lysate of strains ΦN 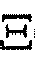 03742, ΦN 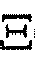 02411, and ΦN 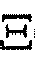 04464, respectively (FIG. 3B). One *bursa aurealis* insertion in the Phoenix library mapped to nucleotide 2 (codon 1) of *esxB* (mutant ΦN 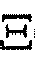 07912), a mutation that abolished the expression of all EsxB immunoreactive species (FIG. 3B). The inability of strain ΦN 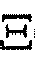 07912 (*esxB::erm*) to produce EsxB abolished all EsxA production and, therefore, secretion. Conversely, the inability of strain ΦN 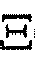 187-01 (*esxA24*) to produce EsxA abolished all EsxB production as well (FIG. 3B). None of the mutants used here abolished the transcription of either *esxA* or *esxB* as judged by RT-PCR, suggesting that the phenotypes observed were not caused by polar effects of mutations or to transcriptional down-regulation.

EsaA is a 1,009-aa polytopic membrane protein with six predicted transmembrane helices that is conserved in staphylococcal and listerial species but absent from mycobacteria (FIG. 1B). *essA* and *esaB* specify 152- and 80-aa long protein products, respectively. Bioinformatic prediction of subcellular localization suggests that EssA contains one transmembrane domain, whereas EsaB may be soluble as it lacks a canonical signal peptide. EssB and EsaB share sequence homology with listerial genes located in a similar ess cluster, and with *B. subtilis* YukC and YukD, respectively (Oudega *et al.,* 1997). The function of YukC and YukD is unknown, but their corresponding genes map to a chromosomal locus with *yukA, yukB,* and *yukE* (Oudega *et al.,* 1997) (FIG. 1B). YukE bears the WXG motif typical of ESAT-6-like proteins.

EssC is homologous to two genes of *M. tuberculosis,* Rv3870 (*snm1*) and Rv3871 (*snm2*), and to *B. subtilis* YukA-YukB. Recent sequence analysis revealed that *yukA-yukB* represents a single ORF (Sao-Jose *et al.,* 2004). EssC, Rv3870, Rv3871, and YukA sequences encompass one or more FSDs (Pallen *et al.,* 2002). Gene SA0276 (*essC,* in the N315 genome) is annotated as "conserved hypothetical protein, similar to diarrheal toxin." The annotation derives from similarity to the *Bacillus cereus* gene *bceT* and appears to be erroneous (Hansen *et al.,* 2003). Bioinformatic analysis predicted two transmembrane helices at the N terminus of the 1,479-aa EssC polypeptide (FIG. 1C). The N and C termini of EssC are presumed to reside in the staphylococcal cytoplasm based on the assumption that the FSDs hydrolyze ATP in this compartment.

***Phenotypic Analysis of Various Insertion Mutants in essC.*** The Phoenix library contains several strains carrying *bursa aurealis* insertions in the *essC* gene (Table 3 and FIG. 4A). Four *essC* mutants were used to analyze the effect of EssC truncations on the secretion of EsxA and EsxB (FIG. 4B). strain ΦN 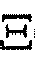 04464 carries a transposon insertion at nucleotide 792 (codon 264, mutant 1), whereas strains ΦN 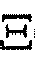 02191, ΦN 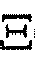 02832, and ΦN 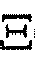 13038 carry *bursa aurealis* insertions at nucleotide positions 1854 (codon 618, mutant 2), 2161 (codon 721, mutant 3), and 3835 (codon 1,279, mutant 4), respectively (FIG. 4A). None of these transposon insertions affected the transcription of the two genes downstream (data not shown). Transposon insertion at codons 264 and 618 of *essC* abolished all production and secretion of EsxA and EsxB. In contrast, the synthesis and secretion of EsxB or EsxA occurred when *bursa aurealis* inserted at codons 721 and 1279 (FIG. 4B). These results imply that a single FSD domain is sufficient for EssC activity, and suggest that EsxA and EsxB secretion may be fueled by EssC mediated ATP hydrolysis.

***esxA, esxB, and essC Mutants Are Defective in the Pathogenesis of Staphylococcal Abscesses.** S. aureus* host infection and dissemination within organ tissues depends on staphylococcal synthesis and secretion of a wide variety of virulence factors (Novick, 2003). Because of the astonishing armament of staphylococci with virulence factors, the pathogenesis of *S*. *aureus* infections is considered multifactorial (Novick, 2003). Thus, with the exception of α-hemolysin (*hla*) mutants (Bhakdi and Tranum-Jensen, 1991), strains carrying mutations that abrogate the expression of individual exoproteins typically do not display significant defects in the pathogenesis of *S*. *aureus* infections (O'Reilly *et al.,* 1986; Jonsson *et al.,* 1985; Patel *et al.,* 1987). *S. aureus* Newman variants ΦN 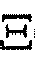 187-01 (*esxA24*), ΦN 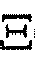 07912 (*esxB::erm*), and ΦN 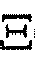 04464 (*essC::erm*) do not synthesize EsxA and EsxB. These strains were chosen for experimental measurement of bacterial virulence. Viable staphylococci of mutant or wild-type Newman strains (∼10⁶ staphylococci) were administered intravenously via retro-orbital injection into mice. The animals were killed 4 days after infection. Internal organs were removed, inspected for abscess formation, and then homogenized and spread on agar medium to quantify staphylococcal replication in host tissues by colony formation (Albus *et al.,* 1991). Mutations in *esxA, esxB,* and *essC* genes caused a significant reduction in the ability of *S. aureus* Newman to establish kidney or liver abscesses in infected mice (FIG. 5 and data not shown). Kidneys of mice infected with Newman carried a mean value of 3.89 x 10⁷ cfu per organ, whereas those of mice infected with strains lacking *esxA, esxB,* or *essC* harbored a mean value of 3.00 x 10⁴ cfu per organ (P < 2.09 x 10⁻⁵), 6.17 x 10⁵ cfu per organ (P < 1.2 x 10⁻²), and 2.51 x 10⁵ cfu per organ (P < 5.53 x 10⁻³), respectively. It should be noted that half of the animals infected with mutant bacteria had cleared most staphylococci from the kidneys after 4 days, suggesting that complete clearing may occur by day 5 after infection with the mutant strains. The formation of liver abscesses in infected animals was even more affected when staphylococci did not produce EsxA and EsxB. Homogenized livers of mice infected with Newman were found to contain a mean value of 2.09 x 10⁶ cfu per organ of staphylococci, whereas the livers of mutants lacking *esxA, esxB,* or *essC* contained a mean value of 9 x 10¹ cfu per organ (P < 3.01 x to⁻⁷), 4.17 x 10² cfu per organ (P < 4.33 x 10⁻⁶) , and 1.35 x 10³ cfu per organ (P < 1.72 x 10⁻⁴), respectively.

### EXAMPLE 5

### Staphylococcus aureus Vaccine Assembled From Surface Protein Antigens

### A. Materials and Methods

**Cloning, Expression and Purification** -- Surface antigens were amplified via PCR from *S*. *aureus* strain N315. Primers were designed excluding the predicted signal peptide sequence and sortase recognition motif. PCR products were cloned into either pET-15b (Novagen) or pGEX-2T to yield recombinant proteins with either a 6-His or glutathione-S-transferase (GST) tag, respectively.

**Animal Immunization and Challenge.** Groups of 25-day-old female BALB/c mice (nine or ten mice per group, Charles River Labs, MA) were immunized by intramuscular injection into the hind leg with purified protein. These proteins (100 µg of each) were administered to mice on days 0 (adsorbed to Complete Freund's adjuvant, CFA) and 11 (adsorbed to Incomplete Freund's adjuvant, IFA). Blood samples were withdrawn via retro-orbital bleeding on days 0, 11 and 20. Following blood clotting, sera were retrieved and used for measurements of antibody production. Immunized animals were challenged on day 21 by retro-orbital injection of 100 µl bacterial suspension (∼10⁶ CFU). For these experiments, *S*. *aureus* strain Newman was grown overnight in tryptic soy broth (TSB) at 37°C, diluted 1:100 in fresh TSB, and grown for 3 h at 37°C. The staphylococci were washed twice and diluted in sterile PBS to yield an OD₆₀₀ of 0.4. The infection inoculum was verified by a plate count. Mice were anaesthetized for this procedure using an intraperitoneal injection of 80-120 mg/kg ketamine and 3-6 mg/kg xylazine. On day 25, mice were euthanized by a compressed CO₂ overdose and their kidneys were excised. The organs were homogenized in 1% Triton X-100 and diluted and plated in triplicate for determination of CFU. Serum immunoglobulin G (IgG) levels with specific antigen binding activity were determined by enzyme-linked immunosorbent assay (ELISA) at the GLRCE Immunology Core, University of Chicago. All animal experiments were performed in accordance with institutional guidelines following experimental protocol review and approval by the Institutional Animal Care and Use Committee.

### B. Results

The pathogenesis of staphylococcal infections relies on many different virulence factors such as secreted exotoxins, exopolysaccharides, and surface adhesins. However, deletion of single genes encoding such factors causes either no defect or results in only modest reduction of virulence. The development of staphylococcal vaccines is hindered by the multifaceted nature of staphylococcal invasion strategies. Previous work demonstrated that immunization of mice with capsular polysaccharide, poly-N-acetylglucosamine (PNAG) exopolysacchride or the surface protein clumping factor A (ClfA) afforded partial protection against staphylococcal replication and disease in murine models of infection. Staphylococci rely on surface protein mediated-adhesion to host cells or invasion of tissues as a strategy for escape from immune defenses. Furthermore, *S*. *aureus* utilize surface proteins to sequester iron from the host during infection. The majority of surface proteins involved in staphylococcal pathogenesis carry C-terminal sorting signals, *i.e*., they are covalently linked to the cell wall envelope by sortase. Further, staphylococcal strains lacking the genes required for surface protein anchoring, *i.e*., sortase A and B, display a dramatic defect in the virulence of several different mouse models of disease. Thus, surface protein antigens represent a validated vaccine target as the corresponding genes are essential for the development of staphylococcal disease.

Using cell wall sorting signals as queries for BLAST searches and previous genome analysis, 22 surface protein genes were identified in six staphylococcal strains. Large portions of N-terminal, surface exposed amino acid sequences of 20 different surface proteins were expressed in *Escherichia coli* and recombinant proteins purified by affinity chromatography. Antigen, 100 µg purified, recombinant protein emulsified with complete Freund's adjuvant, was injected into muscle tissue of the hind leg of 25-day-old female BALB/c mice on day 0. Immune responses of animals were boosted by immunization with 100 µg antigen emulsified in incomplete Freund's adjuvant on day 11. Blood samples were withdrawn via retro-orbital bleeding on days 0, 11 and 20 following immunization and serum antibody titers to individual antigens determined by enzyme linked immuno-sorbent assay (ELISA). Animals were challenged on day 21 with intra-venous injection of 10⁶ colony forming units (cfu) of *S*. *aureus* strain Newman, a human clinical isolate. Four days following infection, animals were euthanized and organ tissues removed. The ability of staphylococci to seed abscesses in kidneys was assessed by macroscopic inspection and by plating tissue homogenate on agar media followed by colony formation and enumeration.

The results show that animals immunized with a buffer control were infected by staphylococci, harboring abscesses with approximately 1×10⁷ cfu of staphylococci in their kidneys (FIG. 8). None of the twenty recombinant surface proteins used as vaccine antigens generated immune responses that afforded complete protection against staphylococcal disease. However, the bacterial load in the kidneys of animals immunized with SdrD, SdrE, IsdA, and IsdB was reduced by three to four logs of cfu, respectively. Immunization with Spa, ClfA, ClfB, SdrC, IsdC or SasF afforded a two to three log reduction in the bacterial burden of kidney tissues, whereas immunization with other antigens generated very little or no vaccine protection.

### EXAMPLE 6

### Vaccine Assembly from Combinatins of Surface Proteins Of Staphylococcus

### A. Methods

*Bioinformatics.* Genome sequences of *S*. *aureus* strains were analyzed for surface proteins using sorting signals as queries in BLAST searches. Signal peptide cleavage sites were predicted with the SignalP 3.0 algorithm (Bendtsen *et al.,* 2004)..

*Bacterial Strains and Growth.* Staphylococci were cultured on tryptic soy agar or broth at 37°C. *S*. *aureus* NRS70 (N315), NRS123 (USA400/MW2), NRS248, NRS252 and NRS382 (USA100) were obtained through the Network on Antimicrobial Resistance in *Staphylococcus aureus* (NARSA, NIAID Contract No. N01-AI-95359). *S. aureus* SEJ2 is a *Δ*(*spa*) variant of strain Newman carrying a deletion of the protein A gene. *Escherichia coli* strains DH5α and BL21(DE3) were cultured on Luria agar or broth at 37°C. Carbenicillin (50 µg/mL) and erythromycin (10 µg/mL) were used for plasmid selection.

*Cloning and Purification.* Coding sequences for surface proteins excluding the signal peptide and sorting signal were PCR amplified using *S*. *aureus* N315 template DNA (see Table 7 for a listing of primer sequences). PCR products were cloned into either pET-15b or pGEX-2T to express recombinant proteins with N-terminal six histidyl tag or glutathione-S-transferase (GST) fusion, respectively. Following bacterial disintegration, proteins were affinity purified from cleared lysates using Ni-NTA or glutathione sepharose. Protein eluate was subjected to endotoxin removal by Triton X-114 phase separation, surface proteins were purified further by gel filtration and protein concentration determined.

*Immunization.* BALB/c mice (24-day-old female, eight to ten mice per group, Charles River Labs, MA) were immunized by intramuscular injection into the hind leg with purified protein. Proteins (100 µg of each) were administered on days 0 (emulsified 1:1 with CFA) and 11 (emulsified 1:1 with IFA). Pilot studies compared aluminum hydroxide, CFA, or IFA as possible adjuvants for surface protein immunization and eliminated aluminum hydroxide because of immune responses that interfere with murine abscess formation. Blood samples were drawn via retro-orbital bleeding on days 0, 11, and 20. Sera were examined by enzyme-linked immunosorbent assay (ELISA) for immunoglobulin G (IgG) titers with specific antigen binding activity. Animal experiments were performed in accordance with institutional guidelines following experimental protocol review and approval by the Institutional Animal Care and Use Committee.

*Renal Abscess.* Immunized animals were challenged on day 21 by retro-orbital injection of 100 µL bacterial suspension (3-5 x 10⁶ CFU). Overnight cultures of *S*. *aureus* Newman were diluted 1:100 into fresh TSB and grown for 3 hours at 37°C. Staphylococci were centrifuged, washed twice and diluted in PBS to yield an OD₆₀₀ of 0.4 (3-5 x 10⁷ CFU/mL). Further dilutions were needed for the desired inoculum, which was experimentally verified by agar plating and colony formation. Mice were anesthetized by intra-peritoneal injection of 80-120 mg/kg ketamine and 3-6 mg/kg xylazine. On day 25, mice were euthanized by compressed CO₂ inhalation. Kidneys were removed and homogenized in 1% Triton X-100, aliquots diluted and plated on agar media for triplicate determination of CFU. For histology, kidney tissue was incubated at room temperature in 10% formalin for 24 hours. Tissues were embedded in paraffin, thin sectioned, hematoxylin/eosin stained and examined by microscopy.

*Lethal Challenge.* Immunized animals were challenged on day 21 by intra-peritoneal injection with 2 x 10¹⁰ CFU of *S*. *aureus* Newman or 3-10 x 10⁹ CFU of clinical *S*. *aureus* isolates. Animals were monitored for seven days and lethal disease recorded.

*Opsonophagocytic Killing.* Polymorphonuclear leukocytes (PMNs) were isolated from healthy human volunteers. Cells were counted, examined for viability (trypan blue exclusion) and diluted to 2-5 x 10⁶ PMN/mL. To remove antibodies that react with the bacterial target, infant rabbit serum was pre-adsorbed with *S*. *aureus* SEJ2 suspensions by mixing at 4°C for 30 minutes. Serum was then centrifuged, filter sterilized and used as a source of complement. *S*. *aureus* SEJ2 was adjusted to 2-5 x 10⁵ CFU/mL. Rabbit antibodies to IsdA, IsdB, SdrD and SdrE were normalized to the same IgG titer. Equal volumes (100 µL) of PMNs, complement, bacteria, and diluted antibodies were mixed and incubated at 37°C for 90 minutes prior to dilution, agar plating and bacterial enumeration. The percent amount of bacterial killing was calculated as (1 - [the number of CFU recovered in the presence of PMNs / the number of CFU recovered in the absence of PMNs]) x 100.

*Statistical Analysis.* One-tailed Student's t tests were performed to analyze statistical significance of renal abscess data. Fisher's exact test was used to analyze statistical significance of the lethal challenge data.

### B. Results

*S. aureus,* a gram-positive bacterial commensal of human skin and nares, is the leading cause of bloodstream, lower respiratory tract and skin/soft tissue infections (Diekema *et al.,* 2001). The broad spectrum of important staphylococcal diseases also includes endocarditis, septic arthritis, toxic shock syndrome, scalded skin syndrome and food poisoning (Archer and Climo, 2001). *S. aureus* strains exhibiting multiple antibiotic resistances are isolated in about 60% of community and up to 80% of hospital infections (Kaplan *et al.,* 2005). For example, *S*. *aureus* strains with intermediate (VISA) or full resistance (VRSA) to vancomycin, which is considered the therapy of last resort for methicillin-resistant *S*. *aureus* (MRSA), have recently emerged (Weigel *et al.,* 2003).

Generation of protective immunity against invasive *S*. *aureus* disease has been a goal since the discovery of this microbe. Whole-cell live or killed vaccines largely fail to generate protective immune responses (Rogers and Melly, 1965). Purified capsular polysaccharides (CP), types 5 and 8 (which represent more than 80% of all capsular types found in clinical isolates (Arbeit *et al.,* 1984)), shows promise when used as a conjugate vaccine in experimental animals or in patients with end-stage renal disease (Fattom *et al.,* 1990; Fattom *et al.,* 2004). Immunization with PNAG, a *S. aureus* surface carbohydrate synthesized by *icaABC* products (Heilmann *et al.,* 1996), also protects mice against staphylococcal disease (McKenney *et al.,* 1999; Maira-Litran *et al.,* 2005). Subunit vaccines composed of individual surface proteins, for example clumping factor A (ClfA) (Josefsson *et al.,* 2001), clumping factor B (ClfB) (Schaffer *et al.,* 2006), iron-regulated surface determinant B (IsdB) (Kuklin *et al.,* 2006) or fibronectin-binding protein (FnBP) (Zhou *et al.,* 2006) generate immune responses that afford partial protection against *S*. *aureus* challenge of experimental animals. However, in order for a *S*. *aureus* vaccine to be successful, genetic determinants for specific antigens must be essential for staphylococcal virulence (Maira-Litran *et al.,* 2004). This has not been observed for mutants lacking the genetic determinants of CP (Albus *et al.,* 1991) or of individual surface proteins (Mazmanian *et al.,* 2001).

Rappuoli and colleagues exploited information encrypted in bacterial genome sequences to distinguish pan-genomes, *i.e*., genes in all strains of a pathogen, from conserved genes found in all members of its species (Medini *et al.,* 2005). Rational vaccine design was achieved by interrogating conserved antigens (secreted or surface displayed) for protective immunity, which led to the identification of multiple surface proteins of group B streptococci (GBS) as candidates (Maione *et al.,* 2005). By combining multiple antigens into a single vaccine, broad spectrum protective immunity against many different clinical isolates was achieved (Maione *et al.,* 2005).

The inventors contemplate a broad spectrum *S*. *aureus* vaccine derived from staphylococcal surface proteins for protective immunity. *S*. *aureus* sortase A (srtA) mutants, which cannot display surface proteins, are unable to establish infections in experimental models, indicating that the sum of all twenty-three surface proteins is essential for pathogenesis (Mazmanian *et al.,* 2000). Sortase A cleaves sorting signals of surface proteins and anchors polypeptides to the cell wall envelope (Mazmanian *et al.,* 2001). Eight staphylococcal genome sequences (Kuroda *et al.,* 2001; Holden *et al.,* 2004; Diep *et al.,* 2006) were examined for the presence of sortase substrate genes using sorting signals as queries in BLAST searches and nineteen conserved surface protein genes were identified (Table 4). These genes were expressed in *E*. *coli* as soluble His-tagged or glutathione S-transferase fusions and recombinant proteins purified by affinity chromatography. Groups of mice were immunized by intra-muscular injection with 100 µg purified protein emulsified in complete Freund's adjuvant (CFA) and eleven days later with protein emulsified in incomplete Freund's adjuvant (IFA). Blood samples were drawn before, during and after immunization and specific serum IgG levels determined by ELISA, demonstrating that surface proteins generated humoral immune responses to immunization (Table 5). Mice were challenged 21 days after the primary immunization with 3-5 x 10⁶ colony forming units (CFU) of *S. aureus* Newman (Duthie and Lorenz, 1952) via retro-orbital injection. Four days after infection, mice were killed, kidneys removed and the bacterial load in homogenized tissues measured (Albus *et al.,* 1991). When compared to mock immunized animals, some recombinant surface proteins generated specific immune responses that afforded partial protection against staphylococcal disease. Bacterial load in kidneys of animals immunized with ClfA, SdrD, SdrE, IsdA or IsdB was reduced by three to four logs, while immunization with Spa, ClfB, IsdC, SdrC, SasD or SasF afforded a two to three log reduction in staphylococcal burden. FnBPA, SasG or IsdH immunization generated even smaller reductions in bacterial load, while immunization with FnBPB, SasA, SasB, SasC or SasK did not result in significant protection (Table 5).

**Table 4. Sortase-anchored surface proteins of S. aureus**

| Surface Protein | AA | Ligand | MRSA 252 | MSSA 476 | Mu50 | MW2 | N315 | NCTC 8325 | Newman | USA 300 |
|---|---|---|---|---|---|---|---|---|---|---|
| Spa | 450 | IgC7,vWF | + | + | + | + | + | + | + | + |
| FnBPA | 1038 | Fibronectin | | + | + | + | + | + | + | + |
| FnBP | 961 | Fibronectin | + | + | + | + | + | + | + | + |
| ClFA | 989 | Fibrinogen | + | + | + | + | + | + | + | + |
| ClfB | 877 | Fibrinogen | + | + | + | + | + | + | + | + |
| SdrC | 953 | unknown | + | + | + | + | + | + | + | + |
| SdrD | 1347 | unknown | + | + | + | + | + | | + | + |
| SdrE | 1141 | unknown | + | + | + | + | + | + | + | + |
| Can | 1183 | Collagen | + | + | | + | | | | |
| PlS | 1166 | unknown | | | | | | | | |
| SasA | 2271 | unknown | + | + | + | + | + | + | + | + |
| SasB | 2481 | unknown | + | + | + | + | + | + | + | + |
| SasC | 2186 | unknown | + | + | + | + | + | + | + | + |
| SasD | 241 | unknown | + | + | + | + | + | | + | + |
| SasE/IsdA | 350 | Heme | + | + | + | + | + | + | + | + |
| SasF | 635 | unknown | + | + | + | + | + | + | + | + |
| SasG/Aap | 525 | unknown | + | + | + | + | + | + | | + |
| SasH Sasl/HarA/IsdH | 772 891 | unknown | + | + | + | + | + | + | + | + |
| SasJ/IsdB | 645 | Hemoglobin | + | + | + | + | + | + | + | + |
| SasK | 211 | unknown | | + | + | + | + | | | |
| SasL | 232 | unknown | | | + | | | | | |
| IsdC | 227 | Heme | + | + | + | + | + | + | + | + |
| Surface proteins were identified as genes harboring a C-terminal sorting signal. | | | | | | | | | | |
| AA, protein length in amino acids. | | | | | | | | | | |
| Motif, consensus motif recognized by sortase and present in C-terminal cell wall sorting signal. | | | | | | | | | | |
| Ligand, molecular component)s) recognized or bound by surface proteins. | | | | | | | | | | |

**Table 5 Immune protection against S. aureus abscess formation conferred by immunization of mice with surface protein**

| **Surface Protein** | **IgG Titer** | ***S. aureus* in Kidneys (log₁₀ CFU/mL)** | **Reduction of Staphylococci (log₁₀ CFU/mL)** | **Significance** |
|---|---|---|---|---|
| **Spa** | ND | 3.911 ±0.978 | 2.951 | *P* = 0.00541 |
| **FnBPA** | 18,000 ± 6,771 | 4.891 ± 0.922 | 1.971 | *P* = 0.02316 |
| **FnBPB** | 24,300 ± 3,600 | 6.172 ± 0.437 | 0.690 | *P* = 0.07434 |
| **ClfA** | 64,800 ± 9,920 | 3.521 ± 0.922 | 3.341 | *P* = 0.00361 |
| **ClfB** | 36,600 ± 12,247 | 4.308 ± 0.797 | 2.554 | *P* = 0.01012 |
| **SdrC** | 12,125 ± 3,770 | 4.026 ± 0.979 | 2.836 | *P* = 0.01012 |
| **SdrD** | 30,000 ± 12,920 | 3.477 ± 1.039 | 3.385 | *P* = 0.00613 |
| **SdrE** | 29,000 ± 6,878 | 2.565 ± 0.913 | 4.297 | *P* = 0.00068 |
| **SasA** | 8,500 ± 1,936 | 5.207 ± 1.048 | 1.655 | *P* = 0.06821 |
| **SasB** | 20,250 ± 6,037 | 5.709 ± 0.780 | 1.153 | *P* = 0.09094 |
| **SasC** | ND | 5.649 ± 0.781 | 1.213 | *P* = 0.09642 |
| **SasD** | 24,500 ± 1,342 | 4.675 ± 1.082 | 2.187 | *P* = 0.03782 |
| **IsdA/SasE** | 45,900 ± 9,156 | 2.518 ± 0.897 | 4.344 | *P* = 0.00057 |
| **SasF** | 16,900 ± 3,932 | 3.916 ± 0.781 | 2.946 | *P* = 0.00187 |
| **SasG/Aap** | 21,875 ± 2,900 | 5.384 ± 0.715 | 1.478 | *P* = 0.03597 |
| **HarA/IsdH/SasI** | 30,375 ± 15,093 | 4.918 ± 0.761 | 1.944 | *P* = 0.02239 |
| **IsdB/SasJ** | 36,300 ± 2,741 | 3.394 ± 0.982 | 3.468 | *P* = 0.00318 |
| **SasK** | 32,800 ± 12,659 | 5.898 ± 0.746 | 0.964 | *P* = 0.11032 |
| **IsdC** | ND | 3.779 ± 1.084 | 3.083 | *P* = 0.00996 |
| **PBS mock** | - | 6.862 ± 0.098 | - | - |
| **IgG titers [mean serum titers ± standard error of mean (SEM)] in response to immunization with surface proteins were determined by ELISA (n=5 animals).** | | | | |
| **Immunized mice challenged with *S. aureus* Newman (n=8-10 animals) and staphylococci in Kidney tissues were enumerated [log₁₀(CFU)/mL ± SEM].** | | | | |
| **Reduction of the number of staphylococci in kidney tissues (log₁₀(CFU)/mL) as a measure for protective immunity and compared to control mice immunized with phosphate buffered saline (PBS)(n=8-10 animals).** | | | | |
| **Statistical significance of reduction in staphylococcal burden was assessed with the one-tailed student's t test and *P* values recorded.** | | | | |
| **ND=not determined** | | | | |

Four surface protein vaccine candidates (IsdA, IsdB, SdrD and SdrE) generated the highest levels of protection against staphylococcal renal infection. To determine the nature of protection, antibodies against IsdA, IsdB, SdrD and SdrE were raised in rabbits and analyzed for their ability to induce opsonophagocytic killing of staphylococci in the presence of white blood cells, an important immunological correlate of protective immunity against S. *aureus* (Fattom *et al.,* 2004; Maira-Litran *et al.,* 2005). Freshly isolated human polymorphonuclear leukocytes (PMNs) were incubated with staphylococci in the presence of complement and specific antibodies. Following incubation, bacterial killing was monitored by spreading sample aliquots on agar media followed by colony formation and enumeration (FIG. 12). Antibodies against all four surface protein antigens induced opsonophagocytic killing of *S*. *aureus* (FIG. 12).

IsdA, IsdB, SdrD and SdrE, were assembled into a combined vaccine. To determine whether antibodies are generated against each of the four antigens in a combined vaccine, serum IgG titers of immunized mice were determined (Table 6). Antibody levels generated via the combined vaccine were similar to those achieved by immunization with individual surface protein antigens. Mice were challenged with 3-5 x 10⁶ CFU of *S*. *aureus* strain Newman. Four days following infection, animals were killed and kidneys removed. In contrast to immunization with individual antigens, the combined vaccine afforded complete protection against staphylococcal challenge [a reduction in bacterial load of 5.062 log₁₀ CFU/mL (P=0.00074) to a level below detection]. Histological analysis of kidney tissues failed to detect staphylococcal abscesses in mice that had been immunized with the combined vaccine (FIG. 9). In contrast, organs removed from mock-immunized animals harbored bacterial abscesses with central concentrations of staphylococci that were surrounded by a large cuff of white blood cells (FIG. 9). Kidney tissue of mice immunized with the combined vaccine revealed the physiological architecture of renal tubules as well as small infiltrates of PMNs, likely the anatomical substrate of opsonophagocytic clearance of staphylococci.

**Table 6. IgG titers for individual surface proteins and the combined vaccine (IsdA, IsdB, SdrD & SdrE)**

| **Surface Protein** | **Individual Immunization** | **Combined Vaccine** | **Significance** |
|---|---|---|---|
| **IsdA/SasE** | **27,000 ± 12,000** | **41,000 ± 14,000** | **0.26964** |
| **IsdB/SasJ** | **11,000 ± 2,449** | **28,000 ± 12,104** | **0.14330** |
| **SdrD** | **7,000 ± 2,000** | **9,000 ± 2,449** | **0.18695** |
| **SdrE** | **15,000 ± 3,162** | **17,000 ± 3,742** | **0.35200** |
| **IgG titers (mean serum titers ± standard error of mean) in response to immunization of mice as determined by ELISA (n=5 animals).** | | | |
| **Specific antibody titers raised against surface proteins were not significantly different when immunizing antigens were administered individually or in combination.** | | | |

**Table 7. Surface protein primers for cloning**

| **Gene** | **Sequence (SEQ ID NO.)** | **Term** | **R.E.** | **Vector** |
|---|---|---|---|---|
| Spa | gctgcaCATATGgcgcaacacgatgaagctcaac (SEQ ID NO:25.) | N | NdeI | pET15b |
| Spa | agtGGATCCttatgcttgagctttgttagcatctgc (SEQ ID NO.26) | C | BamHI | pET15b |
| FnBPA | aaagaaCATATGgcatcagaacaaaagacaactacag (SEQ ID NO.27) | N | NdeI | pET15b |
| FnBPA | tgaGGATCCttaggactcagtgtatcctccaacg (SEQ ID NO28.) | C | BamHI | pET15b |
| FnBPB | gaaCTCGAGgcatcggaacaaaacaatactacag (SEQ ID NO.29) | N | XhoI | pET15b |
| FnBPB | aatGGATCCttaatcatttggtttatctttaccatcg (SEQ ID NO.30) | C | BamHI | pET15b |
| ClfA | gaagcaCATATGagtgaaaatagtgttacgcaatc (SEQ ID NO.31) | N | Ndel | pET15b |
| ClfA | agaGGATCCttagttattaaatgctacttcgttgtc (SEQ ID NO.32) | C | BamHI | pETi 5b |
| ClfB | gcaCTCGAGtcagaacaatcgaacgatacaacg (SEQ ID NO.33) | N | XhoI | pET15b |
| ClfB | tggGGATCCttaatttactgctgaatcaccatcag (SEQ ID NO.34) | C | BamHI | pET15b |
| SdrC | gaagctCATATGgcagaacatacgaatggagaattaaat c (SEQ ID NO.35) | N | NdeI | pET15b |
| SdrC | ttcGGATCCttaattatcaagtgtgaaatcatcatgatc (SEQ ID NO.36) | C | BamHI | pET15b |
| SdrD | gcaCTCGAGgcagaaagtactaataaagaattgaacg (SEQ ID NO.37) | N | XhoI | pET15b |
| SdrD | ttcGGATCCttatttataagttccattttctaatcc (SEQ ID NO.38) | C | BamHI | pET15b |
| SdrE | gaagctCATATGgctgaaaacactagtacagaaaatg (SEQ ID NO.39) | N | NdeI | pET15b |
| SdrE | ttcGGATCCttagttatcaagtgtgaaatcatcatg (SEQ ID NO.40) | C | BamHI | pET15b |
| SasA | caagctCATATGgcttctgatgcaccattaacttct (SEQ ID N0.41) | N | NdeI | pET15b |
| SasA | atcGGATCCgctatttcttgttacttcatatttaaaagt (SEQ ID NO.42) | C | BamHI | pET15b |
| SasB | gcgAGATCTgcagagcaaaatcagcctgcac (SEQ ID NO.43) | N | BglII | pGEX-2T |
| SasB | atcGAATTCttaagttgtggcagcttgcacttga (SEQ ID NO.44) | C | EcoRI | pGEX-2T |
| SasC | gcaGGATCCttaactacggataataatgtacaaag (SEQ ID NO.45) | N | BamHI | pGEX-2T |
| SasC | tatGAATTCttaagctttatcatttacagcattgcg (SEQ ID NO.46) | C | EcoRI-9 | pGEX-2T |
| SasD | gcgCTCGAGgacacgacttcaatgaatgtgc (SEQ ID NO.47) | N | XhoI | pET15b |
| SasD | AgcGGATCCttaaacttttttgttactttggttcatttg (SEQ ID NO.48) | C | BamHI | pET15b |
| SasF | GccGGATCCgcatctgaaaaggatactgaaatttc (SEQ ID NO.49) | N | BamHI | pGEX-2T |
| SasF | tttGAATTCttatacattaccatcagcatttaataatc (SEQ ID NO.50) | C | EcoRI | pGEX-2T |
| SasG/Aap | gaagcaCATATGgctgaaaacaatattgagaatccaac (SEQ ID NO.51) | N | Ndel | pET15b |
| SasG/Aap | tgtGGATCCttatttttgtccttctcttgttactttttc (SEQ ID NO.52) | C | BamHI | pET15b |
| HarA/IsdH/Sasl | gcgCTCGAGgcagaaaatacaaatacttcagataaaatc (SEQ ID NO.53) | N | XhoI | pET15b |
| HarA/IsdH/Sasl | agtGAATTCttacattttagattgactaagtttgttttc (SEQ ID NO.54) | C | EcoRI | pET15b |
| SasK | aatgcgCATATGgaaaataacaaacctgaaggtgtg (SEQ ID NO.55) | N | NdeI | pET15b |
| SasK | tgtGGATCCttattctttcaattgttgcttatgtactg (SEQ ID NO.56) | C | BamHI | pET15b |
| IsdA/SasE, IsdB/SasJ and IsdC have been previously described (Mazmanian *et al*., 2003). Restriction enzyme (R.E.) sites are capitalized. | | | | |

To study the protection afforded by a combined vaccine against lethal infections, mice were immunized with the combined vaccine or with individual purified surface antigens (IsdA, IsdB, SdrD and SdrE). Challenges of 2 x 10¹⁰ CFU of *S*. *aureus* Newman were administered intra-peritoneally and mice were monitored for 7 days. FIG. 10 shows that immunization with individual surface proteins had either no effect on survival or afforded only very modest protection (FIG. 10), as already reported for purified IsdB (Kuklin *et al.,* 2006). In contrast to individual protein antigens, immunization with the combined vaccine afforded complete protection against a lethal challenge with one LD₅₀ unit of *S*. *aureus* Newman (P < 0.03) (FIG. 10). These results suggest that immunization with the combined vaccine can generate increased protection against lethal challenge with a strain expressing all four surface proteins.

In order for staphylococcal vaccines to be effective, protection must be achieved against a wide variety of different strains. By first eliminating surface proteins that are not conserved amongst staphylococcal strains, the inventors aim at developing a vaccine that may be effective against a wide range of *S*. *aureus* isolates. Five *S*. *aureus* strains were selected for assessment. NRS252 is a methicillin-sensitive *S. aureus* (MSSA) strain associated with toxic shock syndrome following burn injury, whereas N315, NRS248, USA100 and USA400 are MRSA strains (Kuroda *et al.,* 2001; McDougal *et al.,* 2003; Baba *et al.,* 2002). NRS248 is the causative agent of necrotizing pneumonia. USA400 carries the Panton-Valentine leukocidin genes associated with lethal lung infections (Gillet *et al.,* 2002). USA100 is the most frequent cause of healthcare-associated infections in the United States and protection against this strain is of crucial importance for vaccine efforts (McDougal *et al.,* 2003). Mice were immunized with the combined vaccine or mock immunized and then challenged by intra-peritoneal injection of *S*. *aureus* suspensions harboring 3-10 x 10⁹ CFU. Survival analysis revealed that the combined vaccine afforded significant protection against lethal challenge with any one of the five clinical *S*. *aureus* isolates (FIG. 11).

The data indicate that a combined vaccine of *S*. *aureus* surface antigens derived from conserved genome sequences can elicit immune responses that achieve greater protective immunity than immunization with its individual components. Immunization with four surface proteins, IsdA, IsdB, SdrD and SdrE, generated the highest level of protection in mice as compared to fifteen other antigens. Three of the four antigens in the combined vaccine are already known to be immunogenic in humans, as antibodies against IsdB, SdrD and SdrE can be found in healthy individuals or in patients with *S*. *aureus* disease (Dryla *et al.,* 2005).

### EXAMPLE 7

### Staphylococcal Infection of Mouse Animal Model

Groups of five 3 week-old mice were infected with various staphylococcal isolates (10⁶ cfu/ml suspended in PBS and administered IV in a 0.1 ml volume) or PBS (as a control for uninfected animals). Twenty-one days following infection, the animals were bled and killed. The presence of anti-EsxA (SAV0282), anti-EsxB (SAV0290), anti-EsaC (SAV0289) IgGs in the sera was tested in an Elisa assay (FIG. 13).

The results of immunization studies using recombinant EsxA and EsxB are summarized in Table 8. For these experiments, mice (groups of 10; 3 week-old)) were immunized with 100 µl of recombinant antigen EsxA or EsxB mixed with complete freund adjuvant (day 0), or incomplete freund adjuvant (day 11). Mice were bled on day 0, 11 and 21. IgG titers are shown for day 21 only (Table 8). On day 21, the animals were challenged with 5 x 10⁶ cfu of strain Newman (suspended in PBS and administered IV in a 0.1 ml volume). On day 25, animals were killed and colony forming units in organs were measured by plating ground organs on agar plates and counting colonies after overnight growth at 37°C. The Average CFU recovered for each kidney is shown in Table 8. The protection conferred upon immunization is measured as *Reduction* in CFU and calculated as the ratio of CFU for animals immunized with PBS (no protein) over CFU for animals immunized with recombinant antigen.

Patients infected with staphylococci generate antibodies against EsxA and EsxB. Fig. 14 illustrates the serum IgG titers of patients infected with *S*. *aureus.* Sera of two non infected individuals were used as a control. Various dilutions of sera were analyzed for EsxA and EsxB specific IgG by custom ELISA.

**Table 8. Immunization with recombinant EsxA and EsxB**

| **Protein** | **Avg log₁₀GFU/ml** | **Reduction** | **Pvalue** | **IgG Titer** |
|---|---|---|---|---|
| ― | 6.21 | 0.00 | | |
| EsxA | 3.85 | 233 | 2.8 v 10⁻¹ | 50000 |
| ― | 6.51 | 0.00 | | |
| EsxB | 4.27 | 2.24 | 2.2 x 10-3 | 75000 |
| EsxAB | 6.35 | 1.16 | 1.9 x 10 | 10000(EsxA) 75000 (EsxB) |
| - | 6.33 | 0.00 | | ― |
| EsxA | 4.31 | 2.05 | 29 x 10-3 | 40500 |
| EsxB | 3.77 | 2.59 | 1.0 x 10-3 | 81000 |
| EsxAB | 4.93 | 1.43 | 5.1 x 10-3 | 18000 (EsxA) 81000 (EsxB) |

All of the compositions and methods disclosed and claimed herein can be made and executed without undue experimentation in light of the present disclosure. While the compositions and methods of this invention have been described in terms of preferred embodiments, it will be apparent to those of skill in the art that variations may be applied to the fusion proteins, compositions, and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

### REFERENCES

The following references, to the extent that they provide exemplary procedural or other details supplementary to those set forth herein, are specifically incorporated herein by reference.
U.S. Patent 3,791,932
U.S. Patent 3,949,064
U.S. Patent 4,174,384
U.S. Patent 4,338,298
U.S. Patent 4,367,110
U.S. Patent 4,452,901
U.S. Patent 4,554,101
U.S. Patent 4,578,770
U.S. Patent 4,596,792
U.S. Patent 4,599,230
U.S. Patent 4,599,231
U.S. Patent 4,601,903
U.S. Patent 4,608,251
U.S. Patent 4,683,195
U.S. Patent 4,683,202
U.S. Patent 4,683,202
U.S. Patent 4,684,611
U.S. Patent 4,690,915
U.S. Patent 4,748,018
U.S. Patent 4,800,159
U.S. Patent 4,879,23
U.S. Patent 4,952,500
U.S. Patent 5,084,269
U.S. Patent 5,199,942
U.S. Patent 5,221,605
U.S. Patent 5,238,808
U.S. Patent 5,302,523
U.S. Patent 5,310,687
U.S. Patent 5,322,783
U.S. Patent 5,360,897
U.S. Patent 5,378,814
U.S. Patent 5,384,253
U.S. Patent 5,464,765
U.S. Patent 5,512,282
U.S. Patent 5,538,877
U.S. Patent 5,538,880
U.S. Patent 5,548,066
U.S. Patent 5,550,318
U.S. Patent 5,563,055
U.S. Patent 5,563,055
U.S. Patent 5,580,859
U.S. Patent 5,589,466
U.S. Patent 5,591,616
U.S. Patent 5,610,042
U.S. Patent 5,620,896
U.S. Patent 5,656,610
U.S. Patent 5,702,932
U.S. Patent 5,736,524
U.S. Patent 5,780,448
U.S. Patent 5,789,215
U.S. Patent 5,843,650
U.S. Patent 5,846,709
U.S. Patent 5,846,783
U.S. Patent 5,849,497
U.S. Patent 5,849,546
U.S. Patent 5,849,547
U.S. Patent 5,858,652
U.S. Patent 5,866,366
U.S. Patent 5,871,986
U.S. Patent 5,916,776
U.S. Patent 5,922,574
U.S. Patent 5,925,565
U.S. Patent 5,928,905
U.S. Patent 5,928,906
U.S. Patent 5,928,906
U.S. Patent 5,932,451
U.S. Patent 5,935,819
U.S. Patent 5,935,825
U.S. Patent 5,939,291
U.S. Patent 5,942,391
U.S. Patent 5,945,100
U.S. Patent 5,958,895
U.S. Patent 5,981,274
U.S. Patent 5,994,624
U.S. Patent 6,333,164
U.S. Patent 6,651,655
U.S. Patent 6,656,462
U.S. Patent 6,699,703
U.S. Patent 6,733,754
U.S. Patent 6,756,361
U.S. Patent 6,756,361
U.S. Patent 6,770,278
U.S. Patent 6,793,923
U.S. Patent 6,800,744
U.S. Patent 6,814,971
U.S. Patent 6,936,258
U.S. Patent Appln. 20030153022

WO 98/18931
WO 98/18930
WO 97/43303
WO 97/37026
WO 02/34771
WO 02/09485I
WO 2005/032582
WO 02/34771
WO 03/093306
WO 04/041157
WO 2005/002619
WO 02/18595
WO 99/58562
WO 99/24578
WO 99/36544
WO 99/57280
WO 00/37494
WO 03/049762
WO 03/068811
WO 05/002619
WO 02/02606

Albus et al., Infect. Immun., 59:1008-1014, 1991.
An, 1997
Anavi, Sc. thesis from the department of Molecular Microbiology and Biotechnology of the Tel-Aviv University, Israel, 1998.
Angel et al., Cell, 49:729, 1987b.
Angel et al., Mol. Cell. Biol., 7:2256, 1987a.
Arbeit et al., Diagn. Microbiol. Infect. Dis. 2, 85-91, 1984.
Archer and Climo, N. Engl. J. Med. 344, 55-56, 2001.
Atchison and Perry, Cell, 46:253, 1986.
Atchison and Perry, Cell, 48:121, 1987.
Ausubel et al., In: Current Protocols in Molecular Biology, John, Wiley & Sons, Inc, New York, 1996.
Baba et al., Lancet 359, 1819-1827, 2002.
Bae et al., Proc. Natl. Acad. Sci. USA, 101:12312-12317, 2004.
Banerji et al., Cell, 27(2 Pt 1):299-308, 1981.
Banerji et al., Cell, 33(3):729-740, 1983.
Barany and Merrifield, In: The Peptides, Gross and Meienhofer (Eds.), Academic Press, NY, 1-284, 1979.
Bellus, J. Macromol. Sci. PureAppl. Chem., A31(1): 1355-1376, 1994.
Bendtsen et al., J. Mol. Biol. 340, 783-795, 2004.
Berkhout et al., Cell, 59:273-282, 1989.
Bhakdi and Tranum-Jensen, Microbiol. Rev., 55:733-751, 1991.
Biochim Biophys Acta. 2004 Nov 1;1702(2):145
Blanar et al., EMBO J., 8:1139, 1989.
Bodine and Ley, EMBO J., 6:2997, 1987.
Borrebaeck, In: Antibody Engineering--A Practical Guide, W. H. Freeman and Co., 1992.
Boshart et al., Cell, 41:521, 1985.
Bosze et al., EMBO J., 5(7):1615-1623, 1986.
Braddock et al., Cell, 58:269, 1989.
Bulla and Siddiqui, J. Virol., 62:1437, 1986.
Burke et al., , J. Inf. Dis., 170:1110-1119, 1994.
Burts et al., Proc Natl Acad Sci USA 102:1169-74, 2005.
Calmette, In: La Vaccination Pre'ventive contre la Tuberculose, Mason-et-Cie, Paris, 251, 1927.
Campbell and Villarreal, Mol. Cell. Biol., 8:1993, 1988.
Campere and Tilghman, Genes and Dev., 3:537, 1989.
Campo et al., Mol. Microbiol., 53:1583-1599, 2004.
Campo et al., Nature, 303:77, 1983.
Can J Biochem Cell Biol. 1984 May;62(5):270-5
Carbonelli et al., FEMS Microbiol. Lett., 177(1):75-82, 1999.
Celander and Haseltine, J. Virology, 61:269, 1987.
Celander et al., J. Virology, 62:1314, 1988.
Chaatwal, Trends Microbiol., 10:205-208, 2002.
Chandler et al., Cell, 33:489, 1983.
Chandler et al., Proc. Natl. Acad. Sci. USA, 94(8):3596-601, 1997.
Chang et al., Mol. Cell. Biol., 9:2153, 1989.
Chang et al., N. Engl. J. Med. 348, 1342-1347, 2003.
Chatterjee et al., Proc. Natl. Acad. Sci. USA, 86:9114, 1989.
Chen and Okayama, Mol. Cell Biol., 7(8):2745-2752, 1987.
Cheung et al., J. Clin. Invest., 94:1815-1822, 1994.
Choi et al., Cell, 53:519, 1988.
Chou and Fasman, Adv. Enzymol., 47:45-148, 1978a.
Chou and Fasman, Annu. Rev. Biochem., 47:251-276, 1978b.
Chou and Fasman, Biochemistry, 13(2):211-222, 1974a.
Chou and Fasman, Biochemistry, 13(2):222-245, 1974b.
Chou and Fasman, Biophys. J., 26(3):385-399, 1979.
Cocea, Biotechniques, 23(5):814-816, 1997.
Cohen et al., J. Cell. Physiol., 5:75, 1987.
Cole et al., Nature, 393:537-544, 1998.
Costa et al., Mol. Cell. Biol., 8:81,1988.
Cripe et al., EMBO J., 6:3745, 1987.
Culotta and Hamer, Mol. Cell. Biol., 9:1376, 1989.
Dale, Vaccine (1999) 17:193-200
Dale, Vaccine 14(10): 944-948
Dandolo et al., J. Virology, 47:55-64, 1983.
De Villiers et al., Nature, 312(5991):242-246, 1984.
Deschamps et al., Science, 230:1174-1177, 1985.
Devereux et al., Nucl. Acid Res., 12:387-395, 1984.
Diekema et al., Clin. Infect. Dis. 32, S114-132, 2001.
Diep et al., Lancet 367, 731-739, 2006.
Dryla et al., Clin. Diagn. Lab. Immunol. 12, 387-398, 2005.
Duthie and Lorenz, J. Gen. Microbiol. 6, 95-107, 1952.
Edbrooke et al., Mol. Cell. Biol., 9:1908, 1989.
Edlund et al., Science, 230:912-916, 1985.
Epitope Mapping Protocols In: Methods in Molecular Biology, Vol. 66, Morris (Ed.), 1996,
Etz et al., Proc. Natl. Acad. Sci. USA, 99:6573-6578, 2002.
Fattom et al., Infect. Immun. 58, 2367-2374, 1990.
Fattom et al., Vaccine 22, 880-887, 2004.
Fechheimer, et al., Proc Natl. Acad. Sci. USA, 84:8463-8467, 1987.
Feng and Holland, Nature, 334:6178, 1988.
Firak and Subramanian, Mol. Cell. Biol., 6:3667, 1986.
Foecking and Hofstetter, Gene, 45(1):101-105, 1986.
Fraley et al., Proc. Natl. Acad. Sci. USA, 76:3348-3352, 1979.
Fujita et al., Cell, 49:357, 1987.
Galan and Collmer, Science, 284:1322-1333, 1999.
Gardy et al., Nucleic Acids Res., 31:3613-3617, 2003.
GB Appln. 2 202 328
Gey Van Pittius et al., Genome Biol., 1-0044.18, 2001.
Gilles et al., Cell, 33:717, 1983.
Gillet et al., Lancet 359, 753-759, 2002.
Gloss et al., EMBO J., 6:3735, 1987.
Godbout et al., Mol. Cell. Biol., 8:1169, 1988.
Goodbourn and Maniatis, Proc. Natl. Acad. Sci. USA, 85:1447, 1988.
Goodbourn et al., Cell, 45:601, 1986.
Gopal, Mol. Cell Biol., 5:1188-1190, 1985.
Graham and Van Der Eb, Virology, 52:456-467, 1973.
Greene et al., Immunology Today, 10:272, 1989
Grosschedl and Baltimore, Cell, 41:885, 1985.
Guinn et al., Mol. Microbiol., 51:359-370, 2004.
Hansen et al., FEMS Microbiol. Lett., 223:21-24, 2003.
Harboe et al., Infect. Immun., 64:16-22, 1996.
Harland and Weintraub, J. Cell Biol., 101 (3):1094-1099, 1985.
Harlow et al., Antibodies: A Laboratory Manual, Cold Spring Harbor Laboratory, Cold Spring Harbor, N.Y., Chapter 8, 1988.
Haslinger and Karin, Proc. Natl. Acad. Sci. USA, 82:8572, 1985.
Hauber and Cullen, J. Virology, 62:673, 1988.
Heilmann et al., Mol. Microbiol. 20, 1083-1091, 1996.
Hen et al., Nature, 321:249, 1986.
Hensel et al., Lymphokine Res., 8:347, 1989.
Herr and Clarke, Cell, 45:461, 1986.
Hirochika et al., J. Virol., 61:2599, 1987.
Hirsch et al., Mol. Cell. Biol., 10:1959, 1990.
Holbrook et al., Virology, 157:211, 1987.
Holden et al., Proc. Natl. Acad. Sci. USA 101, 9786-9791, 2004.
Horlick and Benfield, Mol. Cell. Biol., 9:2396, 1989.
Houghton et al., Hepatology (1991) 14:381
Hsu et al., Proc. Natl. Acad. Sci. USA, 100:12420-12425, 2003.
Huang et al., Cell, 27:245, 1981.
Hug et al., Mol. Cell. Biol., 8:3065, 1988.
Huston et al., In: Methods in Enzymology, Langone (Ed.), Academic Press, NY, 203:46-88, 1991.
Hwang et al., Mol. Cell. Biol., 10:585, 1990.
Imagawa et al., Cell, 51:251, 1987.
Imbra and Karin, Nature, 323:555, 1986.
Imler et al., Mol. Cell. Biol., 7:2558, 1987.
Imperiale and Nevins, Mol. Cell. Biol., 4:875, 1984.
Infect Immun. 1997 Nov; 65(11): 4476-4482
Infect Immun. 1999 Oct; 67(10): 5395
Infect Immun. 2001 May; 69(5): 3323-3334
Infect Immun. 2001 May; 69(5):3510-3515
Infect Immun. 2002 August; 70(8): 4414
Infect Immun. 2003 Jan; 71(1): 374-383
Infect Immun. 2003 Oct;71(10):5498-504
Infect Immun. 2004 July; 72(7): 3829
Infect Immun. 2004 October; 72(10): 6148
Innis et al., Proc Natl Acad Sci USA, 85(24):9436-9440, 1988.
Inouye and Inouye, Nucleic Acids Res., 13: 3101-3109, 1985.
J Autoimmun. 1989 Jun;2 Suppl:81
J Clin Microbiol. 1999 Dec; 37(12): 3997
Jakobovits et al., Mol. Cell. Biol., 8:2555, 1988.
Jameel and Siddiqui, Mol. Cell. Biol., 6:710, 1986.
Janeway et al., In: Immunobiology, 4th Ed., Current Biology Publications, London, 1999.
Jaynes et al., Mol. Cell. Biol., 8:62, 1988.
Johnson et al., Methods in Enzymol., 203:88-99, 1991.
Johnson et al., Mol. Cell. Biol., 9:3393, 1989.
Johnstone et al., In: Immunochemistry in Practice, Blackwell Scientific Publications, Oxford, 1982.
Jonsson et al., Infect. Immun., 49:765-769, 1985.
Jonsson et al., Microb. Infect., 5:775-780, 2003.
Josefsson et al., J. Infect. Dis. 184, 1572-1580, 2001.
Kadesch and Berg, Mol. Cell. Biol., 6:2593, 1986.
Kaeppler et al., Plant Cell Rep., 8:415-418, 1990.
Kaneda et al., Science, 243:375-378, 1989.
Kaplan et al., Clin. Infect. Dis. 40,1785-1791, 2005.
Karin et al., Mol. Cell. Biol., 7:606, 1987.
Katinka et al., Cell, 20:393, 1980.
Kato et al, J. Biol. Chem., 266:3361-3364, 1991.
Kawamoto et al., Mol. Cell. Biol., 8:267, 1988.
Kiledjian et al., Mol. Cell. Biol., 8:145, 1988.
Klamut et al., Mol. Cell. Biol., 10:193, 1990.
Koch et al., Mol. Cell. Biol., 9:303, 1989.
Kriegler and Botchan, In: Eukaryotic Viral Vectors, Gluzman (Ed.), Cold Spring Harbor: Cold Spring Harbor Laboratory, NY, 1982.
Kriegler and Botchan, Mol. Cell. Biol., 3:325, 1983.
Kriegler et al., Cell, 38:483, 1984.
Kriegler et al., Cell, 53:45, 1988.
Kuhl et al., Cell, 50:1057, 1987.
Kuklin et al., Infect. Immun. 74, 2215-2223, 2006.
Kunz et al., Nucl. Acids Res., 17:1121, 1989.
Kuroda et al., Lancet., 357:1225-1240, 2001.
Kyte and Doolittle, J. Mol. BioL, 157(1):105-132, 1982.
Larsen et al., Proc Natl. Acad. Sci. USA., 83:8283, 1986.
Laspia et al., Cell, 59:283, 1989.
Latimer et al., Mol. Cell. Biol., 10:760, 1990.
Lee et al., Nature, 294:228, 1981.
Lee et al., Nucleic Acids Res., 12:4191-206, 1984.
Levenson et al., Hum. Gene Ther., 9(8):1233-1236, 1998.
Levinson et al., Nature, 295:79, 1982.
Lin et al., Mol. Cell. Biol., 10:850, 1990.
Lowy, New Engl. J. Med. 339, 520-532, 1998.
Luria et al., EMBO J., 6:3307, 1987.
Lusky and Botchan, Proc. Natl. Acad. Sci. USA, 83:3609, 1986.
Lusky et al., Mol. Cell. Biol., 3:1108, 1983.
Macejak and Sarnow, Nature, 353:90-94, 1991.
Madden et al., Cell, 104:143-152, 2001.
Mahairas et al., J. Bacteriol., 178:1274-1282, 1996.
Maione et al., Science 309, 148-150, 2005.
Maira-Litran et al., Infect. Immun. 73, 6752-6762, 2005.
Maira-Litran et al., Vaccine 22, 872-879, 2004.
Majors and Varmus, Proc. Natl. Acad. Sci. USA, 80:5866, 1983.
Mazmanian et al., Mol. Microbiol. 40, 1049-1057, 2001.
Mazmanian et al., Proc. Natl. Acad. Sci. USA, 97:5510-5515, 2000.
Mazmanian et al., Science 299, 906-9, 2003.
McDougal et al., J. Clin. Microbiol. 41, 5113-5120, 2003.
McKenney et al., Science 284, 1523-1527, 1999.
McNeall et al., Gene, 76:81, 1989.
Medini et al., Curr. Opin. Genet. Dev. 15, 589-594, 2005.
Mernaugh et al., In: Molecular Methods in Plant Pathology, Singh et al. (Eds.), CRC Press Inc., Boca Raton, FL, 359-365, 1995.
Merrifield, Science, 232(4748):341-347, 1986.
Miksicek et al., Cell, 46:203, 1986.
Mills et al., Proc. Natl. Acad. Sci. USA, 94:12638-12643, 1997.
Mordacq and Linzer, Genes and Dev., 3:760, 1989.
Moreau et al., Nucl. Acids Res., 9:6047, 1981.
Muesing et al., Cell, 48:691, 1987.
Navarre and Schneewind, Microbiol. Mol. Biol Rev., 63:174-229, 1999.
Needleman & Wunsch, J. Mol. Biol., 48:443, 1970.
Ng et al., Nuc. Acids Res., 17:601, 1989.
Ni Eidhin et al., Mol. Microbiol., 30:245-257, 1998.
Nicolau and Sene, Biochim. Biophys. Acta, 721:185-190, 1982.
Nicolau et al., Methods Enzymol., 149:157-176, 1987.
Novick, Mol. Microbiol., 48:1429-1449, 2003.
O'Reilly et al., Microb. Pathog., 1:125-138, 1986.
Okkels and Andersen, J. Bacteriol., 186:2487-2491, 2004.
Omirulleh et al., Plant Mol. Biol., 21(3):415-28, 1993.
Ondek et al., EMBO J., 6:1017, 1987.
Ornitz et al., Mol. Cell. Biol., 7:3466, 1987.
Oudega et al., Microbiology, 143:1489-1491, 1997.
Pallen, Trends Microbiol., 10:209-212, 2002.
Palmiter et al., Nature, 300:611, 1982.
Pancholi and Fischetti, J. Exp. Med., 176:415-426, 1992.
Patel et al., Infect. Immun., 55:3103-3110, 1987.
PCT Appln. PCT/US89/01025
PCT Appln. WO 94/09699
PCT Appln. WO 95/06128
Pearson & Lipman, Proc. Natl. Acad. Sci. USA, 85:2444, 1988.
Pech et al., Mol. Cell. Biol., 9:396, 1989.
Pelletier and Sonenberg, Nature, 334(6180):320-325, 1988.
Perez-Stable and Constantini, Mol. Cell. Biol., 10:1116, 1990.
Philipp et al., Microbiology, 142:3135-3145, 1996.
Picard and Schaffner, Nature, 307:83, 1984.
Pinkert et al., Genes and Dev., 1:268, 1987.
Plante et al., J Infectious Disease (2000) 182:848 - 855
Ponta et al., Pr-oc. Natl. Acad. Sci. USA, 82:1020, 1985.
Porton et al., Mol. Cell. Biol., 10:1076, 1990.
Potrykus et al., Mol. Gen. Genet., 199(2):169-177, 1985.
Prevost et al., Infect. Immun., 63:4121-4129, 1995.
Price et al., Infection and Immunity (2004) 71(1):277 - 283
Proc Natl Acad Sci U S A. 2004 Aug 24; 101 (34): 12652
Pym et al., Mol. Microbiol., 46:709-717, 2002.
Pym et al., Nat. Med., 9:533-539, 2003.
Queen and Baltimore, Cell, 35:741, 1983.
Quinn et al., Mol. Cell. Biol., 9:4713, 1989.
Redondo et al., Science, 247:1225, 1990.
Reisman and Rotter, Mol. Cell. Biol., 9:3571, 1989.
Reznington's Pharmaceutical Sciences, 18th Ed. Mack Printing Company, 1289-1329, 1990.
Resendez Jr. et al., Mol. Cell. Biol., 8:4579, 1988.
Ripe et al., Mol. Cell. Biol., 9:2224, 1989.
Rippe, et al., Mol. Cell Biol., 10:689-695, 1990.
Rittling et al., Nuc. Acids Res., 17:1619, 1989.
Rivas et al., Curr. Opin. Drug Discov. Devel. 7, 223-227, 2004.
Rogers and Melly, Ann. N.Y. Acad. Sci. 128, 45-56, 1965.
Rosch and Caparon, Science, 304:1513-1515, 2004.
Rosen et al., Cell, 41:813, 1988.
Sakai et al., Genes and Dev., 2:1144, 1988.
Sambrook et al., In: Molecular cloning, Cold Spring Harbor Laboratory Press, Cold Spring Harbor, NY, 2001.
Sao-Jose et al., J. Bacteriol., 186:8337-8346, 2004.
Satake et al., J. Virology, 62:970, 1988.
Schaffer et al., Infect. Immun. 74, 2145-2153, 2006.
Schaffner et al., J. Mol. Biol., 201:81, 1988.
Schindler and Schuhardt, Proc. Natl. Acad. Sci. USA, 51:414-421, 1964.
Schneewind et al., Cell, 70:267-281, 1992.
Schneewind et al., Science, 268:103-106, 1995.
Searle et al., Mol. Cell. Biol., 5:1480, 1985.
Sharp and Marciniak, Cell, 59:229, 1989.
Shaul and Ben-Levy, EMBO J., 6:1913, 1987.
Sherman et al., Mol. Cell. Biol., 9:50, 1989.
Shinefield et al., N. Engl. J. Med. 346, 491-496, 2002.
Skaar et al., Science, 305:1626-1628, 2004.
Sleigh and Lockett, J. EMBO, 4:3831, 1985.
Smith & Waterman, Adv. Appl. Math., 2:482, 1981.
Sorensen et al., Infect. Immun., 63:1710-1717, 1995.
Spalholz et al., Cell, 42:183, 1985.
Spandau and Lee, J. Virology, 62:427, 1988.
Spandidos and Wilkie, EMBO J., 2:1193, 1983.
Stanley et al., Proc. Natl. Acad. Sci. USA, 100:13001-13006, 2003.
Stephens and Hentschel, Biochem. J., 248:1, 1987.
Stewart and Young, In: Solid Phase Peptide Synthesis, 2d. ed., Pierce Chemical Co., 1984.
Stuart et al., Nature, 317:828, 1985.
Sullivan and Peterlin, Mol. Cell. Biol., 7:3315, 1987.
Swartzendruber and Lehman, J. Cell. Physiology, 85:179,1975.
Takebe et al., Mol. Cell. Biol., 8:466, 1988.
Tam et al., J. Am. Chem. Soc., 105:6442, 1983.
Tavernier et al., Nature, 301:634, 1983.
Taylor and Kingston, Mol. Cell. Biol., 10:165, 1990a.
Taylor and Kingston, Mol. Cell. Biol., 10:176, 1990b.
Taylor et al., J. Biol. Chem., 264:15160, 1989.
Thakker et al., Infect. Immun., 66:5183-5189, 1998.
Thiesen et al., J. Virology, 62:614, 1988.
Thomas et al., J. Exp. Med., 191:147-155, 2000.
Thomson et al., J. Immunol., 157(2):822-826, 1996.
Tigges et al., J. Immunol., 156(10):3901-3910, 1996.
Treisman, Cell, 42:889, 1985.
Tronche et al., Mol. Biol. Med., 7:173, 1990.
Trudel and Constantini, Genes and Dev., 6:954, 1987.
Tyndell et al., Nuc. Acids. Res., 9:6231, 1981.
Vannice and Levinson, J. Virology, 62:1305, 1988.
Vasseur et al., Proc Natl. Acad. Sci. USA, 77:1068, 1980.
Wang and Calame, Cell, 47:241, 1986.
Weber et al., Cell, 36:983, 1984.
Weichhart et al., Infect. Immun., 71:4633-4641, 2003.
Weigel et al., Science 302,1569-1571, 2003.
Weinberger et al. Mol. Cell. Biol., 8:988, 1984.
Winoto and Baltimore, Cell, 59:649, 1989.
Wong et al., Gene, 10:87-94, 1980.
Yutzey et al. Mol. Cell. Biol., 9:1397, 1989.
Zhou et al., Vaccine 24, 4830-4837, 2006.
Zhu et al., Vaccine (2004) 22:660 - 669

### EMBODIMENTS OF THE INVENTION

Embodiment 1. A method for eliciting an immune response against a staphylococcus bacterium in a subject comprising providing to the subject an effective amount of an EsxA and/or EsxB protein.
Embodiment 2. The method of Embodiment 1, further comprising providing to the subject a SdrD, SdrE, IsdA, and/or IsdB protein.
Embodiment 3. The method of Embodiment 1, wherein the subject is provided with an effective amount of an EsxA or EsxB protein by administering to the subject a composition comprising
   i) an isolated EsxA and/or EsxB protein, or
   ii) at least one isolated recombinant nucleic acid molecule encoding an EsxA or EsxB protein,
   wherein the composition is not a staphylococcus bacterium.
Embodiment 4. The method of Embodiment 2, wherein the subject is provided with an SdrD, SdrE, IsdA, and/or IsdB protein by administering to the subject a composition comprising
   i) an isolated SdrD, SdrE, IsdA, and/or IsdB protein, or
   ii) at least one isolated recombinant nucleic acid molecule encoding a SdrD, SdrE, IsdA, or IsdB protein.
Embodiment 5. The method of Embodiment 4, wherein the subject is provided with more than one of the following: SdrD, SdrE, IsdA, or IsdB protein.
Embodiment 6. The method of Embodiment 3, wherein the composition comprises isolated EsxA and/or EsxB protein.
Embodiment 7. The method of Embodiment 6, wherein the isolated EsxA and/or EsxB proteins are dimerized.
Embodiment 8. The method of Embodiment 1, where the subject is also administered an adjuvant.
Embodiment 9. The method of Embodiment 6, wherein the composition comprises an adjuvant.
Embodiment 10. The method of Embodiment 6, wherein the composition further comprises an adjuvant linked to the protein.
Embodiment 11. The method of Embodiment 10, wherein the adjuvant is chemically conjugated to the protein.
Embodiment 12. The method of Embodiment 1, wherein the EsxA protein is at least 70% identical to SEQ ID NO:2.
Embodiment 13. The method of Embodiment 1, wherein the EsxA protein is at least 80% identical to SEQ ID NO:2.
Embodiment 14. The method of Embodiment 1, wherein the EsxA protein is at least 90% identical to SEQ ID NO:2.
Embodiment 15. The method of Embodiment 1, wherein the EsxB protein is at least 70% identical to SEQ ID NO:4.
Embodiment 16. The method of Embodiment 1, wherein the EsxB protein is at least 80% identical to SEQ ID NO:4.
Embodiment 17. The method of Embodiment 1, wherein the EsxB protein is at least 90% identical to SEQ ID NO:4.
Embodiment 18. The method of Embodiment 6, wherein the composition is formulated in a pharmaceutically acceptable composition.
Embodiment 19. The method of Embodiment 1, wherein the staphylococcus bacterium is an *Staphylococcus aureus* bacterium.
Embodiment 20. The method of Embodiment 1, further comprising administering the composition more than one time to the subject.
Embodiment 21. The method of Embodiment 1, wherein the composition is administered orally.
Embodiment 22. The method of Embodiment 1, wherein the composition is administered parenterally.
Embodiment 23. The method of Embodiment 22, wherein the composition is administered subcutaneously, intramuscularly, or intravenously.
Embodiment 24. The method of Embodiment 1, wherein the method further comprises administering to the subject a composition comprising a non-EsxA or non-EsxB Ess protein.
Embodiment 25. The method of Embodiment 24, wherein the Ess protein is in the same composition as EsxA protein or EsxB protein.
Embodiment 26. The method of Embodiment 6, wherein the composition comprises a recombinant nucleic acid molecule encoding an EsxA protein or a recombinant nucleic acid molecule encoding an EsxB protein.
Embodiment 27. The method of Embodiment 26, wherein the composition comprises a nucleic acid molecule encoding an EsxA protein and a nucleic acid molecule encoding an EsxB protein.
Embodiment 28. The method of Embodiment 27, wherein a nucleic acid molecule encodes both an EsxA protein and an EsxB protein.
Embodiment 29. The method of Embodiment 26, wherein the recombinant nucleic acid molecule encoding an EsxA or EsxB protein contains a heterologous promoter.
Embodiment 30. The method of Embodiment 26, wherein the recombinant nucleic acid molecule is a vector.
Embodiment 31. The method of Embodiment 30, wherein the vector is a plasmid.
Embodiment 32. The method of Embodiment 30, wherein the vector is a viral vector.
Embodiment 33. The method of Embodiment 26, wherein the composition further comprises a nucleic acid encoding another Ess protein.
Embodiment 34. The method of Embodiment 26, wherein the composition includes a recombinant, non-staphylococcus bacterium containing the EsxA or EsxB protein.
Embodiment 35. The method of Embodiment 34, wherein the recombinant non-staphylococcus bacterium is Salmonella.
Embodiment 36. The method of Embodiment 1, wherein the subject is human.
Embodiment 37. The method of Embodiment 1, wherein the immune response is a protective immune response.
Embodiment 38. A method for stimulating in a subject an immune response against a staphylococcus bacterium comprising providing to the subject an effective amount of a combination of Staphylococcus virulence factors, wherein the combination includes more than one of the following Staphylococcus cell surface proteins: SdrD, SdrE, IsdA, or IsdB.
Embodiment 39. The method of Embodiment 38, wherein the combination further includes SdrC, Spa, IsdC, ClfA, ClfB, SasF, or combinations thereof.
Embodiment 40. The method of Embodiment 38, wherein the combination further includes EsxA and/or EsxB.
Embodiment 41. The method of Embodiment 38, wherein the subject is provided with an effective amount of a combination of Staphylococcus virulence factors by administering to the subject an effective amount of a composition including i) an isolated SdrD, SdrE, IsdA, and/or IsdB protein; or, ii) an isolated nucleic acid molecule encoding a SdrD, SdrE, IsdA, and/or IsdB protein.
Embodiment 42. The method of Embodiment 40, wherein the subject is provided with a combination that includes EsxA and/or EsxB by administering to the subject a composition that includes i) an isolated EsxA or EsxB protein; or, ii) an isolated nucleic acid molecule encoding an EsxA and/or EsxB protein, wherein the composition is not a Staphylococcus bacteria.
Embodiment 43. The method of Embodiment 38, wherein the subject is a human.
Embodiment 44. The method of Embodiment 38, wherein the composition is formulated in a pharmaceutically acceptable formulation.
Embodiment 45. The method of Embodiment 38, wherein the Staphylococcus bacteria is Staphylococcus aureus.
Embodiment 46. The method of Embodiment 38, wherein the patient is not provided a Staphylococcus bacterium containing the combination of virulence factors.
Embodiment 47. A vaccine comprising a pharmaceutically acceptable composition having an isolated EsxA and/or EsxB protein, wherein the composition is capable of stimulating an immune response against a Staphylococcus bacteria.
Embodiment 48. The vaccine of Embodiment 47, further comprising an isolated SdrD, SdrE, IsdA, and/or IsdB protein.
Embodiment 49. The vaccine of Embodiment 47, comprising an isolated EsxA and an isolated EsxB protein.
Embodiment 50. The vaccine of Embodiment 47, wherein the isolated EsxA and isolated EsxB proteins are dimerized.
Embodiment 51. The vaccine of Embodiment 48 or 49, wherein the composition is contaminated by less than 1% with other Staphylococcus bacterial proteins.
Embodiment 52. The vaccine of Embodiment 47, further comprising a non-EsxA or EsxB isolated Ess protein.
Embodiment 53. The vaccine of Embodiment 47, further comprising an adjuvant.
Embodiment 54. The vaccine of Embodiment 53, wherein the protein is linked to the adj uvant.
Embodiment 55. The vaccine of Embodiment 54, wherein the adjuvant is chemically conjugated to the protein.
Embodiment 56. A vaccine comprising a pharmaceutically acceptable composition having an isolated recombinant nucleic acid encoding an EsxA or EsxB protein, wherein the composition is capable of stimulating an immune response against a Staphylococcus bacteria.
Embodiment 57. The vaccine of Embodiment 56, further comprising a recombinant nucleic acid encoding a SdrD, SdrE, IsdA or IsdB protein.
Embodiment 58. The vaccine of Embodiment 56, wherein the composition comprises a recombinant nucleic acid encoding an EsxA protein.
Embodiment 59. The vaccine of Embodiment 56, wherein the composition comprises a recombinant nucleic acid encoding an EsxB protein.
Embodiment 60. The vaccine of Embodiment 56, wherein the composition comprises a recombinant nucleic acid encoding an EsxA protein and a recombinant nucleic acid encoding an EsxB protein.
Embodiment 61. The vaccine of Embodiment 60, further comprising an isolated recombinant nucleic acid encoding a SdrD, SdrE, IsdA and/or IsdB protein
Embodiment 62. The vaccine of Embodiment 60, wherein the composition comprises a recombinant nucleic acid encoding both EsxA and EsxB proteins.
Embodiment 63. The vaccine of Embodiment 56, wherein the recombinant nucleic acid contains a heterologous promoter.
Embodiment 64. The vaccine of Embodiment 56, wherein the recombinant nucleic acid is a vector.
Embodiment 65. The vaccine of Embodiment 64, wherein the vector is a plasmid.
Embodiment 66. The vaccine of Embodiment 64, wherein the vector is a viral vector.
Embodiment 67. The vaccine of Embodiment 56, wherein the composition further comprises a nucleic acid encoding another Ess protein.
Embodiment 68. The vaccine of Embodiment 56, wherein the composition includes a recombinant, non-Staphylococcus bacteria containing the nucleic acid.
Embodiment 69. The vaccine of Embodiment 68, wherein the recombinant non-Staphylococcus bacteria is Salmonella.
Embodiment 70. The vaccine of Embodiment 56, wherein the composition further comprises an adjuvant.
Embodiment 71. A method for eliciting an immune response against a staphylococcus bacterium in a subject comprising providing to the subject an effective amount of a sortase substrate and a second protein or polypeptide.
Embodiment 72. The method of Embodiment 71, wherein the second protein or polypeptide is a second sortase substrate.
Embodiment 73. The method of Embodiment 71, wherein the sortase substrate is SdrD, SdrE, IsdA, or IsdB protein.
Embodiment 74. The method of Embodiment 73, wherein the subject is provided with an effective amount of SdrD, SdrE, IsdA, or IsdB protein by administering to the subject a composition comprising
   i) an isolated SdrD, SdrE, IsdA, or IsdB protein, or
   ii) an isolated recombinant nucleic acid molecule encoding a SdrD, SdrE, IsdA, or IsdB protein,
   wherein the composition is not a staphylococcus bacterium.
Embodiment 75. The method of Embodiment 74, wherein three or more of SdrD, SdrE, IsdA, or IsdB proteins are administered to the subject.
Embodiment 76. The method of Embodiment 75, wherein SdrD, SdrE, IsdA, and IsdB proteins are administered to the subject.
Embodiment 77. The method of Embodiment 71, wherein the second polypeptide is a secreted virulence factor.
Embodiment 78. The method of Embodiment 77, wherein the secreted virulence factor is provided to the subject by administering a composition comprising:
   i) an isolated EsxA, and/or EsxB protein, or
   ii) an isolated recombinant nucleic acid molecule encoding a EsxA, and/or EsxB protein.
Embodiment 79. The method of Embodiment 74, wherein the composition comprises isolated SdrD, SdrE, IsdA, or IsdB protein.
Embodiment 80. The method of Embodiment 71, where the subject is also administered an adjuvant.
Embodiment 81. The method of Embodiment 79, wherein the composition comprises an adjuvant.
Embodiment 82. The method of Embodiment 79, wherein the composition further comprises an adjuvant linked to the protein.
Embodiment 83. The method of Embodiment 82, wherein the adjuvant is chemically conjugated to the protein.
Embodiment 84. The method of Embodiment 73, wherein the SdrD protein is at least 70% identical to SEQ ID NO:6.
Embodiment 85. The method of Embodiment 73, wherein the SdrD protein is at least 80% identical to SEQ ID NO: 6.
Embodiment 86. The method of Embodiment 73, wherein the SdrD protein is at least 90% identical to SEQ ID NO:6.
Embodiment 87. The method of Embodiment 73, wherein the SdrE protein is at least 70% identical to SEQ ID NO:8.
Embodiment 88. The method of Embodiment 73, wherein the SdrE protein is at least 80% identical to SEQ ID NO: 8.
Embodiment 89. The method of Embodiment 73, wherein the SdrE protein is at least 90% identical to SEQ ID NO: 8.
Embodiment 90. The method of Embodiment 73, wherein the IsdA protein is at least 70% identical to SEQ ID NO:10.
Embodiment 91. The method of Embodiment 73, wherein the IsdA protein is at least 80% identical to SEQ ID NO:10.
Embodiment 92. The method of Embodiment 73, wherein the IsdA protein is at least 90% identical to SEQ ID NO:10.
Embodiment 93. The method of Embodiment 73, wherein the IsdB protein is at least 70% identical to SEQ ID NO:12.
Embodiment 94. The method of Embodiment 73, wherein the IsdB protein is at least 80% identical to SEQ ID NO:12.
Embodiment 95. The method of Embodiment 73, wherein the IsdB protein is at least 90% identical to SEQ ID NO:12.
Embodiment 96. The method of Embodiment 79, wherein the composition is formulated in a pharmaceutically acceptable composition.
Embodiment 97. The method of Embodiment 71, wherein the staphylococcus bacterium is an *S. aureus* bacterium.
Embodiment 98. The method of Embodiment 71, further comprising administering the composition more than one time to the subject.
Embodiment 99. The method of Embodiment 71, wherein the composition is administered orally.
Embodiment 100. The method of Embodiment 71, wherein the composition is administered parenterally.
Embodiment 101. The method of Embodiment 100, wherein the composition is administered subcutaneously, intramuscularly, or intravenously.
Embodiment 102. The method of Embodiment 74, wherein the composition comprises a recombinant nucleic acid molecule encoding an SdrD protein, a recombinant nucleic acid molecule encoding an SdrE protein, a recombinant nucleic acid molecule encoding an IsdA protein, or a recombinant nucleic acid molecule encoding an IsdB protein.
Embodiment 103. The method of Embodiment 102, wherein the composition comprises a recombinant nucleic acid molecule encoding an SdrD protein, a recombinant nucleic acid molecule encoding an SdrE protein, a recombinant nucleic acid molecule encoding an IsdA protein, and a recombinant nucleic acid molecule encoding an IsdB protein.
Embodiment 104. The method of Embodiment 103, wherein a nucleic acid molecule encodes two or more of a SdrD protein, a SdrE protein, an IsdA protein, or an IsdB protein.
Embodiment 105. The method of Embodiment 102, wherein the recombinant nucleic acid molecule encoding a SdrD, SdrE, IsdA or IsdB protein contains a heterologous promoter.
Embodiment 106. The method of Embodiment 102, wherein the recombinant nucleic acid molecule is a vector.
Embodiment 107. The method of Embodiment 106, wherein the vector is a plasmid.
Embodiment 108. The method of Embodiment 106, wherein the vector is a viral vector.
Embodiment 109. The method of Embodiment 71, wherein the composition includes a recombinant, non-staphylococcus bacterium containing the SdrD, SdrE, IsdA or IsdB protein.
Embodiment 110. The method of Embodiment 109, wherein the recombinant non-staphylococcus bacterium is Salmonella.
Embodiment 111. The method of Embodiment 71, wherein the subject is human.
Embodiment 112. The method of Embodiment 71, wherein the immune response is a protective immune response.
Embodiment 113. A method for stimulating in a subject a protective immune response against a staphylococcus bacterium comprising administering to the subject an effective amount of a composition including i) two or more of SdrD, SdrE, IsdA, or IsdB proteins; or, ii) one or more nucleic acid molecules encoding two or more SdrD, SdrE, IsdA, or IsdB proteins, wherein the composition is not a Staphylococcus bacteria.
Embodiment 114. The method of Embodiment 113, wherein the subject is a human.
Embodiment 115. The method of Embodiment 113, wherein the composition is formulated in a pharmaceutically acceptable formulation.
Embodiment 116. The method of Embodiment 113, wherein the Staphylococcus bacteria is Staphylococcus aureus.
Embodiment 117. A vaccine comprising a pharmaceutically acceptable composition having two or more isolated SdrD, SdrE, IsdA, or IsdB proteins, wherein the composition is capable of stimulating an immune response against a Staphylococcus bacteria.
Embodiment 118. The vaccine of Embodiment 117, further comprising an effective amount of EsxA, EsxB, or EsxA and EsxB.
Embodiment 119. The vaccine of Embodiment 117, comprising an isolated SdrD, an isolated SdrE protein, an isolated IsdA, and an isolated IsdB protein.
Embodiment 120. The vaccine of Embodiment 119, wherein the composition is contaminated by less than 1% with other Staphylococcus bacterial proteins.
Embodiment 121. The vaccine of Embodiment 117, further comprising an adjuvant.
Embodiment 122. The vaccine of Embodiment 121, wherein the protein is linked to the adjuvant.
Embodiment 123. The vaccine of Embodiment 122, wherein the adjuvant is chemically conjugated to the protein.
Embodiment 124. A vaccine comprising a pharmaceutically acceptable composition having a recombinant nucleic acid encoding two or more of SdrD, SdrE, IsdA, or IsdB proteins, wherein the composition is capable of stimulating an immune response against a Staphylococcus bacteria..
Embodiment 125. The vaccine of Embodiment 124, further comprising a recombinant nucleic acid encoding EsxA, EsxB, or EsxA and EsxB.
Embodiment 126. The vaccine of Embodiment 124, wherein the composition comprises a recombinant nucleic acid encoding a SdrD protein, a recombinant nucleic acid encoding a SdrE protein, a recombinant nucleic acid encoding an IsdA protein, or a recombinant nucleic acid encoding an IsdB protein.
Embodiment 127. The vaccine of Embodiment 124, wherein the recombinant nucleic acid contains a heterologous promoter.
Embodiment 128. The vaccine of Embodiment 124, wherein the recombinant nucleic acid is a vector.
Embodiment 129. The vaccine of Embodiment 128, wherein the vector is a plasmid.
Embodiment 130. The vaccine of Embodiment 128, wherein the vector is a viral vector.
Embodiment 131. The vaccine of Embodiment 124, wherein the composition includes a recombinant, non-Staphylococcus bacteria containing the nucleic acid.
Embodiment 132. The vaccine of Embodiment 131, wherein the recombinant non-Staphylococcus bacteria is Salmonella.
Embodiment 133. The vaccine of Embodiment 124, wherein the composition further comprises an adjuvant.

## Claims

1. A composition comprising
(a) a sortase substrate and a second protein or polypeptide, or
(b) a nucleic acid encoding a sortase substrate and a second protein or polypeptide,
for use in a method of eliciting an immune response against a staphylococcus bacterium in a subject.

2. The composition of claim 1, wherein the second protein or polypeptide is a second sortase substrate.

3. The composition of claim 1, wherein the sortase substrate is SdrD, SdrE, IsdA, or IsdB protein.

4. The composition of claim 3, comprising
i) an isolated SdrD, SdrE, IsdA, or IsdB protein, or
ii) an isolated recombinant nucleic acid molecule encoding a SdrD, SdrE, IsdA, or IsdB protein,
wherein the composition is not a staphylococcus bacterium.

5. The composition of any preceding claim, comprising a combination of Staphylococcus virulence factors, wherein the combination includes more than one of the following Staphylococcus cell surface proteins: SdrD, SdrE, IsdA, or IsdB.

6. The composition of claim 5, wherein the combination further includes SdrC, Spa, IsdC, ClfA, ClfB, SasF, or combinations thereof, or further includes EsxA and/or EsxB.

7. The composition of claim 4, comprising
(a) two or more of SdrD, SdrE, IsdA, or IsdB proteins, preferably comprising SdrD, SdrE, IsdA, and IsdB proteins; or
(b) one or more nucleic acid molecules encoding two or more SdrD, SdrE, IsdA, or IsdB proteins.

8. The composition of claim 1, wherein the second polypeptide is a secreted virulence factor, preferably wherein the composition comprises:
i) an isolated EsxA, and/or EsxB protein, or
ii) an isolated recombinant nucleic acid molecule encoding a EsxA, and/or EsxB protein.

9. The composition of claim 1 wherein:
(a) the subject is a human;
(b) the composition is formulated in a pharmaceutically acceptable formulation;
(c) the Staphylococcus bacteria is Staphylococcus aureus;
(d) the patient is not provided a Staphylococcus bacterium containing the combination of virulence factors;
(e) the composition is administered more than one time to the subject;
(f) the composition is administered orally;
(g) the composition is administered parenterally, preferably wherein the composition is administered subcutaneously, intramuscularly, or intravenously;
(h) the composition includes a recombinant, non-staphylococcus bacterium containing the SdrD, SdrE, IsdA or IsdB protein, preferably wherein the recombinant non-staphylococcus bacterium is Salmonella; or
(i) the immune response is a protective immune response.

10. The composition of claim 4, comprising isolated SdrD, SdrE, IsdA, or IsdB protein, preferably comprising an adjuvant or further comprising an adjuvant linked to the protein, more preferably wherein the adjuvant is chemically conjugated to the protein.

11. The composition of claim 3, wherein:
(a) the SdrD protein is at least 70% identical to SEQ ID NO:6;
(b) the SdrD protein is at least 80% identical to SEQ ID NO:6;
(c) the SdrD protein is at least 90% identical to SEQ ID NO:6;
(d) the SdrE protein is at least 70% identical to SEQ ID NO:8;
(e) the SdrE protein is at least 80% identical to SEQ ID NO:8;
(f) the SdrE protein is at least 90% identical to SEQ ID NO:8;
(g) the IsdA protein is at least 70% identical to SEQ ID NO:10;
(h) the IsdA protein is at least 80% identical to SEQ ID NO:10;
(i) the IsdA protein is at least 90% identical to SEQ ID NO:10;
(j) the IsdB protein is at least 70% identical to SEQ ID NO:12;
(k) the IsdB protein is at least 80% identical to SEQ ID NO:12; or
(l) the IsdB protein is at least 90% identical to SEQ ID NO:12.

12. The composition of claim 4, wherein the composition comprises a recombinant nucleic acid molecule encoding an SdrD protein, a recombinant nucleic acid molecule encoding an SdrE protein, a recombinant nucleic acid molecule encoding an IsdA protein, or a recombinant nucleic acid molecule encoding an IsdB protein preferably wherein:
(a) the composition comprises a recombinant nucleic acid molecule encoding an SdrD protein, a recombinant nucleic acid molecule encoding an SdrE protein, a recombinant nucleic acid molecule encoding an IsdA protein, and a recombinant nucleic acid molecule encoding an IsdB protein, preferably wherein a nucleic acid molecule encodes two or more of a SdrD protein, a SdrE protein, an IsdA protein, or an IsdB protein;
(b) the recombinant nucleic acid molecule encoding a SdrD, SdrE, IsdA or IsdB protein contains a heterologous promoter; or
(c) the recombinant nucleic acid molecule is a vector, preferably wherein the vector is a plasmid or a viral vector.

13. A vaccine comprising a pharmaceutically acceptable composition having
(a) two or more isolated SdrD, SdrE, IsdA, or IsdB proteins, or
(b) a recombinant nucleic acid encoding two or more of SdrD, SdrE, IsdA, or IsdB proteins,
wherein the composition is capable of stimulating an immune response against a Staphylococcus bacteria.

14. The vaccine of claim 13(a):
(i) further comprising an effective amount of EsxA, EsxB, or EsxA and EsxB;
(ii) further comprising an isolated SdrC, Spa, IsdC, ClfA, ClfB or SasF protein;
(iii) comprising an isolated SdrD, an isolated SdrE protein, an isolated IsdA, and an isolated IsdB protein, preferably wherein the composition is contaminated by less than 1% with other Staphylococcus bacterial proteins;
(iv) further comprising an adjuvant, preferably wherein the protein is linked to the adjuvant, more preferably wherein the adjuvant is chemically conjugated to the protein.

15. The vaccine of claim 13(b):
(i) further comprising a recombinant nucleic acid encoding EsxA, EsxB, or EsxA and EsxB;
(ii) wherein the composition comprises a recombinant nucleic acid encoding a SdrD protein, a recombinant nucleic acid encoding a SdrE protein, a recombinant nucleic acid encoding an IsdA protein, or a recombinant nucleic acid encoding an IsdB protein;
(iii) further comprising a recombinant nucleic acid encoding an SdrC, Spa, IsdC, ClfA, ClfB or SasF protein;
(iii) wherein the recombinant nucleic acid contains a heterologous promoter;
(iv) wherein the recombinant nucleic acid is a vector, preferably a plasmid or a viral vector;
(v) wherein the composition includes a recombinant, non-Staphylococcus bacteria containing the nucleic acid, preferably wherein the recombinant non-Staphylococcus bacteria is Salmonella; or
(vi) wherein the composition further comprises an adjuvant.


SEQUENCE LISTING

<110> MISSIAKAS, DOMINIQUE
      STRANGER-JONES, YUKIKO
      BURTS, MONICA
      SCHNEEWIND, OLAF

<120> COMPOSITIONS AND METHODS RELATED TO STAPHYLOCOCCAL
      BACTERIUM PROTEINS

<130> ARCD:430WO

<140> PCT/US2007/060720
<141> 2007-01-18

<150> 60/841,52
<151> 2006-08-31

<150> 60/760,008
<151> 2006-08-18

<160> 56

<170> PatentIn Ver. 2.1

<210> 1
<211> 294
<212> DNA
<213> Staphylococcus sp.

<220>
<221> CDS
<222> (1)..(294)

<400> 1
atg gca atg att aag atg agt cca gag gaa atc aga gca aaa tcg caa    48
Met Ala Met Ile Lys Met Ser Pro Glu Glu Ile Arg Ala Lys Ser Gln
  1               5                  10                  15

tct tac ggg caa ggt tca gac caa atc cgt caa att tta tct gat tta    96
Ser Tyr Gly Gln Gly Ser Asp Gln Ile Arg Gln Ile Leu Ser Asp Leu
             20                  25                  30

aca cgt gca caa ggt gaa att gca gcg aac tgg gaa ggt caa gct ttc    144
Thr Arg Ala Gln Gly Glu Ile Ala Ala Asn Trp Glu Gly Gln Ala Phe
         35                  40                  45

agc cgt ttc gaa gag caa ttc caa caa ctt agt cct aaa gta gaa aaa    192
Ser Arg Phe Glu Glu Gln Phe Gln Gln Leu Ser Pro Lys Val Glu Lys
     50                  55                  60

ttt gca caa tta tta gaa gaa att aaa caa caa ttg aat agc act gct    240
Phe Ala Gln Leu Leu Glu Glu Ile Lys Gln Gln Leu Asn Ser Thr Ala
 65                  70                  75                  80

gat gcc gtt caa gaa caa gac caa caa ctt tct aat aat ttc ggt ttg    288
Asp Ala Val Gln Glu Gln Asp Gln Gln Leu Ser Asn Asn Phe Gly Leu
                 85                  90                  95

caa taa                                                            294
Gln

<210> 2
<211> 97
<212> PRT
<213> Staphylococcus sp.

<400> 2
Met Ala Met Ile Lys Met Ser Pro Glu Glu Ile Arg Ala Lys Ser Gln
1               5                   10                  15
Ser Tyr Gly Gln Gly Ser Asp Gln Ile Arg Gln Ile Leu Ser Asp Leu
            20                  25                  30
Thr Arg Ala Gln Gly Glu Ile Ala Ala Asn Trp Glu Gly Gln Ala Phe
        35                  40                  45
Ser Arg Phe Glu Glu Gln Phe Gln Gln Leu Ser Pro Lys Val Glu Lys
    50                  55                  60
Phe Ala Gln Leu Leu Glu Glu Ile Lys Gln Gln Leu Asn Ser Thr Ala
65                  70                  75                  80
Asp Ala Val Gln Glu Gln Asp Gln Gln Leu Ser Asn Asn Phe Gly Leu
                85                  90                  95
Gln

<210> 3
<211> 307
<212> DNA
<213> Staphylococcus sp.

<220>
<221> CDS
<222> (1)..(306)

<400> 3
atg ggt gga tat aaa ggg att aaa gca gat ggt ggc aag gtg aat caa      48
Met Gly Gly Tyr Lys Gly Ile Lys Ala Asp Gly Gly Lys Val Asn Gln
1               5                   10                  15

gcg aaa caa tta gcg gca aaa ata gct aaa gat att gaa gca tgt caa      96
Ala Lys Gln Leu Ala Ala Lys Ile Ala Lys Asp Ile Glu Ala Cys Gln
            20                  25                  30

aag caa acg caa cag ctc gct gag tat atc gaa ggt agt gat tgg gaa      144
Lys Gln Thr Gln Gln Leu Ala Glu Tyr Ile Glu Gly Ser Asp Trp Glu
        35                  40                  45

gga cag ttc gcc aat aag gtg aaa gat gtg tta ctt att atg gca aag      192
Gly Gln Phe Ala Asn Lys Val Lys Asp Val Leu Leu Ile Met Ala Lys
    50                  55                  60

ttt caa gaa gaa tta gta caa ccg atg gct gac cat caa aaa gca att      240
Phe Gln Glu Glu Leu Val Gln Pro Met Ala Asp His Gln Lys Ala Ile
65                  70                  75                  80

gat aac tta agt caa aat cta gcg aaa tac gat aca tta tca att aag      288
Asp Asn Leu Ser Gln Asn Leu Ala Lys Tyr Asp Thr Leu Ser Ile Lys
                85                  90                  95

caa gga ctt gat agg gtg a                                            307
Gln Gly Leu Asp Arg Val
            100

<210> 4
<211> 102
<212> PRT
<213> Staphylococcus sp.

<400> 4
Met Gly Gly Tyr Lys Gly Ile Lys Ala Asp Gly Gly Lys Val Asn Gln
1               5                   10                  15

Ala Lys Gln Leu Ala Ala Lys Ile Ala Lys Asp Ile Glu Ala Cys Gln
            20                  25                  30

Lys Gln Thr Gln Gln Leu Ala Glu Tyr Ile Glu Gly Ser Asp Trp Glu
        35                  40                  45

Gly Gln Phe Ala Asn Lys Val Lys Asp Val Leu Leu Ile Met Ala Lys
    50                  55                  60

Phe Gln Glu Glu Leu Val Gln Pro Met Ala Asp His Gln Lys Ala Ile
65                  70                  75                  80

Asp Asn Leu Ser Gln Asn Leu Ala Lys Tyr Asp Thr Leu Ser Ile Lys
                85                  90                  95

Gln Gly Leu Asp Arg Val
            100

<210> 5
<211> 4158
<212> DNA
<213> Staphylococcus sp.

<220>
<221> CDS
<222> (1)..(4158)

<400> 5
atg cta aac aga gaa aat aaa acg gca ata aca aga aaa ggc atg gta      48
Met Leu Asn Arg Glu Asn Lys Thr Ala Ile Thr Arg Lys Gly Met Val
1               5                   10                  15

tcc aat cga tta aat aaa ttt tcg att aga aag tac aca gtg gga aca      96
Ser Asn Arg Leu Asn Lys Phe Ser Ile Arg Lys Tyr Thr Val Gly Thr
            20                  25                  30

gca tca att tta gta ggt aca aca tta att ttt ggt ctg ggg aac caa      144
Ala Ser Ile Leu Val Gly Thr Thr Leu Ile Phe Gly Leu Gly Asn Gln
        35                  40                  45

gaa gca aag gct gca gaa agt act aat aaa gaa ttg aac gaa gcg aca      192
Glu Ala Lys Ala Ala Glu Ser Thr Asn Lys Glu Leu Asn Glu Ala Thr
    50                  55                  60

act tca gca agt gat aat caa tcg agt gat aaa gtt gat atg cag caa      240
Thr Ser Ala Ser Asp Asn Gln Ser Ser Asp Lys Val Asp Met Gln Gln
65                  70                  75                  80

cta aat caa gaa gac aat act aaa aat gat aat caa aaa gaa atg gta    288
Leu Asn Gln Glu Asp Asn Thr Lys Asn Asp Asn Gln Lys Glu Met Val
                85                  90                  95

tca tct caa ggt aat gaa acg act tca aat ggg aat aaa tca ata gaa    336
Ser Ser Gln Gly Asn Glu Thr Thr Ser Asn Gly Asn Lys Ser Ile Glu
            100                 105                 110

aaa gaa agt gta caa tct acc act gga aat aaa gtt gaa gtt tca act    384
Lys Glu Ser Val Gln Ser Thr Thr Gly Asn Lys Val Glu Val Ser Thr
        115                 120                 125

gcc aaa tca gat gag caa gct tca cca aaa tct acg aat gaa gat tta    432
Ala Lys Ser Asp Glu Gln Ala Ser Pro Lys Ser Thr Asn Glu Asp Leu
    130                 135                 140

aac act aaa caa act ata agt aat caa gaa ggg tta caa cct gat ttg    480
Asn Thr Lys Gln Thr Ile Ser Asn Gln Glu Gly Leu Gln Pro Asp Leu
145                 150                 155                 160

cta gag aat aaa tca gtg gta aat gtt caa cca act aat gag gaa aac    528
Leu Glu Asn Lys Ser Val Val Asn Val Gln Pro Thr Asn Glu Glu Asn
                165                 170                 175

aaa aag gta gat gcg aaa act gaa tca act aca tta aat gtt aaa agt    576
Lys Lys Val Asp Ala Lys Thr Glu Ser Thr Thr Leu Asn Val Lys Ser
            180                 185                 190

gat gct atc aag agt aat gct gaa act ctt gtt gat aac aat agt aat    624
Asp Ala Ile Lys Ser Asn Ala Glu Thr Leu Val Asp Asn Asn Ser Asn
        195                 200                 205

tca aat aat gaa aat aat gca gat atc att ttg cca aaa agt aca gca    672
Ser Asn Asn Glu Asn Asn Ala Asp Ile Ile Leu Pro Lys Ser Thr Ala
    210                 215                 220

cct aaa agt ttg aat aca aga atg cgt atg gca gca ata caa cca aac    720
Pro Lys Ser Leu Asn Thr Arg Met Arg Met Ala Ala Ile Gln Pro Asn
225                 230                 235                 240

tca aca gat tct aaa aat gtt aat gat tta atc aca tca aat aca aca    768
Ser Thr Asp Ser Lys Asn Val Asn Asp Leu Ile Thr Ser Asn Thr Thr
                245                 250                 255

tta act gtc gtt gat gca gat aat agc aaa acg att gta cca gcc caa    816
Leu Thr Val Val Asp Ala Asp Asn Ser Lys Thr Ile Val Pro Ala Gln
            260                 265                 270

gat tat tta tca tta aaa tca caa att aca gtt gat gac aaa gtt aaa    864
Asp Tyr Leu Ser Leu Lys Ser Gln Ile Thr Val Asp Asp Lys Val Lys
        275                 280                 285

tca ggt gat tat ttc aca att aaa tac tca gat aca gta caa gta tat    912
Ser Gly Asp Tyr Phe Thr Ile Lys Tyr Ser Asp Thr Val Gln Val Tyr
    290                 295                 300

gga ttg aat ccg gaa gat att aaa aat att ggt gat att aaa gat cca    960
Gly Leu Asn Pro Glu Asp Ile Lys Asn Ile Gly Asp Ile Lys Asp Pro
305                 310                 315                 320

aat aat ggt gaa aca att gcg act gca aaa cat gat act gca aat aat    1008

Asn Asn Gly Glu Thr Ile Ala Thr Ala Lys His Asp Thr Ala Asn Asn
                325                 330                 335

tta att aca tat aca ttt aca gat tat gtt gat cga ttt aat tca gta 1056
Leu Ile Thr Tyr Thr Phe Thr Asp Tyr Val Asp Arg Phe Asn Ser Val
            340                 345                 350

aaa atg ggt att aat tac tca att tat atg gat gca gat aca att cct 1104
Lys Met Gly Ile Asn Tyr Ser Ile Tyr Met Asp Ala Asp Thr Ile Pro
        355                 360                 365

gtt gac aag aaa gat gtt cct ttt agt gta act att gga aat caa att 1152
Val Asp Lys Lys Asp Val Pro Phe Ser Val Thr Ile Gly Asn Gln Ile
    370                 375                 380

aca act aca aca gca gat atc act tat ccg gct tat aaa gaa gct gac 1200
Thr Thr Thr Thr Ala Asp Ile Thr Tyr Pro Ala Tyr Lys Glu Ala Asp
385                 390                 395                 400

aat aat tca ata gga tca gct ttt aca gag aca gtt tct cat gta gga 1248
Asn Asn Ser Ile Gly Ser Ala Phe Thr Glu Thr Val Ser His Val Gly
                405                 410                 415

aat gtt gaa gac cct ggt tac tat aac cag gta gta tat gtt aat cct 1296
Asn Val Glu Asp Pro Gly Tyr Tyr Asn Gln Val Val Tyr Val Asn Pro
            420                 425                 430

atg gat aag gat tta aaa ggt gct aag tta aaa gtt gaa gcg tac cat 1344
Met Asp Lys Asp Leu Lys Gly Ala Lys Leu Lys Val Glu Ala Tyr His
        435                 440                 445

ccg aaa tat cca act aat att ggt caa att aat caa aat gtt aca aat 1392
Pro Lys Tyr Pro Thr Asn Ile Gly Gln Ile Asn Gln Asn Val Thr Asn
    450                 455                 460

ata aaa ata tat cgt gtt cct gaa gga tat aca ttg aat aaa gga tat 1440
Ile Lys Ile Tyr Arg Val Pro Glu Gly Tyr Thr Leu Asn Lys Gly Tyr
465                 470                 475                 480

gac gtt aat act aat gat ttg gta gac gta act gat gaa ttt aaa aat 1488
Asp Val Asn Thr Asn Asp Leu Val Asp Val Thr Asp Glu Phe Lys Asn
                485                 490                 495

aaa atg acg tat gga tca aat caa agt gtt aat ctt gat ttt ggt gat 1536
Lys Met Thr Tyr Gly Ser Asn Gln Ser Val Asn Leu Asp Phe Gly Asp
            500                 505                 510

att aca tca gca tat gtt gta atg gtt aat aca aaa ttc caa tat aca 1584
Ile Thr Ser Ala Tyr Val Val Met Val Asn Thr Lys Phe Gln Tyr Thr
        515                 520                 525

aat agc gaa agc cca aca ctt gtt caa atg gct act tta tct tca aca 1632
Asn Ser Glu Ser Pro Thr Leu Val Gln Met Ala Thr Leu Ser Ser Thr
    530                 535                 540

ggt aat aaa tcc gtt tct act ggc aat gct tta gga ttt act aat aac 1680
Gly Asn Lys Ser Val Ser Thr Gly Asn Ala Leu Gly Phe Thr Asn Asn
545                 550                 555                 560

caa agt ggc gga gct ggt caa gaa gta tat aaa att ggt aac tac gta 1728
Gln Ser Gly Gly Ala Gly Gln Glu Val Tyr Lys Ile Gly Asn Tyr Val

                565                 570                 575

tgg gaa gat act aat aaa aac ggt gtt caa gaa tta gga gaa aaa ggc 1776
Trp Glu Asp Thr Asn Lys Asn Gly Val Gln Glu Leu Gly Glu Lys Gly
            580                 585                 590

gtt ggc aat gta act gta act gta ttt gat aat aat aca aat aca aaa 1824
Val Gly Asn Val Thr Val Thr Val Phe Asp Asn Asn Thr Asn Thr Lys
        595                 600                 605

gta gga gaa gca gtt act aaa gaa gat ggg tca tac ttg att cca aac 1872
Val Gly Glu Ala Val Thr Lys Glu Asp Gly Ser Tyr Leu Ile Pro Asn
    610                 615                 620

tta cct aat gga gat tac cgt gta gaa ttt tca aac tta cca aaa ggt 1920
Leu Pro Asn Gly Asp Tyr Arg Val Glu Phe Ser Asn Leu Pro Lys Gly
625                 630                 635                 640

tat gaa gta acc cct tca aaa caa ggt aat aac gaa gaa tta gat tca 1968
Tyr Glu Val Thr Pro Ser Lys Gln Gly Asn Asn Glu Glu Leu Asp Ser
                645                 650                 655

aac ggc tta tct tca gtt att aca gtt aat ggc aaa gat aac tta tct 2016
Asn Gly Leu Ser Ser Val Ile Thr Val Asn Gly Lys Asp Asn Leu Ser
            660                 665                 670

gca gac tta ggt att tac aaa cct aaa tac aac tta ggt gac tat gtc 2064
Ala Asp Leu Gly Ile Tyr Lys Pro Lys Tyr Asn Leu Gly Asp Tyr Val
        675                 680                 685

tgg gaa gat aca aat aaa aat ggt atc caa gac caa gat gaa aaa ggt 2112
Trp Glu Asp Thr Asn Lys Asn Gly Ile Gln Asp Gln Asp Glu Lys Gly
    690                 695                 700

ata tct ggc gta acg gta aca tta aaa gat gaa aac ggt aac gtg tta 2160
Ile Ser Gly Val Thr Val Thr Leu Lys Asp Glu Asn Gly Asn Val Leu
705                 710                 715                 720

aaa aca gtt aca aca gac gca gat ggc aaa tat aaa ttt act gat tta 2208
Lys Thr Val Thr Thr Asp Ala Asp Gly Lys Tyr Lys Phe Thr Asp Leu
                725                 730                 735

gat aat ggt aat tat aaa gtt gaa ttt act aca cca gaa ggc tat aca 2256
Asp Asn Gly Asn Tyr Lys Val Glu Phe Thr Thr Pro Glu Gly Tyr Thr
            740                 745                 750

ccg act aca gta aca tct ggt agc gac att gaa aaa gac tct aat ggt 2304
Pro Thr Thr Val Thr Ser Gly Ser Asp Ile Glu Lys Asp Ser Asn Gly
        755                 760                 765

tta aca aca aca ggt gtt att aat ggt gct gat aac atg aca tta gat 2352
Leu Thr Thr Thr Gly Val Ile Asn Gly Ala Asp Asn Met Thr Leu Asp
    770                 775                 780

agt gga ttc tac aaa aca cca aaa tat aat tta ggt aat tat gta tgg 2400
Ser Gly Phe Tyr Lys Thr Pro Lys Tyr Asn Leu Gly Asn Tyr Val Trp
785                 790                 795                 800

gaa gat aca aat aaa gat ggt aag cag gat tca act gaa aaa ggt att 2448
Glu Asp Thr Asn Lys Asp Gly Lys Gln Asp Ser Thr Glu Lys Gly Ile
                805                 810                 815

tca ggc gta aca gtt aca ttg aaa aat gaa aac ggt gaa gtt tta caa    2496
Ser Gly Val Thr Val Thr Leu Lys Asn Glu Asn Gly Glu Val Leu Gln
            820                 825                 830

aca act aaa aca gat aaa gat ggt aaa tat caa ttt act gga tta gaa    2544
Thr Thr Lys Thr Asp Lys Asp Gly Lys Tyr Gln Phe Thr Gly Leu Glu
        835                 840                 845

aat gga act tat aaa gtt gaa ttc gaa aca cca tca ggt tac aca cca    2592
Asn Gly Thr Tyr Lys Val Glu Phe Glu Thr Pro Ser Gly Tyr Thr Pro
    850                 855                 860

aca caa gta ggt tca gga act gat gaa ggt ata gat tca aat ggt aca    2640
Thr Gln Val Gly Ser Gly Thr Asp Glu Gly Ile Asp Ser Asn Gly Thr
865                 870                 875                 880

tca aca aca ggt gtc att aaa gat aaa gat aac gat act att gac tct    2688
Ser Thr Thr Gly Val Ile Lys Asp Lys Asp Asn Asp Thr Ile Asp Ser
                885                 890                 895

ggt ttc tac aaa ccg act tac aac tta ggt gac tat gta tgg gaa gat    2736
Gly Phe Tyr Lys Pro Thr Tyr Asn Leu Gly Asp Tyr Val Trp Glu Asp
            900                 905                 910

aca aat aaa aac ggt gtt caa gat aaa gat gaa aag ggt att tca ggt    2784
Thr Asn Lys Asn Gly Val Gln Asp Lys Asp Glu Lys Gly Ile Ser Gly
        915                 920                 925

gta aca gtt acg tta aaa gat gaa aac gac aaa gtt tta aaa aca gtt    2832
Val Thr Val Thr Leu Lys Asp Glu Asn Asp Lys Val Leu Lys Thr Val
    930                 935                 940

aca aca gat gaa aat ggt aaa tat caa ttc act gat tta aac aat gga    2880
Thr Thr Asp Glu Asn Gly Lys Tyr Gln Phe Thr Asp Leu Asn Asn Gly
945                 950                 955                 960

act tat aaa gtt gaa ttc gag aca cca tca ggt tat aca cca act tca    2928
Thr Tyr Lys Val Glu Phe Glu Thr Pro Ser Gly Tyr Thr Pro Thr Ser
                965                 970                 975

gta act tct gga aat gat act gaa aaa gat tct aat ggt tta aca aca    2976
Val Thr Ser Gly Asn Asp Thr Glu Lys Asp Ser Asn Gly Leu Thr Thr
            980                 985                 990

aca ggt gtc att aaa gat gca gat aac atg aca tta gac agt ggt ttc    3024
Thr Gly Val Ile Lys Asp Ala Asp Asn Met Thr Leu Asp Ser Gly Phe
        995                 1000                1005

tat aaa aca cca aaa tat agt tta ggt gat tat gtt tgg tac gac agt    3072
Tyr Lys Thr Pro Lys Tyr Ser Leu Gly Asp Tyr Val Trp Tyr Asp Ser
    1010                1015                1020

aat aaa gac ggc aaa caa gat tca act gaa aaa ggt atc aaa gat gtt    3120
Asn Lys Asp Gly Lys Gln Asp Ser Thr Glu Lys Gly Ile Lys Asp Val
1025                1030                1035                1040

aaa gtt att tta tta aat gaa aaa ggc gaa gta att gga aca act aaa    3168
Lys Val Ile Leu Leu Asn Glu Lys Gly Glu Val Ile Gly Thr Thr Lys
                1045                1050                1055

aca gat gaa aat ggt aaa tac cgc ttt gat aat tta gat agc ggt aaa    3216
Thr Asp Glu Asn Gly Lys Tyr Arg Phe Asp Asn Leu Asp Ser Gly Lys
            1060                1065                1070

tac aaa gtt att ttt gaa aag cct act ggc tta aca caa aca ggt aca    3264
Tyr Lys Val Ile Phe Glu Lys Pro Thr Gly Leu Thr Gln Thr Gly Thr
        1075                1080                1085

aat aca act gaa gat gat aaa gat gcc gat ggt ggc gaa gtt gat gta    3312
Asn Thr Thr Glu Asp Asp Lys Asp Ala Asp Gly Gly Glu Val Asp Val
    1090                1095                1100

aca att acg gat cat gat gat ttc aca ctt gat aat ggc tac tac gaa    3360
Thr Ile Thr Asp His Asp Asp Phe Thr Leu Asp Asn Gly Tyr Tyr Glu
1105                1110                1115                1120

gaa gaa aca tca gat agc gac tca gat tcg gac agc gat tca gac tca    3408
Glu Glu Thr Ser Asp Ser Asp Ser Asp Ser Asp Ser Asp Ser Asp Ser
                1125                1130                1135

gac agc gat tca gac tca gat agt gat tca gat tca gat agt gat tca    3456
Asp Ser Asp Ser Asp Ser Asp Ser Asp Ser Asp Ser Asp Ser Asp Ser
            1140                1145                1150

gat tca gat agt gat tca gat tca gac agc gac tca gac tca gat agt    3504
Asp Ser Asp Ser Asp Ser Asp Ser Asp Ser Asp Ser Asp Ser Asp Ser
        1155                1160                1165

gac tca gac tca gat agc gat tca gat tca gat agc gat tca gac tca    3552
Asp Ser Asp Ser Asp Ser Asp Ser Asp Ser Asp Ser Asp Ser Asp Ser
    1170                1175                1180

gac agc gat tca gat tca gac agc gac tca gac tca gat agc gac tca    3600
Asp Ser Asp Ser Asp Ser Asp Ser Asp Ser Asp Ser Asp Ser Asp Ser
1185                1190                1195                1200

gat tcg gac agc gat tca gac tca gat agc gac tca gac tca gac agc    3648
Asp Ser Asp Ser Asp Ser Asp Ser Asp Ser Asp Ser Asp Ser Asp Ser
                1205                1210                1215

gat tca gac tca gat agc gac tca gac tca gat agc gat tca gat tca    3696
Asp Ser Asp Ser Asp Ser Asp Ser Asp Ser Asp Ser Asp Ser Asp Ser
            1220                1225                1230

gac agc gat tca gat tca gac agt gat tca gat tca gac agc gac tca    3744
Asp Ser Asp Ser Asp Ser Asp Ser Asp Ser Asp Ser Asp Ser Asp Ser
        1235                1240                1245

gat tca gat agc gat tca gac tca gac tca gat agc gat tca gat tca    3792
Asp Ser Asp Ser Asp Ser Asp Ser Asp Ser Asp Ser Asp Ser Asp Ser
    1250                1255                1260

gac agc gac tca gat tcg gac agc gac tca gac tca gac agt gat tca    3840
Asp Ser Asp Ser Asp Ser Asp Ser Asp Ser Asp Ser Asp Ser Asp Ser
1265                1270                1275                1280

gat tca gat agc gac tca gac tca gat agc gac tca gat tca gac agc    3888
Asp Ser Asp Ser Asp Ser Asp Ser Asp Ser Asp Ser Asp Ser Asp Ser
                1285                1290                1295

gat tca gac tca gat agt gac tca gat tcg gac agc gat tca gac tca    3936

Asp Ser Asp Ser Asp Ser Asp Ser Asp Ser Asp Ser Asp Ser Asp Ser
            1300                1305                1310

gat agc gac tca gat tca gac agt gat tca gac tca gat gca ggt aag    3984
Asp Ser Asp Ser Asp Ser Asp Ser Asp Ser Asp Ser Asp Ala Gly Lys
        1315                1320                1325

cac aca cct gtt aaa cca atg agt act act aaa gac cat cac aat aaa    4032
His Thr Pro Val Lys Pro Met Ser Thr Thr Lys Asp His His Asn Lys
    1330                1335                1340

gca aaa gca tta cca gaa aca ggt aat gaa aat agt ggc tca aat aac    4080
Ala Lys Ala Leu Pro Glu Thr Gly Asn Glu Asn Ser Gly Ser Asn Asn
1345                1350                1355                1360

gca acg tta ttt ggc gga tta ttc gca gca tta gga tca tta ttg tta    4128
Ala Thr Leu Phe Gly Gly Leu Phe Ala Ala Leu Gly Ser Leu Leu Leu
                1365                1370                1375

ttc ggt cgt cgt aaa aaa caa aat aaa taa                            4158
Phe Gly Arg Arg Lys Lys Gln Asn Lys
            1380                1385


<210> 6
<211> 1385
<212> PRT
<213> Staphylococcus sp.

<400> 6
Met Leu Asn Arg Glu Asn Lys Thr Ala Ile Thr Arg Lys Gly Met Val
  1               5                  10                  15
Ser Asn Arg Leu Asn Lys Phe Ser Ile Arg Lys Tyr Thr Val Gly Thr
             20                  25                  30
Ala Ser Ile Leu Val Gly Thr Thr Leu Ile Phe Gly Leu Gly Asn Gln
         35                  40                  45
Glu Ala Lys Ala Ala Glu Ser Thr Asn Lys Glu Leu Asn Glu Ala Thr
     50                  55                  60
Thr Ser Ala Ser Asp Asn Gln Ser Ser Asp Lys Val Asp Met Gln Gln
 65                  70                  75                  80
Leu Asn Gln Glu Asp Asn Thr Lys Asn Asp Asn Gln Lys Glu Met Val
                 85                  90                  95
Ser Ser Gln Gly Asn Glu Thr Thr Ser Asn Gly Asn Lys Ser Ile Glu
            100                 105                 110
Lys Glu Ser Val Gln Ser Thr Thr Gly Asn Lys Val Glu Val Ser Thr
        115                 120                 125
Ala Lys Ser Asp Glu Gln Ala Ser Pro Lys Ser Thr Asn Glu Asp Leu
    130                 135                 140
Asn Thr Lys Gln Thr Ile Ser Asn Gln Glu Gly Leu Gln Pro Asp Leu
145                 150                 155                 160
Leu Glu Asn Lys Ser Val Val Asn Val Gln Pro Thr Asn Glu Glu Asn
                165                 170                 175
Lys Lys Val Asp Ala Lys Thr Glu Ser Thr Thr Leu Asn Val Lys Ser
            180                 185                 190
Asp Ala Ile Lys Ser Asn Ala Glu Thr Leu Val Asp Asn Asn Ser Asn
        195                 200                 205
Ser Asn Asn Glu Asn Asn Ala Asp Ile Ile Leu Pro Lys Ser Thr Ala
    210                 215                 220
Pro Lys Ser Leu Asn Thr Arg Met Arg Met Ala Ala Ile Gln Pro Asn
225                 230                 235                 240
Ser Thr Asp Ser Lys Asn Val Asn Asp Leu Ile Thr Ser Asn Thr Thr

                245                 250                 255
Leu Thr Val Val Asp Ala Asp Asn Ser Lys Thr Ile Val Pro Ala Gln
            260                 265                 270
Asp Tyr Leu Ser Leu Lys Ser Gln Ile Thr Val Asp Asp Lys Val Lys
        275                 280                 285
Ser Gly Asp Tyr Phe Thr Ile Lys Tyr Ser Asp Thr Val Gln Val Tyr
    290                 295                 300
Gly Leu Asn Pro Glu Asp Ile Lys Asn Ile Gly Asp Ile Lys Asp Pro
305                 310                 315                 320
Asn Asn Gly Glu Thr Ile Ala Thr Ala Lys His Asp Thr Ala Asn Asn
                325                 330                 335
Leu Ile Thr Tyr Thr Phe Thr Asp Tyr Val Asp Arg Phe Asn Ser Val
            340                 345                 350
Lys Met Gly Ile Asn Tyr Ser Ile Tyr Met Asp Ala Asp Thr Ile Pro
        355                 360                 365
Val Asp Lys Lys Asp Val Pro Phe Ser Val Thr Ile Gly Asn Gln Ile
    370                 375                 380
Thr Thr Thr Thr Ala Asp Ile Thr Tyr Pro Ala Tyr Lys Glu Ala Asp
385                 390                 395                 400
Asn Asn Ser Ile Gly Ser Ala Phe Thr Glu Thr Val Ser His Val Gly
                405                 410                 415
Asn Val Glu Asp Pro Gly Tyr Tyr Asn Gln Val Val Tyr Val Asn Pro
            420                 425                 430
Met Asp Lys Asp Leu Lys Gly Ala Lys Leu Lys Val Glu Ala Tyr His
        435                 440                 445
Pro Lys Tyr Pro Thr Asn Ile Gly Gln Ile Asn Gln Asn Val Thr Asn
    450                 455                 460
Ile Lys Ile Tyr Arg Val Pro Glu Gly Tyr Thr Leu Asn Lys Gly Tyr
465                 470                 475                 480
Asp Val Asn Thr Asn Asp Leu Val Asp Val Thr Asp Glu Phe Lys Asn
                485                 490                 495
Lys Met Thr Tyr Gly Ser Asn Gln Ser Val Asn Leu Asp Phe Gly Asp
            500                 505                 510
Ile Thr Ser Ala Tyr Val Val Met Val Asn Thr Lys Phe Gln Tyr Thr
        515                 520                 525
Asn Ser Glu Ser Pro Thr Leu Val Gln Met Ala Thr Leu Ser Ser Thr
    530                 535                 540
Gly Asn Lys Ser Val Ser Thr Gly Asn Ala Leu Gly Phe Thr Asn Asn
545                 550                 555                 560
Gln Ser Gly Gly Ala Gly Gln Glu Val Tyr Lys Ile Gly Asn Tyr Val
                565                 570                 575
Trp Glu Asp Thr Asn Lys Asn Gly Val Gln Glu Leu Gly Glu Lys Gly
            580                 585                 590
Val Gly Asn Val Thr Val Thr Val Phe Asp Asn Asn Thr Asn Thr Lys
        595                 600                 605
Val Gly Glu Ala Val Thr Lys Glu Asp Gly Ser Tyr Leu Ile Pro Asn
    610                 615                 620
Leu Pro Asn Gly Asp Tyr Arg Val Glu Phe Ser Asn Leu Pro Lys Gly
625                 630                 635                 640
Tyr Glu Val Thr Pro Ser Lys Gln Gly Asn Asn Glu Glu Leu Asp Ser
                645                 650                 655
Asn Gly Leu Ser Ser Val Ile Thr Val Asn Gly Lys Asp Asn Leu Ser
            660                 665                 670
Ala Asp Leu Gly Ile Tyr Lys Pro Lys Tyr Asn Leu Gly Asp Tyr Val
        675                 680                 685
Trp Glu Asp Thr Asn Lys Asn Gly Ile Gln Asp Gln Asp Glu Lys Gly
    690                 695                 700
Ile Ser Gly Val Thr Val Thr Leu Lys Asp Glu Asn Gly Asn Val Leu
705                 710                 715                 720
Lys Thr Val Thr Thr Asp Ala Asp Gly Lys Tyr Lys Phe Thr Asp Leu
                725                 730                 735

Asp Asn Gly Asn Tyr Lys Val Glu Phe Thr Thr Pro Glu Gly Tyr Thr
            740                 745                 750
Pro Thr Thr Val Thr Ser Gly Ser Asp Ile Glu Lys Asp Ser Asn Gly
        755                 760                 765
Leu Thr Thr Thr Gly Val Ile Asn Gly Ala Asp Asn Met Thr Leu Asp
    770                 775                 780
Ser Gly Phe Tyr Lys Thr Pro Lys Tyr Asn Leu Gly Asn Tyr Val Trp
785                 790                 795                 800
Glu Asp Thr Asn Lys Asp Gly Lys Gln Asp Ser Thr Glu Lys Gly Ile
                805                 810                 815
Ser Gly Val Thr Val Thr Leu Lys Asn Glu Asn Gly Glu Val Leu Gln
            820                 825                 830
Thr Thr Lys Thr Asp Lys Asp Gly Lys Tyr Gln Phe Thr Gly Leu Glu
        835                 840                 845
Asn Gly Thr Tyr Lys Val Glu Phe Glu Thr Pro Ser Gly Tyr Thr Pro
    850                 855                 860
Thr Gln Val Gly Ser Gly Thr Asp Glu Gly Ile Asp Ser Asn Gly Thr
865                 870                 875                 880
Ser Thr Thr Gly Val Ile Lys Asp Lys Asp Asn Asp Thr Ile Asp Ser
                885                 890                 895
Gly Phe Tyr Lys Pro Thr Tyr Asn Leu Gly Asp Tyr Val Trp Glu Asp
            900                 905                 910
Thr Asn Lys Asn Gly Val Gln Asp Lys Asp Glu Lys Gly Ile Ser Gly
        915                 920                 925
Val Thr Val Thr Leu Lys Asp Glu Asn Asp Lys Val Leu Lys Thr Val
    930                 935                 940
Thr Thr Asp Glu Asn Gly Lys Tyr Gln Phe Thr Asp Leu Asn Asn Gly
945                 950                 955                 960
Thr Tyr Lys Val Glu Phe Glu Thr Pro Ser Gly Tyr Thr Pro Thr Ser
                965                 970                 975
Val Thr Ser Gly Asn Asp Thr Glu Lys Asp Ser Asn Gly Leu Thr Thr
            980                 985                 990
Thr Gly Val Ile Lys Asp Ala Asp Asn Met Thr Leu Asp Ser Gly Phe
        995                 1000                1005
Tyr Lys Thr Pro Lys Tyr Ser Leu Gly Asp Tyr Val Trp Tyr Asp Ser
   1010                 1015                1020
Asn Lys Asp Gly Lys Gln Asp Ser Thr Glu Lys Gly Ile Lys Asp Val
1025                1030                1035                1040
Lys Val Ile Leu Leu Asn Glu Lys Gly Glu Val Ile Gly Thr Thr Lys
               1045                1050                1055
Thr Asp Glu Asn Gly Lys Tyr Arg Phe Asp Asn Leu Asp Ser Gly Lys
           1060                1065                1070
Tyr Lys Val Ile Phe Glu Lys Pro Thr Gly Leu Thr Gln Thr Gly Thr
       1075                1080                1085
Asn Thr Thr Glu Asp Asp Lys Asp Ala Asp Gly Gly Glu Val Asp Val
   1090                1095                1100
Thr Ile Thr Asp His Asp Asp Phe Thr Leu Asp Asn Gly Tyr Tyr Glu
1105                1110                1115                1120
Glu Glu Thr Ser Asp Ser Asp Ser Asp Ser Asp Ser Asp Ser Asp Ser
               1125                1130                1135
Asp Ser Asp Ser Asp Ser Asp Ser Asp Ser Asp Ser Asp Ser Asp Ser
           1140                1145                1150
Asp Ser Asp Ser Asp Ser Asp Ser Asp Ser Asp Ser Asp Ser Asp Ser
       1155                1160                1165
Asp Ser Asp Ser Asp Ser Asp Ser Asp Ser Asp Ser Asp Ser Asp Ser
   1170                1175                1180
Asp Ser Asp Ser Asp Ser Asp Ser Asp Ser Asp Ser Asp Ser Asp Ser
1185                1190                1195                1200
Asp Ser Asp Ser Asp Ser Asp Ser Asp Ser Asp Ser Asp Ser Asp Ser
               1205                1210                1215
Asp Ser Asp Ser Asp Ser Asp Ser Asp Ser Asp Ser Asp Ser Asp Ser

1220                1225                1230
Asp Ser Asp Ser Asp Ser Asp Ser Asp Ser Asp Ser Asp Ser Asp Ser
        1235                1240                1245
Asp Ser Asp Ser Asp Ser Asp Ser Asp Ser Asp Ser Asp Ser Asp Ser
    1250                1255                1260
Asp Ser Asp Ser Asp Ser Asp Ser Asp Ser Asp Ser Asp Ser Asp Ser
1265                1270                1275                1280
Asp Ser Asp Ser Asp Ser Asp Ser Asp Ser Asp Ser Asp Ser Asp Ser
                1285                1290                1295
Asp Ser Asp Ser Asp Ser Asp Ser Asp Ser Asp Ser Asp Ser Asp Ser
            1300                1305                1310
Asp Ser Asp Ser Asp Ser Asp Ser Asp Ser Asp Ser Asp Ala Gly Lys
        1315                1320                1325
His Thr Pro Val Lys Pro Met Ser Thr Thr Lys Asp His His Asn Lys
    1330                1335                1340
Ala Lys Ala Leu Pro Glu Thr Gly Asn Glu Asn Ser Gly Ser Asn Asn
1345                1350                1355                1360
Ala Thr Leu Phe Gly Gly Leu Phe Ala Ala Leu Gly Ser Leu Leu Leu
                1365                1370                1375
Phe Gly Arg Arg Lys Lys Gln Asn Lys
            1380                1385

<210> 7
<211> 3426
<212> DNA
<213> Staphylococcus sp.

<220>
<221> CDS
<222> (1)..(3426)

<400> 7
atg att aac agg gat aat aaa aag gca ata aca aaa aag ggt atg att      48
Met Ile Asn Arg Asp Asn Lys Lys Ala Ile Thr Lys Lys Gly Met Ile
1               5                   10                  15

tca aat cgc tta aac aaa ttt tcg att aga aag tat act gta gga act      96
Ser Asn Arg Leu Asn Lys Phe Ser Ile Arg Lys Tyr Thr Val Gly Thr
            20                  25                  30

gca tcg att tta gta ggt acg aca ttg att ttt ggt cta ggg aac caa      144
Ala Ser Ile Leu Val Gly Thr Thr Leu Ile Phe Gly Leu Gly Asn Gln
        35                  40                  45

gaa gct aaa gct gct gaa aac act agt aca gaa aat gcg aaa caa gat      192
Glu Ala Lys Ala Ala Glu Asn Thr Ser Thr Glu Asn Ala Lys Gln Asp
    50                  55                  60

gat gca acg act agt gat aat aaa gaa gta gtg tcg gaa act gaa aat      240
Asp Ala Thr Thr Ser Asp Asn Lys Glu Val Val Ser Glu Thr Glu Asn
65                  70                  75                  80

aat tcg aca aca gaa aat gat tca aca aat cca att aag aaa gaa aca      288
Asn Ser Thr Thr Glu Asn Asp Ser Thr Asn Pro Ile Lys Lys Glu Thr
                85                  90                  95

aat act gat tca caa cca gaa gct aaa gaa gaa tca act aca tca agt      336
Asn Thr Asp Ser Gln Pro Glu Ala Lys Glu Glu Ser Thr Thr Ser Ser
            100                 105                 110

act caa caa cag caa aat aac gtt aca gct aca act gaa act aag cct    384
Thr Gln Gln Gln Gln Asn Asn Val Thr Ala Thr Thr Glu Thr Lys Pro
        115                 120                 125

caa aac att gaa aaa gaa aat gtt aaa cct tca act gat aaa act gcg    432
Gln Asn Ile Glu Lys Glu Asn Val Lys Pro Ser Thr Asp Lys Thr Ala
    130                 135                 140

aca gaa gat aca tct gtt att tta gaa gag aag aaa gca cca aat tat    480
Thr Glu Asp Thr Ser Val Ile Leu Glu Glu Lys Lys Ala Pro Asn Tyr
145                 150                 155                 160

aca aat aac gat gta act aca aaa cca tct aca agt gaa att caa aca    528
Thr Asn Asn Asp Val Thr Thr Lys Pro Ser Thr Ser Glu Ile Gln Thr
                165                 170                 175

aaa cca act aca cct caa gaa tct aca aat att gaa aat tca caa ccg    576
Lys Pro Thr Thr Pro Gln Glu Ser Thr Asn Ile Glu Asn Ser Gln Pro
            180                 185                 190

caa cca acg cct tca aaa gta gac aat caa gtt aca gat gca act aat    624
Gln Pro Thr Pro Ser Lys Val Asp Asn Gln Val Thr Asp Ala Thr Asn
        195                 200                 205

cca aaa gaa cca gta aat gtg tca aaa gaa gaa ctt aaa aat aat cct    672
Pro Lys Glu Pro Val Asn Val Ser Lys Glu Glu Leu Lys Asn Asn Pro
    210                 215                 220

gag aaa tta aaa gaa tta gtt aga aat gat aac aat aca gat cgt tca    720
Glu Lys Leu Lys Glu Leu Val Arg Asn Asp Asn Asn Thr Asp Arg Ser
225                 230                 235                 240

act aaa cca gtt gct aca gct cca aca agt gtt gca cca aaa cga tta    768
Thr Lys Pro Val Ala Thr Ala Pro Thr Ser Val Ala Pro Lys Arg Leu
                245                 250                 255

aat gcg aaa atg cgt ttt gca gtt gca caa cca gca gca gtt gct tca    816
Asn Ala Lys Met Arg Phe Ala Val Ala Gln Pro Ala Ala Val Ala Ser
            260                 265                 270

aat aat gta aat gac tta att aca gtt acg aaa cag acg atc aaa gtt    864
Asn Asn Val Asn Asp Leu Ile Thr Val Thr Lys Gln Thr Ile Lys Val
        275                 280                 285

ggc gat ggt aaa gat aat gtg gca gca gcg cat gac ggt aaa gat att    912
Gly Asp Gly Lys Asp Asn Val Ala Ala Ala His Asp Gly Lys Asp Ile
    290                 295                 300

gaa tat gat aca gag ttt aca att gac aat aaa gtc aaa aaa ggc gat    960
Glu Tyr Asp Thr Glu Phe Thr Ile Asp Asn Lys Val Lys Lys Gly Asp
305                 310                 315                 320

aca atg acg att aat tat gat aag aat gta att cct tcg gat tta aca    1008
Thr Met Thr Ile Asn Tyr Asp Lys Asn Val Ile Pro Ser Asp Leu Thr
                325                 330                 335

gat aaa aat gat cct atc gat att act gat cca tca gga gag gtc att    1056
Asp Lys Asn Asp Pro Ile Asp Ile Thr Asp Pro Ser Gly Glu Val Ile
            340                 345                 350

gcc aaa gga aca ttt gat aaa gcg act aag caa atc aca tat aca ttt    1104
Ala Lys Gly Thr Phe Asp Lys Ala Thr Lys Gln Ile Thr Tyr Thr Phe
        355                 360                 365

aca gat tat gta gat aaa tat gaa gat ata aaa gca cgt tta act tta    1152
Thr Asp Tyr Val Asp Lys Tyr Glu Asp Ile Lys Ala Arg Leu Thr Leu
    370                 375                 380

tac tca tat att gat aag caa gca gta cct aat gaa act agt ttg aat    1200
Tyr Ser Tyr Ile Asp Lys Gln Ala Val Pro Asn Glu Thr Ser Leu Asn
385                 390                 395                 400

tta acg ttt gca aca gca ggt aaa gaa act agc caa aac gtt tct gtt    1248
Leu Thr Phe Ala Thr Ala Gly Lys Glu Thr Ser Gln Asn Val Ser Val
                405                 410                 415

gat tat caa gac cca atg gtt cat ggt gat tca aac att caa tct atc    1296
Asp Tyr Gln Asp Pro Met Val His Gly Asp Ser Asn Ile Gln Ser Ile
            420                 425                 430

ttt aca aag tta gat gaa aac aaa caa act att gaa caa caa att tat    1344
Phe Thr Lys Leu Asp Glu Asn Lys Gln Thr Ile Glu Gln Gln Ile Tyr
        435                 440                 445

gtt aat cct ttg aaa aaa aca gca act aac act aaa gtt gat ata gct    1392
Val Asn Pro Leu Lys Lys Thr Ala Thr Asn Thr Lys Val Asp Ile Ala
    450                 455                 460

ggt agt caa gta gat gat tat gga aat att aaa cta gga aat ggt agt    1440
Gly Ser Gln Val Asp Asp Tyr Gly Asn Ile Lys Leu Gly Asn Gly Ser
465                 470                 475                 480

acc att att gac caa aat aca gaa ata aaa gtt tat aaa gtt aac cct    1488
Thr Ile Ile Asp Gln Asn Thr Glu Ile Lys Val Tyr Lys Val Asn Pro
                485                 490                 495

aat caa caa ttg cct caa agt aat aga atc tat gat ttt agt caa tac    1536
Asn Gln Gln Leu Pro Gln Ser Asn Arg Ile Tyr Asp Phe Ser Gln Tyr
            500                 505                 510

gaa gat gta aca agt caa ttt gat aat aaa aaa tca ttt agt aat aat    1584
Glu Asp Val Thr Ser Gln Phe Asp Asn Lys Lys Ser Phe Ser Asn Asn
        515                 520                 525

gta gca aca ttg gat ttt ggt gat att aat tca gcc tat att atc aaa    1632
Val Ala Thr Leu Asp Phe Gly Asp Ile Asn Ser Ala Tyr Ile Ile Lys
    530                 535                 540

gtt gtt agt aaa tat aca cct aca tca gat ggc gaa cta gat att gct    1680
Val Val Ser Lys Tyr Thr Pro Thr Ser Asp Gly Glu Leu Asp Ile Ala
545                 550                 555                 560

caa ggt act agt atg aga aca act gat aaa tat ggt tat tat aat tat    1728
Gln Gly Thr Ser Met Arg Thr Thr Asp Lys Tyr Gly Tyr Tyr Asn Tyr
                565                 570                 575

gca gga tat tca aac ttc atc gta act tct aat gac act ggc ggt ggc    1776
Ala Gly Tyr Ser Asn Phe Ile Val Thr Ser Asn Asp Thr Gly Gly Gly
            580                 585                 590

gac ggt act gtt aaa cct gaa gaa aag tta tac aaa att ggt gac tat    1824

Asp Gly Thr Val Lys Pro Glu Glu Lys Leu Tyr Lys Ile Gly Asp Tyr
        595                 600                 605

gta tgg gaa gac gtt gat aaa gac ggt gtc caa ggt aca gat tcg aaa     1872
Val Trp Glu Asp Val Asp Lys Asp Gly Val Gln Gly Thr Asp Ser Lys
    610                 615                 620

gaa aag cca atg gca aac gtt tta gtt aca tta act tac ccg gac ggt     1920
Glu Lys Pro Met Ala Asn Val Leu Val Thr Leu Thr Tyr Pro Asp Gly
625                 630                 635                 640

act aca aaa tca gta aga aca gat gct aac ggt cat tat gaa ttc ggt     1968
Thr Thr Lys Ser Val Arg Thr Asp Ala Asn Gly His Tyr Glu Phe Gly
                645                 650                 655

ggt ttg aaa gac gga gaa act tat aca gtt aaa ttc gaa acg cca gct     2016
Gly Leu Lys Asp Gly Glu Thr Tyr Thr Val Lys Phe Glu Thr Pro Ala
            660                 665                 670

gga tat ctt cca aca aaa gta aat gga aca act gat ggt gaa aaa gac     2064
Gly Tyr Leu Pro Thr Lys Val Asn Gly Thr Thr Asp Gly Glu Lys Asp
        675                 680                 685

tca aat ggt agt tct ata act gtt aaa att aat ggt aaa gat gat atg     2112
Ser Asn Gly Ser Ser Ile Thr Val Lys Ile Asn Gly Lys Asp Asp Met
    690                 695                 700

tct tta gac act ggt ttt tat aaa gaa cct aaa tat aat ctt ggt gac     2160
Ser Leu Asp Thr Gly Phe Tyr Lys Glu Pro Lys Tyr Asn Leu Gly Asp
705                 710                 715                 720

tat gta tgg gaa gat aca aat aaa gat ggt atc caa gat gct aat gaa     2208
Tyr Val Trp Glu Asp Thr Asn Lys Asp Gly Ile Gln Asp Ala Asn Glu
                725                 730                 735

cct ggt atc aaa gat gtt aag gtt aca tta aaa gat agt act gga aaa     2256
Pro Gly Ile Lys Asp Val Lys Val Thr Leu Lys Asp Ser Thr Gly Lys
            740                 745                 750

gtt att ggt aca act act act gat gcc tcg ggt aaa tat aaa ttt aca     2304
Val Ile Gly Thr Thr Thr Thr Asp Ala Ser Gly Lys Tyr Lys Phe Thr
        755                 760                 765

gat tta gat aat ggt aac tat aca gta gaa ttt gaa aca cca gca ggt     2352
Asp Leu Asp Asn Gly Asn Tyr Thr Val Glu Phe Glu Thr Pro Ala Gly
    770                 775                 780

tac acg cca acg gtt aaa aat act aca gct gaa gat aaa gat tct aat     2400
Tyr Thr Pro Thr Val Lys Asn Thr Thr Ala Glu Asp Lys Asp Ser Asn
785                 790                 795                 800

ggt tta aca aca aca ggt gtc att aaa gat gca gat aat atg aca tta     2448
Gly Leu Thr Thr Thr Gly Val Ile Lys Asp Ala Asp Asn Met Thr Leu
                805                 810                 815

gac agt ggt ttc tat aaa aca cca aaa tac agt tta ggt gat tat gtt     2496
Asp Ser Gly Phe Tyr Lys Thr Pro Lys Tyr Ser Leu Gly Asp Tyr Val
            820                 825                 830

tgg tac gac agt aat aaa gac ggt aaa caa gat tca act gaa aaa ggt     2544
Trp Tyr Asp Ser Asn Lys Asp Gly Lys Gln Asp Ser Thr Glu Lys Gly

        835                 840                 845

atc aaa gat gtt aaa gtt act tta tta aat gaa aaa ggc gaa gta att 2592
Ile Lys Asp Val Lys Val Thr Leu Leu Asn Glu Lys Gly Glu Val Ile
    850                 855                 860

gga aca act aaa aca gat gaa aat ggt aaa tat cgt ttc gat aat tta 2640
Gly Thr Thr Lys Thr Asp Glu Asn Gly Lys Tyr Arg Phe Asp Asn Leu
865                 870                 875                 880

gat agc ggt aaa tac aaa gtt att ttt gaa aag cct gct ggc tta aca 2688
Asp Ser Gly Lys Tyr Lys Val Ile Phe Glu Lys Pro Ala Gly Leu Thr
                885                 890                 895

caa aca gtt aca aat aca act gaa gat gat aaa gat gcc gat ggt ggc 2736
Gln Thr Val Thr Asn Thr Thr Glu Asp Asp Lys Asp Ala Asp Gly Gly
            900                 905                 910

gaa gtt gac gta aca att acg gat cat gat gat ttc aca ctt gat aac 2784
Glu Val Asp Val Thr Ile Thr Asp His Asp Asp Phe Thr Leu Asp Asn
        915                 920                 925

gga tac ttc gaa gaa gat aca tca gac agt gat tca gac tca gac agt 2832
Gly Tyr Phe Glu Glu Asp Thr Ser Asp Ser Asp Ser Asp Ser Asp Ser
    930                 935                 940

gat tca gac tca gac agc gac tca gat tca gac agt gat tca gac tca 2880
Asp Ser Asp Ser Asp Ser Asp Ser Asp Ser Asp Ser Asp Ser Asp Ser
945                 950                 955                 960

gat agc gat tca gat tca gac agc gac tca gac tca gat agc gac tca 2928
Asp Ser Asp Ser Asp Ser Asp Ser Asp Ser Asp Ser Asp Ser Asp Ser
                965                 970                 975

gac tca gac agc gac tca gac tca gat agc gac tca gat tcg gac agc 2976
Asp Ser Asp Ser Asp Ser Asp Ser Asp Ser Asp Ser Asp Ser Asp Ser
            980                 985                 990

gat tca gac tca gat agc gac tca gat tca gac agc gat tca gac tca 3024
Asp Ser Asp Ser Asp Ser Asp Ser Asp Ser Asp Ser Asp Ser Asp Ser
        995                 1000                1005

gat agc gac tca gat tca gac agt gac tca gac tca gat agc gac tca 3072
Asp Ser Asp Ser Asp Ser Asp Ser Asp Ser Asp Ser Asp Ser Asp Ser
    1010                1015                1020

gac tca gac agt gac tca gac tca gac agc gat tca gat tca gat agc 3120
Asp Ser Asp Ser Asp Ser Asp Ser Asp Ser Asp Ser Asp Ser Asp Ser
1025                1030                1035                1040

gac tca gat tcg gac agt gat tca gac tca gat agc gac tca gat tca 3168
Asp Ser Asp Ser Asp Ser Asp Ser Asp Ser Asp Ser Asp Ser Asp Ser
                1045                1050                1055

gac agc gac tca gac tca gat agc gac tca gac tca gac agt gat tca 3216
Asp Ser Asp Ser Asp Ser Asp Ser Asp Ser Asp Ser Asp Ser Asp Ser
            1060                1065                1070

gac tca gat agc gat tcg gac tcg gat gca gga aaa cat aca cct gtt 3264
Asp Ser Asp Ser Asp Ser Asp Ser Asp Ala Gly Lys His Thr Pro Val
        1075                1080                1085

aaa cca atg agt act act aaa gac cat cac aat aaa gca aaa gca tta    3312
Lys Pro Met Ser Thr Thr Lys Asp His His Asn Lys Ala Lys Ala Leu
   1090                1095                1100

cca gaa aca ggt agt gaa aat aac ggc tca aat aac gca acg tta ttt    3360
Pro Glu Thr Gly Ser Glu Asn Asn Gly Ser Asn Asn Ala Thr Leu Phe
1105                1110                1115                1120

ggt gga tta ttt gca gca tta ggt tca tta ttg tta ttc ggt cgt cgc    3408
Gly Gly Leu Phe Ala Ala Leu Gly Ser Leu Leu Leu Phe Gly Arg Arg
                1125                1130                1135

aaa aaa caa aac aaa taa                                            3426
Lys Lys Gln Asn Lys
            1140

<210> 8
<211> 1141
<212> PRT
<213> Staphylococcus sp.

<400> 8
Met Ile Asn Arg Asp Asn Lys Lys Ala Ile Thr Lys Lys Gly Met Ile
 1               5                  10                  15
Ser Asn Arg Leu Asn Lys Phe Ser Ile Arg Lys Tyr Thr Val Gly Thr
            20                  25                  30
Ala Ser Ile Leu Val Gly Thr Thr Leu Ile Phe Gly Leu Gly Asn Gln
        35                  40                  45
Glu Ala Lys Ala Ala Glu Asn Thr Ser Thr Glu Asn Ala Lys Gln Asp
    50                  55                  60
Asp Ala Thr Thr Ser Asp Asn Lys Glu Val Val Ser Glu Thr Glu Asn
 65                  70                  75                  80
Asn Ser Thr Thr Glu Asn Asp Ser Thr Asn Pro Ile Lys Lys Glu Thr
                85                  90                  95
Asn Thr Asp Ser Gln Pro Glu Ala Lys Glu Glu Ser Thr Thr Ser Ser
            100                 105                 110
Thr Gln Gln Gln Gln Asn Asn Val Thr Ala Thr Thr Glu Thr Lys Pro
        115                 120                 125
Gln Asn Ile Glu Lys Glu Asn Val Lys Pro Ser Thr Asp Lys Thr Ala
    130                 135                 140
Thr Glu Asp Thr Ser Val Ile Leu Glu Glu Lys Lys Ala Pro Asn Tyr
145                 150                 155                 160
Thr Asn Asn Asp Val Thr Thr Lys Pro Ser Thr Ser Glu Ile Gln Thr
                165                 170                 175
Lys Pro Thr Thr Pro Gln Glu Ser Thr Asn Ile Glu Asn Ser Gln Pro
            180                 185                 190
Gln Pro Thr Pro Ser Lys Val Asp Asn Gln Val Thr Asp Ala Thr Asn
        195                 200                 205
Pro Lys Glu Pro Val Asn Val Ser Lys Glu Glu Leu Lys Asn Asn Pro
    210                 215                 220
Glu Lys Leu Lys Glu Leu Val Arg Asn Asp Asn Asn Thr Asp Arg Ser
225                 230                 235                 240
Thr Lys Pro Val Ala Thr Ala Pro Thr Ser Val Ala Pro Lys Arg Leu
                245                 250                 255
Asn Ala Lys Met Arg Phe Ala Val Ala Gln Pro Ala Ala Val Ala Ser
            260                 265                 270
Asn Asn Val Asn Asp Leu Ile Thr Val Thr Lys Gln Thr Ile Lys Val
        275                 280                 285
Gly Asp Gly Lys Asp Asn Val Ala Ala Ala His Asp Gly Lys Asp Ile

290                 295                 300
Glu Tyr Asp Thr Glu Phe Thr Ile Asp Asn Lys Val Lys Lys Gly Asp
305                 310                 315                 320
Thr Met Thr Ile Asn Tyr Asp Lys Asn Val Ile Pro Ser Asp Leu Thr
                325                 330                 335
Asp Lys Asn Asp Pro Ile Asp Ile Thr Asp Pro Ser Gly Glu Val Ile
            340                 345                 350
Ala Lys Gly Thr Phe Asp Lys Ala Thr Lys Gln Ile Thr Tyr Thr Phe
        355                 360                 365
Thr Asp Tyr Val Asp Lys Tyr Glu Asp Ile Lys Ala Arg Leu Thr Leu
    370                 375                 380
Tyr Ser Tyr Ile Asp Lys Gln Ala Val Pro Asn Glu Thr Ser Leu Asn
385                 390                 395                 400
Leu Thr Phe Ala Thr Ala Gly Lys Glu Thr Ser Gln Asn Val Ser Val
                405                 410                 415
Asp Tyr Gln Asp Pro Met Val His Gly Asp Ser Asn Ile Gln Ser Ile
            420                 425                 430
Phe Thr Lys Leu Asp Glu Asn Lys Gln Thr Ile Glu Gln Gln Ile Tyr
        435                 440                 445
Val Asn Pro Leu Lys Lys Thr Ala Thr Asn Thr Lys Val Asp Ile Ala
    450                 455                 460
Gly Ser Gln Val Asp Asp Tyr Gly Asn Ile Lys Leu Gly Asn Gly Ser
465                 470                 475                 480
Thr Ile Ile Asp Gln Asn Thr Glu Ile Lys Val Tyr Lys Val Asn Pro
                485                 490                 495
Asn Gln Gln Leu Pro Gln Ser Asn Arg Ile Tyr Asp Phe Ser Gln Tyr
            500                 505                 510
Glu Asp Val Thr Ser Gln Phe Asp Asn Lys Lys Ser Phe Ser Asn Asn
        515                 520                 525
Val Ala Thr Leu Asp Phe Gly Asp Ile Asn Ser Ala Tyr Ile Ile Lys
    530                 535                 540
Val Val Ser Lys Tyr Thr Pro Thr Ser Asp Gly Glu Leu Asp Ile Ala
545                 550                 555                 560
Gln Gly Thr Ser Met Arg Thr Thr Asp Lys Tyr Gly Tyr Tyr Asn Tyr
                565                 570                 575
Ala Gly Tyr Ser Asn Phe Ile Val Thr Ser Asn Asp Thr Gly Gly Gly
            580                 585                 590
Asp Gly Thr Val Lys Pro Glu Glu Lys Leu Tyr Lys Ile Gly Asp Tyr
        595                 600                 605
Val Trp Glu Asp Val Asp Lys Asp Gly Val Gln Gly Thr Asp Ser Lys
    610                 615                 620
Glu Lys Pro Met Ala Asn Val Leu Val Thr Leu Thr Tyr Pro Asp Gly
625                 630                 635                 640
Thr Thr Lys Ser Val Arg Thr Asp Ala Asn Gly His Tyr Glu Phe Gly
                645                 650                 655
Gly Leu Lys Asp Gly Glu Thr Tyr Thr Val Lys Phe Glu Thr Pro Ala
            660                 665                 670
Gly Tyr Leu Pro Thr Lys Val Asn Gly Thr Thr Asp Gly Glu Lys Asp
        675                 680                 685
Ser Asn Gly Ser Ser Ile Thr Val Lys Ile Asn Gly Lys Asp Asp Met
    690                 695                 700
Ser Leu Asp Thr Gly Phe Tyr Lys Glu Pro Lys Tyr Asn Leu Gly Asp
705                 710                 715                 720
Tyr Val Trp Glu Asp Thr Asn Lys Asp Gly Ile Gln Asp Ala Asn Glu
                725                 730                 735
Pro Gly Ile Lys Asp Val Lys Val Thr Leu Lys Asp Ser Thr Gly Lys
            740                 745                 750
Val Ile Gly Thr Thr Thr Thr Asp Ala Ser Gly Lys Tyr Lys Phe Thr
        755                 760                 765
Asp Leu Asp Asn Gly Asn Tyr Thr Val Glu Phe Glu Thr Pro Ala Gly
    770                 775                 780

Tyr Thr Pro Thr Val Lys Asn Thr Thr Ala Glu Asp Lys Asp Ser Asn
785                 790                 795                 800
Gly Leu Thr Thr Thr Gly Val Ile Lys Asp Ala Asp Asn Met Thr Leu
                805                 810                 815
Asp Ser Gly Phe Tyr Lys Thr Pro Lys Tyr Ser Leu Gly Asp Tyr Val
            820                 825                 830
Trp Tyr Asp Ser Asn Lys Asp Gly Lys Gln Asp Ser Thr Glu Lys Gly
        835                 840                 845
Ile Lys Asp Val Lys Val Thr Leu Leu Asn Glu Lys Gly Glu Val Ile
    850                 855                 860
Gly Thr Thr Lys Thr Asp Glu Asn Gly Lys Tyr Arg Phe Asp Asn Leu
865                 870                 875                 880
Asp Ser Gly Lys Tyr Lys Val Ile Phe Glu Lys Pro Ala Gly Leu Thr
                885                 890                 895
Gln Thr Val Thr Asn Thr Thr Glu Asp Asp Lys Asp Ala Asp Gly Gly
            900                 905                 910
Glu Val Asp Val Thr Ile Thr Asp His Asp Asp Phe Thr Leu Asp Asn
        915                 920                 925
Gly Tyr Phe Glu Glu Asp Thr Ser Asp Ser Asp Ser Asp Ser Asp Ser
    930                 935                 940
Asp Ser Asp Ser Asp Ser Asp Ser Asp Ser Asp Ser Asp Ser Asp Ser
945                 950                 955                 960
Asp Ser Asp Ser Asp Ser Asp Ser Asp Ser Asp Ser Asp Ser Asp Ser
                965                 970                 975
Asp Ser Asp Ser Asp Ser Asp Ser Asp Ser Asp Ser Asp Ser Asp Ser
            980                 985                 990
Asp Ser Asp Ser Asp Ser Asp Ser Asp Ser Asp Ser Asp Ser Asp Ser
        995                 1000                1005
Asp Ser Asp Ser Asp Ser Asp Ser Asp Ser Asp Ser Asp Ser Asp Ser
   1010                 1015                1020
Asp Ser Asp Ser Asp Ser Asp Ser Asp Ser Asp Ser Asp Ser Asp Ser
1025                1030                1035                1040
Asp Ser Asp Ser Asp Ser Asp Ser Asp Ser Asp Ser Asp Ser Asp Ser
               1045                1050                1055
Asp Ser Asp Ser Asp Ser Asp Ser Asp Ser Asp Ser Asp Ser Asp Ser
           1060                1065                1070
Asp Ser Asp Ser Asp Ser Asp Ser Asp Ala Gly Lys His Thr Pro Val
       1075                1080                1085
Lys Pro Met Ser Thr Thr Lys Asp His His Asn Lys Ala Lys Ala Leu
   1090                1095                1100
Pro Glu Thr Gly Ser Glu Asn Asn Gly Ser Asn Asn Ala Thr Leu Phe
1105                1110                1115                1120
Gly Gly Leu Phe Ala Ala Leu Gly Ser Leu Leu Leu Phe Gly Arg Arg
               1125                1130                1135
Lys Lys Gln Asn Lys
           1140

<210> 9
<211> 1052
<212> DNA
<213> Staphylococcus sp.

<220>
<221> CDS
<222> (1)..(1050)

<400> 9
atg aca aaa cat tat tta aac agt aag tat caa tca gaa caa cgt tca    48
Met Thr Lys His Tyr Leu Asn Ser Lys Tyr Gln Ser Glu Gln Arg Ser

1               5                   10                  15

tca gct atg aaa aag att aca atg ggt aca gca tct atc att tta ggt    96
Ser Ala Met Lys Lys Ile Thr Met Gly Thr Ala Ser Ile Ile Leu Gly
                20                  25                  30

tcc ctt gta tac ata ggc gca gac agc caa caa gtc aat gcg gca aca    144
Ser Leu Val Tyr Ile Gly Ala Asp Ser Gln Gln Val Asn Ala Ala Thr
            35                  40                  45

gaa gct acg aac gca act aat aat caa agc aca caa gtt tct caa gca    192
Glu Ala Thr Asn Ala Thr Asn Asn Gln Ser Thr Gln Val Ser Gln Ala
    50                  55                  60

aca tca caa cca att aat ttc caa gtg caa aaa gat ggc tct tca gag    240
Thr Ser Gln Pro Ile Asn Phe Gln Val Gln Lys Asp Gly Ser Ser Glu
65                  70                  75                  80

aag tca cac atg gat gac tat atg caa cac cct ggt aaa gta att aaa    288
Lys Ser His Met Asp Asp Tyr Met Gln His Pro Gly Lys Val Ile Lys
                85                  90                  95

caa aat aat aaa tat tat ttc caa acc gtg tta aac aat gca tca ttc    336
Gln Asn Asn Lys Tyr Tyr Phe Gln Thr Val Leu Asn Asn Ala Ser Phe
            100                 105                 110

tgg aaa gaa tac aaa ttt tac aat gca aac aat caa gaa tta gca aca    384
Trp Lys Glu Tyr Lys Phe Tyr Asn Ala Asn Asn Gln Glu Leu Ala Thr
        115                 120                 125

act gtt gtt aac gat aat aaa aaa gcg gat act aga aca atc aat gtt    432
Thr Val Val Asn Asp Asn Lys Lys Ala Asp Thr Arg Thr Ile Asn Val
    130                 135                 140

gca gtt gaa cct gga tat aag agc tta act act aaa gta cat att gtc    480
Ala Val Glu Pro Gly Tyr Lys Ser Leu Thr Thr Lys Val His Ile Val
145                 150                 155                 160

gtg cca caa att aat tac aat cat aga tat act acg cat ttg gaa ttt    528
Val Pro Gln Ile Asn Tyr Asn His Arg Tyr Thr Thr His Leu Glu Phe
                165                 170                 175

gaa aaa gca att cct aca tta gct gac gca gca aaa cca aac aat gtt    576
Glu Lys Ala Ile Pro Thr Leu Ala Asp Ala Ala Lys Pro Asn Asn Val
            180                 185                 190

aaa ccg gtt caa cca aaa cca gct caa cct aaa aca cct act gag caa    624
Lys Pro Val Gln Pro Lys Pro Ala Gln Pro Lys Thr Pro Thr Glu Gln
        195                 200                 205

act aaa cca gtt caa cct aaa gtt gaa aaa gtt aaa cct act gta act    672
Thr Lys Pro Val Gln Pro Lys Val Glu Lys Val Lys Pro Thr Val Thr
    210                 215                 220

aca aca agc aaa gtt gaa gac aat cac tct act aaa gtt gta agt act    720
Thr Thr Ser Lys Val Glu Asp Asn His Ser Thr Lys Val Val Ser Thr
225                 230                 235                 240

gac aca aca aaa gat caa act aaa aca caa act gct cat aca gtt aaa    768
Asp Thr Thr Lys Asp Gln Thr Lys Thr Gln Thr Ala His Thr Val Lys
                245                 250                 255

aca gca caa act gct caa gaa caa aat aaa gtt caa aca cct gtt aaa    816
Thr Ala Gln Thr Ala Gln Glu Gln Asn Lys Val Gln Thr Pro Val Lys
            260                 265                 270

gat gtt gca aca gcg aaa tct gaa agc aac aat caa gct gta agt gat    864
Asp Val Ala Thr Ala Lys Ser Glu Ser Asn Asn Gln Ala Val Ser Asp
        275                 280                 285

aat aaa tca caa caa act aac aaa gtt aca aaa cat aac gaa acg cct    912
Asn Lys Ser Gln Gln Thr Asn Lys Val Thr Lys His Asn Glu Thr Pro
    290                 295                 300

aaa caa gca tct aaa gct aaa gaa tta cca aaa act ggt tta act tca    960
Lys Gln Ala Ser Lys Ala Lys Glu Leu Pro Lys Thr Gly Leu Thr Ser
305                 310                 315                 320

gtt gat aac ttt att agc aca gtt gcc ttc gca aca ctt gcc ctt tta    1008
Val Asp Asn Phe Ile Ser Thr Val Ala Phe Ala Thr Leu Ala Leu Leu
                325                 330                 335

ggt tca tta tct tta tta ctt ttc aaa aga aaa gaa tct aaa ta         1052
Gly Ser Leu Ser Leu Leu Leu Phe Lys Arg Lys Glu Ser Lys
            340                 345                 350


<210> 10
<211> 350
<212> PRT
<213> Staphylococcus sp.

<400> 10
Met Thr Lys His Tyr Leu Asn Ser Lys Tyr Gln Ser Glu Gln Arg Ser
1               5                   10                  15

Ser Ala Met Lys Lys Ile Thr Met Gly Thr Ala Ser Ile Ile Leu Gly
            20                  25                  30

Ser Leu Val Tyr Ile Gly Ala Asp Ser Gln Gln Val Asn Ala Ala Thr
        35                  40                  45

Glu Ala Thr Asn Ala Thr Asn Asn Gln Ser Thr Gln Val Ser Gln Ala
    50                  55                  60

Thr Ser Gln Pro Ile Asn Phe Gln Val Gln Lys Asp Gly Ser Ser Glu
65                  70                  75                  80

Lys Ser His Met Asp Asp Tyr Met Gln His Pro Gly Lys Val Ile Lys
                85                  90                  95

Gln Asn Asn Lys Tyr Tyr Phe Gln Thr Val Leu Asn Asn Ala Ser Phe
            100                 105                 110

Trp Lys Glu Tyr Lys Phe Tyr Asn Ala Asn Asn Gln Glu Leu Ala Thr
        115                 120                 125

Thr Val Val Asn Asp Asn Lys Lys Ala Asp Thr Arg Thr Ile Asn Val
    130                 135                 140

Ala Val Glu Pro Gly Tyr Lys Ser Leu Thr Thr Lys Val His Ile Val
145                 150                 155                 160

Val Pro Gln Ile Asn Tyr Asn His Arg Tyr Thr Thr His Leu Glu Phe
                165                 170                 175

Glu Lys Ala Ile Pro Thr Leu Ala Asp Ala Ala Lys Pro Asn Asn Val
            180                 185                 190

Lys Pro Val Gln Pro Lys Pro Ala Gln Pro Lys Thr Pro Thr Glu Gln
        195                 200                 205

Thr Lys Pro Val Gln Pro Lys Val Glu Lys Val Lys Pro Thr Val Thr
    210                 215                 220

Thr Thr Ser Lys Val Glu Asp Asn His Ser Thr Lys Val Val Ser Thr
225                 230                 235                 240

Asp Thr Thr Lys Asp Gln Thr Lys Thr Gln Thr Ala His Thr Val Lys
                245                 250                 255

Thr Ala Gln Thr Ala Gln Glu Gln Asn Lys Val Gln Thr Pro Val Lys
            260                 265                 270

Asp Val Ala Thr Ala Lys Ser Glu Ser Asn Asn Gln Ala Val Ser Asp
        275                 280                 285

Asn Lys Ser Gln Gln Thr Asn Lys Val Thr Lys His Asn Glu Thr Pro
    290                 295                 300

Lys Gln Ala Ser Lys Ala Lys Glu Leu Pro Lys Thr Gly Leu Thr Ser
305                 310                 315                 320

Val Asp Asn Phe Ile Ser Thr Val Ala Phe Ala Thr Leu Ala Leu Leu
                325                 330                 335

Gly Ser Leu Ser Leu Leu Leu Phe Lys Arg Lys Glu Ser Lys
            340                 345                 350

<210> 11
<211> 1938
<212> DNA
<213> Staphylococcus sp.

<400> 11
ttagttttta cgttttctag gtaatacgaa tgcaacgatg ctacttaaag ctagtaatgc 60
cattaatggt aatgtcatat ctttatttga ttcttcacca gtttgtggta atgattttgc 120
tttattttct tgtgtatttt tattgttttg gctttgagtg tgtccatcat ttgtgttttt 180
aatgtttgct ttttgtaatg gagcactatc ttttgcttcg ctagaacctg ctgaagtttg 240
aacaacatct tttgttgttt ttgatgaagc agttgttggt tttgcaacat tttgagtcgt 300
agatactacc ttagttggag ttgtactact tgattctact tcacctttag ttggttttgt 360
agcaggcgtt ttgtctttac ctgactcact agatgcgtca ttttcttttt caacacttgg 420
taattgttta ttgtcatctt tttggctgtc ttgtttttgt gattcttttt caacaggtga 480
tggtgttggt ttgctaggcg tagctggagt agcttccttc ttagctgagt tatcttgttg 540
ttcttttttg ttagatttat cggtattggc ttttgtaaat gcttctttat caacgattct 600
gacatggtat tgtccatcat aatcaatcgt ttttacgtga actttaacga tagcatcata 660
tagagtttta ccttcaacat atgggaaaat aattgttcta gtattatttt tagcatcttt 720
gcttatagtt ctaacacgtt gaccttcaac catgaaatct ttccagtaat cgtcattagt 780
agtttccatg accatatatt ttttgccgtt aagcatacct gttttaatag ggtgtttaac 840
aaaagtatcc atcatagatt cgttattctc aacactttca taaacaacat attttgtatc 900
ttgtaaatca gtcatttttt catttgttgg ttgtacattt tggaattcag taatagctga 960

tttcacttgc tcatctaaag ctttctttgt atcctctaat ttcttcttgt actcagcctt 1020
taatttttca ggaagtttat cttgaatttt atttaattca taaacttgtc tttctagtgt 1080
tttcgctttt ttatatggcg ctaataattt ttcagcttta taatcttctt cagttttgaa 1140
tttatctgca ctgttataaa ttggttgtgc gaattccatt aatgtgtaat cgtatttttc 1200
ttctttgtta ttgaagtgag ttgaacttac aattttaacg gcttttgttc catttgaaac 1260
agagaagcga atgtaagcgt aatctttaac agtatcgtat gatactaatt taattggcaa 1320
ctttttgtca ccttcataaa cttcaaattt tctccaaaat tgacctgatt gtaatcctaa 1380
ttcaatttct ggttttgaat cagtgaaaat aactctagca ggtttaacag agctggcata 1440
atgataaaat tgttgctcac catttctttt tttcatttca aaatcaattg gacgagagtt 1500
tggtgcgcta tgatctttat cttttattgc agggttttta atcgcttctc taagttcctg 1560
attcaaaata ggatatgtat tgttagtggc ttttgctgct ggtttaactg cttttgtttc 1620
cttaggggct ttaacttctt taacttcttt agcttctttt gtttcagaag taggggcctc 1680
aacttcttta ttagatactg agacagcatt agctactggt ttagtttctg gagctttttc 1740
agatgttgtt gttggacttg caactgcttc agtttttggt tgtgcttctg tatttgtacc 1800
acctgtttct tcagctgctg cttgtgcttc gccatttgac attaataata aaagtgtact 1860
aatcgctaca gatgcaacgc ctagtgatga ctttctaatt gaataaaatg atttaaattc 1920
tttttgctgt ttgttcat 1938

<210> 12
<211> 645
<212> PRT
<213> Staphylococcus sp.

<400> 12
Met Asn Lys Gln Gln Lys Glu Phe Lys Ser Phe Tyr Ser Ile Arg Lys
1               5                   10                  15

Ser Ser Leu Gly Val Ala Ser Val Ala Ile Ser Thr Leu Leu Leu Leu
            20                  25                  30

Met Ser Asn Gly Glu Ala Gln Ala Ala Ala Glu Glu Thr Gly Gly Thr
        35                  40                  45

Asn Thr Glu Ala Gln Pro Lys Thr Glu Ala Val Ala Ser Pro Thr Thr
    50                  55                  60

Thr Ser Glu Lys Ala Pro Glu Thr Lys Pro Val Ala Asn Ala Val Ser
65                  70                  75                  80

Val Ser Asn Lys Glu Val Glu Ala Pro Thr Ser Glu Thr Lys Glu Ala
                85                  90                  95

Lys Glu Val Lys Glu Val Lys Ala Pro Lys Glu Thr Lys Ala Val Lys
            100                 105                 110

Pro Ala Ala Lys Ala Thr Asn Asn Thr Tyr Pro Ile Leu Asn Gln Glu
        115                 120                 125

Leu Arg Glu Ala Ile Lys Asn Pro Ala Ile Lys Asp Lys Asp His Ser
    130                 135                 140

Ala Pro Asn Ser Arg Pro Ile Asp Phe Glu Met Lys Lys Glu Asn Gly
145                 150                 155                 160

Glu Gln Gln Phe Tyr His Tyr Ala Ser Ser Val Lys Pro Ala Arg Val
                165                 170                 175

Ile Phe Thr Asp Ser Lys Pro Glu Ile Glu Leu Gly Leu Gln Ser Gly
            180                 185                 190

Gln Phe Trp Arg Lys Phe Glu Val Tyr Glu Gly Asp Lys Lys Leu Pro
        195                 200                 205

Ile Lys Leu Val Ser Tyr Asp Thr Val Lys Asp Tyr Ala Tyr Ile Arg
    210                 215                 220

Phe Ser Val Ser Asn Gly Thr Lys Ala Val Lys Ile Val Ser Ser Thr
225                 230                 235                 240

His Phe Asn Asn Lys Glu Glu Lys Tyr Asp Tyr Thr Leu Met Glu Phe
                245                 250                 255

Ala Gln Pro Ile Tyr Asn Ser Ala Asp Lys Phe Lys Thr Glu Glu Asp
            260                 265                 270

Tyr Lys Ala Glu Lys Leu Leu Ala Pro Tyr Lys Lys Ala Lys Thr Leu
        275                 280                 285

Glu Arg Gln Val Tyr Glu Leu Asn Lys Ile Gln Asp Lys Leu Pro Glu
    290                 295                 300

Lys Leu Lys Ala Glu Tyr Lys Lys Lys Leu Glu Asp Thr Lys Lys Ala
305                 310                 315                 320

Leu Asp Glu Gln Val Lys Ser Ala Ile Thr Glu Phe Gln Asn Val Gln
                325                 330                 335

Pro Thr Asn Glu Lys Met Thr Asp Leu Gln Asp Thr Lys Tyr Val Val
            340                 345                 350

Tyr Glu Ser Val Glu Asn Asn Glu Ser Met Met Asp Thr Phe Val Lys
        355                 360                 365

His Pro Ile Lys Thr Gly Met Leu Asn Gly Lys Lys Tyr Met Val Met
    370                 375                 380

Glu Thr Thr Asn Asp Asp Tyr Trp Lys Asp Phe Met Val Glu Gly Gln
385                 390                 395                 400

Arg Val Arg Thr Ile Ser Lys Asp Ala Lys Asn Asn Thr Arg Thr Ile
                405                 410                 415

Ile Phe Pro Tyr Val Glu Gly Lys Thr Leu Tyr Asp Ala Ile Val Lys
            420                 425                 430

Val His Val Lys Thr Ile Asp Tyr Asp Gly Gln Tyr His Val Arg Ile
        435                 440                 445

Val Asp Lys Glu Ala Phe Thr Lys Ala Asn Thr Asp Lys Ser Asn Lys
    450                 455                 460

Lys Glu Gln Gln Asp Asn Ser Ala Lys Lys Glu Ala Thr Pro Ala Thr
465                 470                 475                 480

Pro Ser Lys Pro Thr Pro Ser Pro Val Glu Lys Glu Ser Gln Lys Gln
                485                 490                 495

Asp Ser Gln Lys Asp Asp Asn Lys Gln Leu Pro Ser Val Glu Lys Glu
            500                 505                 510

Asn Asp Ala Ser Ser Glu Ser Gly Lys Asp Lys Thr Pro Ala Thr Lys

515                 520                 525

Pro Thr Lys Gly Glu Val Glu Ser Ser Ser Thr Thr Pro Thr Lys Val
    530                 535                 540

Val Ser Thr Thr Gln Asn Val Ala Lys Pro Thr Thr Ala Ser Ser Lys
545                 550                 555                 560

Thr Thr Lys Asp Val Val Gln Thr Ser Ala Gly Ser Ser Glu Ala Lys
                565                 570                 575

Asp Ser Ala Pro Leu Gln Lys Ala Asn Ile Lys Asn Thr Asn Asp Gly
            580                 585                 590

His Thr Gln Ser Gln Asn Asn Lys Asn Thr Gln Glu Asn Lys Ala Lys
        595                 600                 605

Ser Leu Pro Gln Thr Gly Glu Glu Ser Asn Lys Asp Met Thr Leu Pro
    610                 615                 620

Leu Met Ala Leu Leu Ala Leu Ser Ser Ile Val Ala Phe Val Leu Pro
625                 630                 635                 640

Arg Lys Arg Lys Asn
                645


<210> 13
<211> 1353
<212> DNA
<213> Staphylococcus sp.

<220>
<221> CDS
<222> (1)..(1353)

<400> 13
ttg aaa aag aaa aac att tat tca att cgt aaa cta ggt gta ggt att      48
Leu Lys Lys Lys Asn Ile Tyr Ser Ile Arg Lys Leu Gly Val Gly Ile
1               5                   10                  15

gca tct gta act tta ggt aca tta ctt ata tct ggt ggc gta aca cct      96
Ala Ser Val Thr Leu Gly Thr Leu Leu Ile Ser Gly Gly Val Thr Pro
            20                  25                  30

gct gca aat gct gcg caa cac gat gaa gct caa caa aat gct ttt tat     144
Ala Ala Asn Ala Ala Gln His Asp Glu Ala Gln Gln Asn Ala Phe Tyr
        35                  40                  45

caa gtg tta aat atg cct aac tta aac gct gat caa cgt aat ggt ttt     192
Gln Val Leu Asn Met Pro Asn Leu Asn Ala Asp Gln Arg Asn Gly Phe
    50                  55                  60

atc caa agc ctt aaa gat gat cca agc caa agt gct aac gtt tta ggt     240
Ile Gln Ser Leu Lys Asp Asp Pro Ser Gln Ser Ala Asn Val Leu Gly
65                  70                  75                  80

gaa gct caa aaa ctt aat gac tct caa gct cca aaa gct gat gcg caa     288
Glu Ala Gln Lys Leu Asn Asp Ser Gln Ala Pro Lys Ala Asp Ala Gln
                85                  90                  95

caa aat aac ttc aac aaa gat caa caa agc gcc ttc tat gaa atc ttg 336
Gln Asn Asn Phe Asn Lys Asp Gln Gln Ser Ala Phe Tyr Glu Ile Leu
            100                 105                 110

aac atg cct aac tta aac gaa gcg caa cgt aac ggc ttc att caa agt 384
Asn Met Pro Asn Leu Asn Glu Ala Gln Arg Asn Gly Phe Ile Gln Ser
        115                 120                 125

ctt aaa gac gac cca agc caa agc act aat gtt tta ggt gaa gct aaa 432
Leu Lys Asp Asp Pro Ser Gln Ser Thr Asn Val Leu Gly Glu Ala Lys
    130                 135                 140

aaa tta aac gaa tct caa gca ccg aaa gct gat aac aat ttc aac aaa 480
Lys Leu Asn Glu Ser Gln Ala Pro Lys Ala Asp Asn Asn Phe Asn Lys
145                 150                 155                 160

gaa caa caa aat gct ttc tat gaa atc ttg aat atg cct aac tta aac 528
Glu Gln Gln Asn Ala Phe Tyr Glu Ile Leu Asn Met Pro Asn Leu Asn
                165                 170                 175

gaa gaa caa cgc aat ggt ttc atc caa agc tta aaa gat gac cca agc 576
Glu Glu Gln Arg Asn Gly Phe Ile Gln Ser Leu Lys Asp Asp Pro Ser
            180                 185                 190

caa agt gct aac cta ttg tca gaa gct aaa aag tta aat gaa tct caa 624
Gln Ser Ala Asn Leu Leu Ser Glu Ala Lys Lys Leu Asn Glu Ser Gln
        195                 200                 205

gca ccg aaa gcg gat aac aaa ttc aac aaa gaa caa caa aat gct ttc 672
Ala Pro Lys Ala Asp Asn Lys Phe Asn Lys Glu Gln Gln Asn Ala Phe
    210                 215                 220

tat gaa atc tta cat tta cct aac tta aac gaa gaa caa cgt aac ggc 720
Tyr Glu Ile Leu His Leu Pro Asn Leu Asn Glu Glu Gln Arg Asn Gly
225                 230                 235                 240

ttc atc caa agc ctt aaa gac gat cct tca gtg agc aaa gaa att tta 768
Phe Ile Gln Ser Leu Lys Asp Asp Pro Ser Val Ser Lys Glu Ile Leu
                245                 250                 255

gca gaa gct aaa aag cta aac gat gct caa gca cca aaa gag gaa gac 816
Ala Glu Ala Lys Lys Leu Asn Asp Ala Gln Ala Pro Lys Glu Glu Asp
            260                 265                 270

aac aaa aaa cct ggt aaa gaa gac ggc aac aaa cct ggc aaa gaa gac 864
Asn Lys Lys Pro Gly Lys Glu Asp Gly Asn Lys Pro Gly Lys Glu Asp
        275                 280                 285

ggc aac aag cct ggt aaa gaa gac aac aaa aaa cct ggt aaa gaa gac 912
Gly Asn Lys Pro Gly Lys Glu Asp Asn Lys Lys Pro Gly Lys Glu Asp
    290                 295                 300

ggc aac aag cct ggt aaa gaa gac aac aac aaa cct ggc aaa gaa gac 960
Gly Asn Lys Pro Gly Lys Glu Asp Asn Asn Lys Pro Gly Lys Glu Asp
305                 310                 315                 320

ggc aac aag cct ggt aaa gaa gac aac aac aag cct ggt aaa gaa gac 1008
Gly Asn Lys Pro Gly Lys Glu Asp Asn Asn Lys Pro Gly Lys Glu Asp
                325                 330                 335

ggc aac aag cct ggt aaa gaa gac ggc aac aaa cct ggt aaa gaa gac 1056

Gly Asn Lys Pro Gly Lys Glu Asp Gly Asn Lys Pro Gly Lys Glu Asp
            340                 345                 350

ggc aac gga gta cat gtc gtt aaa cct ggt gat aca gta aat gac att      1104
Gly Asn Gly Val His Val Val Lys Pro Gly Asp Thr Val Asn Asp Ile
        355                 360                 365

gca aaa gca aac ggc act act gct gac aaa att gct gca gat aac aaa      1152
Ala Lys Ala Asn Gly Thr Thr Ala Asp Lys Ile Ala Ala Asp Asn Lys
    370                 375                 380

tta gct gat aaa aac atg atc aaa cct ggt caa gaa ctt gtt gtt gat      1200
Leu Ala Asp Lys Asn Met Ile Lys Pro Gly Gln Glu Leu Val Val Asp
385                 390                 395                 400

aag aag caa cca gca aac cat gca gat gct aac aaa gct caa gca tta      1248
Lys Lys Gln Pro Ala Asn His Ala Asp Ala Asn Lys Ala Gln Ala Leu
                405                 410                 415

cca gaa act ggt gaa gaa aat cca ttc atc ggt aca act gta ttt ggt      1296
Pro Glu Thr Gly Glu Glu Asn Pro Phe Ile Gly Thr Thr Val Phe Gly
            420                 425                 430

gga tta tca tta gcc tta ggt gca gcg tta tta gct gga cgt cgt cgc      1344
Gly Leu Ser Leu Ala Leu Gly Ala Ala Leu Leu Ala Gly Arg Arg Arg
        435                 440                 445

gaa cta taa                                                          1353
Glu Leu
    450


<210> 14
<211> 450
<212> PRT
<213> Staphylococcus sp.

<400> 14
Leu Lys Lys Lys Asn Ile Tyr Ser Ile Arg Lys Leu Gly Val Gly Ile
  1               5                  10                  15
Ala Ser Val Thr Leu Gly Thr Leu Leu Ile Ser Gly Gly Val Thr Pro
            20                  25                  30
Ala Ala Asn Ala Ala Gln His Asp Glu Ala Gln Gln Asn Ala Phe Tyr
        35                  40                  45
Gln Val Leu Asn Met Pro Asn Leu Asn Ala Asp Gln Arg Asn Gly Phe
     50                  55                  60
Ile Gln Ser Leu Lys Asp Asp Pro Ser Gln Ser Ala Asn Val Leu Gly
 65                  70                  75                  80
Glu Ala Gln Lys Leu Asn Asp Ser Gln Ala Pro Lys Ala Asp Ala Gln
                 85                  90                  95
Gln Asn Asn Phe Asn Lys Asp Gln Gln Ser Ala Phe Tyr Glu Ile Leu
            100                 105                 110
Asn Met Pro Asn Leu Asn Glu Ala Gln Arg Asn Gly Phe Ile Gln Ser
        115                 120                 125
Leu Lys Asp Asp Pro Ser Gln Ser Thr Asn Val Leu Gly Glu Ala Lys
    130                 135                 140
Lys Leu Asn Glu Ser Gln Ala Pro Lys Ala Asp Asn Asn Phe Asn Lys
145                 150                 155                 160
Glu Gln Gln Asn Ala Phe Tyr Glu Ile Leu Asn Met Pro Asn Leu Asn
                165                 170                 175
Glu Glu Gln Arg Asn Gly Phe Ile Gln Ser Leu Lys Asp Asp Pro Ser

180                 185                 190
Gln Ser Ala Asn Leu Leu Ser Glu Ala Lys Lys Leu Asn Glu Ser Gln
        195                 200                 205
Ala Pro Lys Ala Asp Asn Lys Phe Asn Lys Glu Gln Gln Asn Ala Phe
    210                 215                 220
Tyr Glu Ile Leu His Leu Pro Asn Leu Asn Glu Glu Gln Arg Asn Gly
225                 230                 235                 240
Phe Ile Gln Ser Leu Lys Asp Asp Pro Ser Val Ser Lys Glu Ile Leu
                245                 250                 255
Ala Glu Ala Lys Lys Leu Asn Asp Ala Gln Ala Pro Lys Glu Glu Asp
            260                 265                 270
Asn Lys Lys Pro Gly Lys Glu Asp Gly Asn Lys Pro Gly Lys Glu Asp
        275                 280                 285
Gly Asn Lys Pro Gly Lys Glu Asp Asn Lys Lys Pro Gly Lys Glu Asp
    290                 295                 300
Gly Asn Lys Pro Gly Lys Glu Asp Asn Asn Lys Pro Gly Lys Glu Asp
305                 310                 315                 320
Gly Asn Lys Pro Gly Lys Glu Asp Asn Asn Lys Pro Gly Lys Glu Asp
                325                 330                 335
Gly Asn Lys Pro Gly Lys Glu Asp Gly Asn Lys Pro Gly Lys Glu Asp
            340                 345                 350
Gly Asn Gly Val His Val Val Lys Pro Gly Asp Thr Val Asn Asp Ile
        355                 360                 365
Ala Lys Ala Asn Gly Thr Thr Ala Asp Lys Ile Ala Ala Asp Asn Lys
    370                 375                 380
Leu Ala Asp Lys Asn Met Ile Lys Pro Gly Gln Glu Leu Val Val Asp
385                 390                 395                 400
Lys Lys Gln Pro Ala Asn His Ala Asp Ala Asn Lys Ala Gln Ala Leu
                405                 410                 415
Pro Glu Thr Gly Glu Glu Asn Pro Phe Ile Gly Thr Thr Val Phe Gly
            420                 425                 430
Gly Leu Ser Leu Ala Leu Gly Ala Ala Leu Leu Ala Gly Arg Arg Arg
        435                 440                 445
Glu Leu
    450

<210> 15
<211> 2634
<212> DNA
<213> Staphylococcus sp.

<220>
<221> CDS
<222> (1)..(2634)

<400> 15
ttg aaa aaa aga att gat tat ttg tcg aat aag cag aat aag tat tcg      48
Leu Lys Lys Arg Ile Asp Tyr Leu Ser Asn Lys Gln Asn Lys Tyr Ser
1               5                   10                  15

att aga cgt ttt aca gta ggt acc aca tca gta ata gta ggg gca act      96
Ile Arg Arg Phe Thr Val Gly Thr Thr Ser Val Ile Val Gly Ala Thr
            20                  25                  30

ata cta ttt ggg ata ggc aat cat caa gca caa gct tca gaa caa tcg      144
Ile Leu Phe Gly Ile Gly Asn His Gln Ala Gln Ala Ser Glu Gln Ser
        35                  40                  45

aac gat aca acg caa tct tcg aaa aat aat gca agt gca gat tcc gaa      192

Asn Asp Thr Thr Gln Ser Ser Lys Asn Asn Ala Ser Ala Asp Ser Glu
50                  55                  60

aaa aac aat atg ata gaa aca cct caa tta aat aca acg gct aat gat     240
Lys Asn Asn Met Ile Glu Thr Pro Gln Leu Asn Thr Thr Ala Asn Asp
65                  70                  75                  80

aca tct gat att agt gca aac aca aac agt gcg aat gta gat agc aca     288
Thr Ser Asp Ile Ser Ala Asn Thr Asn Ser Ala Asn Val Asp Ser Thr
                85                  90                  95

aca aaa cca atg tct aca caa acg agc aat acc act aca aca gag cca     336
Thr Lys Pro Met Ser Thr Gln Thr Ser Asn Thr Thr Thr Thr Glu Pro
            100                 105                 110

gct tca aca aat gaa aca cct caa ccg acg gca att aaa aat caa gca     384
Ala Ser Thr Asn Glu Thr Pro Gln Pro Thr Ala Ile Lys Asn Gln Ala
        115                 120                 125

act gct gca aaa atg caa gat caa act gtt cct caa gaa gca aat tct     432
Thr Ala Ala Lys Met Gln Asp Gln Thr Val Pro Gln Glu Ala Asn Ser
    130                 135                 140

caa gta gat aat aaa aca acg aat gat gct aat agc ata gca aca aac     480
Gln Val Asp Asn Lys Thr Thr Asn Asp Ala Asn Ser Ile Ala Thr Asn
145                 150                 155                 160

agt gag ctt aaa aat tct caa aca tta gat tta cca caa tca tca cca     528
Ser Glu Leu Lys Asn Ser Gln Thr Leu Asp Leu Pro Gln Ser Ser Pro
                165                 170                 175

caa acg att tcc aat gcg caa gga act agt aaa cca agt gtt aga acg     576
Gln Thr Ile Ser Asn Ala Gln Gly Thr Ser Lys Pro Ser Val Arg Thr
            180                 185                 190

aga gct gta cgt agt tta gct gtt gct gaa ccg gta gta aat gct gct     624
Arg Ala Val Arg Ser Leu Ala Val Ala Glu Pro Val Val Asn Ala Ala
        195                 200                 205

gat gct aaa ggt aca aat gta aat gat aaa gtt acg gca agt aat ttc     672
Asp Ala Lys Gly Thr Asn Val Asn Asp Lys Val Thr Ala Ser Asn Phe
    210                 215                 220

aag tta gaa aag act aca ttt gac cct aat caa agt ggt aac aca ttt     720
Lys Leu Glu Lys Thr Thr Phe Asp Pro Asn Gln Ser Gly Asn Thr Phe
225                 230                 235                 240

atg gcg gca aat ttt aca gtg aca gat aaa gtg aaa tca ggg gat tat     768
Met Ala Ala Asn Phe Thr Val Thr Asp Lys Val Lys Ser Gly Asp Tyr
                245                 250                 255

ttt aca gcg aag tta cca gat agt tta act ggt aat gga gac gtg gat     816
Phe Thr Ala Lys Leu Pro Asp Ser Leu Thr Gly Asn Gly Asp Val Asp
            260                 265                 270

tat tct aat tca aat aat acg atg cca att gca gac att aaa agt acg     864
Tyr Ser Asn Ser Asn Asn Thr Met Pro Ile Ala Asp Ile Lys Ser Thr
        275                 280                 285

aat ggc gat gtt gta gct aaa gca aca tat gat atc ttg act aag acg     912
Asn Gly Asp Val Val Ala Lys Ala Thr Tyr Asp Ile Leu Thr Lys Thr

290                 295                 300

tat aca ttt gtc ttt aca gat tat gta aat aat aaa gaa aat att aac     960
Tyr Thr Phe Val Phe Thr Asp Tyr Val Asn Asn Lys Glu Asn Ile Asn
305                 310                 315                 320

gga caa ttt tca tta cct tta ttt aca gac cga gca aag gca cct aaa     1008
Gly Gln Phe Ser Leu Pro Leu Phe Thr Asp Arg Ala Lys Ala Pro Lys
                325                 330                 335

tca gga aca tat gat gcg aat att aat att gcg gat gaa atg ttt aat     1056
Ser Gly Thr Tyr Asp Ala Asn Ile Asn Ile Ala Asp Glu Met Phe Asn
            340                 345                 350

aat aaa att act tat aac tat agt tcg cca att gca gga att gat aaa     1104
Asn Lys Ile Thr Tyr Asn Tyr Ser Ser Pro Ile Ala Gly Ile Asp Lys
        355                 360                 365

cca aat ggc gcg aac att tct tct caa att att ggt gta gat aca gct     1152
Pro Asn Gly Ala Asn Ile Ser Ser Gln Ile Ile Gly Val Asp Thr Ala
    370                 375                 380

tca ggt caa aac aca tac aag caa aca gta ttt gtt aac cct aag caa     1200
Ser Gly Gln Asn Thr Tyr Lys Gln Thr Val Phe Val Asn Pro Lys Gln
385                 390                 395                 400

cga gtt tta ggt aat acg tgg gtg tat att aaa ggc tac caa gat aaa     1248
Arg Val Leu Gly Asn Thr Trp Val Tyr Ile Lys Gly Tyr Gln Asp Lys
                405                 410                 415

atc gaa gaa agt agc ggt aaa gta agt gct aca gat aca aaa ctg aga     1296
Ile Glu Glu Ser Ser Gly Lys Val Ser Ala Thr Asp Thr Lys Leu Arg
            420                 425                 430

att ttt gaa gtg aat gat aca tct aaa tta tca gat agc tac tat gca     1344
Ile Phe Glu Val Asn Asp Thr Ser Lys Leu Ser Asp Ser Tyr Tyr Ala
        435                 440                 445

gat cca aat gac tct aac ctt aaa gaa gta aca gac caa ttt aaa aat     1392
Asp Pro Asn Asp Ser Asn Leu Lys Glu Val Thr Asp Gln Phe Lys Asn
    450                 455                 460

aga atc tat tat gag cat cca aat gta gct agt att aaa ttt ggt gat     1440
Arg Ile Tyr Tyr Glu His Pro Asn Val Ala Ser Ile Lys Phe Gly Asp
465                 470                 475                 480

att act aaa aca tat gta gta tta gta gaa ggg cat tac gac aat aca     1488
Ile Thr Lys Thr Tyr Val Val Leu Val Glu Gly His Tyr Asp Asn Thr
                485                 490                 495

ggt aag aac tta aaa act cag gtt att caa gaa aat gtt gat cct gta     1536
Gly Lys Asn Leu Lys Thr Gln Val Ile Gln Glu Asn Val Asp Pro Val
            500                 505                 510

aca aat aga gac tac agt att ttc ggt tgg aat aat gag aat gtt gta     1584
Thr Asn Arg Asp Tyr Ser Ile Phe Gly Trp Asn Asn Glu Asn Val Val
        515                 520                 525

cgt tat ggt ggt gga agt gct gat ggt gat tca gca gta aat ccg aaa     1632
Arg Tyr Gly Gly Gly Ser Ala Asp Gly Asp Ser Ala Val Asn Pro Lys
    530                 535                 540

gac cca act cca ggg ccg ccg gtt gac cca gaa cca agt cca gac cca    1680
Asp Pro Thr Pro Gly Pro Pro Val Asp Pro Glu Pro Ser Pro Asp Pro
545                 550                 555                 560

gaa cca gaa cca acg cca gat cca gaa cca agt cca gac cca gaa ccg    1728
Glu Pro Glu Pro Thr Pro Asp Pro Glu Pro Ser Pro Asp Pro Glu Pro
                565                 570                 575

gaa cca agc cca gac ccg gat ccg gat tcg gat tca gac agt gac tca    1776
Glu Pro Ser Pro Asp Pro Asp Pro Asp Ser Asp Ser Asp Ser Asp Ser
            580                 585                 590

ggc tca gac agc gac tca ggt tca gat agc gac tca gaa tca gat agc    1824
Gly Ser Asp Ser Asp Ser Gly Ser Asp Ser Asp Ser Glu Ser Asp Ser
        595                 600                 605

gat tcg gat tca gac agt gat tca gat tca gac agc gac tca gaa tca    1872
Asp Ser Asp Ser Asp Ser Asp Ser Asp Ser Asp Ser Asp Ser Glu Ser
    610                 615                 620

gat agc gat tca gaa tca gat agc gac tca gat tca gat agc gat tca    1920
Asp Ser Asp Ser Glu Ser Asp Ser Asp Ser Asp Ser Asp Ser Asp Ser
625                 630                 635                 640

gat tca gat agc gat tca gaa tca gat agc gat tcg gat tca gac agt    1968
Asp Ser Asp Ser Asp Ser Glu Ser Asp Ser Asp Ser Asp Ser Asp Ser
                645                 650                 655

gat tca gat tca gac agc gac tca gaa tca gat agc gac tca gaa tca    2016
Asp Ser Asp Ser Asp Ser Asp Ser Glu Ser Asp Ser Asp Ser Glu Ser
            660                 665                 670

gat agt gag tca gat tca gac agt gac tcg gac tca gac agt gat tca    2064
Asp Ser Glu Ser Asp Ser Asp Ser Asp Ser Asp Ser Asp Ser Asp Ser
        675                 680                 685

gac tca gat agc gat tca gac tca gat agc gat tca gac tca gac agc    2112
Asp Ser Asp Ser Asp Ser Asp Ser Asp Ser Asp Ser Asp Ser Asp Ser
    690                 695                 700

gat tca gat tca gac agc gac tca gaa tca gac agc gac tca gac tca    2160
Asp Ser Asp Ser Asp Ser Asp Ser Glu Ser Asp Ser Asp Ser Asp Ser
705                 710                 715                 720

gat agc gac tca gac tca gac agc gac tca gat tca gat agc gat tca    2208
Asp Ser Asp Ser Asp Ser Asp Ser Asp Ser Asp Ser Asp Ser Asp Ser
                725                 730                 735

gac tca gac agc gac tca gac tca gac agc gac tca gac tca gat agc    2256
Asp Ser Asp Ser Asp Ser Asp Ser Asp Ser Asp Ser Asp Ser Asp Ser
            740                 745                 750

gat tca gac tca gac agc gac tca gat tca gat agc gat tcg gac tca    2304
Asp Ser Asp Ser Asp Ser Asp Ser Asp Ser Asp Ser Asp Ser Asp Ser
        755                 760                 765

gac agc gat tca gat tca gac agc gac tca gac tcg gat agc gat tca    2352
Asp Ser Asp Ser Asp Ser Asp Ser Asp Ser Asp Ser Asp Ser Asp Ser
    770                 775                 780

gat tca gac agc gac tca gac tcg gat agc gac tcg gat tca gat agt     2400
Asp Ser Asp Ser Asp Ser Asp Ser Asp Ser Asp Ser Asp Ser Asp Ser
785                 790                 795                 800

gac tcc gat tca aga gtt aca cca cca aat aat gaa cag aaa gca cca     2448
Asp Ser Asp Ser Arg Val Thr Pro Pro Asn Asn Glu Gln Lys Ala Pro
                805                 810                 815

tca aat cct aaa ggt gaa gta aac cat tct aat aag gta tca aaa caa     2496
Ser Asn Pro Lys Gly Glu Val Asn His Ser Asn Lys Val Ser Lys Gln
            820                 825                 830

cac aaa act gat gct tta cca gaa aca gga gat aag agc gaa aac aca     2544
His Lys Thr Asp Ala Leu Pro Glu Thr Gly Asp Lys Ser Glu Asn Thr
        835                 840                 845

aat gca act tta ttt ggt gca atg atg gca tta tta gga tca tta cta     2592
Asn Ala Thr Leu Phe Gly Ala Met Met Ala Leu Leu Gly Ser Leu Leu
    850                 855                 860

ttg ttt aga aaa cgc aag caa gat cat aaa gaa aaa gcg taa             2634
Leu Phe Arg Lys Arg Lys Gln Asp His Lys Glu Lys Ala
865                 870                 875


<210> 16
<211> 877
<212> PRT
<213> Staphylococcus sp.

<400> 16
Leu Lys Lys Arg Ile Asp Tyr Leu Ser Asn Lys Gln Asn Lys Tyr Ser
1               5                   10                  15
Ile Arg Arg Phe Thr Val Gly Thr Thr Ser Val Ile Val Gly Ala Thr
            20                  25                  30
Ile Leu Phe Gly Ile Gly Asn His Gln Ala Gln Ala Ser Glu Gln Ser
        35                  40                  45
Asn Asp Thr Thr Gln Ser Ser Lys Asn Asn Ala Ser Ala Asp Ser Glu
    50                  55                  60
Lys Asn Asn Met Ile Glu Thr Pro Gln Leu Asn Thr Thr Ala Asn Asp
65                  70                  75                  80
Thr Ser Asp Ile Ser Ala Asn Thr Asn Ser Ala Asn Val Asp Ser Thr
                85                  90                  95
Thr Lys Pro Met Ser Thr Gln Thr Ser Asn Thr Thr Thr Thr Glu Pro
            100                 105                 110
Ala Ser Thr Asn Glu Thr Pro Gln Pro Thr Ala Ile Lys Asn Gln Ala
        115                 120                 125
Thr Ala Ala Lys Met Gln Asp Gln Thr Val Pro Gln Glu Ala Asn Ser
    130                 135                 140
Gln Val Asp Asn Lys Thr Thr Asn Asp Ala Asn Ser Ile Ala Thr Asn
145                 150                 155                 160
Ser Glu Leu Lys Asn Ser Gln Thr Leu Asp Leu Pro Gln Ser Ser Pro
                165                 170                 175
Gln Thr Ile Ser Asn Ala Gln Gly Thr Ser Lys Pro Ser Val Arg Thr
            180                 185                 190
Arg Ala Val Arg Ser Leu Ala Val Ala Glu Pro Val Val Asn Ala Ala
        195                 200                 205
Asp Ala Lys Gly Thr Asn Val Asn Asp Lys Val Thr Ala Ser Asn Phe
    210                 215                 220
Lys Leu Glu Lys Thr Thr Phe Asp Pro Asn Gln Ser Gly Asn Thr Phe
225                 230                 235                 240

Met Ala Ala Asn Phe Thr Val Thr Asp Lys Val Lys Ser Gly Asp Tyr
                245                 250                 255
Phe Thr Ala Lys Leu Pro Asp Ser Leu Thr Gly Asn Gly Asp Val Asp
            260                 265                 270
Tyr Ser Asn Ser Asn Asn Thr Met Pro Ile Ala Asp Ile Lys Ser Thr
        275                 280                 285
Asn Gly Asp Val Val Ala Lys Ala Thr Tyr Asp Ile Leu Thr Lys Thr
    290                 295                 300
Tyr Thr Phe Val Phe Thr Asp Tyr Val Asn Asn Lys Glu Asn Ile Asn
305                 310                 315                 320
Gly Gln Phe Ser Leu Pro Leu Phe Thr Asp Arg Ala Lys Ala Pro Lys
                325                 330                 335
Ser Gly Thr Tyr Asp Ala Asn Ile Asn Ile Ala Asp Glu Met Phe Asn
            340                 345                 350
Asn Lys Ile Thr Tyr Asn Tyr Ser Ser Pro Ile Ala Gly Ile Asp Lys
        355                 360                 365
Pro Asn Gly Ala Asn Ile Ser Ser Gln Ile Ile Gly Val Asp Thr Ala
    370                 375                 380
Ser Gly Gln Asn Thr Tyr Lys Gln Thr Val Phe Val Asn Pro Lys Gln
385                 390                 395                 400
Arg Val Leu Gly Asn Thr Trp Val Tyr Ile Lys Gly Tyr Gln Asp Lys
                405                 410                 415
Ile Glu Glu Ser Ser Gly Lys Val Ser Ala Thr Asp Thr Lys Leu Arg
            420                 425                 430
Ile Phe Glu Val Asn Asp Thr Ser Lys Leu Ser Asp Ser Tyr Tyr Ala
        435                 440                 445
Asp Pro Asn Asp Ser Asn Leu Lys Glu Val Thr Asp Gln Phe Lys Asn
    450                 455                 460
Arg Ile Tyr Tyr Glu His Pro Asn Val Ala Ser Ile Lys Phe Gly Asp
465                 470                 475                 480
Ile Thr Lys Thr Tyr Val Val Leu Val Glu Gly His Tyr Asp Asn Thr
                485                 490                 495
Gly Lys Asn Leu Lys Thr Gln Val Ile Gln Glu Asn Val Asp Pro Val
            500                 505                 510
Thr Asn Arg Asp Tyr Ser Ile Phe Gly Trp Asn Asn Glu Asn Val Val
        515                 520                 525
Arg Tyr Gly Gly Gly Ser Ala Asp Gly Asp Ser Ala Val Asn Pro Lys
    530                 535                 540
Asp Pro Thr Pro Gly Pro Pro Val Asp Pro Glu Pro Ser Pro Asp Pro
545                 550                 555                 560
Glu Pro Glu Pro Thr Pro Asp Pro Glu Pro Ser Pro Asp Pro Glu Pro
                565                 570                 575
Glu Pro Ser Pro Asp Pro Asp Pro Asp Ser Asp Ser Asp Ser Asp Ser
            580                 585                 590
Gly Ser Asp Ser Asp Ser Gly Ser Asp Ser Asp Ser Glu Ser Asp Ser
        595                 600                 605
Asp Ser Asp Ser Asp Ser Asp Ser Asp Ser Asp Ser Asp Ser Glu Ser
    610                 615                 620
Asp Ser Asp Ser Glu Ser Asp Ser Asp Ser Asp Ser Asp Ser Asp Ser
625                 630                 635                 640
Asp Ser Asp Ser Asp Ser Glu Ser Asp Ser Asp Ser Asp Ser Asp Ser
                645                 650                 655
Asp Ser Asp Ser Asp Ser Asp Ser Glu Ser Asp Ser Asp Ser Glu Ser
            660                 665                 670
Asp Ser Glu Ser Asp Ser Asp Ser Asp Ser Asp Ser Asp Ser Asp Ser
        675                 680                 685
Asp Ser Asp Ser Asp Ser Asp Ser Asp Ser Asp Ser Asp Ser Asp Ser
    690                 695                 700
Asp Ser Asp Ser Asp Ser Asp Ser Glu Ser Asp Ser Asp Ser Asp Ser
705                 710                 715                 720
Asp Ser Asp Ser Asp Ser Asp Ser Asp Ser Asp Ser Asp Ser Asp Ser

725                 730                 735
Asp Ser Asp Ser Asp Ser Asp Ser Asp Ser Asp Ser Asp Ser Asp Ser
            740                 745                 750
Asp Ser Asp Ser Asp Ser Asp Ser Asp Ser Asp Ser Asp Ser Asp Ser
        755                 760                 765
Asp Ser Asp Ser Asp Ser Asp Ser Asp Ser Asp Ser Asp Ser Asp Ser
    770                 775                 780
Asp Ser Asp Ser Asp Ser Asp Ser Asp Ser Asp Ser Asp Ser Asp Ser
785                 790                 795                 800
Asp Ser Asp Ser Arg Val Thr Pro Pro Asn Asn Glu Gln Lys Ala Pro
                805                 810                 815
Ser Asn Pro Lys Gly Glu Val Asn His Ser Asn Lys Val Ser Lys Gln
            820                 825                 830
His Lys Thr Asp Ala Leu Pro Glu Thr Gly Asp Lys Ser Glu Asn Thr
        835                 840                 845
Asn Ala Thr Leu Phe Gly Ala Met Met Ala Leu Leu Gly Ser Leu Leu
    850                 855                 860
Leu Phe Arg Lys Arg Lys Gln Asp His Lys Glu Lys Ala
865                 870                 875

<210> 17
<211> 684
<212> DNA
<213> Staphylococcus sp.

<220>
<221> CDS
<222> (1)..(684)

<400> 17
ttg aaa aat att tta aaa gtt ttt aat aca acg att tta gcg tta att      48
Leu Lys Asn Ile Leu Lys Val Phe Asn Thr Thr Ile Leu Ala Leu Ile
1               5                   10                  15

atc atc atc gcg aca ttc agt aat tct gca aat gcc gca gat agc ggt      96
Ile Ile Ile Ala Thr Phe Ser Asn Ser Ala Asn Ala Ala Asp Ser Gly
            20                  25                  30

act ttg aat tat gag gtt tac aaa tac aat acc aat gac acg tca att      144
Thr Leu Asn Tyr Glu Val Tyr Lys Tyr Asn Thr Asn Asp Thr Ser Ile
        35                  40                  45

gct aat gac tat ttt aat aaa ccg gca aag tac att aag aaa aat ggt      192
Ala Asn Asp Tyr Phe Asn Lys Pro Ala Lys Tyr Ile Lys Lys Asn Gly
    50                  55                  60

aaa ttg tat gtt caa ata act gtc aac cac agt cat tgg att act gga      240
Lys Leu Tyr Val Gln Ile Thr Val Asn His Ser His Trp Ile Thr Gly
65                  70                  75                  80

atg agt atc gaa gga cat aaa gaa aat att att agt aaa aac act gcc      288
Met Ser Ile Glu Gly His Lys Glu Asn Ile Ile Ser Lys Asn Thr Ala
                85                  90                  95

aaa gat gaa cgc act tct gaa ttt gaa gta agt aag ttg aac ggt aaa      336
Lys Asp Glu Arg Thr Ser Glu Phe Glu Val Ser Lys Leu Asn Gly Lys
            100                 105                 110

ata gat gga aaa att gac gtt tat atc gat gaa aaa gta aat gga aag      384

Ile Asp Gly Lys Ile Asp Val Tyr Ile Asp Glu Lys Val Asn Gly Lys
        115                 120                 125

cca ttc aaa tat gac cat cat tac aac att aca tat aaa ttt aat gga     432
Pro Phe Lys Tyr Asp His His Tyr Asn Ile Thr Tyr Lys Phe Asn Gly
    130                 135                 140

cca act gat gta gca ggt gct aat gca cca ggt aaa gat gat aaa aat     480
Pro Thr Asp Val Ala Gly Ala Asn Ala Pro Gly Lys Asp Asp Lys Asn
145                 150                 155                 160

tct gct tca ggt agt gac aaa gga tct gat gga acg act act ggt caa     528
Ser Ala Ser Gly Ser Asp Lys Gly Ser Asp Gly Thr Thr Thr Gly Gln
                165                 170                 175

agt gaa tct aat agt tcg aat aaa gac aaa gta gaa aat cca caa aca     576
Ser Glu Ser Asn Ser Ser Asn Lys Asp Lys Val Glu Asn Pro Gln Thr
            180                 185                 190

aat gct ggt aca cct gca tat ata tat gca ata cca gtt gca tcc tta     624
Asn Ala Gly Thr Pro Ala Tyr Ile Tyr Ala Ile Pro Val Ala Ser Leu
        195                 200                 205

gca tta tta atc gca atc aca ttg ttt gtt aga aaa aaa tct aaa ggc     672
Ala Leu Leu Ile Ala Ile Thr Leu Phe Val Arg Lys Lys Ser Lys Gly
    210                 215                 220

aat gtg gaa taa                                                     684
Asn Val Glu
225


<210> 18
<211> 227
<212> PRT
<213> Staphylococcus sp.

<400> 18
Leu Lys Asn Ile Leu Lys Val Phe Asn Thr Thr Ile Leu Ala Leu Ile
  1               5                  10                  15
Ile Ile Ile Ala Thr Phe Ser Asn Ser Ala Asn Ala Ala Asp Ser Gly
             20                  25                  30
Thr Leu Asn Tyr Glu Val Tyr Lys Tyr Asn Thr Asn Asp Thr Ser Ile
         35                  40                  45
Ala Asn Asp Tyr Phe Asn Lys Pro Ala Lys Tyr Ile Lys Lys Asn Gly
     50                  55                  60
Lys Leu Tyr Val Gln Ile Thr Val Asn His Ser His Trp Ile Thr Gly
 65                  70                  75                  80
Met Ser Ile Glu Gly His Lys Glu Asn Ile Ile Ser Lys Asn Thr Ala
                 85                  90                  95
Lys Asp Glu Arg Thr Ser Glu Phe Glu Val Ser Lys Leu Asn Gly Lys
            100                 105                 110
Ile Asp Gly Lys Ile Asp Val Tyr Ile Asp Glu Lys Val Asn Gly Lys
        115                 120                 125
Pro Phe Lys Tyr Asp His His Tyr Asn Ile Thr Tyr Lys Phe Asn Gly
    130                 135                 140
Pro Thr Asp Val Ala Gly Ala Asn Ala Pro Gly Lys Asp Asp Lys Asn
145                 150                 155                 160
Ser Ala Ser Gly Ser Asp Lys Gly Ser Asp Gly Thr Thr Thr Gly Gln
                165                 170                 175
Ser Glu Ser Asn Ser Ser Asn Lys Asp Lys Val Glu Asn Pro Gln Thr

180                 185                 190
Asn Ala Gly Thr Pro Ala Tyr Ile Tyr Ala Ile Pro Val Ala Ser Leu
        195                 200                 205
Ala Leu Leu Ile Ala Ile Thr Leu Phe Val Arg Lys Lys Ser Lys Gly
    210                 215                 220
Asn Val Glu
225

<210> 19
<211> 1908
<212> DNA
<213> Staphylococcus sp.

<220>
<221> CDS
<222> (1)..(1908)

<400> 19
atg gct aaa tat cga ggg aaa ccg ttt caa tta tat gta aag tta tcg      48
Met Ala Lys Tyr Arg Gly Lys Pro Phe Gln Leu Tyr Val Lys Leu Ser
1               5                   10                  15

tgt tcg aca atg atg gcg tca agt atc att tta acg aat atc ttg ccg      96
Cys Ser Thr Met Met Ala Ser Ser Ile Ile Leu Thr Asn Ile Leu Pro
            20                  25                  30

tac gat gcc caa gct gca tct gaa aag gat act gaa att tca aaa gag     144
Tyr Asp Ala Gln Ala Ala Ser Glu Lys Asp Thr Glu Ile Ser Lys Glu
        35                  40                  45

ata tta tct aag caa gat tta tta gac aaa gtt gac aaa gca att cgt     192
Ile Leu Ser Lys Gln Asp Leu Leu Asp Lys Val Asp Lys Ala Ile Arg
    50                  55                  60

caa att gag caa tta aaa cag tta tcg gct tca tct aaa gca cat tat     240
Gln Ile Glu Gln Leu Lys Gln Leu Ser Ala Ser Ser Lys Ala His Tyr
65                  70                  75                  80

aaa gca caa cta aat gaa gcg aaa aca gca tcg caa ata gat gaa atc     288
Lys Ala Gln Leu Asn Glu Ala Lys Thr Ala Ser Gln Ile Asp Glu Ile
                85                  90                  95

ata aaa cga gct aat gag ttg gat agc aaa gaa aat aaa agt tct cac     336
Ile Lys Arg Ala Asn Glu Leu Asp Ser Lys Glu Asn Lys Ser Ser His
            100                 105                 110

act gaa atg aac ggt caa agt gat ata gac agt aaa tta gat caa ttg     384
Thr Glu Met Asn Gly Gln Ser Asp Ile Asp Ser Lys Leu Asp Gln Leu
        115                 120                 125

ctt aaa gat tta aat gag gtt tct tca aat gtt gat agg ggt caa caa     432
Leu Lys Asp Leu Asn Glu Val Ser Ser Asn Val Asp Arg Gly Gln Gln
    130                 135                 140

agt ggc gag gac gat ctt aat gca atg aaa aat gat atg tca caa acg     480
Ser Gly Glu Asp Asp Leu Asn Ala Met Lys Asn Asp Met Ser Gln Thr
145                 150                 155                 160

gct aca aca aaa tat gga gaa aaa gat gat aaa aat gat gaa gca atg     528

Ala Thr Thr Lys Tyr Gly Glu Lys Asp Asp Lys Asn Asp Glu Ala Met
                165                 170                 175

gta aat aag gcg tta gaa gac cta gac cat ttg aat cag caa ata cac     576
Val Asn Lys Ala Leu Glu Asp Leu Asp His Leu Asn Gln Gln Ile His
            180                 185                 190

aaa tcg aaa gat gca ttg aaa gat gca tcg aaa gat ccg gca gtg tct     624
Lys Ser Lys Asp Ala Leu Lys Asp Ala Ser Lys Asp Pro Ala Val Ser
        195                 200                 205

aca aca gat agt aat cat gaa gta gct aaa acg cca aat aat gat ggt     672
Thr Thr Asp Ser Asn His Glu Val Ala Lys Thr Pro Asn Asn Asp Gly
    210                 215                 220

tct gga cat gtt gtg tta aat aaa ttt ctt tca aat gaa gag aat caa     720
Ser Gly His Val Val Leu Asn Lys Phe Leu Ser Asn Glu Glu Asn Gln
225                 230                 235                 240

agc cat agt aat caa ctc act gat aaa tta caa gga agc gat aaa att     768
Ser His Ser Asn Gln Leu Thr Asp Lys Leu Gln Gly Ser Asp Lys Ile
                245                 250                 255

aat cat gct atg att gaa aaa ttg gct aaa agt aat gcc tca acg caa     816
Asn His Ala Met Ile Glu Lys Leu Ala Lys Ser Asn Ala Ser Thr Gln
            260                 265                 270

cat tac aca tat cat aaa ctg aat acg tta caa tct tta gat caa cgt     864
His Tyr Thr Tyr His Lys Leu Asn Thr Leu Gln Ser Leu Asp Gln Arg
        275                 280                 285

att gca aat acg caa ctt cct aaa aat caa aaa tca gac tta atg agc     912
Ile Ala Asn Thr Gln Leu Pro Lys Asn Gln Lys Ser Asp Leu Met Ser
    290                 295                 300

gaa gta aat aag acg aaa gag cgt ata aaa agt caa cga aat att att     960
Glu Val Asn Lys Thr Lys Glu Arg Ile Lys Ser Gln Arg Asn Ile Ile
305                 310                 315                 320

ttg gaa gaa ctt gca cgt act gat gat aaa aag tat gct aca caa agc    1008
Leu Glu Glu Leu Ala Arg Thr Asp Asp Lys Lys Tyr Ala Thr Gln Ser
                325                 330                 335

att tta gaa agt ata ttt aat aaa gac gag gca gat aaa att cta aaa    1056
Ile Leu Glu Ser Ile Phe Asn Lys Asp Glu Ala Asp Lys Ile Leu Lys
            340                 345                 350

gat ata cgt gtt gat ggt aaa aca gat caa caa att gca gat caa att    1104
Asp Ile Arg Val Asp Gly Lys Thr Asp Gln Gln Ile Ala Asp Gln Ile
        355                 360                 365

act cgt cat att gat caa cta tct ctg aca acg agt gat gat tta tta    1152
Thr Arg His Ile Asp Gln Leu Ser Leu Thr Thr Ser Asp Asp Leu Leu
    370                 375                 380

acg tca ttg att gat caa tca caa gat aag tcg cta ttg att tct caa    1200
Thr Ser Leu Ile Asp Gln Ser Gln Asp Lys Ser Leu Leu Ile Ser Gln
385                 390                 395                 400

atc tta caa acg aaa tta gga aaa gct gaa gca gat aaa ttg gct aaa    1248
Ile Leu Gln Thr Lys Leu Gly Lys Ala Glu Ala Asp Lys Leu Ala Lys

                405                 410                 415

gat tgg acg aat aaa gga tta tca aat cgc caa atc gtt gac caa ttg 1296
Asp Trp Thr Asn Lys Gly Leu Ser Asn Arg Gln Ile Val Asp Gln Leu
            420                 425                 430

aag aaa cat ttt gca tca act ggc gac acg tct tca gat gat ata tta 1344
Lys Lys His Phe Ala Ser Thr Gly Asp Thr Ser Ser Asp Asp Ile Leu
        435                 440                 445

aaa gca att ttg aat aat gcc aaa gat aaa aag caa gca att gaa acg 1392
Lys Ala Ile Leu Asn Asn Ala Lys Asp Lys Lys Gln Ala Ile Glu Thr
    450                 455                 460

att tta gca aca cgt ata gaa aga caa aag gca aaa tta ctg gca gat 1440
Ile Leu Ala Thr Arg Ile Glu Arg Gln Lys Ala Lys Leu Leu Ala Asp
465                 470                 475                 480

tta att act aaa ata gaa aca gat caa aat aaa att ttt aat tta gtt 1488
Leu Ile Thr Lys Ile Glu Thr Asp Gln Asn Lys Ile Phe Asn Leu Val
                485                 490                 495

aaa tcg gca ttg aat ggt aaa gcg gat gat tta ttg aat tta caa aag 1536
Lys Ser Ala Leu Asn Gly Lys Ala Asp Asp Leu Leu Asn Leu Gln Lys
            500                 505                 510

aga ctc aat caa acg aaa aaa gat ata gac tat att tta tca cca ata 1584
Arg Leu Asn Gln Thr Lys Lys Asp Ile Asp Tyr Ile Leu Ser Pro Ile
        515                 520                 525

gta aat cgt cca agt tta cta gat cga ttg aat aaa aat ggg aaa aca 1632
Val Asn Arg Pro Ser Leu Leu Asp Arg Leu Asn Lys Asn Gly Lys Thr
    530                 535                 540

acg gat tta aat aag tta gca aat tta atg aat caa gga tca aat tta 1680
Thr Asp Leu Asn Lys Leu Ala Asn Leu Met Asn Gln Gly Ser Asn Leu
545                 550                 555                 560

tta gac agt att cca gat ata ccc aca cca aag cca gaa aag acg tta 1728
Leu Asp Ser Ile Pro Asp Ile Pro Thr Pro Lys Pro Glu Lys Thr Leu
                565                 570                 575

aca ctt ggt aaa ggt aat gga ttg tta agt gga tta tta aat gct gat 1776
Thr Leu Gly Lys Gly Asn Gly Leu Leu Ser Gly Leu Leu Asn Ala Asp
            580                 585                 590

ggt aat gta tct ttg cct aaa gcg ggg gaa acg ata aaa gaa cat tgg 1824
Gly Asn Val Ser Leu Pro Lys Ala Gly Glu Thr Ile Lys Glu His Trp
        595                 600                 605

ttg ccg ata tct gta att gtt ggt gca atg ggt gta cta atg att tgg 1872
Leu Pro Ile Ser Val Ile Val Gly Ala Met Gly Val Leu Met Ile Trp
    610                 615                 620

tta tca cga cgc aat aag ttg aaa aat aaa gca taa 1908
Leu Ser Arg Arg Asn Lys Leu Lys Asn Lys Ala
625                 630                 635

<210> 20
<211> 635

<212> PRT
<213> Staphylococcus sp.

<400> 20
Met Ala Lys Tyr Arg Gly Lys Pro Phe Gln Leu Tyr Val Lys Leu Ser
1               5                   10                  15
Cys Ser Thr Met Met Ala Ser Ser Ile Ile Leu Thr Asn Ile Leu Pro
            20                  25                  30
Tyr Asp Ala Gln Ala Ala Ser Glu Lys Asp Thr Glu Ile Ser Lys Glu
        35                  40                  45
Ile Leu Ser Lys Gln Asp Leu Leu Asp Lys Val Asp Lys Ala Ile Arg
    50                  55                  60
Gln Ile Glu Gln Leu Lys Gln Leu Ser Ala Ser Ser Lys Ala His Tyr
65                  70                  75                  80
Lys Ala Gln Leu Asn Glu Ala Lys Thr Ala Ser Gln Ile Asp Glu Ile
                85                  90                  95
Ile Lys Arg Ala Asn Glu Leu Asp Ser Lys Glu Asn Lys Ser Ser His
            100                 105                 110
Thr Glu Met Asn Gly Gln Ser Asp Ile Asp Ser Lys Leu Asp Gln Leu
        115                 120                 125
Leu Lys Asp Leu Asn Glu Val Ser Ser Asn Val Asp Arg Gly Gln Gln
    130                 135                 140
Ser Gly Glu Asp Asp Leu Asn Ala Met Lys Asn Asp Met Ser Gln Thr
145                 150                 155                 160
Ala Thr Thr Lys Tyr Gly Glu Lys Asp Asp Lys Asn Asp Glu Ala Met
                165                 170                 175
Val Asn Lys Ala Leu Glu Asp Leu Asp His Leu Asn Gln Gln Ile His
            180                 185                 190
Lys Ser Lys Asp Ala Leu Lys Asp Ala Ser Lys Asp Pro Ala Val Ser
        195                 200                 205
Thr Thr Asp Ser Asn His Glu Val Ala Lys Thr Pro Asn Asn Asp Gly
    210                 215                 220
Ser Gly His Val Val Leu Asn Lys Phe Leu Ser Asn Glu Glu Asn Gln
225                 230                 235                 240
Ser His Ser Asn Gln Leu Thr Asp Lys Leu Gln Gly Ser Asp Lys Ile
                245                 250                 255
Asn His Ala Met Ile Glu Lys Leu Ala Lys Ser Asn Ala Ser Thr Gln
            260                 265                 270
His Tyr Thr Tyr His Lys Leu Asn Thr Leu Gln Ser Leu Asp Gln Arg
        275                 280                 285
Ile Ala Asn Thr Gln Leu Pro Lys Asn Gln Lys Ser Asp Leu Met Ser
    290                 295                 300
Glu Val Asn Lys Thr Lys Glu Arg Ile Lys Ser Gln Arg Asn Ile Ile
305                 310                 315                 320
Leu Glu Glu Leu Ala Arg Thr Asp Asp Lys Lys Tyr Ala Thr Gln Ser
                325                 330                 335
Ile Leu Glu Ser Ile Phe Asn Lys Asp Glu Ala Asp Lys Ile Leu Lys
            340                 345                 350
Asp Ile Arg Val Asp Gly Lys Thr Asp Gln Gln Ile Ala Asp Gln Ile
        355                 360                 365
Thr Arg His Ile Asp Gln Leu Ser Leu Thr Thr Ser Asp Asp Leu Leu
    370                 375                 380
Thr Ser Leu Ile Asp Gln Ser Gln Asp Lys Ser Leu Leu Ile Ser Gln
385                 390                 395                 400
Ile Leu Gln Thr Lys Leu Gly Lys Ala Glu Ala Asp Lys Leu Ala Lys
                405                 410                 415
Asp Trp Thr Asn Lys Gly Leu Ser Asn Arg Gln Ile Val Asp Gln Leu
            420                 425                 430
Lys Lys His Phe Ala Ser Thr Gly Asp Thr Ser Ser Asp Asp Ile Leu
        435                 440                 445
Lys Ala Ile Leu Asn Asn Ala Lys Asp Lys Lys Gln Ala Ile Glu Thr

450                 455                 460
Ile Leu Ala Thr Arg Ile Glu Arg Gln Lys Ala Lys Leu Leu Ala Asp
465                 470                 475                 480
Leu Ile Thr Lys Ile Glu Thr Asp Gln Asn Lys Ile Phe Asn Leu Val
                485                 490                 495
Lys Ser Ala Leu Asn Gly Lys Ala Asp Asp Leu Leu Asn Leu Gln Lys
            500                 505                 510
Arg Leu Asn Gln Thr Lys Lys Asp Ile Asp Tyr Ile Leu Ser Pro Ile
        515                 520                 525
Val Asn Arg Pro Ser Leu Leu Asp Arg Leu Asn Lys Asn Gly Lys Thr
    530                 535                 540
Thr Asp Leu Asn Lys Leu Ala Asn Leu Met Asn Gln Gly Ser Asn Leu
545                 550                 555                 560
Leu Asp Ser Ile Pro Asp Ile Pro Thr Pro Lys Pro Glu Lys Thr Leu
                565                 570                 575
Thr Leu Gly Lys Gly Asn Gly Leu Leu Ser Gly Leu Leu Asn Ala Asp
            580                 585                 590
Gly Asn Val Ser Leu Pro Lys Ala Gly Glu Thr Ile Lys Glu His Trp
        595                 600                 605
Leu Pro Ile Ser Val Ile Val Gly Ala Met Gly Val Leu Met Ile Trp
    610                 615                 620
Leu Ser Arg Arg Asn Lys Leu Lys Asn Lys Ala
625                 630                 635

<210> 21
<211> 2862
<212> DNA
<213> Staphylococcus sp.

<220>
<221> CDS
<222> (1)..(2862)

<400> 21
atg aat aat aaa aag aca gca aca aat aga aaa ggc atg ata cca aat    48
Met Asn Asn Lys Lys Thr Ala Thr Asn Arg Lys Gly Met Ile Pro Asn
1               5                   10                  15

cga tta aac aaa ttt tcg ata aga aag tat tct gta ggt act gct tca    96
Arg Leu Asn Lys Phe Ser Ile Arg Lys Tyr Ser Val Gly Thr Ala Ser
            20                  25                  30

att tta gta ggg aca aca ttg att ttt ggg tta agt ggt cat gaa gct    144
Ile Leu Val Gly Thr Thr Leu Ile Phe Gly Leu Ser Gly His Glu Ala
        35                  40                  45

aaa gcg gca gaa cat acg aat gga gaa tta aat caa tca aaa aat gaa    192
Lys Ala Ala Glu His Thr Asn Gly Glu Leu Asn Gln Ser Lys Asn Glu
    50                  55                  60

acg aca gcc cca agt gag aat aaa aca act gaa aaa gtt gat agt cgt    240
Thr Thr Ala Pro Ser Glu Asn Lys Thr Thr Glu Lys Val Asp Ser Arg
65                  70                  75                  80

caa cta aaa gac aat acg caa act gca act gca gat cag cct aaa gtg    288
Gln Leu Lys Asp Asn Thr Gln Thr Ala Thr Ala Asp Gln Pro Lys Val
                85                  90                  95

aca atg agt gat agt gca aca gtt aaa gaa act agt agt aac atg caa    336

Thr Met Ser Asp Ser Ala Thr Val Lys Glu Thr Ser Ser Asn Met Gln
            100                 105                 110

tca cca caa aac gct aca gct agt caa tct act aca caa act agc aat      384
Ser Pro Gln Asn Ala Thr Ala Ser Gln Ser Thr Thr Gln Thr Ser Asn
        115                 120                 125

gta aca aca aat gat aaa tca tca act aca tat agt aat gaa act gat      432
Val Thr Thr Asn Asp Lys Ser Ser Thr Thr Tyr Ser Asn Glu Thr Asp
    130                 135                 140

aaa agt aat tta aca caa gca aaa aac gtt tca act aca cct aaa aca      480
Lys Ser Asn Leu Thr Gln Ala Lys Asn Val Ser Thr Thr Pro Lys Thr
145                 150                 155                 160

acg act att aaa caa aga gct tta aat cgc atg gca gtg aat act gtt      528
Thr Thr Ile Lys Gln Arg Ala Leu Asn Arg Met Ala Val Asn Thr Val
                165                 170                 175

gca gct cca caa caa gga aca aat gtt aat gat aaa gta cat ttt acg      576
Ala Ala Pro Gln Gln Gly Thr Asn Val Asn Asp Lys Val His Phe Thr
            180                 185                 190

aac att gat att gcg att gat aaa gga cat gtt aat aaa aca aca gga      624
Asn Ile Asp Ile Ala Ile Asp Lys Gly His Val Asn Lys Thr Thr Gly
        195                 200                 205

aat act gaa ttt tgg gca act tca agt gat gtt tta aaa tta aaa gcg      672
Asn Thr Glu Phe Trp Ala Thr Ser Ser Asp Val Leu Lys Leu Lys Ala
    210                 215                 220

aat tac aca atc gat gat tct gtt aaa gag ggc gat aca ttt act ttt      720
Asn Tyr Thr Ile Asp Asp Ser Val Lys Glu Gly Asp Thr Phe Thr Phe
225                 230                 235                 240

aaa tat ggt caa tat ttc cgt cca ggt tct gta aga tta cct tca caa      768
Lys Tyr Gly Gln Tyr Phe Arg Pro Gly Ser Val Arg Leu Pro Ser Gln
                245                 250                 255

act caa aat tta tat aat gcc caa ggt aat att att gca aaa ggt att      816
Thr Gln Asn Leu Tyr Asn Ala Gln Gly Asn Ile Ile Ala Lys Gly Ile
            260                 265                 270

tac gat agt aaa aca aat aca aca acg tat act ttt acg aat tat gta      864
Tyr Asp Ser Lys Thr Asn Thr Thr Thr Tyr Thr Phe Thr Asn Tyr Val
        275                 280                 285

gat caa tac aca aat gtt agc ggt agc ttt gaa caa gtc gca ttt gcg      912
Asp Gln Tyr Thr Asn Val Ser Gly Ser Phe Glu Gln Val Ala Phe Ala
    290                 295                 300

aaa cgt gaa aat gca aca act gat aaa act gct tat aaa atg gaa gta      960
Lys Arg Glu Asn Ala Thr Thr Asp Lys Thr Ala Tyr Lys Met Glu Val
305                 310                 315                 320

act tta ggt aat gat aca tat agt aaa gat gtc att gtc gat tat ggt     1008
Thr Leu Gly Asn Asp Thr Tyr Ser Lys Asp Val Ile Val Asp Tyr Gly
                325                 330                 335

aat caa aaa ggt caa caa ctt att tcg agt aca aat tat att aat aat     1056
Asn Gln Lys Gly Gln Gln Leu Ile Ser Ser Thr Asn Tyr Ile Asn Asn

340                 345                 350

gaa gat ttg tca cgt aat atg act gtt tat gta aat caa cct aaa aag     1104
Glu Asp Leu Ser Arg Asn Met Thr Val Tyr Val Asn Gln Pro Lys Lys
        355                 360                 365

acc tat aca aaa gaa aca ttt gta aca aat tta act ggt tat aaa ttt     1152
Thr Tyr Thr Lys Glu Thr Phe Val Thr Asn Leu Thr Gly Tyr Lys Phe
    370                 375                 380

aat cca gat gct aaa aac ttc aaa att tac gaa gtg aca gat caa aat     1200
Asn Pro Asp Ala Lys Asn Phe Lys Ile Tyr Glu Val Thr Asp Gln Asn
385                 390                 395                 400

caa ttt gtg gat agt ttc acc cca gat act tca aaa ctt aaa gat gtt     1248
Gln Phe Val Asp Ser Phe Thr Pro Asp Thr Ser Lys Leu Lys Asp Val
                405                 410                 415

act ggt caa ttc gat gtt att tat agt aat gat aat aag acg gcg aca     1296
Thr Gly Gln Phe Asp Val Ile Tyr Ser Asn Asp Asn Lys Thr Ala Thr
            420                 425                 430

gta gat tta ttg aat ggt caa tct agt agt gat aaa cag tac atc att     1344
Val Asp Leu Leu Asn Gly Gln Ser Ser Ser Asp Lys Gln Tyr Ile Ile
        435                 440                 445

caa caa gtt gct tat cca gat aat agt tca aca gat aat ggg aaa att     1392
Gln Gln Val Ala Tyr Pro Asp Asn Ser Ser Thr Asp Asn Gly Lys Ile
    450                 455                 460

gat tat act tta gaa aca caa aat gga aaa agt agt tgg tca aac agt     1440
Asp Tyr Thr Leu Glu Thr Gln Asn Gly Lys Ser Ser Trp Ser Asn Ser
465                 470                 475                 480

tat tca aat gtg aat ggc tca tca act gca aat ggc gac caa aag aaa     1488
Tyr Ser Asn Val Asn Gly Ser Ser Thr Ala Asn Gly Asp Gln Lys Lys
                485                 490                 495

tat aat cta ggt gac tat gta tgg gaa gat aca aat aaa gat ggt aaa     1536
Tyr Asn Leu Gly Asp Tyr Val Trp Glu Asp Thr Asn Lys Asp Gly Lys
            500                 505                 510

caa gat gcc aat gaa aaa ggg att aaa ggt gtt tat gtc att ctt aaa     1584
Gln Asp Ala Asn Glu Lys Gly Ile Lys Gly Val Tyr Val Ile Leu Lys
        515                 520                 525

gat agt aac ggt aaa gaa tta gat cgt acg aca aca gat gaa aat ggt     1632
Asp Ser Asn Gly Lys Glu Leu Asp Arg Thr Thr Thr Asp Glu Asn Gly
    530                 535                 540

aaa tat cag ttc act ggt tta agc aat gga act tat agt gta gag ttt     1680
Lys Tyr Gln Phe Thr Gly Leu Ser Asn Gly Thr Tyr Ser Val Glu Phe
545                 550                 555                 560

tca aca cca gcc ggt tat aca ccg aca act gca aat gca ggt aca gat     1728
Ser Thr Pro Ala Gly Tyr Thr Pro Thr Thr Ala Asn Ala Gly Thr Asp
                565                 570                 575

gat gct gta gat tct gat gga cta act aca aca ggt gtc att aaa gac     1776
Asp Ala Val Asp Ser Asp Gly Leu Thr Thr Thr Gly Val Ile Lys Asp
            580                 585                 590

gct gac aac atg aca tta gat agt gga ttc tac aaa aca cca aaa tat 1824
Ala Asp Asn Met Thr Leu Asp Ser Gly Phe Tyr Lys Thr Pro Lys Tyr
        595                 600                 605

agt tta ggt gat tat gtt tgg tac gac agt aat aaa gat ggt aaa caa 1872
Ser Leu Gly Asp Tyr Val Trp Tyr Asp Ser Asn Lys Asp Gly Lys Gln
    610                 615                 620

gat tcg act gaa aaa gga att aaa ggt gtt aaa gtt act ttg caa aac 1920
Asp Ser Thr Glu Lys Gly Ile Lys Gly Val Lys Val Thr Leu Gln Asn
625                 630                 635                 640

gaa aaa ggc gaa gta att ggt aca act gaa aca gat gaa aat ggt aaa 1968
Glu Lys Gly Glu Val Ile Gly Thr Thr Glu Thr Asp Glu Asn Gly Lys
                645                 650                 655

tac cgc ttt gat aat tta gat agt ggt aaa tac aaa gtt atc ttt gaa 2016
Tyr Arg Phe Asp Asn Leu Asp Ser Gly Lys Tyr Lys Val Ile Phe Glu
            660                 665                 670

aag cct gct ggt tta act caa aca ggt aca aat aca act gaa gat gat 2064
Lys Pro Ala Gly Leu Thr Gln Thr Gly Thr Asn Thr Thr Glu Asp Asp
        675                 680                 685

aaa gat gcc gat ggt ggc gaa gtt gat gta aca att acg gat cat gat 2112
Lys Asp Ala Asp Gly Gly Glu Val Asp Val Thr Ile Thr Asp His Asp
    690                 695                 700

gat ttc aca ctt gat aat ggc tac tac gaa gaa gaa aca tca gat agt 2160
Asp Phe Thr Leu Asp Asn Gly Tyr Tyr Glu Glu Glu Thr Ser Asp Ser
705                 710                 715                 720

gac tca gat tcg gac agc gat tca gac tca gat agc gac tca gat tca 2208
Asp Ser Asp Ser Asp Ser Asp Ser Asp Ser Asp Ser Asp Ser Asp Ser
                725                 730                 735

gat agt gac tca gac tca gat agc gac tca gac tca gat agc gac tca 2256
Asp Ser Asp Ser Asp Ser Asp Ser Asp Ser Asp Ser Asp Ser Asp Ser
            740                 745                 750

gac agc gac tca gac tca gat agt gat tca gat tcg gac agc gac tca 2304
Asp Ser Asp Ser Asp Ser Asp Ser Asp Ser Asp Ser Asp Ser Asp Ser
        755                 760                 765

gat tca gac agc gaa tca gat tcg gat agc gac tca gac tca gat agc 2352
Asp Ser Asp Ser Glu Ser Asp Ser Asp Ser Asp Ser Asp Ser Asp Ser
    770                 775                 780

gac tca gac agc gac tca gat tca gac agt gac tca gac tca gac agc 2400
Asp Ser Asp Ser Asp Ser Asp Ser Asp Ser Asp Ser Asp Ser Asp Ser
785                 790                 795                 800

gac tca gat tca gac agc gat tca gat tcg gat agc gac tca gat tca 2448
Asp Ser Asp Ser Asp Ser Asp Ser Asp Ser Asp Ser Asp Ser Asp Ser
                805                 810                 815

gat agc gat tcg gac tca gac aac gac tca gat tct gac agc gat tca 2496
Asp Ser Asp Ser Asp Ser Asp Asn Asp Ser Asp Ser Asp Ser Asp Ser
            820                 825                 830

gac tca gat agc gac tca gat tca gac agc gac tca gat tca gac agc     2544
Asp Ser Asp Ser Asp Ser Asp Ser Asp Ser Asp Ser Asp Ser Asp Ser
        835                 840                 845

gat tca gat tca gat agc gat tca gat tca gac agc gac tca gat tca     2592
Asp Ser Asp Ser Asp Ser Asp Ser Asp Ser Asp Ser Asp Ser Asp Ser
    850                 855                 860

gat agc gac tca gac tca gac agc gat tca gac tca gat agc gac tca     2640
Asp Ser Asp Ser Asp Ser Asp Ser Asp Ser Asp Ser Asp Ser Asp Ser
865                 870                 875                 880

gac agc gat tca gat tcg gat agc gat tca gat tca gat gca ggt aaa     2688
Asp Ser Asp Ser Asp Ser Asp Ser Asp Ser Asp Ser Asp Ala Gly Lys
                885                 890                 895

cat act ccg act aaa cca atg agt acg gtt aaa gat cag cat aaa aca     2736
His Thr Pro Thr Lys Pro Met Ser Thr Val Lys Asp Gln His Lys Thr
            900                 905                 910

gct aaa gca tta cca gaa aca ggt agt gaa aat aat aat tca aat aat     2784
Ala Lys Ala Leu Pro Glu Thr Gly Ser Glu Asn Asn Asn Ser Asn Asn
        915                 920                 925

ggc aca tta ttc ggt gga tta ttc gcg gca tta gga tca tta ttg tta     2832
Gly Thr Leu Phe Gly Gly Leu Phe Ala Ala Leu Gly Ser Leu Leu Leu
    930                 935                 940

ttc ggt cgt cgt aaa aaa caa aat aaa taa                             2862
Phe Gly Arg Arg Lys Lys Gln Asn Lys
945                 950


<210> 22
<211> 953
<212> PRT
<213> Staphylococcus sp.

<400> 22
Met Asn Asn Lys Lys Thr Ala Thr Asn Arg Lys Gly Met Ile Pro Asn
1               5                   10                  15
Arg Leu Asn Lys Phe Ser Ile Arg Lys Tyr Ser Val Gly Thr Ala Ser
            20                  25                  30
Ile Leu Val Gly Thr Thr Leu Ile Phe Gly Leu Ser Gly His Glu Ala
        35                  40                  45
Lys Ala Ala Glu His Thr Asn Gly Glu Leu Asn Gln Ser Lys Asn Glu
    50                  55                  60
Thr Thr Ala Pro Ser Glu Asn Lys Thr Thr Glu Lys Val Asp Ser Arg
65                  70                  75                  80
Gln Leu Lys Asp Asn Thr Gln Thr Ala Thr Ala Asp Gln Pro Lys Val
                85                  90                  95
Thr Met Ser Asp Ser Ala Thr Val Lys Glu Thr Ser Ser Asn Met Gln
            100                 105                 110
Ser Pro Gln Asn Ala Thr Ala Ser Gln Ser Thr Thr Gln Thr Ser Asn
        115                 120                 125
Val Thr Thr Asn Asp Lys Ser Ser Thr Thr Tyr Ser Asn Glu Thr Asp
    130                 135                 140
Lys Ser Asn Leu Thr Gln Ala Lys Asn Val Ser Thr Thr Pro Lys Thr
145                 150                 155                 160
Thr Thr Ile Lys Gln Arg Ala Leu Asn Arg Met Ala Val Asn Thr Val
                165                 170                 175

Ala Ala Pro Gln Gln Gly Thr Asn Val Asn Asp Lys Val His Phe Thr
            180                 185                 190
Asn Ile Asp Ile Ala Ile Asp Lys Gly His Val Asn Lys Thr Thr Gly
        195                 200                 205
Asn Thr Glu Phe Trp Ala Thr Ser Ser Asp Val Leu Lys Leu Lys Ala
    210                 215                 220
Asn Tyr Thr Ile Asp Asp Ser Val Lys Glu Gly Asp Thr Phe Thr Phe
225                 230                 235                 240
Lys Tyr Gly Gln Tyr Phe Arg Pro Gly Ser Val Arg Leu Pro Ser Gln
                245                 250                 255
Thr Gln Asn Leu Tyr Asn Ala Gln Gly Asn Ile Ile Ala Lys Gly Ile
            260                 265                 270
Tyr Asp Ser Lys Thr Asn Thr Thr Thr Tyr Thr Phe Thr Asn Tyr Val
        275                 280                 285
Asp Gln Tyr Thr Asn Val Ser Gly Ser Phe Glu Gln Val Ala Phe Ala
    290                 295                 300
Lys Arg Glu Asn Ala Thr Thr Asp Lys Thr Ala Tyr Lys Met Glu Val
305                 310                 315                 320
Thr Leu Gly Asn Asp Thr Tyr Ser Lys Asp Val Ile Val Asp Tyr Gly
                325                 330                 335
Asn Gln Lys Gly Gln Gln Leu Ile Ser Ser Thr Asn Tyr Ile Asn Asn
            340                 345                 350
Glu Asp Leu Ser Arg Asn Met Thr Val Tyr Val Asn Gln Pro Lys Lys
        355                 360                 365
Thr Tyr Thr Lys Glu Thr Phe Val Thr Asn Leu Thr Gly Tyr Lys Phe
    370                 375                 380
Asn Pro Asp Ala Lys Asn Phe Lys Ile Tyr Glu Val Thr Asp Gln Asn
385                 390                 395                 400
Gln Phe Val Asp Ser Phe Thr Pro Asp Thr Ser Lys Leu Lys Asp Val
                405                 410                 415
Thr Gly Gln Phe Asp Val Ile Tyr Ser Asn Asp Asn Lys Thr Ala Thr
            420                 425                 430
Val Asp Leu Leu Asn Gly Gln Ser Ser Ser Asp Lys Gln Tyr Ile Ile
        435                 440                 445
Gln Gln Val Ala Tyr Pro Asp Asn Ser Ser Thr Asp Asn Gly Lys Ile
    450                 455                 460
Asp Tyr Thr Leu Glu Thr Gln Asn Gly Lys Ser Ser Trp Ser Asn Ser
465                 470                 475                 480
Tyr Ser Asn Val Asn Gly Ser Ser Thr Ala Asn Gly Asp Gln Lys Lys
                485                 490                 495
Tyr Asn Leu Gly Asp Tyr Val Trp Glu Asp Thr Asn Lys Asp Gly Lys
            500                 505                 510
Gln Asp Ala Asn Glu Lys Gly Ile Lys Gly Val Tyr Val Ile Leu Lys
        515                 520                 525
Asp Ser Asn Gly Lys Glu Leu Asp Arg Thr Thr Thr Asp Glu Asn Gly
    530                 535                 540
Lys Tyr Gln Phe Thr Gly Leu Ser Asn Gly Thr Tyr Ser Val Glu Phe
545                 550                 555                 560
Ser Thr Pro Ala Gly Tyr Thr Pro Thr Thr Ala Asn Ala Gly Thr Asp
                565                 570                 575
Asp Ala Val Asp Ser Asp Gly Leu Thr Thr Thr Gly Val Ile Lys Asp
            580                 585                 590
Ala Asp Asn Met Thr Leu Asp Ser Gly Phe Tyr Lys Thr Pro Lys Tyr
        595                 600                 605
Ser Leu Gly Asp Tyr Val Trp Tyr Asp Ser Asn Lys Asp Gly Lys Gln
    610                 615                 620
Asp Ser Thr Glu Lys Gly Ile Lys Gly Val Lys Val Thr Leu Gln Asn
625                 630                 635                 640
Glu Lys Gly Glu Val Ile Gly Thr Thr Glu Thr Asp Glu Asn Gly Lys
                645                 650                 655
Tyr Arg Phe Asp Asn Leu Asp Ser Gly Lys Tyr Lys Val Ile Phe Glu

                660                 665                 670
Lys Pro Ala Gly Leu Thr Gln Thr Gly Thr Asn Thr Thr Glu Asp Asp
        675                 680                 685
Lys Asp Ala Asp Gly Gly Glu Val Asp Val Thr Ile Thr Asp His Asp
    690                 695                 700
Asp Phe Thr Leu Asp Asn Gly Tyr Tyr Glu Glu Glu Thr Ser Asp Ser
705                 710                 715                 720
Asp Ser Asp Ser Asp Ser Asp Ser Asp Ser Asp Ser Asp Ser Asp Ser
                725                 730                 735
Asp Ser Asp Ser Asp Ser Asp Ser Asp Ser Asp Ser Asp Ser Asp Ser
            740                 745                 750
Asp Ser Asp Ser Asp Ser Asp Ser Asp Ser Asp Ser Asp Ser Asp Ser
        755                 760                 765
Asp Ser Asp Ser Glu Ser Asp Ser Asp Ser Asp Ser Asp Ser Asp Ser
    770                 775                 780
Asp Ser Asp Ser Asp Ser Asp Ser Asp Ser Asp Ser Asp Ser Asp Ser
785                 790                 795                 800
Asp Ser Asp Ser Asp Ser Asp Ser Asp Ser Asp Ser Asp Ser Asp Ser
                805                 810                 815
Asp Ser Asp Ser Asp Ser Asp Asn Asp Ser Asp Ser Asp Ser Asp Ser
            820                 825                 830
Asp Ser Asp Ser Asp Ser Asp Ser Asp Ser Asp Ser Asp Ser Asp Ser
        835                 840                 845
Asp Ser Asp Ser Asp Ser Asp Ser Asp Ser Asp Ser Asp Ser Asp Ser
    850                 855                 860
Asp Ser Asp Ser Asp Ser Asp Ser Asp Ser Asp Ser Asp Ser Asp Ser
865                 870                 875                 880
Asp Ser Asp Ser Asp Ser Asp Ser Asp Ser Asp Ser Asp Ala Gly Lys
                885                 890                 895
His Thr Pro Thr Lys Pro Met Ser Thr Val Lys Asp Gln His Lys Thr
            900                 905                 910
Ala Lys Ala Leu Pro Glu Thr Gly Ser Glu Asn Asn Asn Ser Asn Asn
        915                 920                 925
Gly Thr Leu Phe Gly Gly Leu Phe Ala Ala Leu Gly Ser Leu Leu Leu
    930                 935                 940
Phe Gly Arg Arg Lys Lys Gln Asn Lys
945                 950

<210> 23
<211> 2970
<212> DNA
<213> Staphylococcus sp.

<220>
<221> CDS
<222> (1)..(2970)

<400> 23
atg aat atg aag aaa aaa gaa aaa cac gca att cgg aaa aaa tcg att      48
Met Asn Met Lys Lys Lys Glu Lys His Ala Ile Arg Lys Lys Ser Ile
  1               5                  10                  15

ggc gtg gct tca gtg ctt gta ggt acg tta atc ggt ttt gga cta ctc      96
Gly Val Ala Ser Val Leu Val Gly Thr Leu Ile Gly Phe Gly Leu Leu
             20                  25                  30

agc agt aaa gaa gca gat gca agt gaa aat agt gtt acg caa tct gat     144
Ser Ser Lys Glu Ala Asp Ala Ser Glu Asn Ser Val Thr Gln Ser Asp
         35                  40                  45

agc gca agt aac gaa agc aaa agt aat gat tca agt agc gtt agt gct    192
Ser Ala Ser Asn Glu Ser Lys Ser Asn Asp Ser Ser Ser Val Ser Ala
     50                  55                  60

gca cct aaa aca gac gac aca aac gtg agt gat act aaa aca tcg tca    240
Ala Pro Lys Thr Asp Asp Thr Asn Val Ser Asp Thr Lys Thr Ser Ser
 65                  70                  75                  80

aac act aat aat ggc gaa acg agt gtg gcg caa aat cca gca caa cag    288
Asn Thr Asn Asn Gly Glu Thr Ser Val Ala Gln Asn Pro Ala Gln Gln
                 85                  90                  95

gaa acg aca caa tca tca tca aca aat gca act acg gaa gaa acg ccg    336
Glu Thr Thr Gln Ser Ser Ser Thr Asn Ala Thr Thr Glu Glu Thr Pro
            100                 105                 110

gta act ggt gaa gct act act acg aca acg aat caa gct aat aca ccg    384
Val Thr Gly Glu Ala Thr Thr Thr Thr Thr Asn Gln Ala Asn Thr Pro
        115                 120                 125

gca aca act caa tca agc aat aca aat gcg gag gaa tta gtg aat caa    432
Ala Thr Thr Gln Ser Ser Asn Thr Asn Ala Glu Glu Leu Val Asn Gln
    130                 135                 140

aca agt aat gaa acg act tct aat gat act aat aca gta tca tct gta    480
Thr Ser Asn Glu Thr Thr Ser Asn Asp Thr Asn Thr Val Ser Ser Val
145                 150                 155                 160

aat tca cct caa aat tct aca aat gcg gaa aat gtt tca aca acg caa    528
Asn Ser Pro Gln Asn Ser Thr Asn Ala Glu Asn Val Ser Thr Thr Gln
                165                 170                 175

gat act tca act gaa gca aca cct tca aac aat gaa tca gct cca cag    576
Asp Thr Ser Thr Glu Ala Thr Pro Ser Asn Asn Glu Ser Ala Pro Gln
            180                 185                 190

aat aca gat gca agt aat aaa gat gta gtt agt caa gcg gtt aat cca    624
Asn Thr Asp Ala Ser Asn Lys Asp Val Val Ser Gln Ala Val Asn Pro
        195                 200                 205

agt acg cct aga atg aga gca ttt agt tta gcg gca gta gct gca gat    672
Ser Thr Pro Arg Met Arg Ala Phe Ser Leu Ala Ala Val Ala Ala Asp
    210                 215                 220

gca ccg gca gct ggc aca gat att acg aat cag ttg aca gat gtg aaa    720
Ala Pro Ala Ala Gly Thr Asp Ile Thr Asn Gln Leu Thr Asp Val Lys
225                 230                 235                 240

gtt act att gac tct ggt acg act gtg tat ccg cac caa gca ggt tat    768
Val Thr Ile Asp Ser Gly Thr Thr Val Tyr Pro His Gln Ala Gly Tyr
                245                 250                 255

gtc aaa ctg aat tat ggt ttt tca gtg cct aat tct gct gtt aaa ggt    816
Val Lys Leu Asn Tyr Gly Phe Ser Val Pro Asn Ser Ala Val Lys Gly
            260                 265                 270

gac aca ttc aaa ata act gta cct aaa gaa tta aac tta aat ggt gta    864
Asp Thr Phe Lys Ile Thr Val Pro Lys Glu Leu Asn Leu Asn Gly Val
        275                 280                 285

act tca act gct aaa gtg cca cca att atg gct gga gat caa gta ttg     912
Thr Ser Thr Ala Lys Val Pro Pro Ile Met Ala Gly Asp Gln Val Leu
    290                 295                 300

gca aat ggt gta atc gat agt gat ggt aat gtt att tat aca ttt aca     960
Ala Asn Gly Val Ile Asp Ser Asp Gly Asn Val Ile Tyr Thr Phe Thr
305                 310                 315                 320

gac tat gtt gat aat aaa gaa aat gta aca gct aat att act atg cca    1008
Asp Tyr Val Asp Asn Lys Glu Asn Val Thr Ala Asn Ile Thr Met Pro
                325                 330                 335

gct tat att gac cct gaa aat gtt aca aag aca ggt aat gtg aca ttg    1056
Ala Tyr Ile Asp Pro Glu Asn Val Thr Lys Thr Gly Asn Val Thr Leu
            340                 345                 350

aca act ggc ata gga acc aat act gct agt aag aca gta tta atc gac    1104
Thr Thr Gly Ile Gly Thr Asn Thr Ala Ser Lys Thr Val Leu Ile Asp
        355                 360                 365

tat gag aaa tat gga caa ttc cat aat tta tca att aaa ggt acg att    1152
Tyr Glu Lys Tyr Gly Gln Phe His Asn Leu Ser Ile Lys Gly Thr Ile
    370                 375                 380

gat caa atc gat aaa aca aat aat acg tat cgc caa aca att tat gtc    1200
Asp Gln Ile Asp Lys Thr Asn Asn Thr Tyr Arg Gln Thr Ile Tyr Val
385                 390                 395                 400

aat cca agc gga gat aac gtt gtg tta cct gcc tta aca ggt aat tta    1248
Asn Pro Ser Gly Asp Asn Val Val Leu Pro Ala Leu Thr Gly Asn Leu
                405                 410                 415

att cct aat aca aag agt aat gcg tta ata gat gca aaa aac act gat    1296
Ile Pro Asn Thr Lys Ser Asn Ala Leu Ile Asp Ala Lys Asn Thr Asp
            420                 425                 430

att aaa gtt tat aga gtc gat aat gct aat gat tta tct gaa agt tat    1344
Ile Lys Val Tyr Arg Val Asp Asn Ala Asn Asp Leu Ser Glu Ser Tyr
        435                 440                 445

tat gtg aat cct agc gat ttt gaa gat gta act aat caa gtt aga att    1392
Tyr Val Asn Pro Ser Asp Phe Glu Asp Val Thr Asn Gln Val Arg Ile
    450                 455                 460

tca ttt cca aat gct aat caa tac aaa gta gaa ttt cct acg gac gat    1440
Ser Phe Pro Asn Ala Asn Gln Tyr Lys Val Glu Phe Pro Thr Asp Asp
465                 470                 475                 480

gac caa att aca aca ccg tat att gta gtt gtt aat ggc cat att gat    1488
Asp Gln Ile Thr Thr Pro Tyr Ile Val Val Val Asn Gly His Ile Asp
                485                 490                 495

cct gct agt aca ggt gat tta gca cta cgt tcg aca ttt tat ggt tat    1536
Pro Ala Ser Thr Gly Asp Leu Ala Leu Arg Ser Thr Phe Tyr Gly Tyr
            500                 505                 510

gat tct aat ttt ata tgg aga tct atg tca tgg gac aac gaa gta gca    1584
Asp Ser Asn Phe Ile Trp Arg Ser Met Ser Trp Asp Asn Glu Val Ala
        515                 520                 525

ttt aat aac gga tca ggt tct ggt gac ggt atc gat aaa cca gtt gtt    1632

Phe Asn Asn Gly Ser Gly Ser Gly Asp Gly Ile Asp Lys Pro Val Val
    530                 535                 540

cct gaa caa cct gat gag cct ggt gaa att gaa cca att cca gag gat    1680
Pro Glu Gln Pro Asp Glu Pro Gly Glu Ile Glu Pro Ile Pro Glu Asp
545                 550                 555                 560

tca gat tct gac cca ggt tca gat tct ggc agc gat tct aat tca gat    1728
Ser Asp Ser Asp Pro Gly Ser Asp Ser Gly Ser Asp Ser Asn Ser Asp
                565                 570                 575

agc ggt tca gat tct ggc agt gat tct aca tca gat agt ggt tca gat    1776
Ser Gly Ser Asp Ser Gly Ser Asp Ser Thr Ser Asp Ser Gly Ser Asp
            580                 585                 590

tca gcg agt gat tca gat tca gca agt gat tca gac tca gcg agt gat    1824
Ser Ala Ser Asp Ser Asp Ser Ala Ser Asp Ser Asp Ser Ala Ser Asp
        595                 600                 605

tca gat tca gca agt gat tca gat tca gca agt gat tca gat tca gca    1872
Ser Asp Ser Ala Ser Asp Ser Asp Ser Ala Ser Asp Ser Asp Ser Ala
    610                 615                 620

agt gat tca gac tca gca agt gat tca gat tca gca agt gat tca gat    1920
Ser Asp Ser Asp Ser Ala Ser Asp Ser Asp Ser Ala Ser Asp Ser Asp
625                 630                 635                 640

tca gca agc gat tca gat tca gcg agc gat tca gat tca gcg agc gat    1968
Ser Ala Ser Asp Ser Asp Ser Ala Ser Asp Ser Asp Ser Ala Ser Asp
                645                 650                 655

tca gat tca gcg agt gat tcc gac tca gcg agc gat tca gac tca gat    2016
Ser Asp Ser Ala Ser Asp Ser Asp Ser Ala Ser Asp Ser Asp Ser Asp
            660                 665                 670

agt gac tca gat tcc gat agc gat tcc gac tca gat agc gac tca gat    2064
Ser Asp Ser Asp Ser Asp Ser Asp Ser Asp Ser Asp Ser Asp Ser Asp
        675                 680                 685

tca gac agc gat tct gac tca gac agc gat tct gac tca gac agt gac    2112
Ser Asp Ser Asp Ser Asp Ser Asp Ser Asp Ser Asp Ser Asp Ser Asp
    690                 695                 700

tca gat tcc gat agc gat tcc gac tca gac agt gac tca gat tcc gat    2160
Ser Asp Ser Asp Ser Asp Ser Asp Ser Asp Ser Asp Ser Asp Ser Asp
705                 710                 715                 720

agc gat tcc gac tca gac agt gac tca gat tcc gat agc gat tca gat    2208
Ser Asp Ser Asp Ser Asp Ser Asp Ser Asp Ser Asp Ser Asp Ser Asp
                725                 730                 735

tcc gac agt gat tcc gac tca gat agc gat tcc gac tca gat agc gac    2256
Ser Asp Ser Asp Ser Asp Ser Asp Ser Asp Ser Asp Ser Asp Ser Asp
            740                 745                 750

tca gat tca gac agc gat tca gat tca gac agc gat tct gac tca gac    2304
Ser Asp Ser Asp Ser Asp Ser Asp Ser Asp Ser Asp Ser Asp Ser Asp
        755                 760                 765

agt gac tca gat tcc gat agc gat tca gat tca gac agt gat tca gac    2352
Ser Asp Ser Asp Ser Asp Ser Asp Ser Asp Ser Asp Ser Asp Ser Asp

770                 775                 780

tca gat agc gat tca gat tcc gac agt gac tca gac tca gac agc gat     2400
Ser Asp Ser Asp Ser Asp Ser Asp Ser Asp Ser Asp Ser Asp Ser Asp
785                 790                 795                 800

tca gat tcc gat agc gat tca gat tcc gac agt gac tca gat tcc gat     2448
Ser Asp Ser Asp Ser Asp Ser Asp Ser Asp Ser Asp Ser Asp Ser Asp
                805                 810                 815

agt gac tcg gat tca gcg agt gat tca gat tca gat agc gat tca gaa     2496
Ser Asp Ser Asp Ser Ala Ser Asp Ser Asp Ser Asp Ser Asp Ser Glu
            820                 825                 830

tca gat agt gac tca gac tca gac agt gat tca gat tca gat agt gac     2544
Ser Asp Ser Asp Ser Asp Ser Asp Ser Asp Ser Asp Ser Asp Ser Asp
        835                 840                 845

tca gac tca gac agc gat tca gaa tca gat agt gac tcc gat tca gac     2592
Ser Asp Ser Asp Ser Asp Ser Glu Ser Asp Ser Asp Ser Asp Ser Asp
    850                 855                 860

agc gat tca gaa tca gat agt gac tcc gat tca gat agc gat tcg gat     2640
Ser Asp Ser Glu Ser Asp Ser Asp Ser Asp Ser Asp Ser Asp Ser Asp
865                 870                 875                 880

tca gcg agt gat tca gac tca ggt agt gac tcc gat tca tca agt gat     2688
Ser Ala Ser Asp Ser Asp Ser Gly Ser Asp Ser Asp Ser Ser Ser Asp
                885                 890                 895

tca gat tcc gat tca acg agt gac aca gga tca gac aac gac tca gac     2736
Ser Asp Ser Asp Ser Thr Ser Asp Thr Gly Ser Asp Asn Asp Ser Asp
            900                 905                 910

agt gat tca aat agc gat tcc gag tca ggt tct aac aat aat gta gtt     2784
Ser Asp Ser Asn Ser Asp Ser Glu Ser Gly Ser Asn Asn Asn Val Val
        915                 920                 925

ccg cct aat tca cct aaa aat ggt act aat gct tct aat aaa aat gag     2832
Pro Pro Asn Ser Pro Lys Asn Gly Thr Asn Ala Ser Asn Lys Asn Glu
    930                 935                 940

gct aaa gat agt aaa gaa cca tta cca gat aca ggt tct gaa gat gaa     2880
Ala Lys Asp Ser Lys Glu Pro Leu Pro Asp Thr Gly Ser Glu Asp Glu
945                 950                 955                 960

gcg aat acg tca cta att tgg gga tta tta gca tca tta ggt tca tta     2928
Ala Asn Thr Ser Leu Ile Trp Gly Leu Leu Ala Ser Leu Gly Ser Leu
                965                 970                 975

cta ctt ttc aga aga aaa aaa gaa aat aaa gat aag aaa taa             2970
Leu Leu Phe Arg Arg Lys Lys Glu Asn Lys Asp Lys Lys
            980                 985

<210> 24
<211> 989
<212> PRT
<213> Staphylococcus sp.

<400> 24
Met Asn Met Lys Lys Lys Glu Lys His Ala Ile Arg Lys Lys Ser Ile

1               5                   10                  15
Gly Val Ala Ser Val Leu Val Gly Thr Leu Ile Gly Phe Gly Leu Leu
            20                  25                  30
Ser Ser Lys Glu Ala Asp Ala Ser Glu Asn Ser Val Thr Gln Ser Asp
        35                  40                  45
Ser Ala Ser Asn Glu Ser Lys Ser Asn Asp Ser Ser Ser Val Ser Ala
    50                  55                  60
Ala Pro Lys Thr Asp Asp Thr Asn Val Ser Asp Thr Lys Thr Ser Ser
65                  70                  75                  80
Asn Thr Asn Asn Gly Glu Thr Ser Val Ala Gln Asn Pro Ala Gln Gln
                85                  90                  95
Glu Thr Thr Gln Ser Ser Ser Thr Asn Ala Thr Thr Glu Glu Thr Pro
            100                 105                 110
Val Thr Gly Glu Ala Thr Thr Thr Thr Thr Asn Gln Ala Asn Thr Pro
        115                 120                 125
Ala Thr Thr Gln Ser Ser Asn Thr Asn Ala Glu Glu Leu Val Asn Gln
    130                 135                 140
Thr Ser Asn Glu Thr Thr Ser Asn Asp Thr Asn Thr Val Ser Ser Val
145                 150                 155                 160
Asn Ser Pro Gln Asn Ser Thr Asn Ala Glu Asn Val Ser Thr Thr Gln
                165                 170                 175
Asp Thr Ser Thr Glu Ala Thr Pro Ser Asn Asn Glu Ser Ala Pro Gln
            180                 185                 190
Asn Thr Asp Ala Ser Asn Lys Asp Val Val Ser Gln Ala Val Asn Pro
        195                 200                 205
Ser Thr Pro Arg Met Arg Ala Phe Ser Leu Ala Ala Val Ala Ala Asp
    210                 215                 220
Ala Pro Ala Ala Gly Thr Asp Ile Thr Asn Gln Leu Thr Asp Val Lys
225                 230                 235                 240
Val Thr Ile Asp Ser Gly Thr Thr Val Tyr Pro His Gln Ala Gly Tyr
                245                 250                 255
Val Lys Leu Asn Tyr Gly Phe Ser Val Pro Asn Ser Ala Val Lys Gly
            260                 265                 270
Asp Thr Phe Lys Ile Thr Val Pro Lys Glu Leu Asn Leu Asn Gly Val
        275                 280                 285
Thr Ser Thr Ala Lys Val Pro Pro Ile Met Ala Gly Asp Gln Val Leu
    290                 295                 300
Ala Asn Gly Val Ile Asp Ser Asp Gly Asn Val Ile Tyr Thr Phe Thr
305                 310                 315                 320
Asp Tyr Val Asp Asn Lys Glu Asn Val Thr Ala Asn Ile Thr Met Pro
                325                 330                 335
Ala Tyr Ile Asp Pro Glu Asn Val Thr Lys Thr Gly Asn Val Thr Leu
            340                 345                 350
Thr Thr Gly Ile Gly Thr Asn Thr Ala Ser Lys Thr Val Leu Ile Asp
        355                 360                 365
Tyr Glu Lys Tyr Gly Gln Phe His Asn Leu Ser Ile Lys Gly Thr Ile
    370                 375                 380
Asp Gln Ile Asp Lys Thr Asn Asn Thr Tyr Arg Gln Thr Ile Tyr Val
385                 390                 395                 400
Asn Pro Ser Gly Asp Asn Val Val Leu Pro Ala Leu Thr Gly Asn Leu
                405                 410                 415
Ile Pro Asn Thr Lys Ser Asn Ala Leu Ile Asp Ala Lys Asn Thr Asp
            420                 425                 430
Ile Lys Val Tyr Arg Val Asp Asn Ala Asn Asp Leu Ser Glu Ser Tyr
        435                 440                 445
Tyr Val Asn Pro Ser Asp Phe Glu Asp Val Thr Asn Gln Val Arg Ile
    450                 455                 460
Ser Phe Pro Asn Ala Asn Gln Tyr Lys Val Glu Phe Pro Thr Asp Asp
465                 470                 475                 480
Asp Gln Ile Thr Thr Pro Tyr Ile Val Val Val Asn Gly His Ile Asp
                485                 490                 495

Pro Ala Ser Thr Gly Asp Leu Ala Leu Arg Ser Thr Phe Tyr Gly Tyr
            500                 505                 510
Asp Ser Asn Phe Ile Trp Arg Ser Met Ser Trp Asp Asn Glu Val Ala
        515                 520                 525
Phe Asn Asn Gly Ser Gly Ser Gly Asp Gly Ile Asp Lys Pro Val Val
    530                 535                 540
Pro Glu Gln Pro Asp Glu Pro Gly Glu Ile Glu Pro Ile Pro Glu Asp
545                 550                 555                 560
Ser Asp Ser Asp Pro Gly Ser Asp Ser Gly Ser Asp Ser Asn Ser Asp
                565                 570                 575
Ser Gly Ser Asp Ser Gly Ser Asp Ser Thr Ser Asp Ser Gly Ser Asp
            580                 585                 590
Ser Ala Ser Asp Ser Asp Ser Ala Ser Asp Ser Asp Ser Ala Ser Asp
        595                 600                 605
Ser Asp Ser Ala Ser Asp Ser Asp Ser Ala Ser Asp Ser Asp Ser Ala
    610                 615                 620
Ser Asp Ser Asp Ser Ala Ser Asp Ser Asp Ser Ala Ser Asp Ser Asp
625                 630                 635                 640
Ser Ala Ser Asp Ser Asp Ser Ala Ser Asp Ser Asp Ser Ala Ser Asp
                645                 650                 655
Ser Asp Ser Ala Ser Asp Ser Asp Ser Ala Ser Asp Ser Asp Ser Asp
            660                 665                 670
Ser Asp Ser Asp Ser Asp Ser Asp Ser Asp Ser Asp Ser Asp Ser Asp
        675                 680                 685
Ser Asp Ser Asp Ser Asp Ser Asp Ser Asp Ser Asp Ser Asp Ser Asp
    690                 695                 700
Ser Asp Ser Asp Ser Asp Ser Asp Ser Asp Ser Asp Ser Asp Ser Asp
705                 710                 715                 720
Ser Asp Ser Asp Ser Asp Ser Asp Ser Asp Ser Asp Ser Asp Ser Asp
                725                 730                 735
Ser Asp Ser Asp Ser Asp Ser Asp Ser Asp Ser Asp Ser Asp Ser Asp
            740                 745                 750
Ser Asp Ser Asp Ser Asp Ser Asp Ser Asp Ser Asp Ser Asp Ser Asp
        755                 760                 765
Ser Asp Ser Asp Ser Asp Ser Asp Ser Asp Ser Asp Ser Asp Ser Asp
    770                 775                 780
Ser Asp Ser Asp Ser Asp Ser Asp Ser Asp Ser Asp Ser Asp Ser Asp
785                 790                 795                 800
Ser Asp Ser Asp Ser Asp Ser Asp Ser Asp Ser Asp Ser Asp Ser Asp
                805                 810                 815
Ser Asp Ser Asp Ser Ala Ser Asp Ser Asp Ser Asp Ser Asp Ser Glu
            820                 825                 830
Ser Asp Ser Asp Ser Asp Ser Asp Ser Asp Ser Asp Ser Asp Ser Asp
        835                 840                 845
Ser Asp Ser Asp Ser Asp Ser Glu Ser Asp Ser Asp Ser Asp Ser Asp
    850                 855                 860
Ser Asp Ser Glu Ser Asp Ser Asp Ser Asp Ser Asp Ser Asp Ser Asp
865                 870                 875                 880
Ser Ala Ser Asp Ser Asp Ser Gly Ser Asp Ser Asp Ser Ser Ser Asp
                885                 890                 895
Ser Asp Ser Asp Ser Thr Ser Asp Thr Gly Ser Asp Asn Asp Ser Asp
            900                 905                 910
Ser Asp Ser Asn Ser Asp Ser Glu Ser Gly Ser Asn Asn Asn Val Val
        915                 920                 925
Pro Pro Asn Ser Pro Lys Asn Gly Thr Asn Ala Ser Asn Lys Asn Glu
    930                 935                 940
Ala Lys Asp Ser Lys Glu Pro Leu Pro Asp Thr Gly Ser Glu Asp Glu
945                 950                 955                 960
Ala Asn Thr Ser Leu Ile Trp Gly Leu Leu Ala Ser Leu Gly Ser Leu
                965                 970                 975
Leu Leu Phe Arg Arg Lys Lys Glu Asn Lys Asp Lys Lys

980                 985

<210> 25
<211> 34
<212> DNA
<213> Artificial Sequence

<220>
<223> Description of Artificial Sequence:  Synthetic
      Primer

<400> 25
gctgcacata tggcgcaaca cgatgaagct caac                                34

<210> 26
<211> 36
<212> DNA
<213> Artificial Sequence

<220>
<223> Description of Artificial Sequence:  Synthetic
      Primer

<400> 26
agtggatcct tatgcttgag ctttgttagc atctgc                              36

<210> 27
<211> 37
<212> DNA
<213> Artificial Sequence

<220>
<223> Description of Artificial Sequence:  Synthetic
      Primer

<400> 27
aaagaacata tggcatcaga acaaaagaca actacag                             37

<210> 28
<211> 34
<212> DNA
<213> Artificial Sequence

<220>
<223> Description of Artificial Sequence:  Synthetic
      Primer

<400> 28
tgaggatcct taggactcag tgtatcctcc aacg                                34

<210> 29
<211> 34
<212> DNA
<213> Artificial Sequence

<220>
<223> Description of Artificial Sequence:  Synthetic
      Primer

<400> 29
gaactcgagg catcggaaca aaacaatact acag                                34


<210> 30
<211> 37
<212> DNA
<213> Artificial Sequence

<220>
<223> Description of Artificial Sequence:  Synthetic
      Primer

<400> 30
aatggatcct taatcatttg gtttatcttt accatcg                             37


<210> 31
<211> 35
<212> DNA
<213> Artificial Sequence

<220>
<223> Description of Artificial Sequence:  Synthetic
      Primer

<400> 31
gaagcacata tgagtgaaaa tagtgttacg caatc                               35


<210> 32
<211> 36
<212> DNA
<213> Artificial Sequence

<220>
<223> Description of Artificial Sequence:  Synthetic
      Primer

<400> 32
agaggatcct tagttattaa atgctacttc gttgtc                              36


<210> 33
<211> 33
<212> DNA
<213> Artificial Sequence

<220>
<223> Description of Artificial Sequence:  Synthetic
      Primer

<400> 33
gcactcgagt cagaacaatc gaacgataca acg                                 33


<210> 34

<211> 35
<212> DNA
<213> Artificial Sequence

<220>
<223> Description of Artificial Sequence:  Synthetic
      Primer

<400> 34
tggggatcct taatttactg ctgaatcacc atcag                                35


<210> 35
<211> 40
<212> DNA
<213> Artificial Sequence

<220>
<223> Description of Artificial Sequence:  Synthetic
      Primer

<400> 35
gaagctcata tggcagaaca tacgaatgga gaattaaatc                           40


<210> 36
<211> 39
<212> DNA
<213> Artificial Sequence

<220>
<223> Description of Artificial Sequence:  Synthetic
      Primer

<400> 36
ttcggatcct taattatcaa gtgtgaaatc atcatgatc                            39


<210> 37
<211> 37
<212> DNA
<213> Artificial Sequence

<220>
<223> Description of Artificial Sequence:  Synthetic
      Primer

<400> 37
gcactcgagg cagaaagtac taataaagaa ttgaacg                              37


<210> 38
<211> 36
<212> DNA
<213> Artificial Sequence

<220>
<223> Description of Artificial Sequence:  Synthetic
      Primer

<400> 38

ttcggatcct tatttataag ttccattttc taatcc                                36

<210> 39
<211> 37
<212> DNA
<213> Artificial Sequence

<220>
<223> Description of Artificial Sequence:  Synthetic
      Primer

<400> 39
gaagctcata tggctgaaaa cactagtaca gaaaatg                               37

<210> 40
<211> 36
<212> DNA
<213> Artificial Sequence

<220>
<223> Description of Artificial Sequence:  Synthetic
      Primer

<400> 40
ttcggatcct tagttatcaa gtgtgaaatc atcatg                                36

<210> 41
<211> 36
<212> DNA
<213> Artificial Sequence

<220>
<223> Description of Artificial Sequence:  Synthetic
      Primer

<400> 41
caagctcata tggcttctga tgcaccatta acttct                                36

<210> 42
<211> 39
<212> DNA
<213> Artificial Sequence

<220>
<223> Description of Artificial Sequence:  Synthetic
      Primer

<400> 42
atcggatccg ctatttcttg ttacttcata tttaaaagt                             39

<210> 43
<211> 31
<212> DNA
<213> Artificial Sequence

<220>

<223> Description of Artificial Sequence:  Synthetic
      Primer

<400> 43
gcgagatctg cagagcaaaa tcagcctgca c                                    31


<210> 44
<211> 34
<212> DNA
<213> Artificial Sequence

<220>
<223> Description of Artificial Sequence:  Synthetic
      Primer

<400> 44
atcgaattct taagttgtgg cagcttgcac ttga                                 34


<210> 45
<211> 35
<212> DNA
<213> Artificial Sequence

<220>
<223> Description of Artificial Sequence:  Synthetic
      Primer

<400> 45
gcaggatcct taactacgga taataatgta caaag                                35


<210> 46
<211> 36
<212> DNA
<213> Artificial Sequence

<220>
<223> Description of Artificial Sequence:  Synthetic
      Primer

<400> 46
tatgaattct taagctttat catttacagc attgcg                               36


<210> 47
<211> 31
<212> DNA
<213> Artificial Sequence

<220>
<223> Description of Artificial Sequence:  Synthetic
      Primer

<400> 47
gcgctcgagg acacgacttc aatgaatgtg c                                    31


<210> 48
<211> 39

<212> DNA
<213> Artificial Sequence

<220>
<223> Description of Artificial Sequence:  Synthetic
      Primer

<400> 48
agcggatcct taaacttttt tgttactttg gttcatttg                              39


<210> 49
<211> 35
<212> DNA
<213> Artificial Sequence

<220>
<223> Description of Artificial Sequence:  Synthetic
      Primer

<400> 49
gccggatccg catctgaaaa ggatactgaa atttc                                  35


<210> 50
<211> 38
<212> DNA
<213> Artificial Sequence

<220>
<223> Description of Artificial Sequence:  Synthetic
      Primer

<400> 50
tttgaattct tatacattac catcagcatt taataatc                               38


<210> 51
<211> 38
<212> DNA
<213> Artificial Sequence

<220>
<223> Description of Artificial Sequence:  Synthetic
      Primer

<400> 51
gaagcacata tggctgaaaa caatattgag aatccaac                               38


<210> 52
<211> 39
<212> DNA
<213> Artificial Sequence

<220>
<223> Description of Artificial Sequence:  Synthetic
      Primer

<400> 52
tgtggatcct tatttttgtc cttctcttgt tactttttc                              39

<210> 53
<211> 39
<212> DNA
<213> Artificial Sequence

<220>
<223> Description of Artificial Sequence:  Synthetic
      Primer

<400> 53
gcgctcgagg cagaaaatac aaatacttca gataaaatc                              39


<210> 54
<211> 39
<212> DNA
<213> Artificial Sequence

<220>
<223> Description of Artificial Sequence:  Synthetic
      Primer

<400> 54
agtgaattct tacattttag attgactaag tttgttttc                              39


<210> 55
<211> 36
<212> DNA
<213> Artificial Sequence

<220>
<223> Description of Artificial Sequence:  Synthetic
      Primer

<400> 55
aatgcgcata tggaaaataa caaacctgaa ggtgtg                                 36


<210> 56
<211> 38
<212> DNA
<213> Artificial Sequence

<220>
<223> Description of Artificial Sequence:  Synthetic
      Primer

<400> 56
tgtggatcct tattctttca attgttgctt atgtactg                               38